# EUROPEAN PATENT APPLICATION

(11) **EP 4 582 414 A1**
(43) Date of publication of application: **09.07.2025**
(21) Application number: 23859390.9
(22) Date of filing: 30.08.2023
(51) Int. Cl.: C07D 267/14, C07D 401/14, C07D 401/06, C07D 405/06, C07D 403/06, C07D 513/04, C07D 498/04, C07D 495/04, A61P 35/00, A61P 1/00, A61P 29/00, A61P 17/00, A61P 25/00, A61P 7/00, A61P 27/02, A61K 31/16

(54) **AZEPINE FUSED RING COMPOUND AS RIPK1 INHIBITOR AND USE THEREOF**

(30) Priority: 02.09.2022 CN 202211077307
(71) Applicant: Artivila Biopharma, Wuxi, Jiangsu 214000 (CN)
(72) Inventor: NIU, Deqiang, Wuxi, Jiangsu 214000 (CN); ZHU, Zhendong, Wuxi, Jiangsu 214000 (CN); YE, Yibin, Wuxi, Jiangsu 214000 (CN); ZHANG, Qing, Wuxi, Jiangsu 214000 (CN); HE, Yongping, Wuxi, Jiangsu 214000 (CN); WANG, Yongjie, Wuxi, Jiangsu 214000 (CN)
(74) Representative: Boult Wade Tennant LLP
(86) International application number: PCT/CN2023/115773
(87) International publication number: WO 2024/046361

(57) **Abstract**

The present disclosure provides a compound of formula (I), a pharmaceutical composition thereof, and a use thereof in treating and/or preventing diseases or conditions related to the activity of RIPK1 kinase.

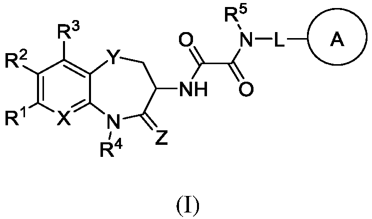

## Description

### FIELD

The present application relates to compounds that can be used to inhibit RIPK1 kinase activity, and/or the use of these compounds in treating and/or preventing diseases or conditions associated with RIPK1 kinase activity.

### BACKGROUND

Protein kinases are enzyme proteins widely present in cells and on the cell surface. So far, nearly 600 protein kinases have been discovered and identified. They belong to a structurally related protein family, and their known members are related to almost all cell signal transduction activities. The catalytic function of protein kinase is to transfer the γ-phosphate group in the ATP molecule to the specific threonine, serine or tyrosine group of the target protein, so that the conformation of the target protein changes, resulting in the function of the target protein changing from a static state to an activated state. According to the specificity of the target amino acid of protein kinase, protein kinases are divided into two major categories: serine/threonine protein kinases and tyrosine protein kinases.

The signal transduction and regulation involved in protein kinases play an extremely important role in the normal function of cells and organs, including cell growth, differentiation, proliferation, angiogenesis, apoptosis, cytoskeletal arrangement, regulation of metabolic reactions, membrane transport and cell movement. In addition, the non-catalytic functions of protein kinases also play an indispensable role, including allosteric effects, subcellular targeting, protein complex scaffolds, competitive protein interactions and DNA binding. On the other hand, when gene mutations or protein kinase overexpression occur, dysregulated protein kinases can lead to a variety of pathological changes, including cancer, inflammation, autoimmune diseases, cardiovascular and nervous system diseases. Therefore, protein kinases have become one of the most important targets in drug development today, and the success of protein kinase inhibitors in clinical treatment in recent years has further demonstrated the feasibility of this strategy and revealed the good prospects of using protein kinases as therapeutic targets.

Receptor-interacting protein kinase 1 (RIPK1) belongs to the TKL serine/threonine protein kinase family, including an N-terminal kinase domain, a RHIM (receptor-interacting protein kinase homotypic interaction motif) domain, and a C-terminal death domain. The C-terminal death domain of RIPK1 binds to other proteins containing death domains (such as Fas, TNFR-1, TRAIL-R1, TRAIL-R2, and TRADD) and initiates downstream signal transduction. The RHIM domain mainly binds to other proteins (such as TRIF and RIP3) containing the RHIM domain to initiate downstream signals. RIPK1 is mainly activated by signals released by death receptors (such as TNFR-1, TRAILR, and FasR), Toll-like receptors (TLR3/4), interferon receptor 1 (IFNAR1), Z-DNA binding protein 1 (ZBP), Dectin-1, or RIPK3. Once activated by upstream signals, RIPK1 will autophosphorylate and exert kinase activity-dependent biological functions, such as Caspase 8 (CASP8)-dependent apoptosis, RIPK3/MLKL-dependent necrosis and inflammation. In addition, RIPK1 can also play a kinase-independent scaffolding function, such as promoting cell survival and inflammatory gene expression. Other members of the RIP kinase family are involved in different physiological activities. RIPK2 usually regulates innate immunity and adaptive immune responses. RIPK3 interacts with RIPK1 to activate necrosis and apoptosis and regulate the activities of some metabolism-related enzymes. RIPK4 is involved in the development of stratified epithelial tissue and the NF-κB signaling pathway. The biological functions of other RIP kinase family members have not yet been clearly elucidated. Among the RIP kinase family, RIPK1 is essential for the innate immune response and is involved in downstream signals initiated by TNF-α. After TNF-α stimulation induces the aggregation of TNF receptors, multiple proteins (such as linear K63-linked polyubiquitinated RIP1, TRAF2/5, TRADD and cIAPs) are recruited to the cytoplasmic tail of TNF receptors and form complex I, which will participate in cell survival through NF-κB and MAPK kinase signaling pathways. In addition, deubiquitination of RIP1 promotes the formation of complex II or DISC (death-inducing signaling complex) (RIPK1, TRADD, FADD and caspase 8). After the formation of the DISC, RIPK3 is expressed, inhibiting cell apoptosis, and RIPK3 enters complex II, is phosphorylated by RIPK1 and initiates cell necroptosis after activation of MLKL and PGAM5. Necroptosis is a regulated, caspase-independent cell death pathway with morphological features similar to necrosis. It can be induced by a variety of stimuli (such as TNF-α and Fas ligand) and can occur in various cell types, such as monocytes, fibroblasts, lymphocytes, macrophages, epithelial cells, and neurons. Under pathological conditions of excessive cell stress, rapid energy loss, and the production of a large number of oxidative species, necroptosis may be an important pathway of cell death, and it is the main mode of cell death in some cases where highly energy-dependent processes do not work. Studies have shown that RIPK1 is a key molecule in the necroptosis pathway. The dysregulated activation of RIPK1 lead to necroptosis, which has become an important pathogenic factor in many diseases, including neuronal degenerative diseases and inflammatory diseases, stroke, coronary heart disease and myocardial infarction, retinal degenerative diseases, inflammatory bowel disease, kidney disease, liver disease, and lesions caused by COVID-19.

Effective and selective small molecule inhibitors of RIPK1 will block RIPK1-dependent pro-inflammatory signaling, thereby providing therapeutic benefits for inflammatory diseases characterized by dysregulated RIPK1 kinase activity. There is an urgent need for such RIPK1 inhibitors in the prior art.

### SUMMARY

After long-term research, the inventors unexpectedly discovered a class of compounds with significant inhibitory effects of RIPK1 activity, which show highly efficient and highly selective RIPK1 kinase inhibitory effects and can be used to treat or prevent diseases related to RIPK1 activity.

In particular, the present invention provides a compound of formula (I): 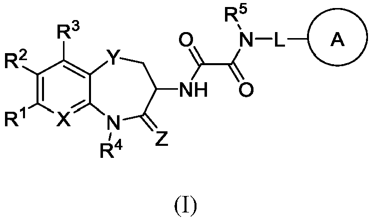
or a mesomer, a racemate, an enantiomer, a diastereoisomer or a mixture thereof, or a pharmaceutically acceptable salt thereof,
wherein,
X is selected from the group consisting of CH and N;
Y is selected from the group consisting of -CR^{a}R^{b}-, -O-, -NR^{a}- and -S(O)ₘ-;
Z is selected from the group consisting of -O and -S;
L is selected from the group consisting of a single bond, -(CR^{a}R^{b})ₙ-, -(CR^{a}R^{b})ₙO-, -(CR^{a}R^{b})ₙS-, -(CR^{a}R^{b})ₙNR^{a}-, -O-, -NR^{a}- and -S(O)ₘ-;
ring A is selected from the group consisting of C₅-C₁₀ aryl, heteroaryl, cycloalkyl and heterocyclyl, wherein the aryl, heteroaryl, cycloalkyl and heterocyclyl are optionally further substituted by one or more R⁶;
R¹ is independently selected from the group consisting of hydrogen, halogen, amino, nitro, cyano, oxo, hydroxyl, sulfydryl, alkyl, alkoxy, alkenyl, alkynyl, aryl, heteroaryl, cycloalkyl, heterocyclyl, -NR^{a}R^{b}, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, wherein the alkyl, alkoxy, alkenyl, alkynyl, aryl, heteroaryl, cycloalkyl, heterocyclyl is optionally further substituted by one or more groups selected from the group consisting of deuterium atom, halogen, amino, nitro, cyano, oxo, hydroxyl, sulfhydryl, carboxyl, ester group, alkyl, alkoxy, alkenyl, alkynyl, aryl, heteroaryl, cycloalkyl, heterocycly; preferably, R¹ is selected from the group consisting of the following alkynyl, alkenyl and allenyl groups: 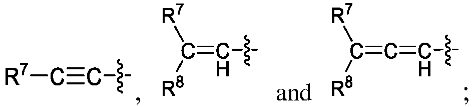
R² and R³ are each independently selected from the group consisting of hydrogen, halogen, amino, nitro, cyano, oxo, hydroxyl, sulfydryl, alkyl, alkoxy, alkenyl, alkynyl, aryl, heteroaryl, cycloalkyl and heterocyclyl; or
R¹ and R² together with the atoms to which they are attached form a C₅-C₁₀ aryl, heteroaryl, heterocyclyl or cycloalkyl, or
R² and R³ together with the atoms to which they are attached form a C₅-C₁₀ aryl, heteroaryl, heterocyclyl or cycloalkyl, wherein the C₅-C₁₀ aryl, heteroaryl, heterocyclyl or cycloalkyl is optionally further substituted by one or more R⁹;
R⁴ and R⁵ are each independently selected from the group consisting of hydrogen, alkyl, haloalkyl, cycloalkyl and halocycloalkyl;
R⁶, R⁷, R⁸ and R⁹ are each independently selected from the group consisting of hydrogen, halogen, amino, nitro, cyano, oxo, hydroxyl, sulfydryl, alkyl, alkoxy, alkenyl, alkynyl, aryl, heteroaryl, cycloalkyl, heterocyclyl, -NR^{a}R^{b}, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, wherein the alkyl, alkoxy, alkenyl, alkynyl, aryl, heteroaryl, cycloalkyl, heterocyclyl is optionally further substituted by one or more groups selected from the group consisting of deuterium atom, halogen, amino, nitro, cyano, oxo, hydroxyl, sulfydryl, carboxyl, ester group, alkyl, alkoxy, hydroxyalkyl, alkenyl, alkynyl, aryl, aryloxy, heteroaryl, cycloalkyl and heterocyclyl; R^{a} and R^{b} are each independently selected from the group consisting of hydrogen, deuterium atom, halogen, amino, cyano, oxo, hydroxyl, sulfydryl, alkyl, alkoxy, aryl, heteroaryl, cycloalkyl and heterocyclyl; or
R^{a} and R^{b} together with the atoms to which they are attached form a cycloalkyl or heterocyclyl, wherein the cycloalkyl or heterocyclyl is optionally further substituted by one or more groups selected from the group consisting of deuterium atom, halogen, amino, nitro, cyano, oxo, hydroxyl, sulfydryl, carboxyl, ester group, alkyl, alkoxy, alkenyl, alkynyl, aryl, heteroaryl, cycloalkyl and heterocyclyl;
m is 0, 1 or 2; and
n is 0, 1, 2 or 3.

In a preferred embodiment, the compound represented by general formula (I) or a mesomer, a racemate, an enantiomer, a diastereomer, or a mixture thereof, or a pharmaceutically acceptable salt thereof according to the present invention, is a compound represented by general formula (II) or a mesomer, a racemate, an enantiomer, a diastereomer, or a mixture thereof, or a pharmaceutically acceptable salt thereof: 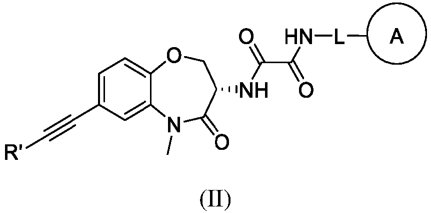 wherein,
R' is selected from the group consisting of hydrogen, halogen, amino, nitro, cyano, oxo, hydroxyl, sulfydryl, alkyl, alkoxy, alkenyl, alkynyl, aryl, heteroaryl, cycloalkyl, heterocyclyl, -NR^{a}R^{b}, -C(O)R^{a}, -O(O)CR^{a}, -C(O)OR^{a}, -C(O)NR^{a}R^{b}, -NHC(O)R^{a}, -S(O)ₘR^{a}, -S(O)mNR^{a}R^{b} and -NHS(O)ₘR^{a}, wherein the alkyl, alkoxy, alkenyl, alkynyl, aryl, heteroaryl, cycloalkyl, heterocyclyl is optionally further substituted by one or more groups selected from the group consisting of deuterium atom, halogen, amino, nitro, cyano, oxo, hydroxyl, sulfydryl, carboxyl, ester group, alkyl, alkoxy, hydroxyalkyl, alkenyl, alkynyl, aryl, aryloxy, heteroaryl, cycloalkyl and heterocyclyl;
m is 0, 1 or 2;
L and ring A are as defined in general formula (I).

In another preferred embodiment, the compound represented by the general formula (I) or a mesomer, a racemate, an enantiomer, a diastereomer, or a mixture thereof, or a pharmaceutically acceptable salt thereof according to the present invention, is a compound represented by the general formula (III) or a mesomer, a racemate, an enantiomer, a diastereomer, or a mixture thereof, or a pharmaceutically acceptable salt thereof: 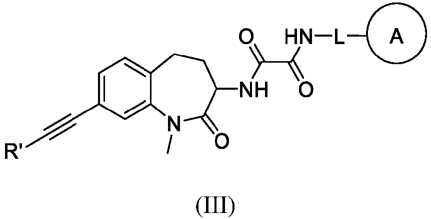 wherein,
L and ring A are defined as in the general formula (I);
R' is defined as in the general formula (II).

Typical compounds of the present invention include, but are not limited to:

| Compound No. | Structure | Name |
|---|---|---|
| 1 | 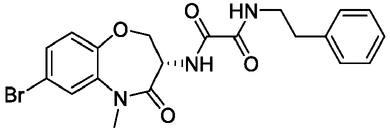 | (*S*)-*N*¹-(7-bromo-5-methyl-4-o xo-2,3,4,5-tetrahydrobenzo[*b*][ 1,4]oxazepin-3-yl)-*N*²-pheneth yloxalamide |
| 2 | 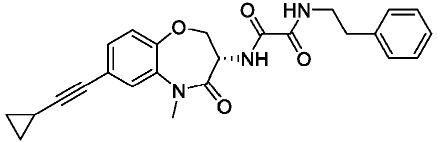 | (*S*)-*N*¹-(7-(cyclopropylethynyl )-5-methyl-4-oxo-2,3,4,5-tetra hydrobenzo[*b*][1,4]oxazepin-3 -yl)-*N*²-phenethyloxalamide |
| 3 | 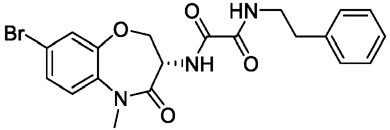 | (*S*)-*N*¹-(8-bromo-5-methyl-4-o xo-2,3,4,5-tetrahydrobenzo[*b*][ 1,4]oxazepin-3-yl)-*N*²-pheneth yloxalamide |
| 4 | 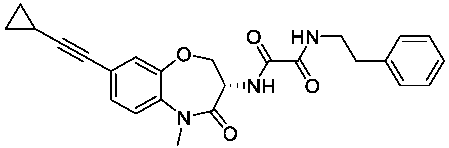 | (*S*)-*N*¹-(8-(cyclopropylethynyl )-5-methyl-4-oxo-2,3,4,5-tetra hydrobenzo[*b*][1,4]oxazepin-3 -yl)-*N*²-phenethyloxalamide |
| 5 | 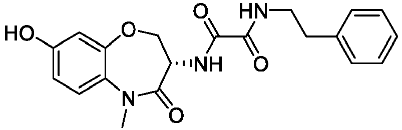 | (*S*)-*N*¹-(8-hydroxy-5-methyl-4 -oxo-2,3,4,5-tetrahydrobenzo[ *b*][1,4]oxazepin-3-yl)-*N*²-phen ethyloxalamide |
| 6 | 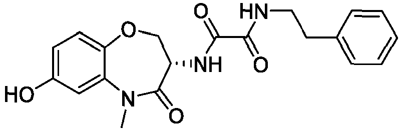 | (*S*)-*N*¹-(7-hydroxy-5-methyl-4 -oxo-2,3,4,5-tetrahydrobenzo[ *b*][1,4]oxazepin-3-yl)-*N*²-phen ethyloxalamide |
| 7 | 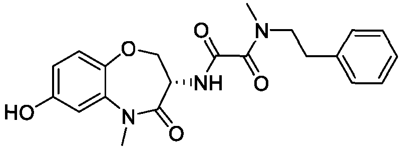 | (*S*)-*N*¹-(7-hydroxy-5-methyl-4 -oxo-2,3,4,5-tetrahydrobenzo[ *b*][1,4]oxazepin-3-yl)-*N*²-meth yl-*N*²-phenethyloxalamide |
| 8 | 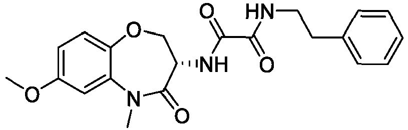 | (*S*)-*N*¹-(7-methoxy-5-methyl-4 -oxo-2,3,4,5-tetrahydrobenzo [ *b*][1,4]oxazepin-3-yl)-*N*²-phen ethyloxalamide |
| 9 | 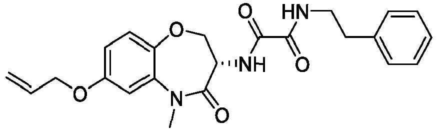 | (*S*)-*N*¹-(7-(allyloxy)-5-methyl-4-oxo-2,3,4,5-tetrahydrobenzo [*b*][1,4]oxazepin-3-yl)-*N*²-phe nethyloxalamide |
| 10 | 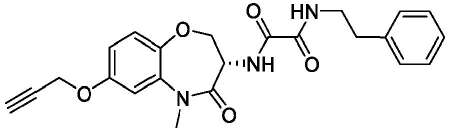 | (*S*)-*N*¹-(5-methyl-4-oxo-7-(pro p-2-yn-1-yloxy)-2,3,4,5-tetrah ydrobenzo[*b*][1,4]oxazepin-3-yl)-*N*²-phenethyloxalamide |
| 11 | 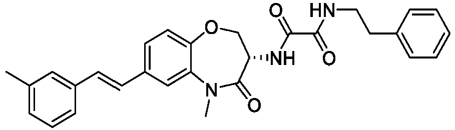 | (*S*,*E*)-*N*¹-(5-methyl-7-(3-meth ylstyryl)-4-oxo-2,3,4,5-tetrahy drobenzo[*b*][1,4]oxazepin-3-yl )-*N*²-phenethyloxalamide |
| 12 | 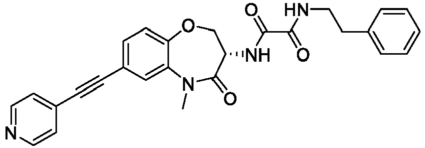 | (*S*)-*N*¹-(5-methyl-4-oxo-7-(pyr idin-4-ylethynyl)-2,3,4,5-tetra hydrobenzo[*b*][1,4]oxazepin-3 -yl)-*N*²-phenethyloxalamide |
| 13 | 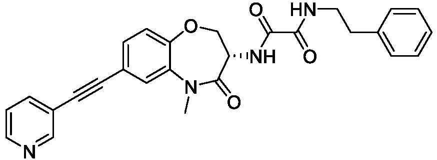 | (*S*)-*N*¹-(5-methyl-4-oxo-7-(pri din-3-ylethynyl)-2,3,4,5-tetrah ydrobenzo[*b*][1,4]oxazepin-3-yl)-*N*²-phenethyloxalamide |
| 14 | 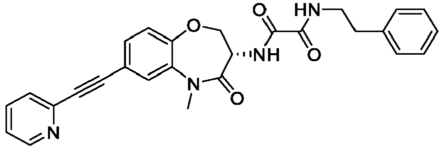 | (*S*)-*N*¹-(5-methyl-4-oxo-7-(pyr idin-2-ylethynyl)-2,3,4,5-tetra hydrobenzo[*b*][1,4]oxazepin-3 -yl)-*N*²-phenethyloxalamide |
| 15 | 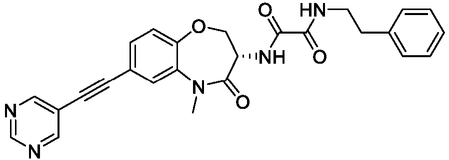 | (*S*)-*N*¹-(5-methyl-4-oxo-7-(pyr imidin-5-ylethynyl)-2,3,4,5-tet rahydrobenzo[*b*][1,4]oxazepin -3-yl)-*N*²-phenethyloxalamide |
| 16 | 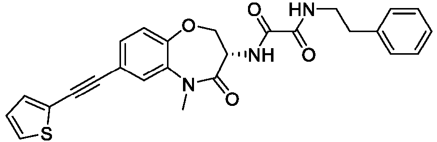 | (*S*)-*N*¹-(5-methyl-4-oxo-7-(thi ophen-2-ylethynyl)-2,3,4,5-tet rahydrobenzo[*b*][1,4]oxazepin -3-yl)-*N*²-phenethyloxalamide |
| 17 | 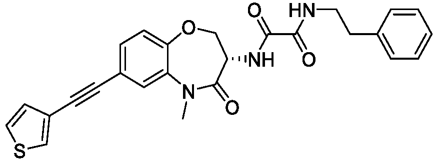 | (*S*)-*N*¹-(5-methyl-4-oxo-7-(thi ophen-3-ylethynyl)-2,3,4,5-tet rahydrobenzo[*b*][1,4]oxazepin -3-yl)-*N*²-phenethyloxalamide |
| 18 | 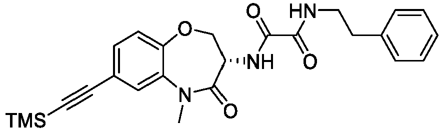 | (*S*)-*N*¹-(5-methyl-4-oxo-7-((tri methylsilyl)ethynyl)-2,3,4,5-te trahydrobenzo[*b*][1,4]oxazepi n-3-yl)-*N*²-phenethyloxalamid e |
| 19 | 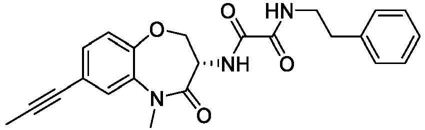 | (*S*)-*N*¹-(5-methyl-4-oxo-7-(pro p-1-yn-1-yl)-2,3,4,5-tetrahydr obenzo[*b*][1,4]oxazepin-3-yl)-*N*²-phenethyloxalamide |
| 20 | 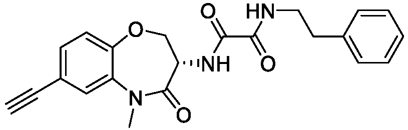 | (*S*)-*N*¹-(7-ethynyl-5-methyl-4-oxo-2,3,4,5-tetrahydrobenzo[*b* ][1,4]oxazepin-3-yl)-*N*²-phene thyloxalamide |
| 21 | 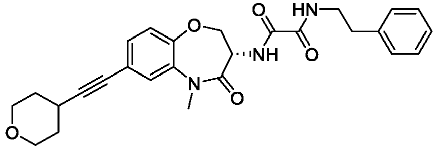 | (*S*)-*N*¹-(5-methyl-4-oxo-7-((tet rahydro-2*H*-pyran-4-yl)ethyny l)-2,3,4,5-tetrahydrobenzo[*b*][ 1,4]oxazepin-3-yl)-*N*²-pheneth yloxalamide |
| 22 | 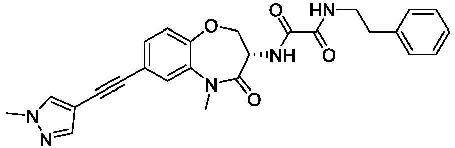 | (*S*)-*N*¹(5-methyl-7-((1-methyl -1*H*-pyrazol-4-yl)ethynyl)-4-o xo-2,3,4,5-tetrahydrobenzo[*b*][ 1,4]oxazepin-3-yl)-*N*²-pheneth yloxalamide |
| 23 | 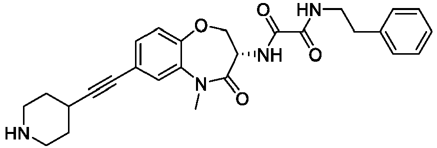 | (*S*)-*N*¹(5-methyl-4-oxo-7-(pip eridin-4-ylethynyl)-2,3,4,5-tetr ahydrobenzo[*b*][1,4]oxazepin-3-yl)-*N*²-phenethyloxalamide |
| 24 | 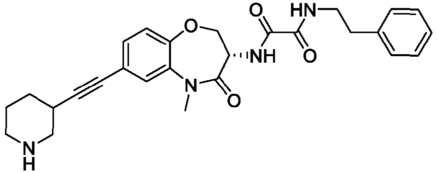 | *N*¹-((3*S*)-5-methyl-4-oxo-7-(pi peridin-3-ylethynyl)-2,3,4,5-te trahydrobenzo[*b*][1,4]oxazepi n-3-yl)-*N*²-phenethyloxalamid e |
| 25 | 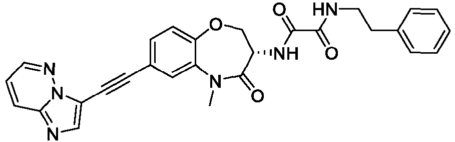 | (*S*)-*N*¹(7-(imidazo[1,2-b]pyrid azin-3-ylethynyl)-5-methyl-4-oxo-2,3,4,5-tetrahydrobenzo[*b* ][1,4]oxazepin-3-yl)-*N*²-phene thyloxalamide |
| 26 | 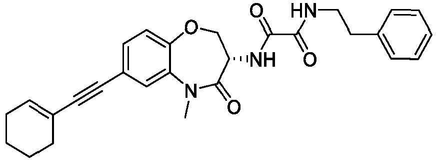 | (*S*)-*N*¹-(7-(cyclohex-1en-1yl ethynyl)-5-methyl-4-oxo-2,3,4 ,5-tetrahydrobenzo[*b*][1,4]oxa zepin-3-yl)-*N*²-phenethyloxala mide |
| 27 | 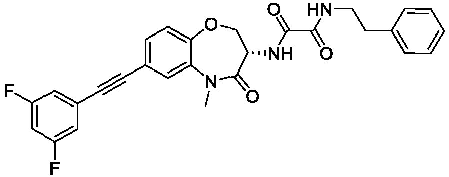 | (*S*)-*N*¹-(7-((3,5-difluorophenyl )ethynyl)-5-methyl-4-oxo-2,3, 4,5-tetrahydrobenzo[*b*][1,4]ox azepin-3-yl)-*N*²-phenethyloxal amide |
| 28 | 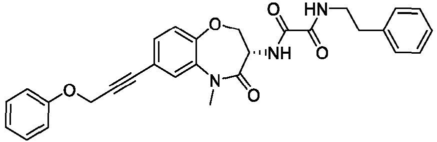 | (*S*)-*N*¹-(5-methyl-4-oxo-7-(3-p henyloxyprop-1-yn-1-yl)-2,3,4 ,5-tetrahydrobenzo[*b*][1,4]oxa zepin-3-yl)-*N*²-phenethyloxala mide |
| 29 | 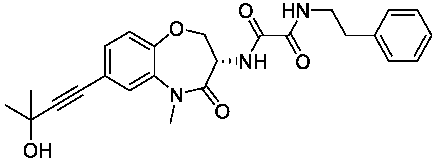 | (*S*)-*N*¹-(7-(3-hydroxy-3-methy 1ut-1-yn-1-yl)-5-methyl-4-ox o-2,3,4,5-tetrahydrobenzo[*b*][1 ,4]oxazepin-3-yl)-*N*²-phenethy loxalamide |
| 30 | 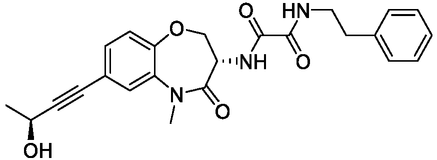 | *N*¹-((*S*)-7-((*S*)-3-hydroxybut-1 -yn-1-yl)-5-methyl-4-oxo-2,3, 4,5-tetrahydrobenzo[*b*][1,4]ox azepin-3-yl)-*N*²-phenethyloxal amide |
| 31 | 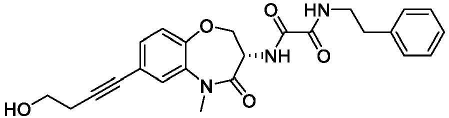 | (*S*)-*N*¹-(7-(4-hydroxybut-1-yn-1-yl)-5-methyl-4-oxo-2,3,4,5-t etrahydrobenzo[*b*][1,4]oxazepi n-3-yl)-*N*²-phenethyloxalamid e |
| 32 | 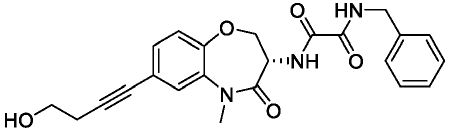 | (*S*)-*N*¹-benzyl-*N*²-(7-(4-hydrox ybut-1-yn-1-yl)-5-methyl-4-ox o-2,3,4,5-tetrahydrobenzo[*b*][1 ,4]oxazepin-3-yl)oxalamide |
| 33 | 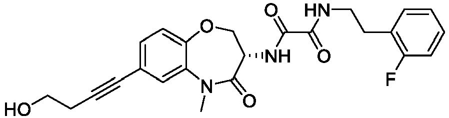 | (*S*)-*N*¹-(2-fluorophenethyl)-*N*²-(7-(4-hydroxybut-1-yn-1-yl)-5 -methyl-4-oxo-2,3,4,5-tetrahy drobenzo[*b*][1,4]oxazepin-3-yl )oxalamide |
| 34 | 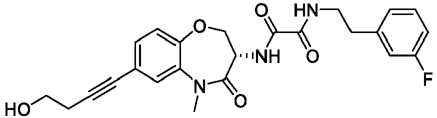 | (*S*)-*N*¹-(3-fluorophenethyl)-*N*²-(7-(4-hydroxybut-1-yn-1-yl)-5 -methyl-4-oxo-2,3,4,5-tetrahy drobenzo[*b*][1,4]oxazepin-3-yl)oxalamide |
| 35 | 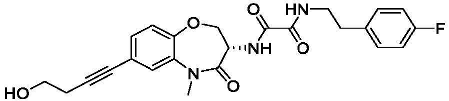 | (*S*)-*N*¹-(4-fluorophenethyl)-*N*²-(7-(4-hydroxybut-1-yn-1-yl)-5 -methyl-4-oxo-2,3,4,5-tetrahy drobenzo[*b*][1,4]oxazepin-3-yl )oxalamide |
| 36 | 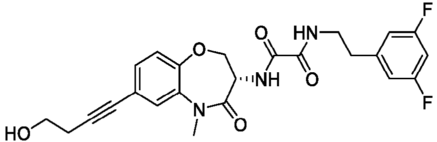 | (*S*)-*N*¹-(3,5-difluorophenethyl) -*N*²-(7-(4-hydroxybut-1-yn-1-yl)-5-methyl-4-oxo-2,3,4,5-tet rahydrobenzo[*b*][1,4]oxazepin -3-yl)oxalamide |
| 37 | 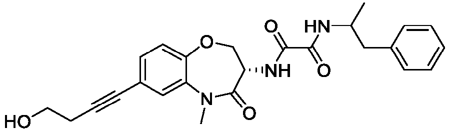 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-(1-phenylpropan-2-yl)oxalamide |
| 38 | 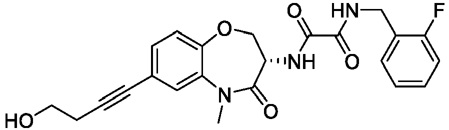 | (*S*)-*N*¹-(2-fluorobenzyl)-*N*²-(7-(4-hydroxybut-1-yn-1-yl)-5-m ethyl-4-oxo-2,3,4,5-tetrahydro benzo[*b*][1,4]oxazepin-3-yl)ox alamide |
| 39 | 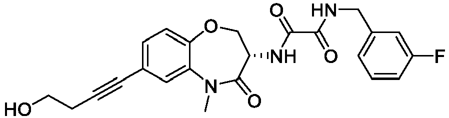 | (*S*)-*N*¹-(3-fluorobenzyl)-*N*²-(7-(4-hydroxybut-1-yn-1-yl)-5-m ethyl-4-oxo-2,3,4,5-tetrahydro benzo[*b*][1,4]oxazepin-3-yl)ox alamide |
| 40 | 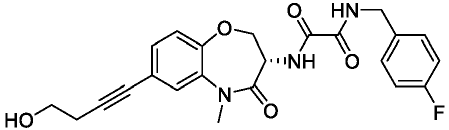 | (*S*)-*N*¹-(4-fluorobenzyl)-*N*²-(7-(4-hydroxybut-1-yn-1-yl)-5-m ethyl-4-oxo-2,3,4,5-tetrahydro benzo[*b*][1,4]oxazepin-3-yl)ox alamide |
| 41 | 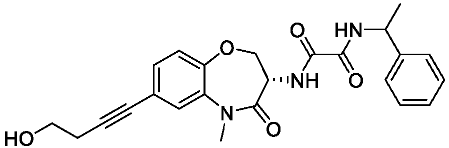 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-(1-phenylethyl)oxal amide |
| 42 | 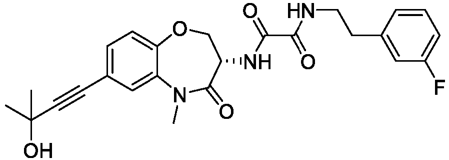 | (*S*)-*N*¹-(3-fluorophenethyl)-*N*²-(7-(3-hydroxy-3-methylbut-1-yn-1-yl)-5-methyl-4-oxo-2,3,4 ,5-tetrahydrobenzo[*b*][1,4]oxa zepin-3-yl)oxalamide |
| 43 | 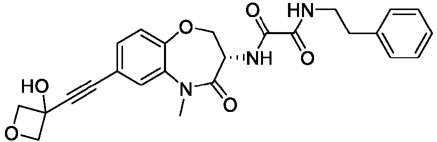 | (*S*)-*N*¹-(7-((3-hydroxyoxetan-3 -yl)ethynyl)-5-methyl-4-oxo-2 ,3,4,5-tetrahydrobenzo[*b*][1,4] oxazepin-3-yl)-*N*²-phenethylo xalamide |
| 44 | 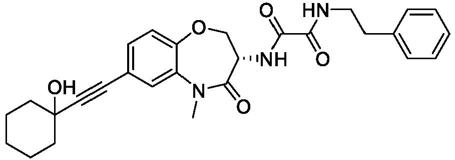 | (*S*)-*N*¹-(7-((1-hydroxycyclohe xyl)ethynyl)-5-methyl-4-oxo-2 ,3,4,5-tetrahydrobenzo[*b*][1,4] oxazepin-3-yl)-*N*²-phenethylo xalamide |
| 45 | 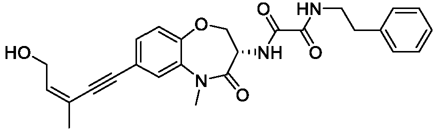 | (*S,Z*)-*N*¹-(7-(5-hydroxy-3-met hylpent-3-en-1-yn-1-yl)-5-met hyl-4-oxo-2,3,4,5-tetrahydrobenzo[*b*][1,4]oxazepin-3-yl)-*N*²-phenethyloxalamide |
| 46 | 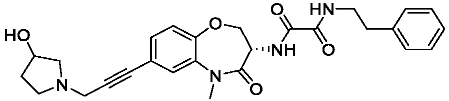 | *N*¹-((3*S*)-7-(3-(3-hydroxypyrro lidin-1-yl)prop-1-yn-1-yl)-5-m ethyl-4-oxo-2,3,4,5-tetrahydro benzo[*b*][1,4]oxazepin-3-yl)-*N* ²-phenethyloxalamide |
| 47 | 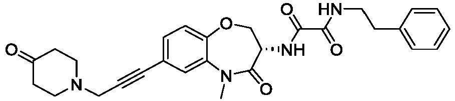 | (*S*)-*N*¹-(5-methyl-4-oxo-7-(3-( 4-oxopiperidin-1-yl)prop-1-yn -1-yl)-2,3,4,5-tetrahydrobenzo [*b*][1,4]oxazepin-3-yl)-*N*²-phe nethyloxalamide |
| 48 | 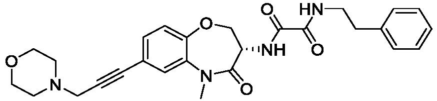 | (*S*)-*N*¹-(5-methyl-7-(3-morpho linoprop-1-yn-1-yl)-4-oxo-2,3, 4,5-tetrahydrobenzo[*b*][1,4]ox azepin-3-yl)-*N*²-phenethyloxal amide |
| 49 | 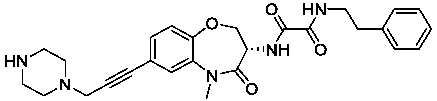 | (*S*)-*N*¹-(5-methyl-4-oxo-7-(3-( piperazin-1-yl)prop-1-yn-1-yl) -2,3,4,5-tetrahydrobenzo[*b*][1, 4]oxazepin-3-yl)-*N*²-phenethyl oxalamide |
| 50 | 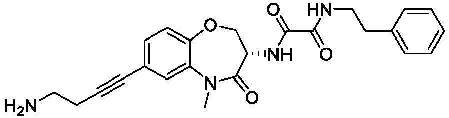 | (*S*)-*N*¹-(7-(4-aminobut-1-yn-1-yl)-5-methyl-4-oxo-2,3,4,5-tet rahydrobenzo[*b*][1,4]oxazepin -3-yl)-*N*²-phenethyloxalamide |
| 51 | 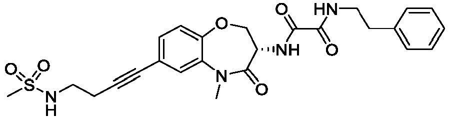 | (*S*)-*N*¹-(5-methyl-7-(4-(methyl sulfonamido)but-1-yn-1-yl)-4-oxo-2,3,4,5-tetrahydrobenzo[*b* ][1,4]oxazepin-3-yl)-*N*²-phene thyloxalamide |
| 52 | 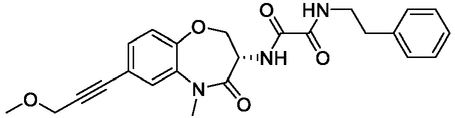 | (*S*)-*N*¹-(7-(3-methoxyprop-1-y n-1-yl)-5-methyl-4-oxo-2,3,4, 5-tetrahydrobenzo[*b*][1,4]oxaz epin-3-yl)-*N*²-phenethyloxala mide |
| 53 | 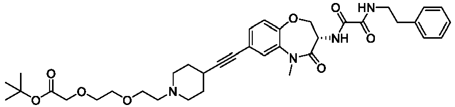 | *tert*-butyl (*S*)-2-(2-(2-(4-((5-methyl-4-ox o-3-(2-oxo-2-(phenethylamino )acetamido)-2,3,4,5-tetrahydro benzo[*b*][1,4]oxazepin-7-yl)et hynyl)piperidin-1-yl)ethoxy)et hoxy)acetate |
| 54 | 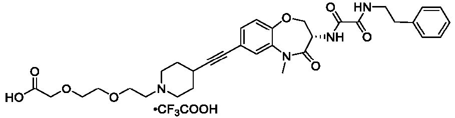 | (*S*)-1-(2-(2-(carboxymethoxy) ethoxy)ethyl)-4-((5-methyl-4-oxo-3-(2-oxo-2-(phenethylami no)acetamido)-2,3,4,5-tetrahy drobenzo[*b*][1,4]oxazepin-7-yl )ethynyl)piperidin-1-ium 2,2,2-trifluoroacetate |
| 55 | 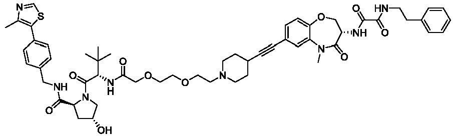 | *N*¹-((*S*)-7-((1-(2-(2-(2-(((*S*)-1-( (2*S*,4*R*)-4-hydroxy-2-((4-(4-m ethylthiazol-5-yl)benzyl)carba moyl)pyrrolidin-1-yl)-3,3-dimethyl-1-oxobutan-2-yl)amino)-2-oxoethoxy)ethoxy)ethyl)pip eridin-4-yl)ethynyl)-5-methyl-4-oxo-2,3,4,5-tetrahydrobenzo [*b*][1,4]oxazepin-3-yl)-*N*²-phe nethyloxalamide |
| 56 | 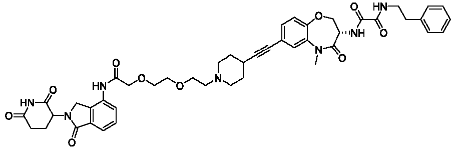 | *N*¹-((3*S*)-7-((1-(2-(2-(2-((2-(2, 6-dioxopiperidin-3-yl)-1-oxois oindolin-4-yl)amino)-2-oxoeth oxy)ethoxy)ethyl)piperidin-4-yl)ethynyl)-5-methyl-4-oxo-2, 3,4,5-tetrahydrobenzo[*b*][1,4]o xazepin-3-yl)-*N*²-phenethylox alamide |
| 57 | 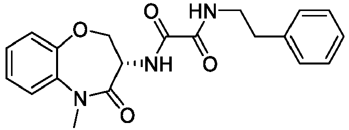 | (*S*)-*N*¹-(5-methyl-4-oxo-2,3,4, 5-tetrahydrobenzo[*b*][1,4]oxaz epin-3-yl)-*N*²-phenethyloxala mide |
| 58 | 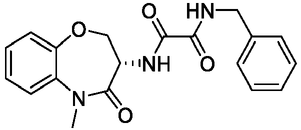 | (*S*)-*N*¹-benzyl-*N*²-(5-methyl-4-oxo-2,3,4,5-tetrahydrobenzo[*b* ][1,4]oxazepin-3-yl)oxalamide |
| 59 | 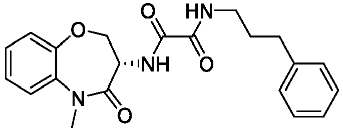 | (*S*)-*N*¹-(5-methyl-4-oxo-2,3,4, 5-tetrahydrobenzo[*b*][1,4]oxaz epin-3-yl)-*N*²-(3-phenylpropyl)oxalamide |
| 60 | 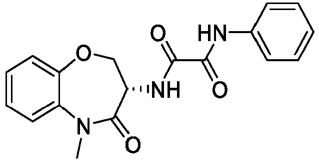 | (*S*)-*N*¹-(5-methyl-4-oxo-2,3,4, 5-tetrahydrobenzo[*b*][1,4]oxaz epin-3-yl)-*N*²-phenyloxalamid e |
| 61 | 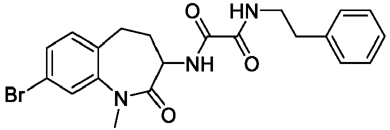 | *N*¹-(8-bromo-1-methyl-2-oxo-2,3,4,5-tetrahydro-1*H*-benzo[*b* ]azepin-3-yl)-*N*²-phenethyloxa lamide |
| 62 | 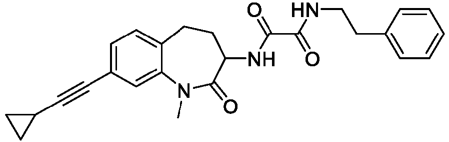 | *N*¹-(8-(cyclopropylethynyl)-1-methyl-2-oxo-2,3,4,5-tetrahyd ro-1*H*-benzo[*b*] azepin-3-yl)-*N* ² -phenethyloxalamide |
| 63 | 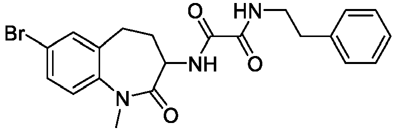 | *N*¹-(7-bromo-1-methyl-2-oxo-2,3,4,5-tetrahydro-1*H*-benzo [*b*]azepin-3-yl)-*N*²-phenethylo xalamide |
| 64 | 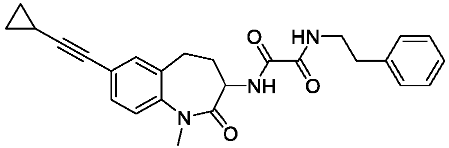 | *N*¹-(7-(cyclopropylethynyl)-1-methyl-2-oxo-2,3,4,5-tetrahyd ro-1*H*-benzo[*b*] azepin-3-yl)-*N* ²-phenethyloxalamide |
| 65 | 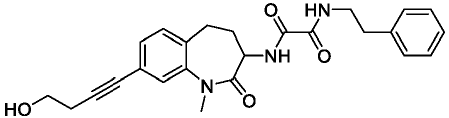 | *N*¹-(8-(4-hydroxybut-1-yn-1-yl )-1-methyl-2-oxo-2,3,4,5-tetra hydro-1*H*-benzo[*b*]azepin-3-yl )-*N*²-phenethyloxalamide |
| 66 | 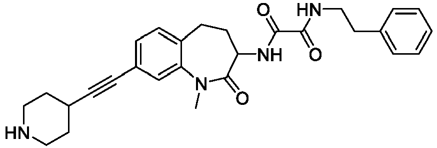 | *N*¹-(1-methyl-2-oxo-8-(piperid in-4-ylethynyl)-2,3,4,5-tetrahy dro-1*H*-benzo[*b*] azepin-3-yl)-*N*²-phenethyloxalamide |
| 67 | 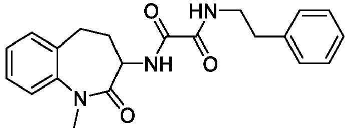 | *N*¹-(1-methyl-2-oxo-2,3,4,5-tet rahydro-1*H*-benzo[*b*]azepin-3-yl)-*N*²-phenethyloxalamide |
| 68 | 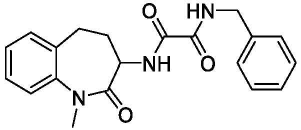 | *N*¹-benzyl-*N*²-(1-methyl-2-oxo -2,3,4,5-tetrahydro-1*H*-benzo[ *b*]azepin-3-yl)oxalamide |
| 69 | 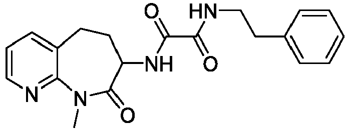 | *N*¹-(9-methyl-8-oxo-6,7,8,9-tet rahydro-5*H*-pyrido[2,3-*b*]azep in-7-yl)-*N*²-phenethyloxalamid e |
| 70 | 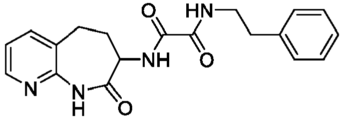 | *N*¹-(8-oxo-6,7,8,9-tetrahydro-5 *H*-pyrido[2,3-*b*]azepin-7-yl)-*N* ²-phenethyloxalamide |
| 71 | 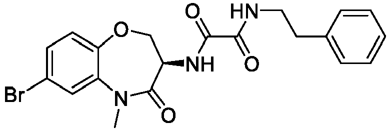 | (*R*)-*N*¹-(7-bromo-5-methyl-4-o xo-2,3,4,5-tetrahydrobenzo[*b*][ 1,4]oxazepin-3-yl)-*N*²-pheneth yloxalamide |
| 72 | 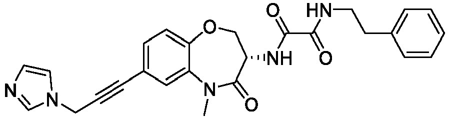 | (*S*)-*N*¹-(7-(3-(1*H*-imidazol-1-y 1)prop-1-yn-1-yl)-5-methyl-4-oxo-2,3,4,5-tetrahydrobenzo[*b* ][1,4]oxazepin-3-yl)-*N*²-phene thyloxalamide |
| 73 | 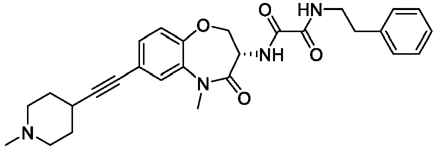 | (*S*)-*N*¹-(5-methyl-7-((1-methyl piperidin-4-yl)ethynyl)-4-oxo-2,3,4,5-tetrahydrobenzo[*b*][1,4 ]oxazepin-3-yl)-*N*²-phenethylo xalamide |
| 74 | 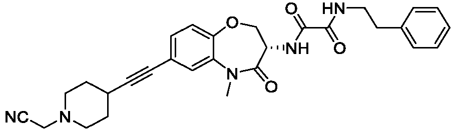 | (*S*)-*N*¹-(7-((1-(cyanomethyl)pi peridin-4-yl)ethynyl)-5-methyl -4-oxo-2,3,4,5-tetrahydrobenz o[*b*][1,4]oxazepin-3-yl)-*N*²-ph enethyloxalamide |
| 75 | 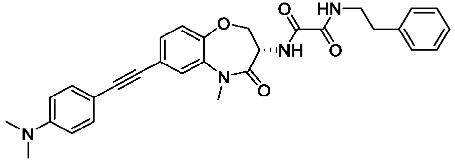 | (*S*)-*N*¹-(7-((4-(dimethylamino) phenyl)ethynyl)-5-methyl-4-o xo-2,3,4,5-tetrahydrobenzo[*b*][ 1,4]oxazepin-3-yl)-*N*²-pheneth yloxalamide |
| 76 | 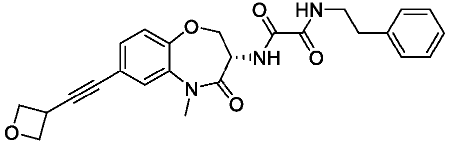 | (*S*)-*N*¹-(5-methyl-7-(oxetan-3-ylethynyl)-4-oxo-2,3,4,5-tetra hydrobenzo[*b*][1,4]oxazepin-3 -yl)-*N*²-phenethyloxalamide |
| 77 | 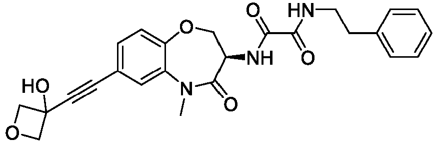 | (*R*)-*N*¹-(7-((3-hydroxyoxetan-3-yl)ethynyl)-5-methyl-4-oxo-2,3,4,5-tetrahydrobenzo[*b*][1,4 ]oxazepin-3-yl)-*N*²-phenethyloxalamide |
| 78 | 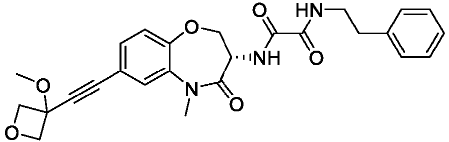 | (*S*)-*N*¹-(7-((3-methoxyoxetan-3-yl)ethynyl)-5-methyl-4-oxo-2,3,4,5-tetrahydrobenzo[*b*][1,4 ]oxazepin-3-yl)-*N*²-phenethylo xalamide |
| 79 | 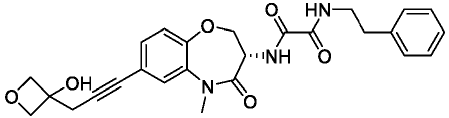 | (*S*)-*N*¹-(7-(3-(3-hydroxyoxetan -3-yl)prop-1-yn-1-yl)-5-methy 1-4-oxo-2,3,4,5-tetrahydrobenz o[*b*][1,4]oxazepin-3-yl)-*N*²-ph enethyloxalamide |
| 80 | 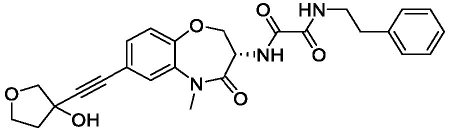 | *N*¹-((3*S*)-7-((3-hydroxytetrahy drofuran-3-yl)ethynyl)-5-meth yl-4-oxo-2,3,4,5-tetrahydroben zo[*b*][1,4]oxazepin-3-yl)-*N*²-p henethyloxalamide |
| 81 | 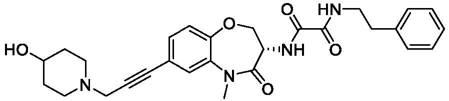 | (*S*)-*N*¹-(7-(3-(4-hydroxypiperi din-1-yl)prop-1-yn-1-yl)-5-me thyl-4-oxo-2,3,4,5-tetrahydrob enzo[*b*][1,4]oxazepin-3-yl)-*N*² -phenethyloxalamide |
| 82 | 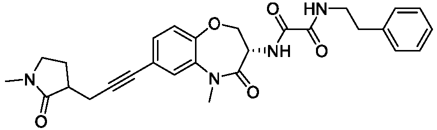 | *N*¹-((3*S*)-5-methyl-7-(3-(1-met hyl-2-oxopyrrolidin-3-yl)prop-1-yn-1-yl)-4-oxo-2,3,4,5-tetra hydrobenzo[*b*][1,4]oxazepin-3 -yl)-*N*²-phenethyloxalamide |
| 83 | 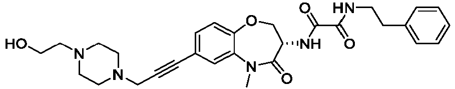 | (*S*)-*N*¹-(7-(3-(4-(2-hydroxyeth yl)piperazin-1-yl)prop-1-yn-1-yl)-5-methyl-4-oxo-2,3,4,5-tet rahydrobenzo[*b*][1,4]oxazepin -3-yl)-*N*²-phenethyloxalamide |
| 84 | 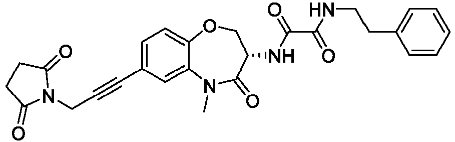 | (*S*)-*N*¹-(7-(3-(2,5-dioxopyrroli din-1-yl)prop-1-yn-1-yl)-5-me thyl-4-oxo-2,3,4,5-tetrahydrob enzo[*b*][1,4]oxazepin-3-yl)-*N*² -phenethyloxalamide |
| 85 | 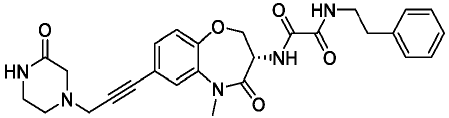 | (*S*)-*N*¹-(5-methyl-4-oxo-7-(3-( 3-oxopiperazin-1-yl)prop-1-yn -1-yl)-2,3,4,5-tetrahydrobenzo [*b*][1,4]oxazepin-3-yl)-*N*²-phe nethyloxalamide |
| 86 | 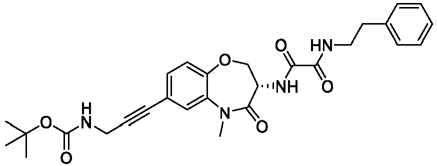 | *tert*-butyl (*S*)-(3-(5-methyl-4-oxo-3-(2-o xo-2-(phenethylamino)acetami do)-2,3,4,5-tetrahydrobenzo[*b*] [1,4]oxazepin-7-yl)prop-2-yn-1-yl)carbamate |
| 87 | 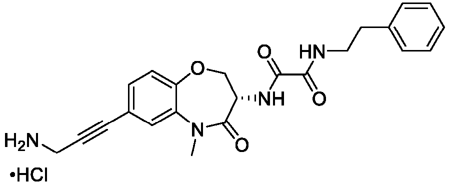 | (*S*)-*N*¹-(7-(3-aminoprop-1-yn-1-yl)-5-methyl-4-oxo-2,3,4,5-t etrahydrobenzo[*b*][1,4]oxazepi n-3-yl)-*N*²-phenethyloxalamid e hydrochloride |
| 88 | 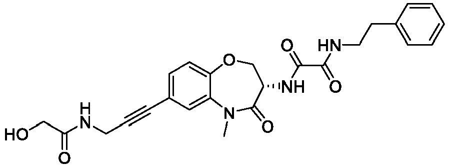 | (*S*)-*N*¹-(7-(3-(2-hydroxyaceta mido)prop-1-yn-1-yl)-5-methy 1-4-oxo-2,3,4,5-tetrahydrobenz o[*b*][1,4]oxazepin-3-yl)-*N*²-ph enethyloxalamide |
| 89 | 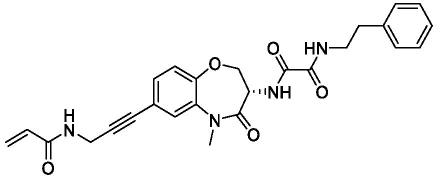 | (*S*)-*N*¹-(7-(3-acrylamidoprop-1 -yn-1-yl)-5-methyl-4-oxo-2,3, 4,5-tetrahydrobenzo[*b*][1,4]ox azepin-3-yl)-*N*²-phenethyloxal amide |
| 90 | 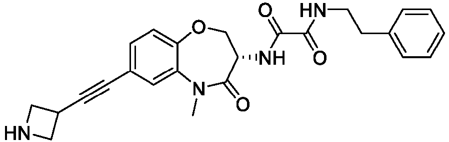 | (*S*)-*N*¹-(7-(azetidin-3-ylethyny 1)-5-methyl-4-oxo-2,3,4,5-tetra hydrobenzo[*b*][1,4]oxazepin-3 -yl)-*N*²-phenethyloxalamide |
| 91 | 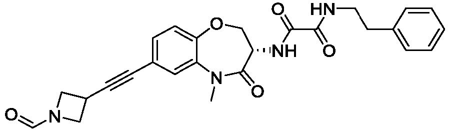 | (*S*)-*N*¹-(7-((1-formylazetidin-3 -yl)ethynyl)-5-methyl-4-oxo-2 ,3,4,5-tetrahydrobenzo[*b*][1,4] oxazepin-3-yl)-*N*²-phenethylo xalamide |
| 92 | 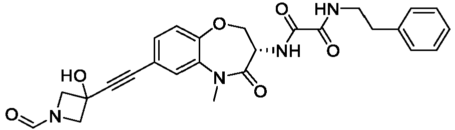 | (*S*)-*N*¹-(7-((1-formyl-3-hydrox yazetidin-3-yl)ethynyl)-5-met hyl-4-oxo-2,3,4,5-tetrahydrobe nzo[*b*][1,4]oxazepin-3-yl)-*N*²-phenethyloxalamide |
| 93 | 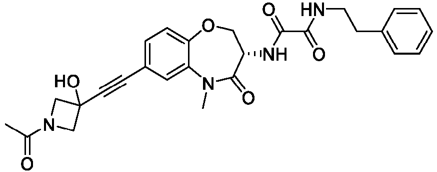 | (*S*)-*N*¹-(7-((1-acetyl-3-hydroxy azetidin-3-yl)ethynyl)-5-meth yl-4-oxo-2,3,4,5-tetrahydroben zo[*b*][1,4]oxazepin-3-yl)-*N*²-phenethyloxalamide |
| 94 | 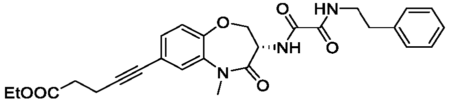 | ethyl (*S*)-5-(5-methyl-4-oxo-3-(2-ox o-2-(phenethylamino)acetamid o)-2,3,4,5-tetrahydrobenzo[*b*][ 1,4]oxazepin-7-yl)pent-4-ynoa te |
| 95 | 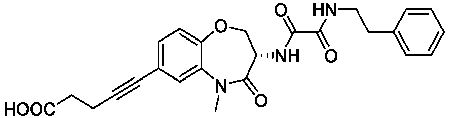 | (*S*)-5-(5-methyl-4-oxo-3-(2-ox o-2-(phenethylamino)acetamid o)-2,3,4,5-tetrahydrobenzo[*b*][ 1,4]oxazepin-7-yl)pent-4-ynoi c acid |
| 96 | 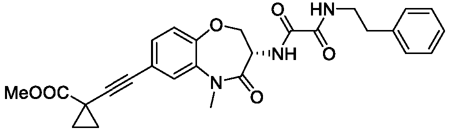 | methyl (*S*)-1-((5-methyl-4-oxo-3-(2-o xo-2-(phenethylamino)acetami do)-2,3,4,5-tetrahydrobenzo[*b*] [1,4]oxazepin-7-yl)ethynyl)cy clopropane-1-carboxylate |
| 97 | 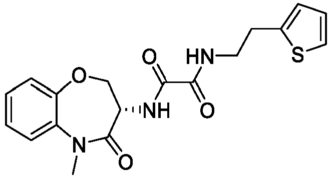 | (*S*)-*N*¹-(5-methyl-4-oxo-2,3,4, 5-tetrahydrobenzo[*b*][1,4]oxaz epin-3-yl)-*N*²-(2-(thiophen-2-y l)ethyl)oxalamide |
| 98 | 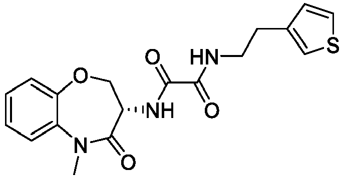 | (*S*)-*N*¹-(5-methyl-4-oxo-2,3,4, 5-tetrahydrobenzo[*b*][1,4]oxaz epin-3-yl)-*N*²-(2-(thiophen-3-y 1)ethyl)oxalamide |
| 99 | 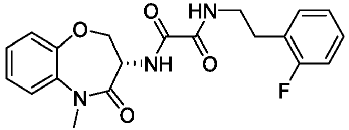 | (*S*)-*N*¹-(2-fluorophenethyl)-*N*²-(5-methyl-4-oxo-2,3,4,5-tetrah ydrobenzo[*b*][1,4]oxazepin-3-yl)oxalamide |
| 100 | 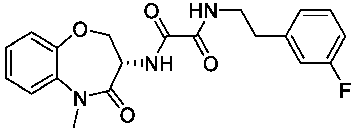 | (*S*)-*N*¹-(3-fluorophenethyl)-*N*²-(5-methyl-4-oxo-2,3,4,5-tetrah ydrobenzo[*b*][1,4]oxazepin-3-yl)oxalamide |
| 101 | 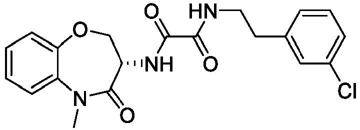 | (*S*)-*N*¹-(3-chlorophenethyl)-*N*² -(5-methyl-4-oxo-2,3,4,5-tetra hydrobenzo[*b*][1,4]oxazepin-3 -yl)oxalamide |
| 102 | 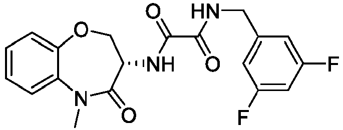 | (*S*)-*N*¹-(3,5-difluorobenzyl)-*N*² -(5-methyl-4-oxo-2,3,4,5-tetra hydrobenzo[*b*][1,4]oxazepin-3 -yl)oxalamide |
| 103 | 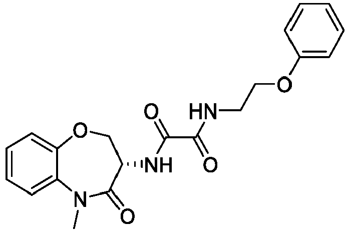 | (*S*)-*N*¹-(5-methyl-4-oxo-2,3,4, 5-tetrahydrobenzo[*b*][1,4]oxaz epin-3-yl)-*N*²-(2-phenoxyethyl )oxalamide |
| 104 | 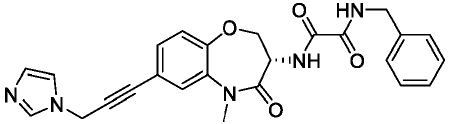 | (*S*)-*N*¹-(7-(3-(1*H*-imidazol-1-y 1)prop-1-yn-1-yl)-5-methyl-4-oxo-2,3,4,5-tetrahydrobenzo[*b* ][1,4]oxazepin-3-yl)-*N*²-benzy loxalamide |
| 105 | 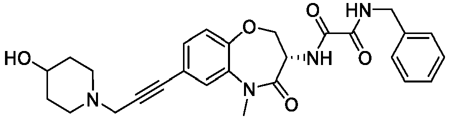 | (*S*)-*N*¹-benzyl-*N*²-(7-(3-(4-hyd roxypiperidin-1-yl)prop-1-yn-1-yl)-5-methyl-4-oxo-2,3,4,5-t etrahydrobenzo[*b*][1,4]oxazepi n-3-yl)oxalamide |
| 106 | 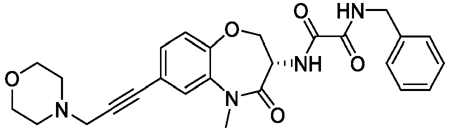 | (*S*)-*N*¹-benzyl-*N*²-(5-methyl-7-(3-morpholinoprop-1-yn-1-yl) -4-oxo-2,3,4,5-tetrahydrobenz o[*b*][1,4]oxazepin-3-yl)oxalam ide |
| 107 | 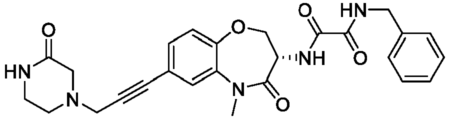 | (*S*)-*N*¹-benzyl-*N*²-(5-methyl-4-oxo-7-(3-(3-oxopiperazin-1-yl )prop-1-yn-1-yl)-2,3,4,5-tetrah ydrobenzo[*b*][1,4]oxazepin-3-yl)oxalamide |
| 108 | 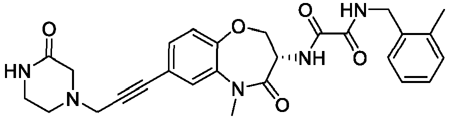 | (*S*)-*N*¹-(5-methyl-4-oxo-7-(3-( 3-oxopiperazin-1-yl)prop-1-yn -1-yl)-2,3,4,5-tetrahydrobenzo [*b*][1,4]oxazepin-3-yl)-*N*²-(2-methylbenzyl)oxalamide |
| 109 | 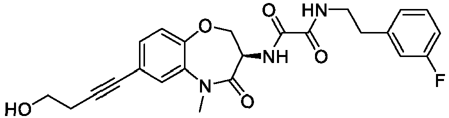 | (*R*)-*N*¹-(3-fluorophenethyl)-*N*² -(7-(4-hydroxybut-1-yn-1-yl)-5-methyl-4-oxo-2,3,4,5-tetrah ydrobenzo[*b*][1,4]oxazepin-3-yl)oxalamide |
| 110 | 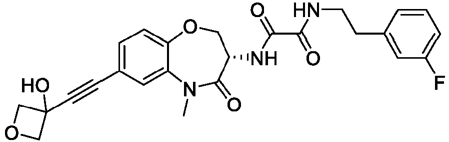 | (*S*)-*N*¹-(3-fluorophenethyl)-*N*²-(7-((3-hydroxyoxetan-3-yl)eth ynyl)-5-methyl-4-oxo-2,3,4,5-t etrahydrobenzo[*b*][1,4]oxazepi n-3-yl)oxalamide |
| 111 | 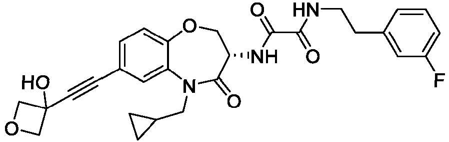 | (*S*)-*N*¹-(5-(cyclopropylmethyl) -7-((3-hydroxyoxetan-3-yl)eth ynyl)-4-oxo-2,3,4,5-tetrahydro benzo[*b*][1,4]oxazepin-3-yl)-*N* ²-(3-fluorophenethyl)oxalamid e |
| 112 | 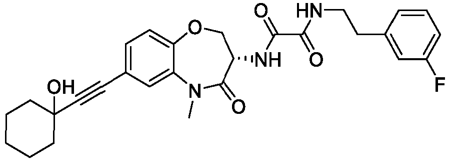 | (*S*)-*N*¹-(3-fluorophenethyl)-*N*²-(7-((1-hydroxycyclohexyl)eth ynyl)-5-methyl-4-oxo-2,3,4,5-t etrahydrobenzo[*b*][1,4]oxazepi n-3-yl)oxalamide |
| 113 | 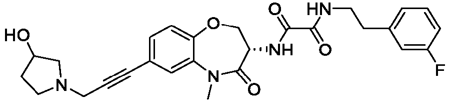 | *N*¹-(3-fluorophenethyl)-*N*²-((3 *S*)-7-(3-(3-hydroxypyrrolidin-1-yl)prop-1-yn-1-yl)-5-methyl -4-oxo-2,3,4,5-tetrahydrobenz o[*b*][1,4]oxazepin-3-yl)oxalam ide |
| 114 | 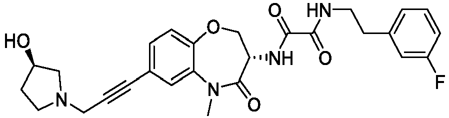 | *N*¹-(3-fluorophenethyl)-*N*²-((*S*) -7-(3-((*R*)-3-hydroxypyrrolidi n-1-yl)prop-1-yn-1-yl)-5-meth yl-4-oxo-2,3,4,5-tetrahydroben zo[*b*][1,4]oxazepin-3-yl)oxala mide |
| 115 | 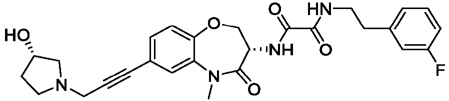 | *N*¹-(3-fluorophenethyl)-*N*²-((*S*) -7-(3-((*S*)-3-hydroxypyrrolidin -1-yl)prop-1-yn-1-yl)-5-methy 1-4-oxo-2,3,4,5-tetrahydrobenz o[*b*][1,4]oxazepin-3-yl)oxalam ide |
| 116 | 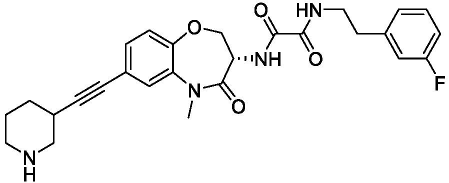 | *N*¹-(3-fluorophenethyl)-*N*²-((3 *S*)-5-methyl-4-oxo-7-(piperidi n-3-ylethynyl)-2,3,4,5-tetrahy drobenzo[*b*][1,4]oxazepin-3-yl )oxalamide |
| 117 | 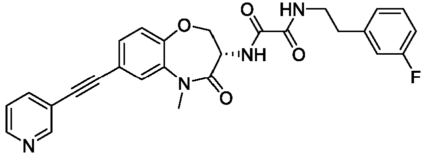 | (*S*)-*N*¹-(3-fluorophenethyl)-*N*²-(5-methyl-4-oxo-7-(pyridin-3-ylethynyl)-2,3,4,5-tetrahydrob enzo[*b*][1,4]oxazepin-3-yl)oxa lamide |
| 118 | 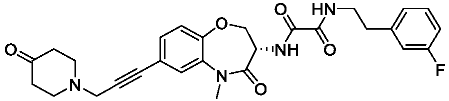 | (*S*)-*N*¹-(3-fluorophenethyl)-*N*²-(5-methyl-4-oxo-7-(3-(4-oxopi peridin-1-yl)prop-1-yn-1-yl)-2 ,3,4,5-tetrahydrobenzo[*b*][1,4] oxazepin-3-yl)oxalamide |
| 119 | 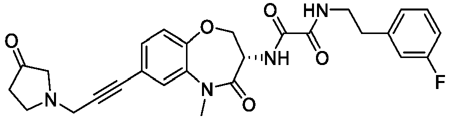 | (*S*)-*N*¹-(3-fluorophenethyl)-*N*²-(5-methyl-4-oxo-7-(3-(3-oxop yrrolidin-1-yl)prop-1-yn-1-yl)-2,3,4,5-tetrahydrobenzo[*b*][1,4 ]oxazepin-3-yl)oxalamide |
| 120 | 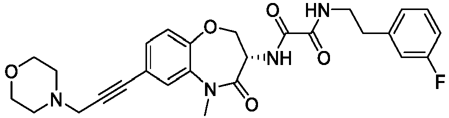 | (*S*)-*N*¹-(3-fluorophenethyl)-*N*²-(5-methyl-7-(3-morpholinopro p-1-yn-1-yl)-4-oxo-2,3,4,5-tetr ahydrobenzo[*b*][1,4]oxazepin-3-yl)oxalamide |
| 121 | 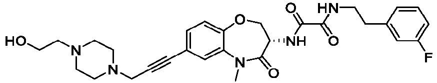 | (*S*)-*N*¹-(3-fluorophenethyl)-*N*²-(7-(3-(4-(2-hydroxyethyl)piper azin-1-yl)prop-1-yn-1-yl)-5-m ethyl-4-oxo-2,3,4,5-tetrahydro benzo[*b*][1,4]oxazepin-3-yl)ox alamide |
| 122 | 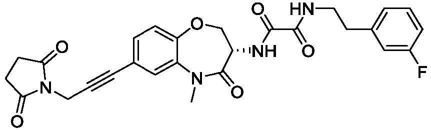 | (*S*)-*N*¹-(7-(3-(2,5-dioxopyrroli din-1-yl)prop-1-yn-1-yl)-5-me thyl-4-oxo-2,3,4,5-tetrahydrob enzo[*b*][1,4]oxazepin-3-yl)-*N*² -(3-fluorophenethyl)oxalamide |
| 123 | 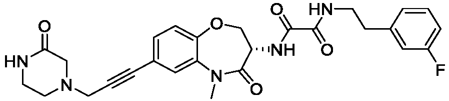 | (*S*)-*N*¹-(3-fluorophenethyl)-*N*²-(5-methyl-4-oxo-7-(3-(3-oxopiperazin-1-yl)prop-1-yn-1-yl)-2 ,3,4,5-tetrahydrobenzo[*b*][1,4] oxazepin-3-yl)oxalamide |
| 124 | 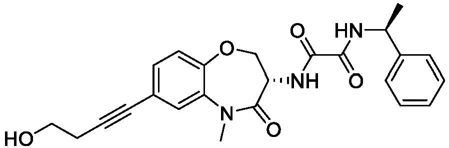 | *N*¹-((*S*)-7-(4-hydroxybut-1-yn-1-yl)-5-methyl-4-oxo-2,3,4,5-t etrahydrobenzo[*b*][1,4]oxazepi *n*-3-yl)-*N*²-((*S*)-1-phenylethyl) oxalamide |
| 125 | 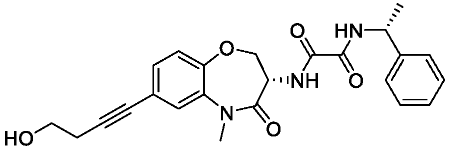 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*R*)-1-phenylethyl) oxalamide |
| 126 | 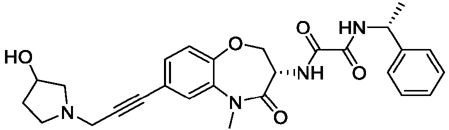 | *N*¹-((3*S*)-7-(3-(3-hydroxypyrro lidin-1-yl)prop-1-yn-1-yl)-5-m ethyl-4-oxo-2,3,4,5-tetrahydro benzo[b] [1,4]oxazepin-3-yl)-*N* ²-((*R*)-1-phenylethyl)oxalamid e |
| 127 | 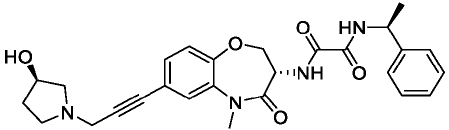 | *N*¹-((*S*)-7-(3-((*R*)-3-hydroxypy rrolidin-1-yl)prop-1-yn-1-yl)-5 -methyl-4-oxo-2,3,4,5-tetrahy drobenzo[*b*][1,4]oxazepin-3-yl )-*N*²-((*S*)-1-phenylethyl)oxala mide |
| 128 | 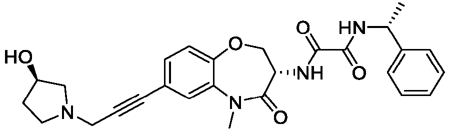 | *N*¹-((*S*)-7-(3-((*R*)-3-hydroxypy rrolidin-1-yl)prop-1-yn-1-yl)-5 -methyl-4-oxo-2,3,4,5-tetrahydrobenzo[*b*][1,4]oxazepin-3-yl )-*N*²-((*R*)-1-phenylethyl)oxala mide |
| 129 | 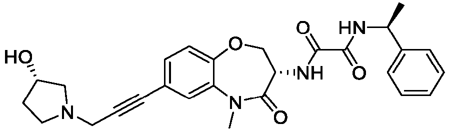 | *N*¹-((*S*)-7-(3-((*S*)-3-hydroxypy rrolidin-1-yl)prop-1-yn-1-yl)-5 -methyl-4-oxo-2,3,4,5-tetrahy drobenzo[*b*][1,4]oxazepin-3-yl )-*N*²-((*S*)-1-phenylethyl)oxala mide |
| 130 | 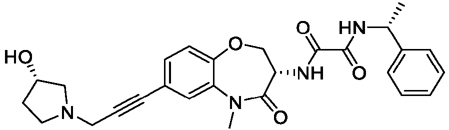 | *N*¹-((*S*)-7-(3-((*S*)-3-hydroxypy rrolidin-1-yl)prop-1-yn-1-yl)-5 -methyl-4-oxo-2,3,4,5-tetrahy drobenzo[*b*][1,4]oxazepin-3-yl )-*N*²-((*R*)-1-phenylethyl)oxala mide |
| 131 | 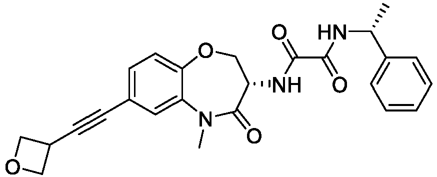 | (*S*)-*N*¹-(5-methyl-7-(oxetan-3-ylethynyl)-4-oxo-2,3,4,5-tetra hydrobenzo[*b*][1,4]oxazepin-3 -yl)-*N*²-((*R*)-1-phenylethyl)oxa lamide |
| 132 | 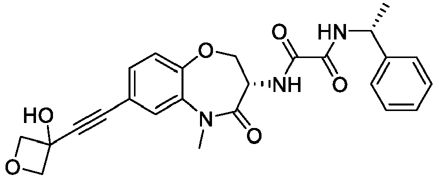 | *N*¹-((*S*)-7-((3-hydroxyoxetan-3 -yl)ethynyl)-5-methyl-4-oxo-2 ,3,4,5-tetrahydrobenzo[*b*][1,4] oxazepin-3-yl)-*N*²-((*R*)-1-phen ylethyl)oxalamide |
| 133 | 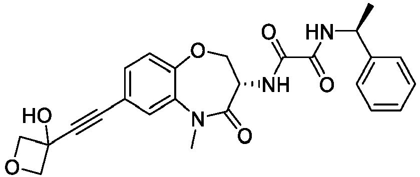 | *N*¹-((*S*)-7-((3-hydroxyoxetan-3 -yl)ethynyl)-5-methyl-4-oxo-2 ,3,4,5-tetrahydrobenzo[*b*][1,4] oxazepin-3-yl)-*N*²-((*S*)-1-phen ylethyl)oxalamide |
| 134 | 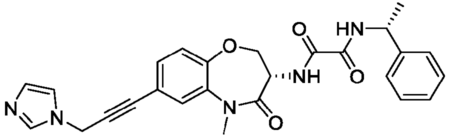 | *N*¹-((*S*)-7-(3-(1*H*-imidazol-1-y 1)prop-1-yn-1-yl)-5-methyl-4-oxo-2,3,4,5-tetrahydrobenzo[*b* ][1,4]oxazepin-3-yl)-*N*²-((*R*)-1 -phenylethyl)oxalamide |
| 135 | 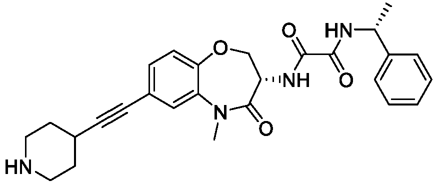 | *N*¹-((*S*)-5-methyl-4-oxo-7-(pip eridin-4-ylethynyl)-2,3,4,5-tetr ahydrobenzo[*b*][1,4]oxazepin-3-yl)-*N*²-((*R*)-1-phenylethyl)o xalamide |
| 136 | 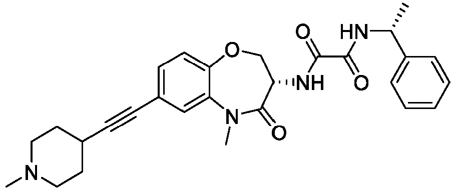 | *N*¹-((*S*)-5-methyl-7-((1-methyl piperidin-4-yl)ethynyl)-4-oxo-2,3,4,5-tetrahydrobenzo[*b*][1,4 ]oxazepin-3-yl)-*N*²-((*R*)-1-phe nylethyl)oxalamide |
| 137 | 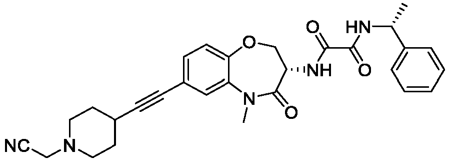 | *N*¹-((*S*)-7-((1-(cyanomethyl)pi peridin-4-yl)ethynyl)-5-methyl -4-oxo-2,3,4,5-tetrahydrobenz o[*b*][1,4]oxazepin-3-yl)-*N*²-((*R* )-1-phenylethyl)oxalamide |
| 138 | 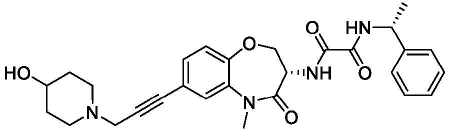 | *N*¹-((*S*)-7-(3-(4-hydroxypiperi din-1-yl)prop-1-yn-1-yl)-5-me thyl-4-oxo-2,3,4,5-tetrahydrobenzo[*b*][1,4]oxazepin-3-yl)-*N*² -((*R*)-1-phenylethyl)oxalamide |
| 139 | 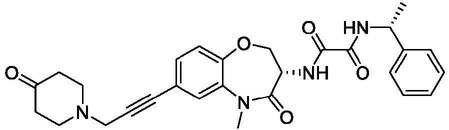 | *N*¹-((*S*)-5-methyl-4-oxo-7-(3-( 4-oxopiperidin-1-yl)prop-1-yn -1-yl)-2,3,4,5-tetrahydrobenzo [*b*][1,4]oxazepin-3-yl)-*N*²-((*R*) -1-phenylethyl)oxalamide |
| 140 | 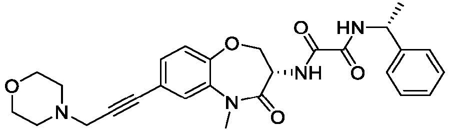 | *N*¹-((*S*)-5-methyl-7-(3-morpho linoprop-1-yn-1-yl)-4-oxo-2,3, 4,5-tetrahydrobenzo[*b*][1,4]ox azepin-3-yl)-*N*²-((*R*)-1-phenyl ethyl)oxalamide |
| 141 | 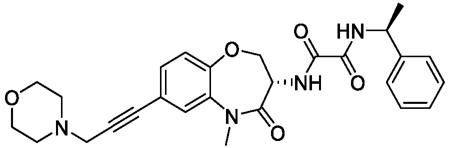 | *N*¹-((*S*)-5-methyl-7-(3-morpho linoprop-1-yn-1-yl)-4-oxo-2,3, 4,5-tetrahydrobenzo[*b*][1,4]ox azepin-3-yl)-*N*²-((*S*)-1-phenyle thyl)oxalamide |
| 142 | 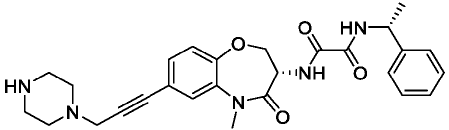 | *N*¹-((*S*)-5-methyl-4-oxo-7-(3-( piperazin-1-yl)prop-1-yn-1-yl) -2,3,4,5-tetrahydrobenzo[*b*][1, 4]oxazepin-3-yl)-*N*²-((*R*)-1-ph enylethyl)oxalamide |
| 143 | 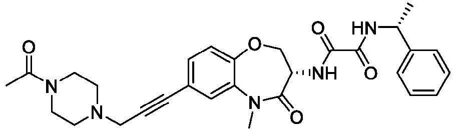 | *N*¹-((*S*)-7-(3-(4-acetylpiperazi n-1-yl)prop-1-yn-1-yl)-5-meth yl-4-oxo-2,3,4,5-tetrahydroben zo[*b*][1,4]oxazepin-3-yl)-*N*²-(( *R*)-1-phenylethyl)oxalamide |
| 144 | 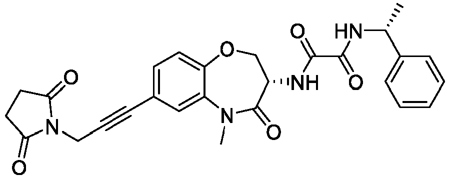 | *N*¹-((*S*)-7-(3-(2,5-dioxopyrroli din-1-yl)prop-1-yn-1-yl)-5-me thyl-4-oxo-2,3,4,5-tetrahydrob enzo[*b*][1,4]oxazepin-3-yl)-*N*² -((*R*)-1-phenylethyl)oxalamide |
| 145 | 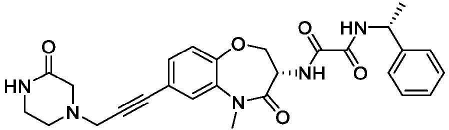 | *N*¹-((*S*)-5-methyl-4-oxo-7-(3-( 3-oxopiperazin-1-yl)prop-1-yn -1-yl)-2,3,4,5-tetrahydrobenzo [*b*][1,4]oxazepin-3-yl)-*N*²-((*R*) -1-phenylethyl)oxalamide |
| 146 | 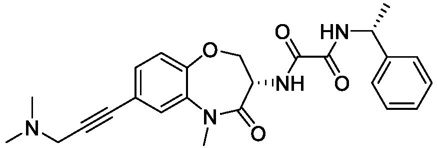 | *N*¹-((*S*)-7-(3-(dimethylamino)p rop-1-yn-1-yl)-5-methyl-4-oxo -2,3,4,5-tetrahydrobenzo[*b*] [1, 4]oxazepin-3-yl)-*N*²-((*R*)-1-ph enylethyl)oxalamide |
| 147 | 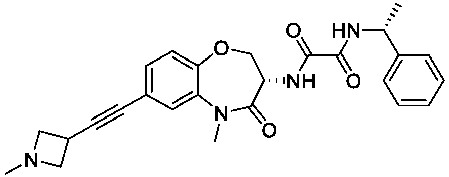 | *N*¹-((*S*)-5-methyl-7-((1-methyl azetidin-3-yl)ethynyl)-4-oxo-2 ,3,4,5-tetrahydrobenzo[*b*][1,4] oxazepin-3-yl)-*N*²-((*R*)-1-phen ylethyl)oxalamide |
| 148 | 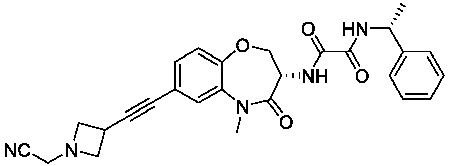 | *N*¹-((*S*)-7-((1-(cyanomethyl)az etidin-3-yl)ethynyl)-5-methyl-4-oxo-2,3,4,5-tetrahydrobenzo [*b*][1,4]oxazepin-3-yl)-*N*²-((*R*) -1-phenylethyl)oxalamide |
| 149 | 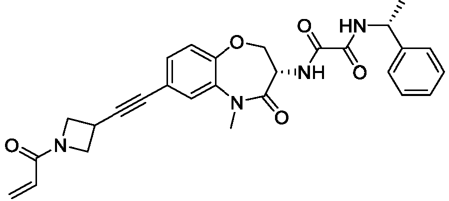 | *N*¹-((*S*)-7-((1-acryloylazetidin-3-yl)ethynyl)-5-methyl-4-oxo-2,3,4,5-tetrahydrobenzo[*b*][1,4 ]oxazepin-3-yl)-*N*²-((*R*)-1-phe nylethyl)oxalamide |
| 150 | 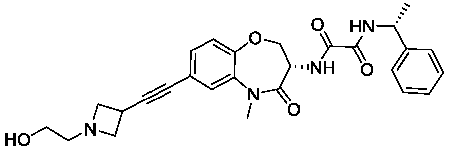 | *N*¹-((*S*)-7-((1-(2-hydroxyethyl) azetidin-3-yl)ethynyl)-5-meth yl-4-oxo-2,3,4,5-tetrahydroben zo[*b*][1,4]oxazepin-3-yl)-*N*²-(( *R*)-1-phenylethyl)oxalamide |
| 151 | 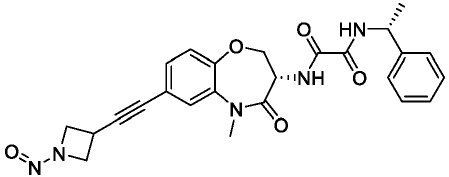 | *N*¹-((*S*)-5-methyl-7-((1-nitroso azetidin-3-yl)ethynyl)-4-oxo-2 ,3,4,5-tetrahydrobenzo[*b*][1,4] oxazepin-3-yl)-*N*²-((*R*)-1-phen ylethyl)oxalamide |
| 152 | 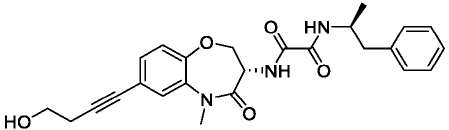 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*S*)-1-phenylpropa n-2-yl)oxalamide |
| 153 | 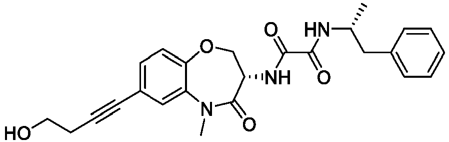 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*R*)-1-phenylpropa n-2-yl)oxalamide |
| 154 | 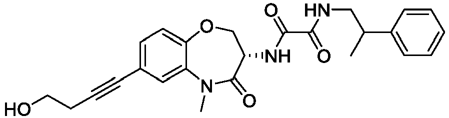 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-(2-phenylpropyl)ox alamide |
| 155 | 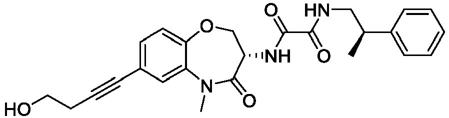 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*R*)-2-phenylpropyl)oxalamide |
| 156 | 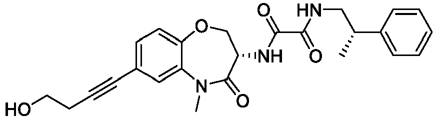 | *N¹*-((*S*)-7-(4-hydroxybut-1-yn-1-yl)-5-methyl-4-oxo-2,3,4,5-t etrahydrobenzo[*b*][1,4]oxazepi n-3-yl)-*N*²-((*S*)-2-phenylpropyl)oxalamide |
| 157 | 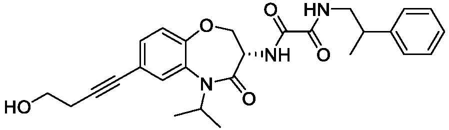 | *N*¹-((*S*)-7-(4-hydroxybut-1-yn-1-yl)-5-isopropyl-4-oxo-2,3,4, 5-tetrahydrobenzo[*b*][1,4]oxaz epin-3-yl)-*N*²-(2-phenylpropyl )oxalamide |
| 158 | 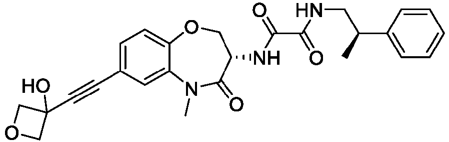 | *N*¹-((*S*)-7-((3-hydroxyoxetan-3 -yl)ethynyl)-5-methyl-4-oxo-2 ,3,4,5-tetrahydrobenzo[*b*][1,4] oxazepin-3-yl)-*N*²-((*R*)-2-phen ylpropyl)oxalamide |
| 159 | 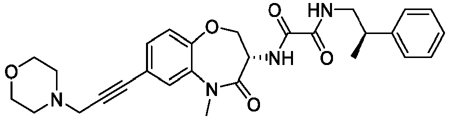 | *N*¹-((*S*)-5-methyl-7-(3-morpho linoprop-1-yn-1-yl)-4-oxo-2,3, 4,5-tetrahydrobenzo[*b*][1,4]ox azepin-3-yl)-*N*²-((*R*)-2-phenyl propyl)oxalamide |
| 160 | 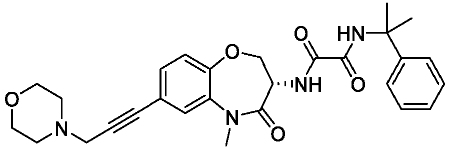 | (*S*)-*N*¹-(5-methyl-7-(3-morpho linoprop-1-yn-1-yl)-4-oxo-2,3, 4,5-tetrahydrobenzo[*b*][1,4]ox azepin-3-yl)-*N*²-(2-phenylpropan-2-yl)oxalamide |
| 161 | 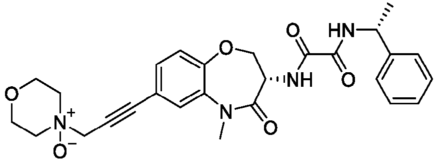 | 4-(3-((*S*)-5-methyl-4-oxo-3-(2 -oxo-2-(((*R*)-1-phenylethyl)am ino)acetamido)-2,3,4,5-tetrahy drobenzo[*b*][1,4]oxazepin-7-yl )prop-2-yn-1-yl)morpholine 4-oxide |
| 162 | 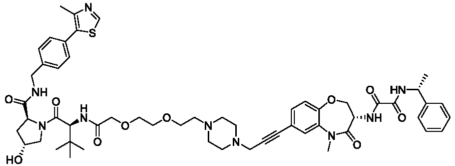 | *N*¹-((*S*)-7-(3-(4-(2-(2-(2-(((*S*)-1-((2*S*,4*R*)-4-hydroxy-2-((4-(4 -methylthiazol-5-yl)benzyl)car bamoyl)pyrrolidin-1-yl)-3,3-di methyl-1-oxobutan-2-yl)amin o)-2-oxoethoxy)ethoxy)ethyl) piperazin-1-yl)prop-1-yn-1-yl) -5-methyl-4-oxo-2,3,4,5-tetrah ydrobenzo[*b*][1,4]oxazepin-3-yl)-*N*²-((*R*)-1-phenylethyl)oxal amide |
| 163 | 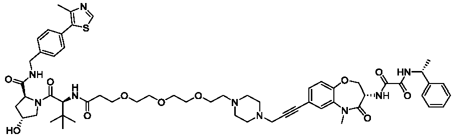 | *N*¹-((*S*)-7-(3-(4-((*S*)-14-((2*S*,4 *R*)-4-hydroxy-2-((4-(4-methylt hiazol-5-yl)benzyl)carbamoyl) pyrrolidine-1-carbonyl)-15,15-dimethyl-12-oxo-3,6,9-trioxa-13-azahexadecyl)piperazin-1-yl)prop-1-yn-1-yl)-5-methyl-4 -oxo-2,3,4,5-tetrahydrobenzo[ *b*][1,4]oxazepin-3-yl)-*N*²-((*R*)-1-phenylethyl)oxalamide |
| 164 | 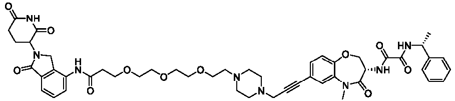 | *N*¹-((3*S*)-7-(3-(4-(2-(2-(2-(3-(( 2-(2,6-dioxopiperidin-3-yl)-1-oxoisoindolin-4-yl)amino)-3-o xopropoxy)ethoxy)ethoxy)eth yl)piperazin-1-yl)prop-1-yn-1-yl)-5-methyl-4-oxo-2,3,4,5-tet rahydrobenzo[*b*][1,4]oxazepin -3-yl)-*N*²-((*R*)- 1-phenylethyl)o xalamide |
| 165 | 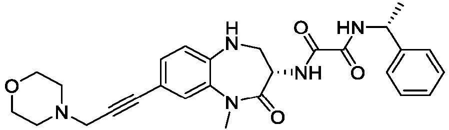 | *N*¹-((*S*)-1-methyl-8-(3-morpho linoprop-1-yn-1-yl)-2-oxo-2,3, 4,5-tetrahydro-1*H*-benzo[*b*][1, 4]diazepin-3-yl)-*N*²-((*R*)-1-phe nylethyl)oxalamide |
| 166 | 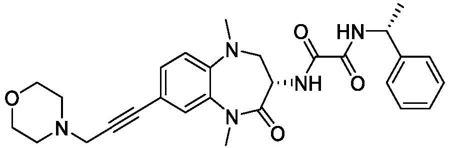 | *N*¹-((*S*)-1,5-dimethyl-8-(3-mor pholinoprop-1-yn-1-yl)-2-oxo-2,3,4,5-tetrahydro-1*H*-benzo[*b* ][1,4]diazepin-3-yl)-*N*²-((*R*)-1-phenylethyl)oxalamide |
| 167 | 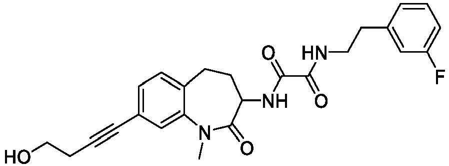 | *N*¹-(3-fluorophenethyl)-*N*²-(8-( 4-hydroxybut-1-yn-1-yl)-1-me thyl-2-oxo-2,3,4,5-tetrahydro-1*H*-benzo[*b*]azepin-3-yl)oxala mide |
| 168 | 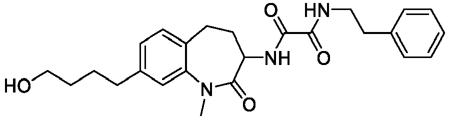 | *N*¹-(8-(4-hydroxybutyl)-1-met hyl-2-oxo-2,3,4,5-tetrahydro-1 *H*-benzo[*b*]azepin-3-yl)-*N*²-ph enethyloxalamide |

or their mesomers, racemates, enantiomers, diastereomers, or mixtures thereof, or pharmaceutically acceptable salts.

The present invention further provides a method for preparing the compounds represented by the general formula (I), (II) and (III) according to the present invention or their mesomers, racemates, enantiomers, diastereomers, or mixtures thereof, or pharmaceutically acceptable salts thereof, which comprises the following steps:

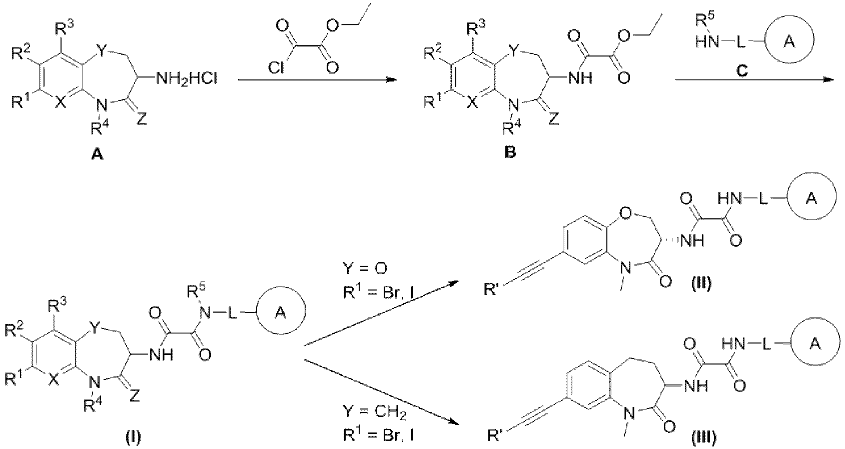

The parent core compound A reacts with monoethyl oxalyl chloride to obtain intermediate B, which then reacts with compound C to obtain the compound of formula (I) through aminolysis. When R¹ in the formula (I) is bromine or iodine, a Sonogashira coupling reaction is performed to obtain the compound of formula (II) or (III).

wherein, X, Y, Z, L, ring A, R¹, R², R³, R⁴ and R⁵ are defined as in general formula (I), and R' is defined as in general formula (II).

### Description of the terms of the invention

Unless otherwise stated, the terms used in the specification and claims have the following meanings.

In the present invention, when referring to a "compound" having a specific structural formula, it generally also covers its pharmaceutically acceptable salts, stereoisomers, diastereomers, enantiomers, racemic mixtures and isotopic derivatives.

It is well known to those skilled in the art that in addition to salts of a compound, solvates and hydrates are alternative forms of existence of the compound, which can all be converted into the compound under certain conditions. Therefore, when referring to a compound in the present invention, it generally also includes its solvates and hydrates.

The "pharmaceutically acceptable salt" described in the present invention refers to a salt of the compound of the present invention, which is suitable for contact with human and mammalian tissues within the scope of reasonable medical judgment without undue toxicity, irritation, allergic reaction, etc., and has the appropriate biological activity, which can be regarded as a reasonable benefit/risk ratio. The salt can be prepared in situ during the final separation and purification of the compound of the present invention, or prepared separately by reacting with a free base or a free acid with a suitable reagent. For example, the free base can react with a suitable acid. Examples of pharmaceutically acceptable acid addition salts are salts formed between an amino group (amine group) and an inorganic acid (e.g., hydrochloric acid, hydrobromic acid, phosphoric acid, sulfuric acid, and perchloric acid) or an organic acid (e.g., acetic acid, oxalic acid, maleic acid, tartaric acid, citric acid, succinic acid, or malonic acid), or by using other methods known in the art such as ion exchange. The pharmaceutically acceptable salts of the present invention can be prepared by conventional methods, for example, by dissolving the compound of the present invention in an organic solvent miscible with water (e.g., methanol, ethanol, acetone, and acetonitrile), adding an excess of an organic acid or an inorganic acid aqueous solution thereto, so that the salt is precipitated from the resulting mixture, removing the solvent and the remaining free acid therefrom, and then isolating the precipitated salt. Other pharmaceutically acceptable salts include sodium alginate, ascorbate, benzenesulfonate, adipate, camphorsulfonate, aspartate, benzoate, bisulfate, borate, butyrate, camphorate, citrate, dodecylsulfate, ethanesulfonate, formate, fumarate, glucoheptonate, glycerophosphate, gluconate, heptanoate, hexanoate, hydroiodide, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate and the like.

In the specification and claims, a given chemical formula or name shall encompass all stereo and optical isomers and racemates in which such isomers exist. Unless otherwise indicated, all chiral (enantiomers and diastereoisomers) and racemic forms are within the scope of the present invention. There may also be many geometric isomers of C=C double bonds, C=N double bonds, ring systems and the like in the compounds described, and all such stable isomers are encompassed by the present invention. The present invention describes *cis-* and *trans-* (or *E*- and *Z*-) geometric isomers of the compounds of the present invention, and they may be separated into mixtures of isomers or into separate isomeric forms.

The compounds of the present invention may be isolated in optically active or racemic form. All processes for preparing the compounds of the present invention and intermediates prepared therein are considered part of the present invention. When enantiomeric or diastereomeric products are prepared, they may be separated by conventional methods (e.g., by chromatography or fractional crystallization). It should be understood that all tautomeric forms that may exist are included in the present invention. The compounds of the present invention may be commercially available when they are known compounds in the prior art.

The term "alkyl" refers to a branched and straight-chain saturated aliphatic hydrocarbon group having a specified number of carbon atoms. The alkyl group in the present invention is preferably C₁-C₁₂ alkyl, C₁-C₁₀ alkyl, C₁-C₈ alkyl, more preferably C₁-C₆ alkyl, particularly preferably C₁-C₄ alkyl, and especially C₁-C₃ alkyl. For example, "C₁-C₆ alkyl" means an alkyl group having 1 to 6 carbon atoms. Examples of alkyl groups include, but are not limited to, methyl, ethyl, propyl (e.g., n-propyl and isopropyl), butyl (e.g., n-butyl, isobutyl, tert-butyl) and pentyl (e.g., n-pentyl, isopentyl, neopentyl). The alkyl group may be substituted or unsubstituted. When substituted, the substituent may be substituted at any available connection point, and the substituent is preferably one or more of the following groups, which are independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkyloxy, heterocycloalkyloxy, cycloalkylthio, heterocycloalkylthio, oxo, carboxyl or carboxylate. For the C₁-C₁₂ alkyl group in the present invention, 1 to 4 -CH₂- units therein are optionally replaced by O atoms, S atoms or -NH-.

The term "alkoxy" refers to -O-(alkyl) or -O-(unsubstituted cycloalkyl). For example, "C₁-C₆ alkoxy" refers to C₁, C₂, C₃, C₄, C₅, C₆ alkoxy. Preferred alkoxy is C₁-C₁₀ alkoxy, C₁-C₈ alkoxy, more preferably C₁-C₆ alkoxy, particularly preferably C₁-C₄ alkoxy, especially C₁-C₃ alkoxy. Examples of alkoxy groups include, but are not limited to, methoxy, ethoxy, propoxy (e.g., n-propoxy and isopropoxy), tert-butoxy, cyclopropyl, cyclobutyl, cyclopentyl, and cyclohexyl. Alkoxy groups may be optionally substituted or unsubstituted, and when substituted, the substituents are preferably one or more of the following groups, independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxyl, or carboxylate groups. Similarly, "alkylthio" refers to an alkyl group as defined above connected *via* a sulfur bridge with a specified number of carbon atoms; for example, methyl-S- and ethyl-S-. Likewise, preferred alkylthio is C₁-C₁₀ alkylthio, C₁-C₈ alkylthio, more preferably C₁-C₆ alkylthio, particularly preferably C₁-C₄ alkylthio, and especially C₁-C₃ alkylthio.

The term "alkenyl" refers to an alkyl group as defined above consisting of at least two carbon atoms and at least one carbon-carbon double bond, such as vinyl, 1-propenyl, 2-propenyl, 1-, 2- or 3-butenyl, etc. The alkenyl group may be substituted or unsubstituted, and when substituted, the substituent is preferably one or more of the following groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkyloxy, heterocycloalkyloxy, cycloalkylthio, heterocycloalkylthio. Preferred are C₂-C₆ alkenyl or C₂-C₄ alkenyl.

The term "alkynyl" refers to an alkyl group as defined above consisting of at least two carbon atoms and at least one carbon-carbon triple bond, such as ethynyl, propynyl, butynyl, etc. Alkynyl may be substituted or unsubstituted, and when substituted, the substituent is preferably one or more of the following groups independently selected from alkyl, alkenyl, alkynyl, alkoxy, alkylthio, alkylamino, halogen, sulfydryl, hydroxyl, nitro, cyano, cycloalkyl, heterocycloalkyl, aryl, heteroaryl, cycloalkyloxy, heterocycloalkyloxy, cycloalkylthio, heterocycloalkylthio. Preferred are C₂-C₆ alkynyl or C₂-C₄ alkynyl.

The term "halo" or "halogen" includes fluorine, chlorine, bromine and iodine. In the present invention, one or more halogens may be independently selected from fluorine, chlorine, bromine and iodine.

The term "haloalkyl" refers to a branched and straight-chain saturated aliphatic hydrocarbon group having a specified number of carbon atoms and substituted with one or more halogens. Examples of haloalkyl groups include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Preferred haloalkyl groups include halo(C₁-C₆ alkyl) or halo(C₁-C₄ alkyl).

The term "oxo" or "carbonyl" refers to an organic functional group formed by a double bond between carbon and oxygen atoms (C=O or C(O)).

The term "benzyl" refers to -CH₂-phenyl or "Bn".

The term "hydroxyl" refers to an -OH group.

The term "amino" refers to -NH₂.

The term "cyano" refers to -CN.

The term "nitro" refers to -NO₂.

The term "carboxyl" refers to -C(O)OH.

The term "thiol" refers to -SH.

The term "ester" or "carboxylate" refers to -C(O)O-(alkyl) or -C(O)O(cycloalkyl), wherein alkyl and cycloalkyl are as defined above.

The term "acyl" refers to a compound containing a -C(O)R group, wherein R is an alkyl, cycloalkyl, heterocyclyl, aryl, or heteroaryl group.

The term "cycloalkyl" refers to a saturated or partially unsaturated monocyclic or polycyclic hydrocarbon substituent, wherein the cycloalkyl ring contains 3 to 20 carbon atoms, and the cycloalkyl of the present invention is preferably C₃-C₈ cycloalkyl or C₃-C₆ cycloalkyl. Monocyclic cycloalkyl includes, but is not limited to, cyclopropyl, cyclobutyl, cyclopentyl, cyclopentenyl, cyclohexyl, cyclohexenyl, cyclohexadienyl, cycloheptyl, cycloheptatrienyl, and cyclooctyl, and polycyclic cycloalkyl includes, but is not limited to, spirocyclic, fused, and bridged cycloalkyl, such as norbornyl.

The cycloalkyl ring may be fused to an aryl, heteroaryl or heterocyclic ring, wherein the ring connected to the parent structure is a cycloalkyl, non-limiting examples of which include but are not limited to:

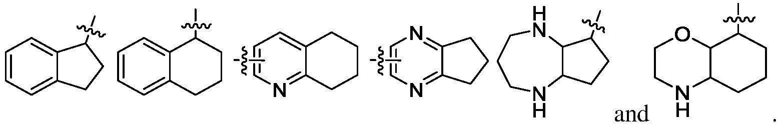

Cycloalkyl may be optionally substituted or unsubstituted, and when substituted, the substituents are preferably one or more of the following groups independently selected from alkyl, alkenyl, alkynyl, alkoxy, hydroxyalkyl, alkylthio, alkylamino, halogen, sulfydryl, hydroxyl, nitro, cyano, cycloalkyl, heterocycloalkyl, aryl, aryloxy, heteroaryl, cycloalkyloxy, heterocycloalkyloxy, cycloalkylthio, heterocycloalkylthio, oxo, carboxyl or carboxylate.

The term "heterocyclyl" refers to a saturated or partially unsaturated monocyclic or polycyclic cyclic hydrocarbon substituent containing 3 to 20 ring atoms, one or more of which is a heteroatom selected from N, O and S (the N and S heteroatoms may be optionally oxidized), but excluding the ring portion of -O-O-, -O-S- or -S-S-, and the remaining ring atoms are carbon. Preferably, it contains 3 to 12 ring atoms, of which 1 to 4 are heteroatoms; most preferably, it contains 3 to 8 ring atoms, of which 1 to 3 are heteroatoms; most preferably, it contains 5 to 7 ring atoms, of which 1 to 2 or 1 to 3 are heteroatoms. Examples of monocyclic heterocyclyl includes, but is not limited to, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and tetrahydropyranyl, and polycyclic heterocyclyl includes, but is not limited to, spirocyclic, fused and bridged heterocyclyl.

The heterocyclic ring may be fused to an aryl, heteroaryl or cycloalkyl ring, wherein the ring connected to the parent structure is a heterocyclyl, and its non-limiting examples include, but are not limited to:

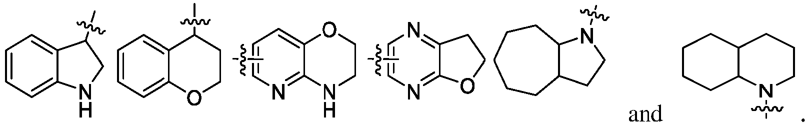

The heterocyclyl group may be optionally substituted or unsubstituted, and when substituted, the substituent is preferably one or more of the following groups, which are independently selected from alkyl, alkenyl, alkynyl, alkoxy, hydroxyalkyl, alkylthio, alkylamino, halogen, sulfydryl, hydroxyl, nitro, cyano, cycloalkyl, heterocycloalkyl, aryl, aryloxy, heteroaryl, cycloalkyloxy, heterocycloalkyloxy, cycloalkylthio, heterocycloalkylthio, oxo, carboxyl or carboxylate ester group.

The term "aryl" refers to a monocyclic, bicyclic or tricyclic ring system with a total of 6 to 14 ring atoms having a conjugated π electron system, wherein at least one ring in the system is aromatic and wherein each ring in the system contains 3 to 7 ring atoms. In certain embodiments of the present invention, "aryl" refers to an aromatic ring system, which includes but is not limited to phenyl, naphthyl, biphenyl, indanyl, 1-naphthyl, 2-naphthyl and tetrahydronaphthyl. The aryl group of the present invention is preferably C₆-C₁₀ aryl. The aryl group may be substituted or unsubstituted, and when substituted, the substituents are preferably one or more of the following groups, which are independently selected from alkyl, alkenyl, alkynyl, alkoxy, hydroxyalkyl, alkylthio, alkylamino, halogen, sulfydryl, hydroxyl, nitro, cyano, cycloalkyl, heterocycloalkyl, aryl, aryloxy, heteroaryl, cycloalkyloxy, heterocycloalkyloxy, cycloalkylthio, heterocycloalkylthio, carboxyl or carboxylate ester group.

The term "heteroaryl" refers to a stable 3-, 4-, 5-, 6-, or 7-membered aromatic monocyclic ring or a 7-, 8-, 9-, or 10-membered aromatic bicyclic or aromatic polycyclic heterocyclic ring, which is fully unsaturated or partially unsaturated and contains carbon atoms and 1, 2, 3 or 4 heteroatoms independently selected from N, O and S (the N and S heteroatoms may be optionally oxidized). The nitrogen atom is substituted or unsubstituted (i.e., N or NR, where R is H or, if defined, another substituent). The heterocyclic ring can be connected to its side group at any heteroatom or carbon atom to obtain a stable structure. If the resulting compound is stable, the heterocyclyl of the present invention can be substituted on carbon or nitrogen atom. The nitrogen in the heterocyclic ring can be optionally quaternized. Preferably, when the total number of S and O atoms in the heterocyclic ring exceeds 1, these heteroatoms are not adjacent to each other. Preferably, the total number of S and O atoms in the heterocyclic ring is not more than 1. When the term "heterocyclic ring" is used, it is intended to include heteroaryl. The examples of heteroaryl include but are not limited to acridinyl, imidazolyl, furyl, thienyl, oxazolyl, thiazolyl, benzotriazolyl, benzotetrazolyl, benzisoxazolyl, benzisothiazolyl, indolyl, indolizinyl, indazolyl, pyrimidinyl, phenazinyl, piperazinyl, piperidinyl, purinyl, pyranyl, pyrazinyl, pyrrolyl and quinolyl. The term "heteroaryl" may also include biaryl structures formed by the above-defined "aryl", "heterocyclyl" or "cycloalkyl" and monocyclic "heteroaryl", such as but not limited to "-phenylbipyridyl-", "-phenylbipyrimidyl-", "-pyridylbinaphthyl-", "-pyrimidylbinaphthyl-" and "-pyridylbipyrimidyl-", wherein the present invention also includes spirocyclic, fused and bridged ring compounds containing, for example, the above-mentioned heterocycles.

"Optional" or "optionally" in the present invention means that the event or environment described subsequently may but not necessarily occur, and the description includes occasions where the event or environment occurs or does not occur. For example, "heterocyclyl optionally substituted by alkyl" means that alkyl may but not necessarily exist, and the description includes the case where the heterocyclyl is substituted by alkyl and the case where the heterocyclyl is not substituted by an alkyl.

"Substitution" or "substituted" as used herein refers to one or more hydrogen atoms in a group, preferably up to 5, more preferably 1 to 3, being replaced independently of each other by a corresponding number of substituents, provided that normal valence is maintained and the substitution results in a stable compound. It goes without saying that the substituents are only in their possible chemical positions, and those skilled in the art are able to determine (by experiment or theory) possible or impossible substitutions without undue effort. For example, an amino or hydroxyl group with free hydrogen may be unstable when combined with a carbon atom with an unsaturated bond (such as olefin).

"Pharmaceutical composition" means a mixture containing one or more compounds of the present invention or their physiologically/pharmaceutically acceptable salts or prodrugs and other chemical components, as well as other components such as physiologically/pharmaceutically acceptable carriers and excipients. The purpose of a pharmaceutical composition is to facilitate administration to an organism, facilitate the absorption of the active ingredient, and thus exert biological activity.

### DETAILED DESCRIPTION

In order to better illustrate the technical means and effects of the present invention, the present invention is further illustrated in conjunction with non-limiting examples. The embodiments of the present invention, including the descriptions provided in the examples, are intended to illustrate the embodiments of the present invention and are not intended to limit the scope of any claims. According to the present invention, those skilled in the art will understand that many changes can be made to the disclosed specific embodiments without departing from the spirit and scope of the present invention and the same or similar results can still be obtained.

Unless otherwise stated, all materials/reagents were obtained from commercial suppliers and used without further purification. The structures of the compounds in the following examples were characterized and determined by nuclear magnetic resonance (NMR) and/or mass spectrometry (MS).

The ¹H NMR spectra were recorded at room temperature on a Bruker Avance 400 MHz spectrometer, and the determination solvents were deuterated dimethyl sulfoxide (DMSO-*d*₆), deuterated chloroform (CDCl₃), deuterated methanol (CD₃OD) or deuterated water (D₂O). Chemical shift values (*δ*) are in ppm, with tetramethylsilane (TMS) or residual solvent peak as internal standard, coupling constant (*J*) is in Hertz (Hz), and the abbreviations of the multiplicity of peaks in ¹H NMR spectra are as follows: s (singlet), d (doublet), t (triplet), q (quartet), qn (quintet), m (multiplet), br (broad peak).

The instrument used for liquid chromatography-mass spectrometry (LC-MS) was Shimadzu LCMS-2020, and the instrument used for preparative high performance liquid chromatography (Prep-HPLC) was Bonna-Agela FLEXA FL-H100G. The thin layer chromatography silica gel plate used for thin layer chromatography (TLC) was Yantai Huanghai HSGF254 thin layer chromatography silica gel plate, the type used for reaction monitoring was 2.5 × 8 cm with 0.2 ± 0.03 mm coating thickness, the type used for separation and purification was 20 × 20 cm with 0.4 - 0.5 mm coating thickness. The silica gel used as the carrier for column chromatography was Qingdao Ocean Silica Gel 100 - 200 mesh or 200 - 300 mesh silica gel.

Unless otherwise defined, all professional and scientific terms used in the text have the same meaning as those familiar to those skilled in the art. In addition, any methods and materials similar or equivalent to the content described can be applied to the methods of the present invention.

### Example 1: Synthesis of (S)-N¹-(5-methyl-4-oxo-2,3,4,5-tetrahydrobenzo[b][1,4]oxazepin-3-yl)-N²-phenethyloxalamide (1)

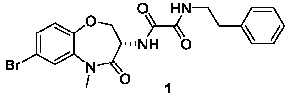

### Synthesis route:

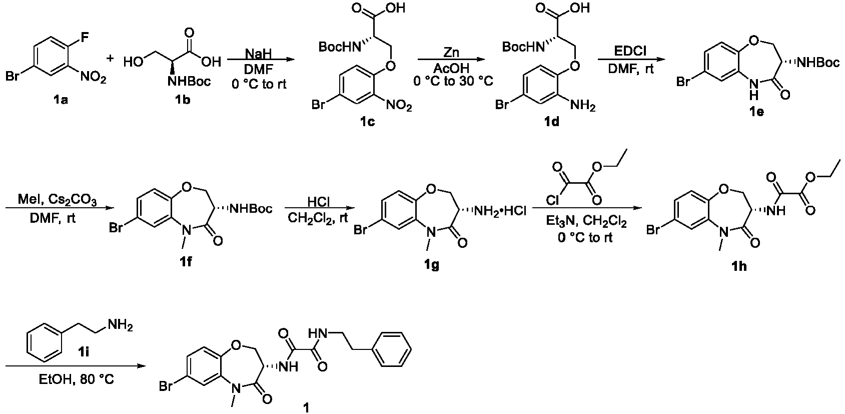

### Step 1: Synthesis of O-(4-bromo-2-nitrophenyl)-N-(tert-butoxycarbonyl)-L-serine (1c)

To a suspension of sodium hydride (60% w/w dispersed in mineral oil, 1.74 g, 47.39 mmol) in DMF (*N,N*-dimethylformamide, 20 mL) at 0 °C in an ice bath, a solution of (*tert*-butoxycarbonyl)-*L*-serine **1b** (4.24 g, 20.66 mmol) in DMF (5 mL) was added. After the reaction mixture was stirred for 1 hour, 4-bromo-1-fluoro-2-nitrobenzene **1a** (5 g, 22.73 mmol) in DMF (10 mL) was added dropwise to the reaction solution, and then the reaction solution was slowly warmed to room temperature and stirred for another 15 hours. TLC and LC-MS showed that the reaction was complete, and the reaction mixture was poured into ice water (300 mL), the aqueous phase was acidified to pH = 4 with dilute hydrochloric acid (1 N), and then extracted with ethyl acetate (100 mL × 5). The combined organic layers were washed with water (300 mL × 2) and saturated brine (100 mL) in turn, and then dried over anhydrous sodium sulfate, and concentrated under reduced pressure to obtain a crude product, which was then purified by silica gel column chromatography (dichloromethane containing 1% methanol as the eluent) to obtain compound **1c** (3.5 g, 38% yield) as a yellow solid.

MS (ES⁺): *m*/*z* 347.9 [M-^{t}Bu+H]⁺.

### Step 2: Synthesis of O-(2-amino-4-bromophenyl)-N-(tert-butoxycarbonyl)-L-serine (1d)

Zinc powder (2.8 g, 43.2 mmol) was added to a solution of compound **1c** (3.5 g, 8.6 mmol) in acetic acid (50 mL) at 0 °C in an ice bath, and the resulting mixture was stirred at 30 °C for 3 hours. TLC and LC-MS showed that the reaction was complete. The reaction mixture was filtered through celite, the filter cake was rinsed with dichloromethane (300 mL), and the filtrate was concentrated under reduced pressure. The residue after concentration was then dissolved in water (50 mL), neutralized with saturated aqueous solution of sodium bicarbonate to pH = 7, and then extracted with dichloromethane (50 mL). The organic layers were combined, washed with saturated brine (20 mL), dried over anhydrous sodium sulfate and concentrated under reduced pressure to obtain a crude product of compound **1d** (3.4 g) as a black solid, which was used directly in the next step without further purification.

MS (ES⁺): *m*/*z* 374.9 [M+H]⁺.

### Step 3: Synthesis of tert-butyl (S)-(7-bromo-4-oxo-2,3,4,5-tetrahydrobenzo[b][1,4]oxazepin-3-yl)carbamate (1e)

1-Ethyl-3-(3-dimethylaminopropyl)carbodiimide hydrochloride (EDCI, 1.74 g, 9.1 mmol) was added to a DMF (50 mL) solution of compound **1d** (3.4 g, 9.1 mmol) at room temperature, and the reaction solution was stirred at room temperature for 2 hours. TLC and LC-MS showed that the reaction was complete, and the reaction mixture was poured into water (300 mL) and extracted with ethyl acetate (100 mL × 3). The organic layers were combined, washed with saturated brine (100 mL), dried over anhydrous sodium sulfate and concentrated under reduced pressure, and then purified by silica gel column chromatography (petroleum ether containing 15% ethyl acetate as the eluent) to obtain compound **1e** (880 mg, 28% two-step yield) as a light yellow solid.

MS (ES⁺): *m*/*z* 300.7 [M-^{t}Bu+H]⁺.

¹H NMR (400 MHz, DMSO-*d*₆): *δ* 10.03 (s, 1H), 7.29 - 7.25 (m, 2H), 7.14 (d, *J =* 7.6 Hz, 1H), 7.07 (d, *J =* 8.4 Hz, 1H), 4.35 - 4.26 (m, 3H), 1.36 (s, 9H).

### Step 4: Synthesis of tert-butyl (S)-(7-bromo-5-methyl-4-oxo-2,3,4,5-tetrahydrobenzo[b][1,4]oxazepin-3-yl)carbamate (1f)

To a solution of compound **1e** (2.1 g, 5.88 mmol) and cesium carbonate (2.5 g, 7.6 mmol) in DMF (30 mL) solution was added iodomethane (0.9 g, 6.5 mmol), and the reaction mixture was stirred at room temperature for 15 hours. TLC and LC-MS showed that the reaction was complete, and the reaction mixture was poured into water (400 mL) and extracted with ethyl acetate (100 mL × 3). The combined organic layers were washed with water (50 mL × 2) and saturated brine (100 mL) in turn, dried over anhydrous sodium sulfate and concentrated under reduced pressure, and then purified by silica gel column chromatography (petroleum ether containing 15% ethyl acetate as the eluent) to obtain compound **1f** (1.3 g, 60% yield) as a light yellow solid.

MS (ES⁺): *m*/*z* 370.9 [M+H]⁺.

¹H NMR (400 MHz, DMSO-*d*₆): *δ* 7.74 (d, *J =* 2.0 Hz, 1H), 7.43 (dd, *J* = 8.6, 2.2 Hz, 1H), 7.19 (d, *J* = 8.4 Hz, 1H), 7.15 (d, *J =* 8.8 Hz, 1H), 4.40 - 4.25 (m, 3H), 3.27 (s, 3H), 1.34 (s, 9H).

### Step 5: Synthesis of (S)-3-amino-7-bromo-5-methyl-2,3-dihydrobenzo[b][1,4]oxazepine-4(5H)-one hydrochloride (1g)

A solution of hydrogen chloride in 1,4-dioxane (4 N, 25 mL) was added dropwise to a solution of compound **1f** (1.6 g, 4.3 mmol) in dichloromethane (20 mL) at room temperature, and the reaction solution was stirred at room temperature for 3 hours. TLC and LC-MS showed that the reaction was complete, and the solvent and other volatiles were removed by concentration under reduced pressure to obtain compound **1g** (1.3 g, 98% yield) as a white solid.

MS (ES⁺): *m*/*z* 272.2 [M+H]⁺.

¹H NMR (400 MHz, DMSO-*d*₆): *δ* 8.66 (s, 3H), 7.80 (d, *J =* 2.4 Hz, 1H), 7.48 (dd, *J* = 8.6, 2.2 Hz, 1H), 7.23 (d, *J =* 8.8 Hz, 1H), 4.60 (dd, *J =* 10.0, 7.3 Hz, 1H), 4.46 (t, *J =* 10.6 Hz, 1H), 4.34 (dd, *J* = 11.2, 7.6 Hz, 1H), 3.34 (s, 3H).

### Step 6: Synthesis of ethyl (S)-2-((7-bromo-5-methyl-4-oxo-2,3,4,5-tetrahydrobenzo[b][1,4]oxazepin-3-yl)amino)-2-oxoacet ate (1h)

To a mixture of compound **1g** (1.3 g, 4.2 mmol) and triethylamine (1.3 g, 12.8 mmol) in dichloromethane (30 mL), a solution of monoethyl oxalyl chloride (0.7 g, 5.1 mmol) in dichloromethane (5 mL) was added dropwise at 0 °C in an ice bath. The reaction solution was slowly warmed to room temperature and stirred for 3 hours. TLC and LC-MS showed that the reaction was complete. The reaction mixture was poured into water (200 mL) and extracted with dichloromethane (100 mL × 3). The organic layers were combined, washed with saturated brine (100 mL), dried over anhydrous sodium sulfate and concentrated under reduced pressure, and then purified by silica gel column chromatography (petroleum ether containing 20% ethyl acetate as the eluent) to obtain compound **1h** (1.4 g, 90% yield) as a white solid.

MS (ES⁺): *m*/*z* 370.8 [M+H]⁺.

¹H NMR (400 MHz, CDCl₃): *δ* 7.98 (d, *J =* 6.6 Hz, 1H), 7.38 - 7.35 (m, 2H), 7.09 - 7.07 (m, 1H), 4.86 (dt, *J =* 11.1, 7.2 Hz, 1H), 4.67 (dd, *J* = 9.7, 7.5 Hz, 1H), 4.35 (q, *J =* 7.1 Hz, 2H), 4.21 (t, *J* = 10.5 Hz, 1H), 3.41 (s, 3H), 1.37 (t, *J =* 7.1 Hz, 3H).

### Step 7: Synthesis of (S)-N¹-(7-bromo-5-methyl-4-oxo-2,3,4,5-tetrahydrobenzo[b][1,4]oxazepin-3-yl)-N²-phenethyloxa lamide (1)

To a solution of compound **1h** (1.4 g, 3.8 mmol) in ethanol (20 mL), 2-phenylethylamine **1i** (1.0 g, 8.3 mmol) was added, the reaction solution was heated to 80 °C and stirred for 4 hours. TLC and LC-MS showed that the reaction was complete and a large amount of white solid precipitated in the reaction solution. The reaction solution was cooled to room temperature and filtered, and the filter cake was rinsed with petroleum ether (10 mL) containing 15% ethyl acetate. After the filter cake was dried, the title compound **1** (1.3 g, 77% yield) was obtained as a white solid.

MS (ES⁺): *m*/*z* 446.0 [M+H]⁺.

¹H NMR (400 MHz, CDCl₃): *δ* 8.22 (d, *J* = 7.5 Hz, 1H), 7.35 - 7.28 (m, 5H), 7.25 - 7.21 (m, 1H), 7.18 (d, *J* = 7.3 Hz, 2H), 7.06 (d, *J* = 8.9 Hz, 1H), 4.81 (dt, *J* = 11.2, 7.4 Hz, 1H), 4.58 (dd, *J* = 9.6, 7.5 Hz, 1H), 4.23 (t, *J* = 10.5 Hz, 1H), 3.59 - 3.54 (m, 2H), 3.40 (s, 3H), 2.84 (t, *J =* 7.1 Hz, 2H).

### Example 2: Synthesis of (S)-N¹-(7-(cyclopropylethynyl)-5-methyl-4-oxo-2,3,4,5-tetrahydrobenzo[b][1,4]oxazepin-3-yl)-N ²-phenethyloxalamide (2)

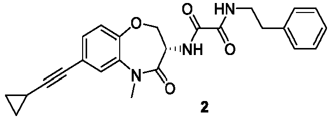

### Synthesis route:

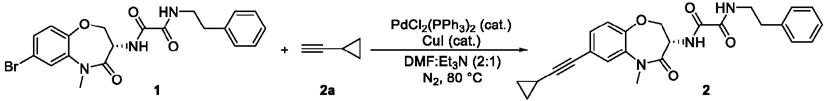

Compound **1** (44.6 mg, 0.1 mmol), bis(triphenylphosphine)palladium(II) dichloride (7.0 mg, 0.01 mmol), cuprous iodide (1.9 mg, 0.01 mmol), triethylamine (1 mL), DMF (2 mL) and cyclopropylacetylene **2a** (13.2 mg, 0.2 mmol) were added in turn to a sealed tube at room temperature. After the air in the tube was quickly replaced with nitrogen three times, the reaction solution was stirred at 80 °C for 15 hours. TLC and LC-MS showed that the reaction was complete. After the reaction solution was cooled to room temperature, water (20 mL) was added to quench it and extracted with ethyl acetate (10 mL × 3). The combined organic layers were washed with saturated brine (10 mL), dried over anhydrous sodium sulfate, and concentrated under reduced pressure to obtain a crude product, which was then purified by silica gel column chromatography (petroleum ether containing 20% ethyl acetate as the eluent) to obtain the title compound **2** (38.8 mg, 90% yield) as a light yellow solid.

MS (ES⁺): *m*/*z* 432.2 [M+H]⁺.

¹H NMR (400 MHz, CDCl₃): *δ* 8.26 (d, *J* = 7.2 Hz, 1H), 7.37 - 7.27 (m, 4H), 7.23 - 7.20 (m, 2H), 7.17 (d, *J* = 7.4 Hz, 2H), 7.05 (d, *J* = 8.7 Hz, 1H), 4.79 (dt, *J* = 11.1, 7.4 Hz, 1H), 4.56 (dd, *J* = 9.5, 7.5 Hz, 1H), 4.22 (t, *J* = 10.5 Hz, 1H), 3.57 - 3.52 (m, 2H), 3.38 (s, 3H), 2.83 (t, *J* = 7.0 Hz, 2H), 1.48 - 4.41 (m, 1H), 0.91 - 0.86 (m, 2H), 0.83 - 0.80 (m, 2H).

### Example 3: Synthesis of (S)-N¹-(8-bromo-5-methyl-4-oxo-2,3,4,5-tetrahydrobenzo[b][1,4]oxazepin-3-yl)-N²-phenethyloxa lamide (3)

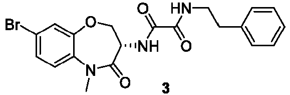

### Synthesis route:

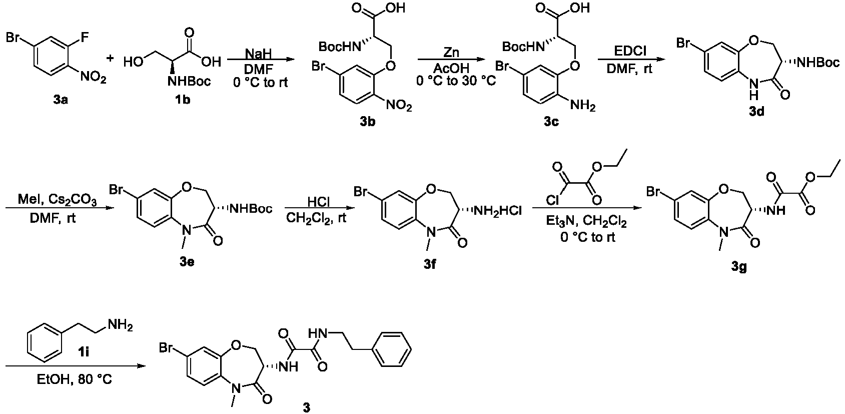

The synthesis route and method were the same as Example 1, except that compound 4-bromo-1-fluoro-2-nitrobenzene **1a** in step 1 was replaced with an equivalent amount of 4-bromo-2-fluoro-1-nitrobenzene **3a** to obtain the title compound **3.**

MS (ES⁺): *m*/*z* 445.9 [M+H]⁺.

¹H NMR (400 MHz, CDCl₃): *δ* 8.23 (d, *J* = 7.1 Hz, 1H), 7.39 - 7.34 (m, 2H), 7.32 - 7.28 (m, 3H), 7.25 - 7.21 (m, 1H), 7.18 (d, *J* = 7.3 Hz, 2H), 7.08 (d, *J* = 8.4 Hz, 1H), 4.81 (dt, *J* = 11.3, 7.4 Hz, 1H), 4.59 (dd, *J* = 9.7, 7.4 Hz, 1H), 4.24 (t, *J* = 10.5 Hz, 1H), 3.59 - 3.54 (m, 2H), 3.39 (s, 3H), 2.84 (t, *J* = 7.1 Hz, 2H).

### Example 4: Synthesis of (S)-N¹-(8-(cyclopropylethynyl)-5-methyl-4-oxo-2,3,4,5-tetrahydrobenzo[b][1,4]oxazepin-3-yl)-N ²-phenethyloxalamide (4)

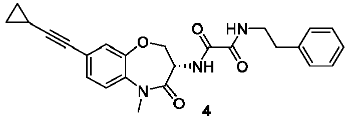

### Synthesis route:

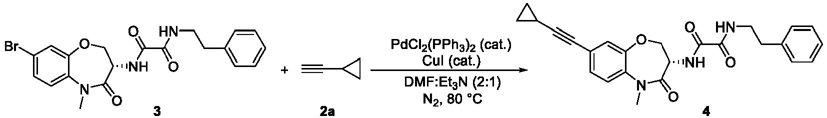

The synthesis route and method were the same as Example 3, except that compound **1** was replaced with an equivalent amount of compound **3** to obtain the title compound **4.**

MS (ES⁺): *m*/*z* 432.1 [M+H]⁺.

¹H NMR (400 MHz, CDCl₃): *δ* 8.23 (d, *J* = 7.4 Hz, 1H), 7.34 - 7.28 (m, 3H), 7.24 - 7.20 (m, 2H), 7.18 - 7.16 (m, 2H), 7.09 (d, *J =* 8.3 Hz, 1H), 4.79 (dt, *J =* 11.1, 7.5 Hz, 1H), 4.56 (dd, *J* = 9.7, 7.5 Hz, 1H), 4.21 (t, *J =* 10.5 Hz, 1H), 3.58 - 3.52 (m, 2H), 3.38 (s, 3H), 2.83 (t, *J* = 7.1 Hz, 2H), 1.47 - 4.42 (m, 1H), 0.92 - 0.87 (m, 2H), 0.83 - 0.79 (m, 2H).

### Example 5: Synthesis of (S)-N¹-(8-hydroxy-5-methyl-4-oxo-2,3,4,5-tetrahydrobenzo[b][1,4]oxazepin-3-yl)-N²-phenethylo xalamide (5)

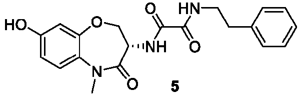

### Synthesis route:

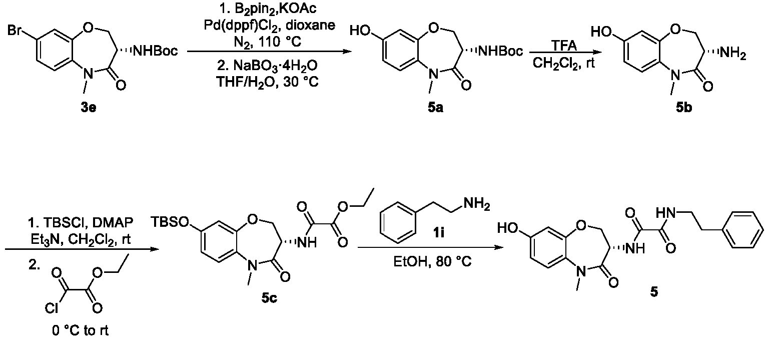

### Step 1: Synthesis of tert-butyl (S)-(8-hydroxy-5-methyl-4-oxo-2,3,4,5-tetrahydrobenzo[b][1,4]oxazepin-3-yl)carbamate (5a)

At room temperature, Pd(dppf)Cl₂ (102 mg, 0.14 mmol) was added to a mixture of a solution of compound **3e** (1.0 g, 2.7 mmol), bis(pinacolato)diboron (863 mg, 3.4mmol) and potassium acetate (540 mg, 5.4 mmol) in 1,4-dioxane (20 mL). After the air in the flask was quickly replaced with nitrogen three times, the reaction solution was kept stirring at 110 °C for 15 hours. TLC and LC-MS showed that the reaction was complete. After the reaction solution was cooled to room temperature, it was diluted with ethyl acetate (10 mL) and filtered. The filtrate was concentrated under reduced pressure. The residue after concentration was then dissolved in a mixed solvent of THF/H₂O (2:1, 30 mL), sodium perborate tetrahydrate (831 mg, 5.4 mmol) was added, and the reaction solution was kept stirring at 30 °C for 2 hours. TLC and LC-MS showed that the reaction was complete, saturated aqueous solution of ammonium chloride (100 mL) was added to quench the reaction, and extraction was performed with ethyl acetate (100 mL × 3). The combined organic layers were washed with saturated brine (100 mL), dried over anhydrous sodium sulfate, and concentrated under reduced pressure to obtain a crude product, which was then purified by silica gel column chromatography (petroleum ether containing 25% ethyl acetate as the eluent) to obtain compound **5a** (591 mg, 71% yield) as a gray solid.

MS (ES⁺): *m*/*z* 309.0 [M+H]⁺.

### Step 2: Synthesis of (S)-3-amino-8-hydroxy-5-methyl-2,3-dihydrobenzo[b][1,4]oxazepine-4(5H)-one (5b)

Trifluoroacetic acid (2 mL) was added dropwise to a solution of compound **5a** (339 mg, 1.1 mmol) in dichloromethane (5 mL) at room temperature, and the reaction solution was stirred at room temperature for 2 hours. TLC and LC-MS showed that the reaction was complete, and then it was neutralized with saturated aqueous solution of sodium bicarbonate to pH = 7, directly concentrated under reduced pressure, and then purified by silica gel column chromatography (dichloromethane containing 6% methanol as the eluent) to obtain compound **5b** (160 mg, 70% yield) as a light red solid.

MS (ES⁺): *m*/*z* 209.2 [M+H]⁺.

¹H NMR (400 MHz, DMSO-*d*₆): *δ* 9.68 (s, 1H), 7.18 (d, *J =* 8.8 Hz, 1H), 6.63 (dd, *J* = 8.4, 2.8 Hz, 1H), 6.52 (d, *J* = 2.4 Hz, 1H), 4.20 (dd, *J* = 10.0, 7.2 Hz, 1H), 3.91 (dd, *J* = 11.6, 10.0 Hz, 1H), 3.58 (dd, *J* = 11.6, 7.2 Hz, 1H), 3.21 (s, 3H), 1.75 (br, 2H).

### Step 3: Synthesis of ethyl (S)-2-((8-((tert-butyldimethylsilyl)oxy)-5-methyl-4-oxo-2,3,4,5-tetrahydrobenzo[b][1,4]oxazepin -3-yl)amino)-2-oxoacetate (5c)

To a mixture of a solution of compound **5b** (150 mg, 0.72 mmol), N,N-dimethylpyridin-4-amine (9 mg, 0.07 mmol) and triethylamine (219 mg, 2.16 mmol) in dichloromethane (3 mL), a solution of *tert*-butyldimethylchlorosilane (136 mg, 0.90 mmol) in dichloromethane (1 mL) was added dropwise at room temperature. The reaction solution was stirred at room temperature for 5 hours. TLC and LC-MS showed that the reaction was complete. The reaction solution was then cooled to 0 °C in an ice bath, and a solution of monoethyl oxalyl chloride (196 mg, 1.44 mmol) in dichloromethane (1 mL) was added dropwise. The reaction solution was slowly warmed to room temperature and stirred for another 3 hours. TLC and LC-MS showed that the reaction was complete. Dichloromethane (30 mL) was added to the reaction solution for dilution, which was then washed with water (15 mL), saturated aqueous solution of ammonium chloride (20 mL) and saturated brine (20 mL) in sequence. The organic layer was dried over anhydrous sodium sulfate, concentrated under reduced pressure, and then purified by silica gel column chromatography (petroleum ether containing 10 - 30% ethyl acetate as the eluent) to obtain compound **5c** (230 mg, 76% yield) as a white solid.

MS (ES⁺): *m*/*z* 423.1 [M+H]⁺.

### Step 4: Synthesis of (S)-N¹-(8-hydroxy-5-methyl-4-oxo-2,3,4,5-tetrahydrobenzo[b][1,4]oxazepin-3-yl)-N²-phenethylo xalamide (5)

To a solution of compound **5c** (200 mg, 0.47 mmol) in ethanol (5 mL), 2-phenylethylamine (190 mg, 1.56 mmol) was added, the reaction solution was heated to 80 °C and stirred for 15 hours. TLC and LC-MS showed that the reaction was complete, the reaction solution was concentrated under reduced pressure, and then purified by silica gel column chromatography (petroleum ether containing 10 - 50% ethyl acetate as the eluent) to obtain the title compound **5** (150 mg, 83% yield) as a white solid.

MS (ES⁺): *m*/*z* 384.0 [M+H]⁺.

¹H NMR (400 MHz, CDCl₃): *δ* 8.27 (d, *J* = 7.6 Hz, 1H), 7.32 - 7.28 (m, 3H), 7.24 - 7.21 (m, 1H), 7.18 (d, *J* = 7.6 Hz, 2H), 7.06 (d, *J* = 8.6 Hz, 1H), 6.71 - 6.66 (m, 2H), 4.83 (dt, *J* = 10.8, 7.4 Hz, 1H), 4.58 (dd, *J* = 9.7, 7.4 Hz, 1H), 4.20 (t, *J* = 10.5 Hz, 1H), 3.60 - 3.54 (m, 2H), 3.37 (s, 3H), 2.84 (t, *J* = 6.9 Hz, 2H).

### Example 6: Synthesis of (S)-N¹-(7-hydroxy-5-methyl-4-oxo-2,3,4,5-tetrahydrobenzo[b][1,4]oxazepin-3-yl)-N²-phenethylo xalamide (6)

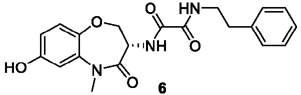

### Synthesis route:

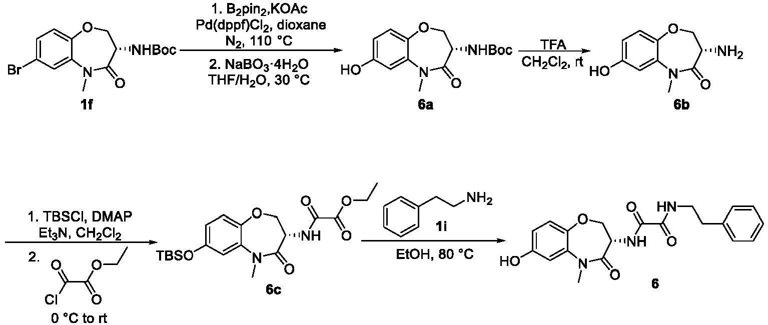

The synthesis route and method were the same as Example 5, except that compound **3e** was replaced with an equivalent amount of compound **1f** to obtain the title compound **6.**

MS (ES⁺): *m*/*z* 384.1 [M+H]⁺.

¹H NMR (400 MHz, CDCl₃): *δ* 8.28 (d, *J* = 7.5 Hz, 1H), 7.32 - 7.28 (m, 3H), 7.24 - 7.21 (m, 1H), 7.18 (d, *J* = 7.1 Hz, 2H), 7.01 (d, *J* = 8.6 Hz, 1H), 6.67 - 6.62 (m, 2H), 5.59 (br, 1H), 4.82 (dt, *J* = 11.1, 7.6 Hz, 1H), 4.56 (dd, *J* = 9.7, 7.6 Hz, 1H), 4.16 (t, *J* = 10.1 Hz, 1H), 3.60 - 3.54 (m, 2H), 3.38 (s, 3H), 2.84 (t, *J* = 7.1 Hz, 2H).

### Example 7: Synthesis of (S)-N¹-(7-hydroxy-5-methyl-4-oxo-2,3,4,5-tetrahydrobenzo[b][1,4]oxazepin-3-yl)-N²-methyl-N²-phenethyloxalamide (7)

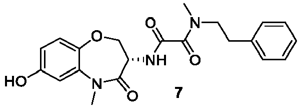

### Synthesis route:

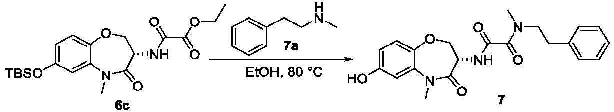

The synthesis route and method were the same as step 4 of Example 5, except that compound **1i** was replaced with an equivalent amount of compound **7a** to obtain the title compound **7.**

MS (ES⁺): *m*/*z* 398.1 [M+H]⁺.

¹H NMR (400 MHz, CDCl₃): *δ* 7.92 (minor), 7.80 (major) (d, *J* = 7.5 Hz, 1H), 7.32 - 7.27 (m, 1H), 7.24 - 7.17 (m, 2H), 7.13 (d, *J* = 7.1 Hz, 1H), 7.03 - 6.99 (m, 1H), 6.67 - 6.62 (m, 2H), 5.97 (s, 1H), 4.85 (minor), 4.74 (major) (dt, *J* = 11.0, 7.4 Hz, 1H), 4.59 (minor), 4.53 (major) (dd, *J* = 9.6, 7.7 Hz, 1H), 4.17 (minor), 4.06 (major) (t, *J* = 10.4 Hz, 1H), 3.96 - 3.82 (major), 3.63 - 3.59 (minor) (m, 2H), 3.37 (minor), 3.36 (major) (s, 3H), 3.16 (minor), 2.99 (major) (s, 3H), 2.91 - 2.86 (m, 2H).

### Example 8: Synthesis of (S)-N¹-(7-methoxy-5-methyl-4-oxo-2,3,4,5-tetrahydrobenzo[b][1,4]oxazepin-3-yl)-N²-phenethylo xalamide (8)

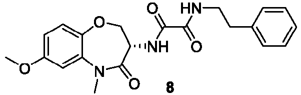

### Synthesis route:

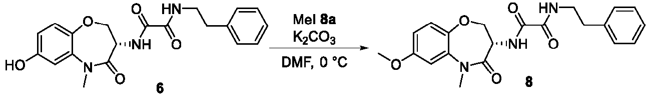

To a mixture of a solution of compound **6** (35 mg, 0.09 mmol) and potassium carbonate (25 mg, 0.18 mmol) in DMF (0.5 mL) was added a solution of iodomethane **8a** (17 mg, 0.12 mmol) in DMF (0.1 mL) at 0°C in an ice bath. The reaction solution was stirred for 1.5 hours at 0°C in an ice bath. TLC and LC-MS showed that the reaction was complete. The reaction solution was filtered, and the filtrate was purified by Prep-HPLC (water containing 30 - 95% acetonitrile as the mobile phase) to obtain compound 8 (27 mg, 75% yield) as a white solid.

MS (ES⁺): *m*/*z* 398.1 [M+H]⁺.

¹H NMR (400 MHz, CDCl₃): *δ*: 8.24 (d, *J* = 7.4 Hz, 1H), 7.31 - 7.28 (m, 3H), 7.24 - 7.20 (m, 1H), 7.17 (d, *J* = 7.2 Hz, 2H), 7.09 (d, *J* = 8.6 Hz, 1H), 6.76 - 6.72 (m, 2H), 4.82 (dt, *J* = 11.1, 7.6 Hz, 1H), 4.56 (dd, *J* = 9.6, 7.7 Hz, 1H), 4.16 (t, *J* = 10.4 Hz, 1H), 3.81 (s, 3H), 3.59 - 3.53 (m, 2H), 3.40 (s, 3H), 2.84 (t, *J =* 7.1 Hz, 2H).

### Example 9: Synthesis of (S)-N¹-(7-(allyloxy)-5-methyl-4-oxo-2,3,4,5-tetrahydrobenzo[b][1,4]oxazepin-3-yl)-N²-phenethyl oxalamide (9)

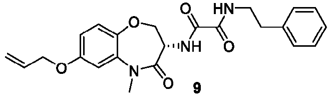

### Synthesis route:

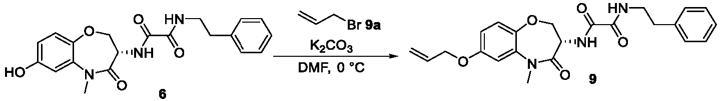

The synthesis route and method were the same as Example 8, except that iodomethane 8a was replaced with an equivalent amount of allyl bromide **9a** to obtain the title compound **9.**

MS (ES+): *m*/*z* 424.0 [M+H]⁺.

¹H NMR (400 MHz, CDCl₃): *δ*: 8.25 (d, *J* = 7.2 Hz, 1H), 7.31 - 7.28 (m, 3H), 7.25 - 7.22 (m, 1H), 7.17 (d, *J* = 7.1 Hz, 2H), 7.09 - 7.06 (m, 1H), 6.76 - 6.74 (m, 2H), 6.09 - 5.99 (m, 1H), 5.42 (d, *J* = 17.3 Hz, 1H), 5.32 (d, *J* = 10.5 Hz, 1H), 4.82 (dt, *J* = 11.1, 7.6 Hz, 1H), 4.58 - 4.52 (m, 3H), 4.16 (t, *J* = 10.4 Hz, 1H), 3.58 - 3.53 (m, 2H), 3.39 (s, 3H), 2.83 (t, *J* = 7.0 Hz, 2H).

### Example 10: Synthesis of (S)-N¹-(5-methyl-4-oxo-7-(prop-2-yn-1-yloxy)-2,3,4,5-tetrahydrobenzo[b][1,4]oxazepin-3-yl)-N² -phenethyloxalamide (10)

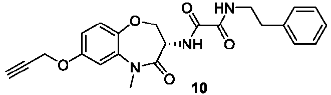

### Synthesis route:

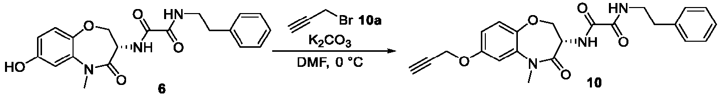

The synthesis route and method were the same as Example 8, except that iodomethane **8a** was replaced with an equivalent amount of propargyl bromide **10a** to obtain the title compound **10.**

MS (ES⁺): *m*/*z* 422.0 [M+H]⁺.

¹H NMR (400 MHz, CDCl₃): *δ*: 8.25 (d, *J* = 7.3 Hz, 1H), 7.33 - 7.28 (m, 3H), 7.25 - 7.22 (m, 1H), 7.17 (d, *J* = 7.2 Hz, 2H), 7.10 (d, *J* = 8.5 Hz, 1H), 6.85 - 6.82 (m, 2H), 4.83 (dt, *J* = 11.0, 7.6 Hz, 1H), 4.69 (d, *J* = 2.2 Hz, 2H), 4.56 (dd, *J* = 9.7, 7.6 Hz, 1H), 4.18 (t, *J* = 10.5 Hz, 1H), 3.58 - 3.53 (m, 2H), 3.40 (s, 3H), 2.83 (t, *J* = 7.0 Hz, 2H), 2.56 (t, *J* = 2.2 Hz, 1H).

### Example 11: Synthesis of (S)-N¹-(5-methyl-4-oxo-7-(prop-2-yn-1-oxy)-2,3,4,5-tetrahydrobenzo[b][1,4]oxazepin-3-yl)-N²-p henethyloxalamide (11)

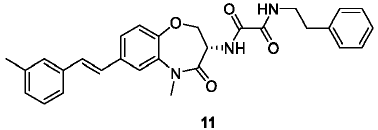

### Synthesis route:

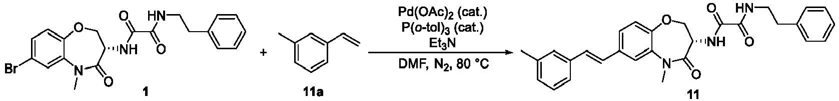

Compound **1** (44.6 mg, 0.1 mmol), palladium acetate (2.2 mg, 0.01 mmol), tris(o-methylphenyl)phosphine (6.1 mg, 0.02 mmol), triethylamine (28 mg, 0.28 mmol), DMF (1 mL) and 1-methyl-3-vinylbenzene **11a** (13 mg, 0.11 mmol) were added to a sealed tube in turn at room temperature. After the air in the tube was quickly replaced with nitrogen three times, the reaction solution was kept stirring at 80 °C for 15 hours. TLC and LC-MS showed that the reaction was complete. After the reaction solution was cooled to room temperature, it was directly concentrated under reduced pressure, first purified by preparative TLC, and then purified by Prep-HPLC (water containing 30 - 95% acetonitrile as the mobile phase) to obtain compound **11** (4 mg, 8% yield) as a white solid.

MS (ES⁻): *m*/*z* 482.1 [M-H]⁻.

¹H NMR (400 MHz, CDCl₃): *δ*: 8.26 (d, *J =* 7.6 Hz, 1H), 7.38 - 7.28 (m, 7H), 7.24 - 6.93 (m, 8H), 4.86 (dt, *J* = 11.4, 7.4 Hz, 1H), 4.60 (dd, *J* = 9.7, 7.4 Hz, 1H), 4.24 (t, *J* = 10.5 Hz, 1H), 3.59 - 3.51 (m, 2H), 3.46 (s, 3H), 2.84 (t, *J* = 7.0 Hz, 2H), 2.39 (s, 3H).

### Example 12: Synthesis of (S)-N¹-(5-methyl-4-oxo-7-(pyridin-4-ylethynyl)-2,3,4,5-tetrahydrobenzo[b][1,4]oxazepin-3-yl)-N ²-phenethyloxalamide (12)

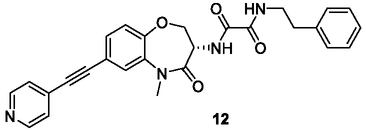

### Synthesis route:

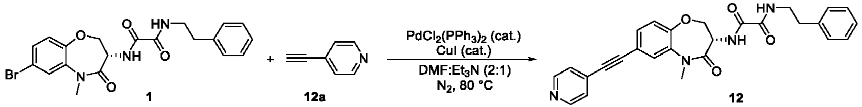

The synthesis route and method were the same as Example 2, except that cyclopropylacetylene **2a** was replaced with an equivalent amount of 4-ethynylpyridine **12a** to obtain the title compound **12.**

MS (ES⁺): *m*/*z* 469.1 [M+H]⁺.

¹H NMR (400 MHz, CDCl₃): *δ* 8.63 (s, 2H), 8.26 (d, *J* = 7.3 Hz, 1H), 7.43 - 7.28 (m, 7H), 7.24 - 7.15 (m, 4H), 4.84 (dt, *J =* 11.2, 7.3 Hz, 1H), 4.62 (dd, *J* = 9.5, 7.4 Hz, 1H), 4.28 (t, *J* = 10.5 Hz, 1H), 3.59 - 3.52 (m, 2H), 3.44 (s, 3H), 2.84 (t, *J* = 6.9 Hz, 2H).

### Example 13: Synthesis of (S)-N¹-(5-methyl-4-oxo-7-(pyridin-3-ylethynyl)-2,3,4,5-tetrahydrobenzo[b][1,4]oxazepin-3-yl)-N ²-phenethyloxalamide (13)

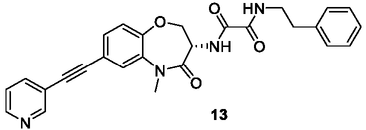

### Synthesis route:

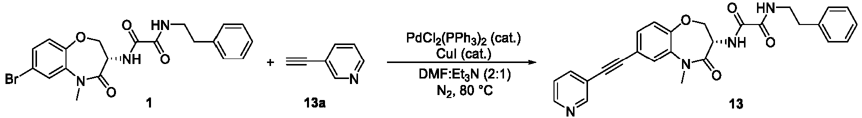

The synthesis route and method were the same as Example 2, except that cyclopropylacetylene **2a** was replaced with an equivalent amount of 3-ethynylpyridine **13a** to obtain the title compound **13.**

MS (ES⁺): *m*/*z* 469.1 [M+H]⁺.

¹H NMR (400 MHz, CDCl₃): *δ* 8.76 (s, 1H), 8.56 (s, 1H), 8.25 (d, *J* = 7.4 Hz, 1H), 7.80 (d, *J* = 7.0 Hz, 1H), 7.40 - 7.28 (m, 6H), 7.22 - 7.13 (m, 4H), 4.83 (dt, *J* = 11.2, 7.4 Hz, 1H), 4.59 (dd, *J* = 9.6, 7.4 Hz, 1H), 4.26 (t, *J* = 10.5 Hz, 1H), 3.57 - 3.52 (m, 2H), 3.42 (s, 3H), 2.82 (t, *J =* 6.9 Hz, 2H).

### Example 14: Synthesis of (S)-N¹-(5-methyl-4-oxo-7-(pyridin-2-ylethynyl)-2,3,4,5-tetrahydrobenzo[b][1,4]oxazepin-3-yl)-N ²-phenethyloxalamide (14)

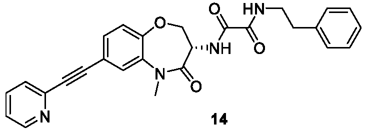

### Synthesis route:

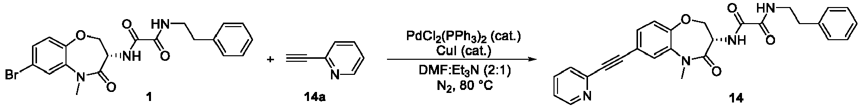

The synthesis route and method were the same as Example 2, except that cyclopropylacetylene **2a** was replaced with an equivalent amount of 2-ethynylpyridine **14a** to obtain the title compound **14.**

MS (ES⁺): *m*/*z* 469.1 [M+H]⁺.

¹H NMR (400 MHz, CDCl₃): *δ* 8.63 (t, *J* = 4.9 Hz, 1H), 8.26 (d, *J* = 7.3 Hz, 1H), 7.73 - 7.68 (m, 1H), 7.55 - 7.45 (m, 2H), 7.35 - 7.28 (m, 5H), 7.22 - 7.15 (m, 4H), 4.84 (dt, *J* = 11.2, 7.3 Hz, 1H), 4.62 (dd, *J* = 9.6, 7.4 Hz, 1H), 4.27 (t, *J* = 10.5 Hz, 1H), 3.59 - 3.52 (m, 2H), 3.43 (s, 3H), 2.83 (t, *J* = 7.0 Hz, 2H).

### Example 15: Synthesis of (S)-N¹-(5-methyl-4-oxo-7-(pyrimidin-5-ylethynyl)-2,3,4,5-tetrahydrobenzo[b][1,4]oxazepin-3-yl) -N²-phenethyloxalamide (15)

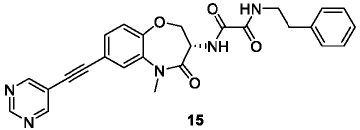

### Synthesis route:

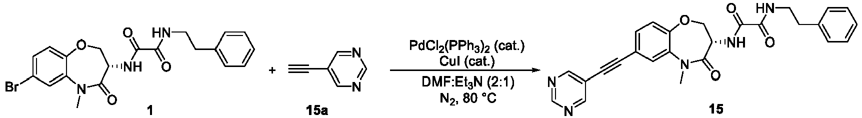

The synthesis route and method were the same as Example 2, except that cyclopropylacetylene **2a** was replaced with an equivalent amount of 5-ethynylpyrimidine **15a** to obtain the title compound **15.**

MS (ES⁺): *m*/*z* 470.1 [M+H]⁺.

¹H NMR (400 MHz, CDCl₃): *δ* 9.16 (d, *J* = 8.8 Hz, 1H), 8.86 (d, *J* = 9.1 Hz, 2H), 8.25 (d, *J =* 7.6 Hz, 1H), 7.41 (s, 1H), 7.32 - 7.28 (m, 3H), 7.23 - 7.15 (m, 5H), 4.85 (dt, *J* = 11.0, 7.4 Hz, 1H), 4.62 (dd, *J* = 9.7, 7.2 Hz, 1H), 4.28 (t, *J* = 10.5 Hz, 1H), 3.59 - 3.48 (m, 2H), 3.45 (s, 3H), 2.86 - 2.80 (m, 2H).

### Example 16: Synthesis of (S)-N¹-(5-methyl-4-oxo-7-(thiophen-2-ylethynyl)-2,3,4,5-tetrahydrobenzo[b][1,4]oxazepin-3-yl)-N²-phenethyloxalamide (16)

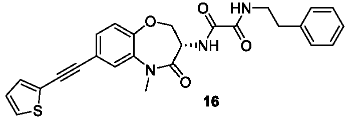

### Synthesis route:

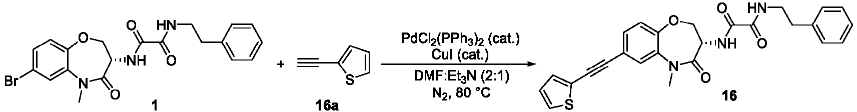

The synthesis route and method were the same as Example 2, except that cyclopropylacetylene **2a** was replaced with an equivalent amount of 2-ethynylthiophene **16a** to obtain the title compound **16.**

MS (ES⁺): *m*/*z* 474.0 [M+H]⁺.

¹H NMR (400 MHz, CDCl₃): *δ* 8.23 (d, *J* = 7.3 Hz, 1H), 7.76 - 7.71 (m, 1H), 7.37 - 7.28 (m, 6H), 7.24 - 7.21 (m, 2H), 7.18 (d, *J* = 7.1 Hz, 2H), 7.06 (d, *J* = 8.6 Hz, 1H), 4.81 (dt, *J* = 11.2, 7.4 Hz, 1H), 4.57 (dd, *J* = 9.7, 7.4 Hz, 1H), 4.23 (t, *J =* 10.6 Hz, 1H), 3.59 - 3.54 (m, 2H), 3.40 (s, 3H), 2.84 (t, *J* = 7.1 Hz, 2H).

### Example 17: Synthesis of (S)-N¹-(5-methyl-4-oxo-7-(thiophen-3-ylethynyl)-2,3,4,5-tetrahydrobenzo[b][1,4]oxazepin-3-yl)-N²-phenethyloxalamide (17)

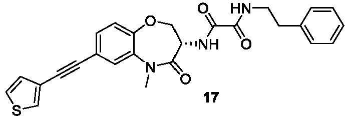

### Synthesis route:

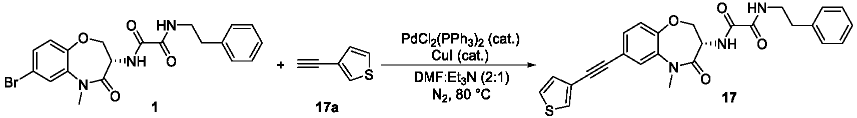

The synthesis route and method were the same as Example 2, except that cyclopropylacetylene **2a** was replaced with an equivalent amount of 3-ethynylthiophene **17a** to obtain the title compound **17.**

MS (ES⁺): *m*/*z* 474.0 [M+H]⁺.

¹H NMR (400 MHz, CDCl₃): *δ* 8.27 (d, *J* = 6.8 Hz, 1H), 7.55 (d, *J* = 2.2 Hz, 1H), 7.37 - 7.35 (m, 2H), 7.33 - 7.28 (m, 4H), 7.24 - 7.13 (m, 5H), 4.84 (dt, *J* = 11.2, 7.4 Hz, 1H), 4.61 (dd, *J =* 9.6, 7.4 Hz, 1H), 4.26 (t, *J =* 10.5 Hz, 1H), 3.59 - 3.54 (m, 2H), 3.43 (s, 3H), 2.84 (t, *J* = 7.1 Hz, 2H).

### Example 18: Synthesis of (S)-N¹-(5-methyl-4-oxo-7-((trimethylsilyl)ethynyl)-2,3,4,5-tetrahydrobenzo[b][1,4]oxazepin-3-yl )-N²-phenethyloxalamide (18)

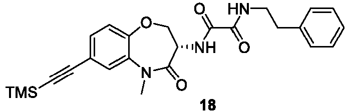

### Synthesis route:

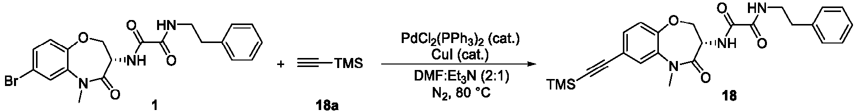

The synthesis route and method were the same as Example 2, except that cyclopropylacetylene **2a** was replaced with an equivalent amount of (trimethylsilyl)acetylene **18a** to obtain the title compound **18.**

MS (ES⁺): *m*/*z* 464.2 [M+H]⁺.

¹H NMR (400 MHz, CDCl₃): *δ* 8.24 (d, *J* = 7.5 Hz, 1H), 7.33 - 7.28 (m, 5H), 7.24 - 7.24 (m, 1H), 7.18 (d, *J* = 6.8 Hz, 2H), 7.10 (d, *J =* 8.8 Hz, 1H), 4.79 (dt, *J* = 11.3, 7.3 Hz, 1H), 4.58 (dd, *J* = 9.7, 7.3 Hz, 1H), 4.23 (t, *J* = 10.6 Hz, 1H), 3.59 - 3.52 (m, 2H), 3.41 (s, 3H), 2.84 (t, *J =* 7.0 Hz, 2H), 0.26 (s, 9H).

### Example 19: Synthesis of (S)-N¹-(5-methyl-4-oxo-7-(prop-1-yn-1-yl)-2,3,4,5-tetrahydrobenzo[b][1,4]oxazepin-3-yl)-N²-ph enethyloxalamide (19)

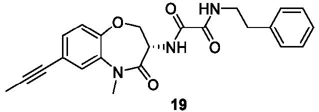

### Synthesis route:

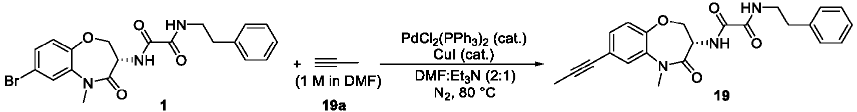

The synthesis route and method were the same as Example 2, except that cyclopropylacetylene **2a** was replaced with an equivalent amount of ethynyltrimethylsilane **19a** (1 M in DMF) to obtain the title compound **19.**

MS (ES⁺): *m*/*z* 406.1 [M+H]⁺.

¹H NMR (400 MHz, CDCl₃): *δ* 8.24 (d, *J* = 7.5 Hz, 1H), 7.34 - 7.28 (m, 4H), 7.24 - 7.20 (m, 2H), 7.18 (d, *J* = 7.3 Hz, 2H), 7.08 (d, *J =* 8.7 Hz, 1H), 4.81 (dt, *J* = 11.2, 7.4 Hz, 1H), 4.58 (dd, *J* = 9.6, 7.5 Hz, 1H), 4.22 (t, *J* = 10.5 Hz, 1H), 3.59 - 3.53 (m, 2H), 3.40 (s, 3H), 2.84 (t, *J =* 7.1 Hz, 2H), 2.05 (s, 3H).

### Example 20: Synthesis of (S)-N¹-(7-ethynyl-5-methyl-4-oxo-2,3,4,5-tetrahydrobenzo[b][1,4]oxazepin-3-yl)-N²-phenethylox alamide (20)

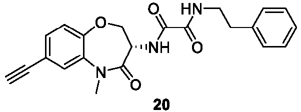

### Synthesis route:

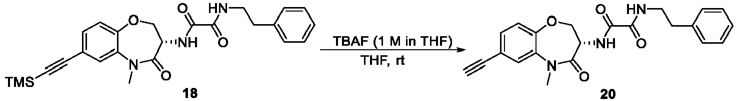

To a solution of compound **18** (18 mg, 0.04 mmol) in tetrahydrofuran (1.0 mL) at room temperature, a solution of tetrabutylammonium fluoride in tetrahydrofuran (1 M, 0.3 mL) was added, and the reaction solution was stirred at room temperature for 0.5 hours. TLC and LC-MS showed that the reaction was complete, and water (5 mL) was added to quench the reaction, and the mixture was extracted with ethyl acetate (5 mL × 3). The combined organic layers were washed with saturated brine (5 mL), dried over anhydrous sodium sulfate, and concentrated under reduced pressure to obtain a crude product, which was then purified by silica gel column chromatography (petroleum ether containing 25 - 40% ethyl acetate as the eluent) to obtain the title compound **20** (8 mg, 51% yield) as a white solid.

MS (ES⁺): *m*/*z* 392.0 [M+H]⁺.

¹H NMR (400 MHz, CDCl₃): *δ* 8.24 (d, *J* = 7.3 Hz, 1H), 7.36 - 7.28 (m, 5H), 7.24 - 7.20 (m, 1H), 7.18 (d, *J* = 7.2 Hz, 2H), 7.14 - 7.09 (m, 1H), 4.81 (dt, *J* = 11.3, 7.4 Hz, 1H), 4.60 (dd, *J* = 9.7, 7.4 Hz, 1H), 4.25 (t, *J* = 10.5 Hz, 1H), 3.59 - 3.53 (m, 2H), 3.41 (s, 3H), 3.11 (s, 1H), 2.84 (t, *J =* 7.0 Hz, 2H).

### Example 21: Synthesis of (S)-N¹-(5-methyl-4-oxo-7-((tetrahydro-2H-pyran-4-yl)ethynyl)-2,3,4,5-tetrahydrobenzo[b][1,4]ox azepin-3-yl)-N²-phenethyloxalamide (21)

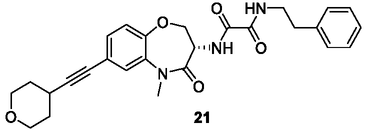

### Synthesis route:

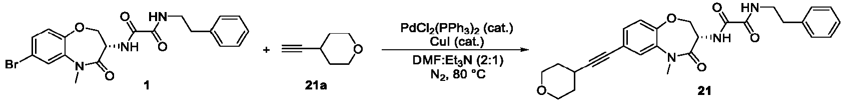

The synthesis route and method were the same as Example 2, except that cyclopropylacetylene **2a** was replaced with an equivalent amount of 4-ethynyl-2H-pyran 21a to obtain the title compound 21.

MS (ES⁺): *m*/*z* 476.1 [M+H]⁺.

¹H NMR (400 MHz, CDCl₃): *δ* 8.24 (d, *J* = 7.3 Hz, 1H), 7.32 - 7.28 (m, 4H), 7.25 - 7.21 (m, 2H), 7.18 (d, *J* = 7.2 Hz, 2H), 7.09 (d, *J* = 8.5 Hz, 1H), 4.80 (dt, *J* = 11.1, 7.4 Hz, 1H), 4.58 (dd, *J* = 9.6, 7.4 Hz, 1H), 4.23 (t, *J* = 10.5 Hz, 1H), 3.97 - 3.92 (m, 2H), 3.59 - 3.52 (m, 4H), 3.41 (s, 3H), 2.85 - 2.79 (m, 3H), 1.93 - 1.89 (m, 2H), 1.80 - 1.71 (m, 2H).

### Example 22: Synthesis of (S)-N¹-(5-methyl-7-((1-methyl-1H-pyrazol-4-yl)ethynyl)-4-oxo-2,3,4,5-tetrahydrobenzo[b][1,4]o xazepin-3-yl)-N²-phenethyloxalamide (22)

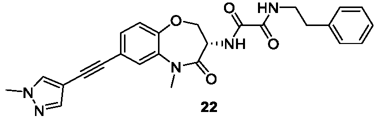

### Synthesis route:

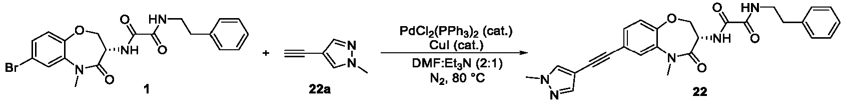

The synthesis route and method were the same as Example 2, except that cyclopropylacetylene **2a** was replaced with an equivalent amount of 4-ethynyl-1-methyl-1H-pyrazole **22a** to obtain the title compound **22.**

MS (ES⁺): *m*/*z* 472.2 [M+H]⁺.

¹H NMR (400 MHz, CDCl₃): *δ* 8.26 (d, *J* = 7.4 Hz, 1H), 7.65 (s, 1H), 7.56 (s, 1H), 7.34 - 7.28 (m, 5H), 7.24 - 7.20 (m, 1H), 7.17 (d, *J* = 7.2 Hz, 2H), 7.12 (d, *J* = 8.7 Hz, 1H), 4.83 (dt, *J* = 11.2, 7.4 Hz, 1H), 4.60 (dd, *J* = 9.6, 7.5 Hz, 1H), 4.24 (t, *J* = 10.5 Hz, 1H), 3.92 (s, 3H), 3.58 - 3.54 (m, 2H), 3.42 (s, 3H), 2.84 (t, *J =* 7.1 Hz, 2H).

### Example 23: Synthesis of (S)-N¹-(5-methyl-4-oxo-7-(piperidin-4-ylethynyl)-2,3,4,5-tetrahydrobenzo[b][1,4]oxazepin-3-yl)-N²-phenethyloxalamide (23)

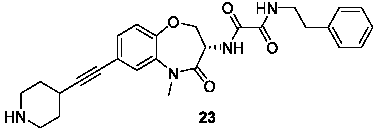

### Synthesis route:

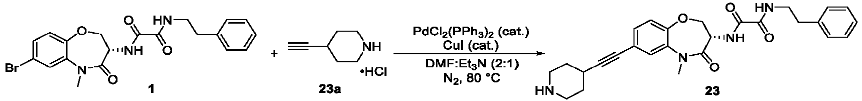

The synthesis route and method were the same as Example 2, except that cyclopropylacetylene **2a** was replaced with an equivalent amount of 4-ethynylpiperidine hydrochloride **23a** to obtain the title compound **23.**

MS (ES⁺): *m*/*z* 475.5 [M+H]⁺.

¹H NMR (400 MHz, DMSO-*d*₆): *δ* 8.81 (t, *J* = 5.9 Hz, 1H), 8.77 (d, *J* = 7.4 Hz, 1H), 7.54 (s, 1H), 7.33 - 7.27 (m, 3H), 7.22 - 7.18 (m, 4H), 4.68 - 4.58 (m, 2H), 4.40 - 4.32 (m, 1H), 3.40 - 3.35 (m, 2H), 3.30 (s, 3H), 3.22 - 3.19 (m, 2H), 2.98 - 2.93 (m, 3H), 2.78 (t, *J* = 7.4 Hz, 2H), 2.03 - 1.98 (m, 2H), 1.79 - 1.69 (m, 2H).

### Example 24: Synthesis of N¹-((3S)-5-methyl-4-oxo-7-(piperidin-3-ylethynyl)-2,3,4,5-tetrahydrobenzo[b][1,4]oxazepin-3-yl )-N²-phenethyloxalamide (24)

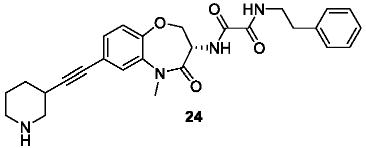

### Synthesis route:

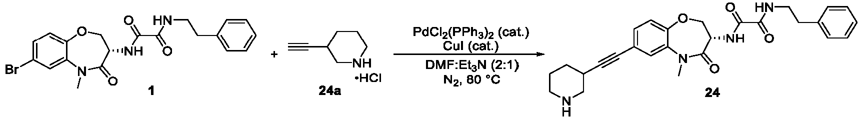

The synthesis route and method were the same as Example 2, except that cyclopropylacetylene **2a** was replaced with an equivalent amount of 3-ethynylpiperidine hydrochloride **24a** to obtain the title compound **24.**

MS (ES⁺): *m*/*z* 475.5 [M+H]⁺.

¹H NMR (400 MHz, DMSO-*d*₆): *δ* 8.82 (t, *J* = 6.1 Hz, 1H), 8.76 (d, *J* = 7.1 Hz, 1H), 7.52 (s, 1H), 7.30 - 7.27 (m, 3H), 7.21 - 7.18 (m, 4H), 4.68 - 4.57 (m, 2H), 4.38 - 4.34 (m, 1H), 3.40 - 3.36 (m, 2H), 3.30 (s, 3H), 3.14 - 3.09 (m, 1H), 2.90 - 2.85 (m, 1H), 2.78 (t, *J* = 7.4 Hz, 2H), 2.70 - 2.67 (m, 2H), 2.64 - 2.58 (m, 1H), 2.01 - 1.97 (m, 1H), 1.69 - 1.63 (m, 1H), 1.60 - 1.52 (m, 1H), 1.48 - 1.42 (m, 1H).

### Example 25: Synthesis of (S)-N¹-(7-(imidazo[1,2-b]pyridazin-3-ylethynyl)-5-methyl-4-oxo-2,3,4,5-tetrahydrobenzo[b][1,4] oxazepin-3-yl)-N²-phenethyloxalamide (25)

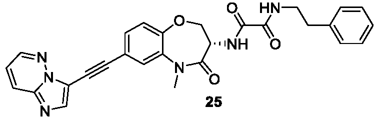

### Synthesis route:

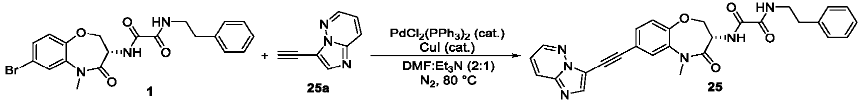

The synthesis route and method were the same as Example 2, except that cyclopropylacetylene **2a** was replaced with an equivalent amount of 3-ethynylimidazo[1,2-b]pyridazine **25a** to obtain the title compound **25.**

MS (ES⁺): *m*/*z* 509.1 [M+H]⁺.

¹H NMR (400 MHz, CDCl₃): *δ* 8.53 (d, *J* = 3.8 Hz, 1H), 8.26 (d, *J* = 7.2 Hz, 1H), 8.15 - 8.10 (m, 2H), 7.52 - 7.48 (m, 2H), 7.32 - 7.28 (m, 3H), 7.24 - 7.17 (m, 5H), 4.85 (dt, *J* = 11.2, 7.3 Hz, 1H), 4.63 (dd, *J* = 9.5, 7.5 Hz, 1H), 4.28 (t, *J* = 10.5 Hz, 1H), 3.59 - 3.54 (m, 2H), 3.45 (s, 3H), 2.84 (t, *J =* 7.0 Hz, 2H).

### Example 26: Synthesis of (S)-N¹-(7-(cyclohex-1-en-1-ylethynyl)-5-methyl-4-oxo-2,3,4,5-tetrahydrobenzo[b][1,4]oxazepin-3-yl)-N²-phenethyloxalamide (26)

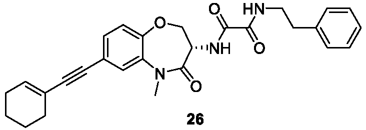

### Synthesis route:

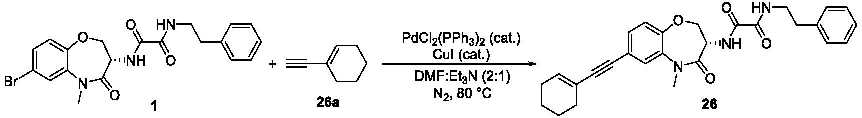

The synthesis route and method were the same as Example 2, except that cyclopropylacetylene **2a** was replaced with an equivalent amount of 1-ethynyl-1-cyclohexene **26a** to obtain the title compound **26.**

MS (ES⁺): *m*/*z* 472.1 [M+H]⁺.

¹H NMR (400 MHz, CDCl₃): *δ* 8.24 (d, *J* = 7.5 Hz, 1H), 7.32 - 7.27 (m, 5H), 7.24 - 7.21 (m, 1H), 7.18 (d, *J* = 7.2 Hz, 2H), 7.09 (d, *J* = 8.8 Hz, 1H), 6.25 - 6.22 (m, 1H), 4.82 (dt, *J* = 11.2, 7.4 Hz, 1H), 4.59 (dd, *J* = 9.9, 7.7 Hz, 1H), 4.23 (t, *J* = 10.5 Hz, 1H), 3.58 - 3.54 (m, 2H), 3.40 (s, 3H), 2.84 (t, *J* = 7.1 Hz, 2H), 2.24 - 2.13 (m, 4H), 1.71 - 1.61 (m, 4H).

### Example 27: Synthesis of (S)-N¹-(7-((3,5-difluorophenyl)ethynyl)-5-methyl-4-oxo-2,3,4,5-tetrahydrobenzo[b][1,4]oxazepin -3-yl)-N²-phenethyloxalamide (27)

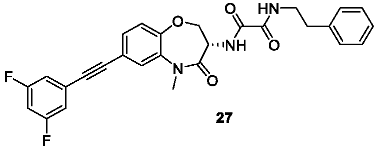

### Synthesis route:

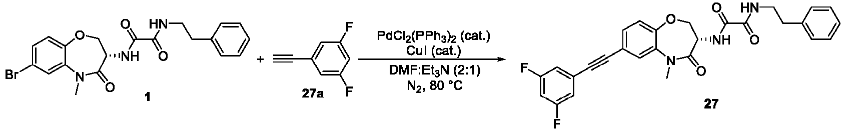

The synthesis route and method were the same as Example 2, except that cyclopropylacetylene **2a** was replaced with an equivalent amount of 1-ethynyl-3,5-difluorobenzene **27a** to obtain the title compound **27.**

MS (ES⁺): *m*/*z* 504.1 [M+H]⁺.

¹H NMR (400 MHz, CDCl₃): *δ* 8.25 (d, *J* = 7.4 Hz, 1H), 7.40 - 7.38 (m, 2H), 7.32 - 7.28 (m, 3H), 7.25 - 7.21 (m, 1H), 7.19 - 7.16 (m, 3H), 7.07 - 7.02 (m, 2H), 6.83 (tt, *J* = 8.9, 2.3 Hz, 1H), 4.84 (dt, *J =* 11.3, 7.3 Hz, 1H), 4.62 (dd, *J* = 9.7, 7.3 Hz, 1H), 4.27 (t, *J =* 10.5 Hz, 1H), 3.60 - 3.54 (m, 2H), 3.44 (s, 3H), 2.84 (t, *J =* 7.1 Hz, 2H).

### Example 28: Synthesis of (S)-N¹-(5-methyl-4-oxo-7-(3-phenyloxyprop-1-yn-1-yl)-2,3,4,5-tetrahydrobenzo[b][1,4]oxazepin -3-yl)-N²-phenethyloxalamide (28)

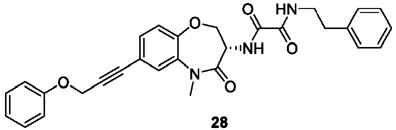

### Synthesis route:

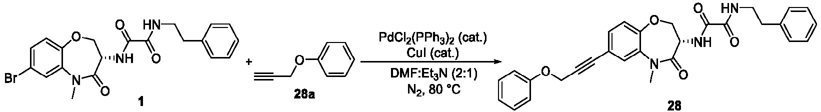

The synthesis route and method are the same as Example 2, except that cyclopropylacetylene **2a** was replaced with an equivalent amount of phenyl propargyl ether **28a** to obtain the title compound **28.**

MS (ES⁺): *m*/*z* 498.2 [M+H]⁺.

¹H NMR (400 MHz, CDCl₃): *δ* 8.23 (d, *J* = 7.0 Hz, 1H), 7.35 - 7.28 (m, 7H), 7.24 - 7.21 (m, 1H), 7.18 (d, *J =* 7.3 Hz, 2H), 7.11 (d, *J* = 7.9 Hz, 1H), 7.04 - 7.00 (m, 3H), 4.92 (s, 2H), 4.80 (dt, *J* = 11.4, 7.5 Hz, 1H), 4.58 (dd, *J* = 9.9, 7.3 Hz, 1H), 4.24 (t, *J* = 10.5 Hz, 1H), 3.59 - 3.54 (m, 2H), 3.39 (s, 3H), 2.84 (t, *J =* 7.1 Hz, 2H).

### Example 29: Synthesis of (S)-N¹-(7-(3-hydroxy-3-methylbut-1-yn-1-yl)-5-methyl-4-oxo-2,3,4,5-tetrahydrobenzo[b][1,4]ox azepin-3-yl)-N²-phenethyloxalamide (29)

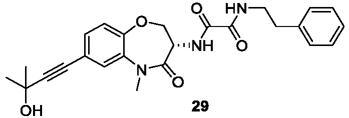

### Synthesis route:

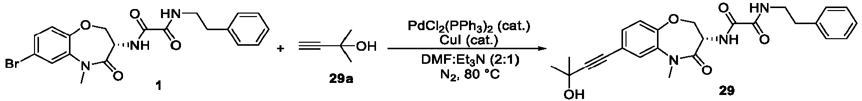

The synthesis route and method were the same as Example 2, except that cyclopropylacetylene **2a** was replaced with an equivalent amount of 2-methyl-3-butyn-2-ol **29a** to obtain the title compound **29.**

MS (ES⁺): *m*/*z* 450.1 [M+H]⁺.

¹H NMR (400 MHz, CDCl₃): *δ* 8.24 (d, *J* = 7.3 Hz, 1H), 7.32 - 7.27 (m, 5H), 7.24 - 7.21 (m, 1H), 7.18 (d, *J* = 7.0 Hz, 2H), 7.10 (d, *J* = 8.7 Hz, 1H), 4.80 (dt, *J* = 11.4, 7.3 Hz, 1H), 4.59 (dd, *J* = 9.7, 7.4 Hz, 1H), 4.23 (t, *J* = 10.5 Hz, 1H), 3.59 - 3.54 (m, 2H), 3.41 (s, 3H), 2.84 (t, *J =* 7.1 Hz, 2H), 1.86 (br, 1H), 1.62 (s, 6H).

### Example 30: Synthesis of N¹-((S)-7-((S)-3-hydroxybut-1-yn-1-yl)-5-methyl-4-oxo-2,3,4,5-tetrahydrobenzo[b][1,4]oxazepin -3-yl)-N²-phenethyloxalamide (30)

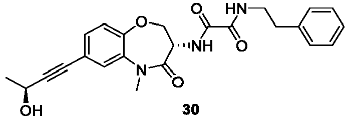

### Synthesis route:

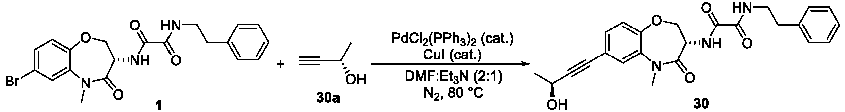

The synthesis route and method were the same as Example 2, except that cyclopropylacetylene **2a** was replaced with an equivalent amount of (S)-3-butyn-2-ol **30a** to obtain the title compound **30.**

MS (ES⁺): *m*/*z* 436.0 [M+H]⁺.

¹H NMR (400 MHz, CDCl₃): *δ* 8.24 (d, *J* = 7.3 Hz, 1H), 7.32 - 7.27 (m, 5H), 7.24 - 7.21 (m, 1H), 7.18 (d, *J* = 7.2 Hz, 2H), 7.11 (d, *J* = 8.8 Hz, 1H), 4.84 - 4.73 (m, 2H), 4.59 (dd, *J* = 9.7, 7.4 Hz, 1H), 4.24 (t, *J* = 10.5 Hz, 1H), 3.59 - 3.54 (m, 2H), 3.41 (s, 3H), 2.84 (t, *J =* 7.1 Hz, 2H), 1.90 (d, *J* = 5.4 Hz, 1H), 1.56 (d, *J* = 6.0 Hz, 3H).

### Example 31: Synthesis of (S)-N¹-(7-(4-hydroxybut-1-yn-1-yl)-5-methyl-4-oxo-2,3,4,5-tetrahydrobenzo[b][1,4]oxazepin-3-y l)-N²-phenethyloxalamide (31)

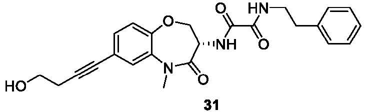

### Synthesis route:

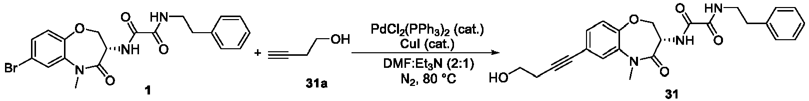

The synthesis route and method were the same as Example 2, except that cyclopropylacetylene **2a** was replaced with an equivalent amount of 3-butyn-1-ol **31a** to obtain the title compound **31.**

MS (ES⁺): *m*/*z* 436.0 [M+H]⁺.

¹H NMR (400 MHz, CDCl₃): *δ* 8.23 (d, *J =* 7.4 Hz, 1H), 7.32 - 7.27 (m, 5H), 7.24 - 7.21 (m, 1H), 7.18 (d, *J =* 7.3 Hz, 2H), 7.09 (d, *J =* 8.8 Hz, 1H), 4.81 (dt, *J =* 11.1, 7.3 Hz, 1H), 4.59 (dd, *J* = 9.8, 7.5 Hz, 1H), 4.23 (t, *J* = 10.5 Hz, 1H), 3.83 (t, *J =* 6.2 Hz, 2H), 3.59 - 3.54 (m, 2H), 3.40 (s, 3H), 2.84 (t, *J* = 7.1 Hz, 2H), 2.70 (t, *J* = 6.3 Hz, 2H).

### Example 32: Synthesis of (S)-N¹-benzyl-N²-(7-(4-hydroxybut-1-yn-1-yl)-5-methyl-4-oxo-2,3,4,5-tetrahydrobenzo[b][1,4]ox azepin-3-yl)oxalamide (32)

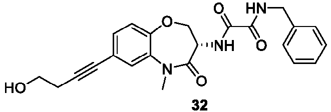

### Synthesis route:

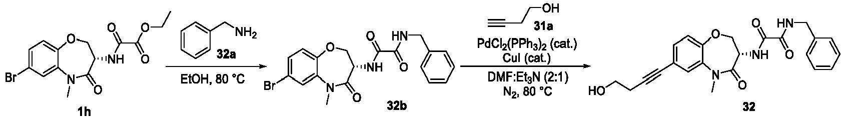

### Step 1: Synthesis of (S)-N¹-benzyl-N²-(7-bromo-5-methyl-4-oxo-2,3,4,5-tetrahydrobenzo[b][1,4]oxazepin-3-yl)oxala mide (32b)

To a solution of compound **1h** (111 mg, 0.3 mmol) in ethanol (5 mL) was added benzylamine **32a** (96 mg, 0.9 mmol), and the reaction solution was heated to 80 °C and stirred for 4 hours. TLC and LC-MS showed that the reaction was complete and a large amount of white solid precipitated in the reaction solution. The reaction solution was cooled to room temperature and filtered, and the filter cake was rinsed with petroleum ether (5 mL) containing 15% ethyl acetate. After the filter cake was dried, compound **32b** (118 mg, 91% yield) was obtained as a white solid.

MS (ES⁺): *m*/*z* 432.0 [M+H]⁺.

### Step 2: Synthesis of (S)-N¹-benzyl-N²-(7-(4-hydroxybut-1-yn-1-yl)-5-methyl-4-oxo-2,3,4,5-tetrahydrobenzo[b][1,4]ox azepin-3-yl)oxalamide (32)

Compound **32b** (43.2 mg, 0.1 mmol), bis(triphenylphosphine)palladium(II) dichloride (7.0 mg, 0.01 mmol), cuprous iodide (1.9 mg, 0.01 mmol), triethylamine (1 mL), DMF (2 mL) and 3-butyn-1-ol **31a** (14.0 mg, 0.2 mmol) were added to a sealed tube in turn at room temperature. After rapidly replacing the air in the tube with nitrogen three times, the reaction solution was stirred at 80 °C for 15 hours. TLC and LC-MS showed that the reaction was complete. After the reaction solution was cooled to room temperature, it was quenched with water (20 mL) and extracted with ethyl acetate (10 mL × 3). The combined organic layers were washed with saturated brine (10 mL), dried over anhydrous sodium sulfate, and concentrated under reduced pressure to obtain a crude product, which was then purified by silica gel column chromatography (petroleum ether containing 25 - 50% ethyl acetate as the eluent) to obtain the title compound **32** (35.8 mg, 85% yield) as a white solid.

MS (ES⁺): *m*/*z* 422.1 [M+H]⁺.

¹H NMR (400 MHz, CDCl₃): *δ* 8.29 (d, *J* = 7.4 Hz, 1H), 7.56 (t, *J* = 5.7 Hz, 1H), 7.36 - 7.29 (m, 3H), 7.28 - 7.25 (m, 4H), 7.10 (d, *J* = 8.7 Hz, 1H), 4.82 (dt, *J* = 11.2, 7.4 Hz, 1H), 4.60 (dd, *J* = 9.7, 7.4 Hz, 1H), 4.48 (d, *J =* 6.2 Hz, 2H), 4.24 (t, *J* = 10.5 Hz, 1H), 3.83 (t, *J =* 6.3 Hz, 2H), 3.41 (s, 3H), 2.70 (t, *J* = 6.3 Hz, 2H).

### Example 33: Synthesis of (S)-N¹-(2-fluorophenethyl)-N²-(7-(4-hydroxybut-1-yn-1-yl)-5-methyl-4-oxo-2,3,4,5-tetrahydrobe nzo[b][1,4]oxazepin-3-yl)oxalamide (33)

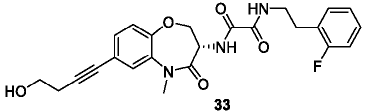

### Synthesis route:

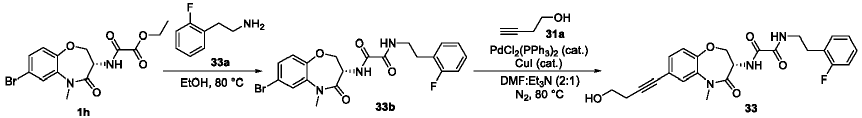

The synthesis route and method were the same as Example 32, except that the benzylamine **32a** in step 1 was replaced with an equivalent amount of 2-fluorophenethylamine **33a** to obtain the title compound **33.**

MS (ES⁺): *m*/*z* 453.9 [M+H]⁺.

¹H NMR (400 MHz, CDCl₃): *δ* 8.23 (d, *J* = 7.4 Hz, 1H), 7.34 (t, *J* = 6.0 Hz, 1H), 7.28 - 7.26 (m, 2H), 7.24 - 7.15 (m, 2H), 7.10 - 7.00 (m, 3H), 4.81 (dt, *J* = 11.4, 7.4 Hz, 1H), 4.59 (dd, *J* = 9.7, 7.4 Hz, 1H), 4.23 (t, *J* = 10.5 Hz, 1H), 3.83 (q, *J* = 6.1 Hz, 2H), 3.59 - 3.52 (m, 2H), 3.40 (s, 3H), 2.89 (t, *J* = 6.9 Hz, 2H), 2.70 (t, *J* = 6.2 Hz, 2H), 1.82 (t, *J* = 6.2 Hz, 1H)..

### Example 34: Synthesis of (S)-N¹-(3-fluorophenethyl)-N²-(7-(4-hydroxybut-1-yn-1-yl)-5-methyl-4-oxo-2,3,4,5-tetrahydrobe nzo[b][1,4]oxazepin-3-yl)oxalamide (34)

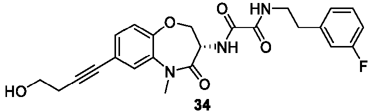

### Synthesis route:

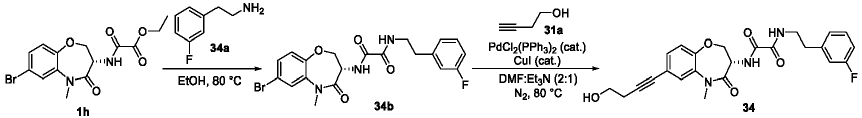

The synthesis route and method were the same as Example 32, except that the benzylamine **32a** in step 1 was replaced with an equivalent amount of 3-fluorophenethylamine **34a** to obtain the title compound **34.**

MS (ES⁺): *m*/*z* 454.2 [M+H]⁺.

¹H NMR (400 MHz, CDCl₃): *δ* 8.23 (d, *J =* 7.3 Hz, 1H), 7.31 - 7.23 (m, 4H), 7.10 (d, *J* = 8.8 Hz, 1H), 6.96 - 6.87 (m, 3H), 4.80 (dt, *J =* 11.1, 7.5 Hz, 1H), 4.59 (dd, *J* = 9.6, 7.4 Hz, 1H), 4.23 (t, *J =* 10.5 Hz, 1H), 3.83 (q, *J =* 6.2 Hz, 2H), 3.58 - 3.53 (m, 2H), 3.40 (s, 3H), 2.84 (t, *J* = 7.1 Hz, 2H), 2.70 (t, *J* = 6.2 Hz, 2H), 1.85 (t, *J* = 6.2 Hz, 1H).

### Example 35: Synthesis of (S)-N¹-(4-fluorophenethyl)-N²-(7-(4-hydroxybut-1-yn-1-yl)-5-methyl-4-oxo-2,3,4,5-tetrahydrobe nzo[b][1,4]oxazepin-3-yl)oxalamide (35)

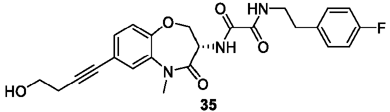

### Synthesis route:

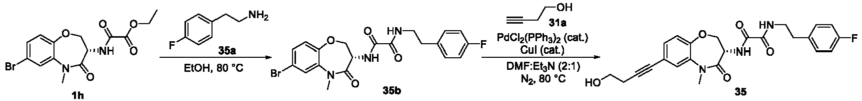

The synthesis route and method were the same as Example 32, except that the benzylamine **32a** in step 1 was replaced with an equivalent amount of 4-fluorophenethylamine **35a** to obtain the title compound **35.**

MS (ES⁺): *m*/*z* 454.0 [M+H]⁺.

¹H NMR (400 MHz, CDCl₃): *δ* 8.23 (d, *J* = 7.4 Hz, 1H), 7.30 - 7.26 (m, 3H), 7.15 - 7.08 (m, 3H), 6.98 (t, *J* = 8.6 Hz, 2H), 4.80 (dt, *J =* 11.2, 7.4 Hz, 1H), 4.58 (dd, *J* = 9.7, 7.4 Hz, 1H), 4.23 (t, *J* = 10.5 Hz, 1H), 3.83 (q, *J* = 6.0 Hz, 2H), 3.58 - 3.49 (m, 2H), 3.40 (s, 3H), 2.81 (t, *J* = 7.1 Hz, 2H), 2.70 (t, *J* = 6.2 Hz, 2H), 1.80 (t, *J =* 6.1 Hz, 1H).

### Example 36: Synthesis of (S)-N¹-(3,5-difluorophenethyl)-N²-(7-(4-hydroxybut-1-yn-1-yl)-5-methyl-4-oxo-2,3,4,5-tetrahydr obenzo[b][1,4]oxazepin-3-yl)oxalamide (36)

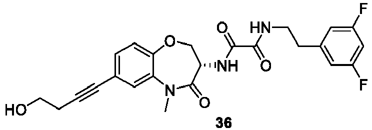

### Synthesis route:

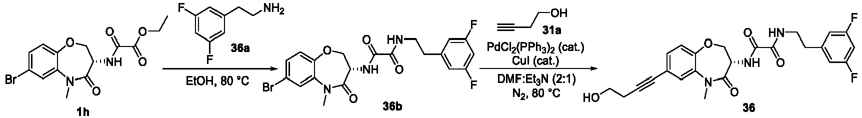

The synthesis route and method were the same as Example 32, except that the benzylamine **32a** in step 1 was replaced with an equivalent amount of 3,5-difluorophenethylamine **36a** to obtain the title compound **36.**

MS (ES⁺): *m*/*z* 472.0 [M+H]⁺.

¹H NMR (400 MHz, CDCl₃): *δ* 8.23 (d, *J =* 7.5 Hz, 1H), 7.33 - 7.27 (m, 3H), 7.10 (d, *J* = 8.7 Hz, 1H), 6.72 - 6.66 (m, 3H), 4.80 (dt, *J* = 11.1, 7.4 Hz, 1H), 4.59 (dd, *J* = 9.6, 7.5 Hz, 1H), 4.23 (t, *J =* 10.6 Hz, 1H), 3.83 (q, *J =* 6.2 Hz, 2H), 3.58 - 3.53 (m, 2H), 3.41 (s, 3H), 2.83 (t, *J* = 7.1 Hz, 2H), 2.70 (t, *J* = 6.1 Hz, 2H), 1.78 (t, *J* = 6.3 Hz, 1H).

### Example 37: Synthesis of N¹-((S)-7-(4-hydroxybut-1-yn-1-yl)-5-methyl-4-oxo-2,3,4,5-tetrahydrobenzo[b][1,4]oxazepin-3-y 1)-N²-(1-phenylpropan-2-yl)oxalamide (37)

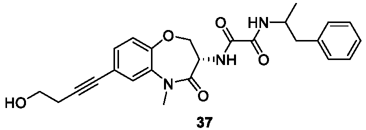

### Synthesis route:

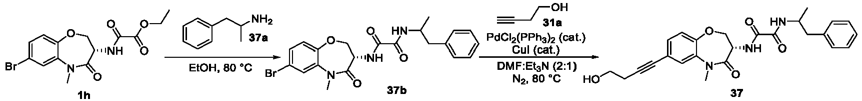

The synthesis route and method were the same as Example 32, except that the benzylamine **32a** in step 1 was replaced with an equivalent amount of 1-phenylpropan-2-amine **37a** to obtain the title compound **37.**

MS (ES⁺): *m*/*z* 450.2 [M+H]⁺.

¹H NMR (400 MHz, CDCl₃): *δ* 8.26 - 8.20 (m, 1H), 7.31 - 7.21 (m, 5H), 7.17 - 7.13 (m, 3H), 7.10 (d, *J* = 8.8 Hz, 1H), 4.80 (dt, *J* = 11.1, 7.5 Hz, 1H), 4.60 - 4.55 (m, 1H), 4.25 - 4.19 (m, 2H), 3.86 - 3.81 (m, 2H), 3.40 (s, 3H), 2.89 - 2.82 (m, 1H), 2.76 - 2.69 (m, 3H), 1.81 (t, *J =* 6.0 Hz, 1H), 1.17 - 1.14 (m, 3H).

### Example 38: Synthesis of (S)-N¹-(2-fluorobenzyl)-N²-(7-(4-hydroxybut-1-yn-1-yl)-5-methyl-4-oxo-2,3,4,5-tetrahydrobenzo [b][1,4]oxazepin-3-yl)oxalamide (38)

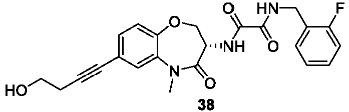

### Synthesis route:

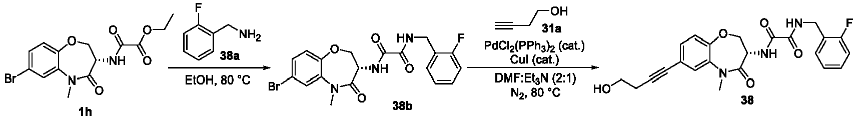

The synthesis route and method were the same as Example 32, except that the benzylamine **32a** in step 1 was replaced with an equivalent amount of 2-fluorobenzylamine **38a** to obtain the title compound **38.**

MS (ES⁺): *m*/*z* 440.0 [M+H]⁺.

¹H NMR (400 MHz, CDCl₃): *δ* 8.26 (d, *J* = 7.3 Hz, 1H), 7.58 (t, *J* = 6.0 Hz, 1H), 7.33 - 7.27 (m, 4H), 7.12 - 7.03 (m, 3H), 4.81 (dt, *J* = 11.2, 7.4 Hz, 1H), 4.59 (dd, *J* = 9.7, 7.4 Hz, 1H), 4.53 (dd, *J* = 6.1, 2.8 Hz, 2H), 4.23 (t, *J* = 10.5 Hz, 1H), 3.83 (q, *J* = 6.0 Hz, 2H), 3.40 (s, 3H), 2.70 (t, *J =* 6.2 Hz, 2H), 1.77 (t, *J* = 6.0 Hz, 1H)..

### Example 39: Synthesis of (S)-N¹-(3-fluorobenzyl)-N²-(7-(4-hydroxybut-1-yn-1-yl)-5-methyl-4-oxo-2,3,4,5-tetrahydrobenzo [b][1,4]oxazepin-3-yl)oxalamide (39)

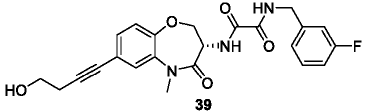

### Synthesis route:

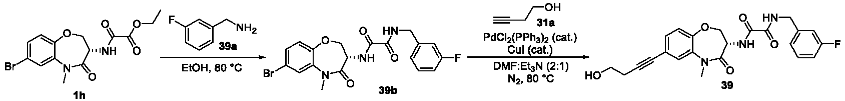

The synthesis route and method were the same as Example 32, except that the benzylamine **32a** in step 1 was replaced with an equivalent amount of 3-fluorobenzylamine **39a** to obtain the title compound **39.**

MS (ES⁺): *m*/*z* 440.0 [M+H]⁺.

¹H NMR (400 MHz, CDCl₃): *δ* 8.28 (d, *J* = 7.3 Hz, 1H), 7.58 (t, *J* = 6.3 Hz, 1H), 7.31 - 7.25 (m, 3H), 7.12 - 6.96 (m, 4H), 4.82 (dt, *J* = 11.3, 7.4 Hz, 1H), 4.61 (dd, *J* = 9.7, 7.3 Hz, 1H), 4.48 (dd, *J* = 6.2, 1.4 Hz, 2H), 4.23 (dd, *J =* 11.1, 9.9 Hz, 1H), 3.83 (q, *J =* 6.1 Hz, 2H), 3.41 (s, 3H), 2.70 (t, *J* = 6.3 Hz, 2H), 1.78 (t, *J* = 6.2 Hz, 1H).

### Example 40: Synthesis of (S)-N¹-(4-fluorobenzyl)-N²-(7-(4-hydroxybut-1-yn-1-yl)-5-methyl-4-oxo-2,3,4,5-tetrahydrobenzo [b][1,4]oxazepin-3-yl)oxalamide (40)

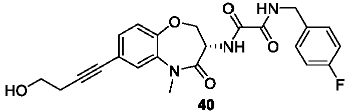

### Synthesis route:

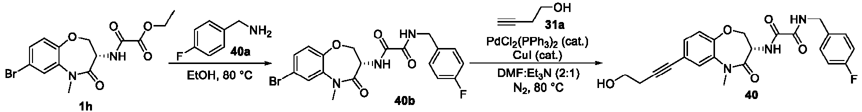

The synthesis route and method were the same as Example 32, except that the benzylamine **32a** in step 1 was replaced with an equivalent amount of 4-fluorobenzylamine **40a** to obtain the title compound **40.**

MS (ES⁺): *m*/*z* 440.0 [M+H]⁺.

¹H NMR (400 MHz, CDCl₃): *δ* 8.28 (d, *J* = 7.4 Hz, 1H), 7.55 (t, *J* = 5.8 Hz, 1H), 7.28 - 7.22 (m, 4H), 7.10 (d, *J* = 8.8 Hz, 1H), 7.01 (t, *J* = 8.6 Hz, 2H), 4.81 (dt, *J* = 11.3, 7.4 Hz, 1H), 4.59 (dd, *J* = 9.7, 7.4 Hz, 1H), 4.44 (d, *J* = 6.2 Hz, 2H), 4.24 (t, *J* = 10.5 Hz, 1H), 3.83 (q, *J =* 6.2 Hz, 2H), 3.41 (s, 3H), 2.70 (t, *J* = 6.2 Hz, 2H), 1.80 (t, *J* = 6.2 Hz, 1H).

### Example 41: Synthesis of N¹-((S)-7-(4-hydroxybut-1-yn-1-yl)-5-methyl-4-oxo-2,3,4,5-tetrahydrobenzo[b][1,4]oxazepin-3-y 1)-N²-(1-phenylethyl)oxalamide (41)

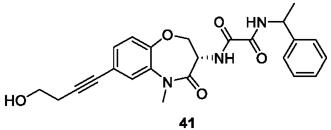

### Synthesis route:

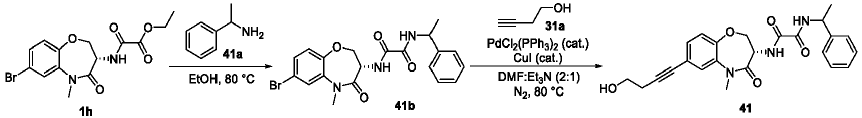

The synthesis route and method were the same as Example 32, except that the benzylamine **32a** in step 1 was replaced with an equivalent amount of 1-phenylethylamine **41a** to obtain the title compound **41** (*dr =* 1:1).

MS (ES⁺): *m*/*z* 436.0 [M+H]⁺.

¹H NMR (400 MHz, CDCl₃): *δ* 8.28 - 8.25 (m, 1H), 7.49 (d, *J* = 8.4 Hz, 1H), 7.36 - 7.26 (m, 7H), 7.12 - 7.09 (m, 1H), 5.10 - 5.03 (m, 1H), 4.84 - 4.76 (m, 1H), 4.62 - 4.54 (m, 1H), 4.27 - 4.18 (m, 1H), 3.83 (t, *J* = 5.9 Hz, 2H), 3.40, 3.39 (1:1, s, 3H), 2.70 (t, *J* = 6.2 Hz, 2H), 1.82 (br, 1H), 1.54, 1.53 (1:1, d, *J* = 6.6 Hz, 3H).

### Example 42: Synthesis of (S)-N¹-(3-fluorophenethyl)-N²-(7-(3-hydroxy-3-methylbut-1-yn-1-yl)-5-methyl-4-oxo-2,3,4,5-tetr ahydrobenzo[b][1,4]oxazepin-3-yl)oxalamide (42)

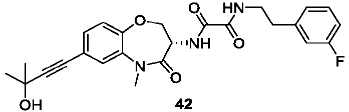

### Synthesis route:

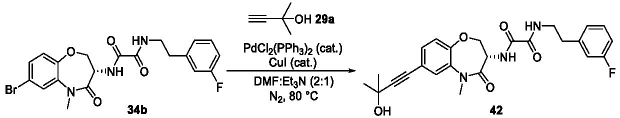

The synthesis route and method were the same as step 2 of Example 32, except that 3-butyn-1-ol **31a** was replaced with an equivalent amount of 2-methyl-3-butyn-2-ol **29a** to obtain the title compound **42.**

MS (ES⁺): *m*/*z* 468.0 [M+H]⁺.

¹H NMR (400 MHz, CDCl₃): *δ* 8.23 (d, *J =* 8.0 Hz, 1H), 7.31 - 7.23 (m, 4H), 7.11 (d, *J* = 8.8 Hz, 1H), 6.96 - 6.88 (m, 3H), 4.80 (dt, *J* = 11.4, 7.4 Hz, 1H), 4.59 (dd, *J* = 9.7, 7.4 Hz, 1H), 4.24 (t, *J* = 10.5 Hz, 1H), 3.58 - 3.53 (m, 2H), 3.41 (s, 3H), 2.84 (t, *J* = 7.0 Hz, 2H), 2.00 (s, 1H), 1.63 (s, 6H).

### Example 43: Synthesis of (S)-N¹-(7-((3-hydroxyoxetan-3-yl)ethynyl)-5-methyl-4-oxo-2,3,4,5-tetrahydrobenzo[b][1,4]oxaze pin-3-yl)-N²-phenethyloxalamide (43)

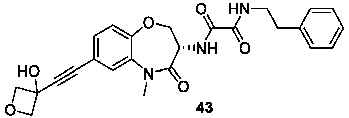

### Synthesis route:

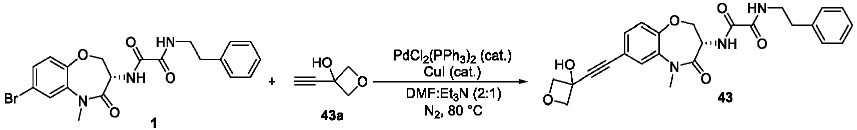

The synthesis route and method are the same as Example 2, except that cyclopropylacetylene **2a** was replaced with an equivalent amount of 3-ethynyloxetan-3-ol **43a** to obtain the title compound **43.**

MS (ES⁺): *m*/*z* 464.1 [M+H]⁺.

¹H NMR (400 MHz, CDCl₃): *δ* 8.24 (d, *J* = 7.0 Hz, 1H), 7.32 - 7.27 (m, 5H), 7.25 - 7.21 (m, 1H), 7.18 (d, *J =* 7.2 Hz, 2H), 7.14 (d, *J* = 8.8 Hz, 1H), 4.94 (d, *J =* 6.6 Hz, 2H), 4.85 - 4.78 (m, 3H), 4.60 (dd, *J* = 9.7, 7.2 Hz, 1H), 4.26 (t, *J* = 10.5 Hz, 1H), 3.60 - 3.54 (m, 2H), 3.42 (s, 3H), 2.84 (t, *J* = 7.0 Hz, 2H), 2.60 (br, 1H).

### Example 44: Synthesis of (S)-N¹-(7-((1-hydroxycyclohexyl)ethynyl)-5-methyl-4-oxo-2,3,4,5-tetrahydrobenzo[b][1,4]oxaze pin-3-yl)-N²-phenethyloxalamide (44)

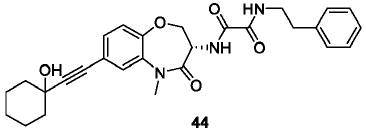

### Synthesis route:

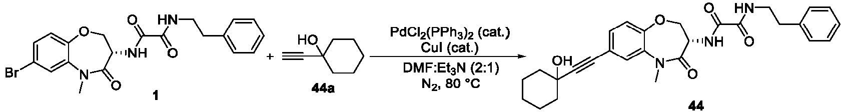

The synthesis route and method were the same as Example 2, except that cyclopropylacetylene **2a** was replaced with an equivalent amount of 1-ethynyl-1-cyclohexanol **44a** to obtain the title compound **44.**

MS (ES⁻): *m*/*z* 488.1 [M-H]⁻.

¹H NMR (400 MHz, CDCl₃): *δ* 8.23 (d, *J* = 7.4 Hz, 1H), 7.32 - 7.27 (m, 5H), 7.24 - 7.21 (m, 1H), 7.18 (d, *J* = 7.0 Hz, 2H), 7.11 (d, *J =* 8.6 Hz, 1H), 4.80 (dt, *J* = 11.3, 7.4 Hz, 1H), 4.58 (dd, *J* = 9.7, 7.3 Hz, 1H), 4.24 (t, *J* = 10.5 Hz, 1H), 3.59 - 3.54 (m, 2H), 3.41 (s, 3H), 2.84 (t, *J* = 7.1 Hz, 2H), 2.02 - 1.99 (m, 2H), 1.77 - 1.55 (m, 8H).

### Example 45: Synthesis of (S,Z)-N¹-(7-(5-hydroxy-3-methylpent-3-en-1-yn-1-yl)-5-methyl-4-oxo-2,3,4,5-tetrahydrobenzo[b ][1,4]oxazepin-3-yl)-N²-phenethyloxalamide (45)

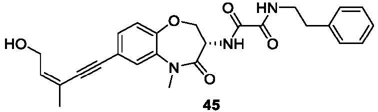

### Synthesis route:

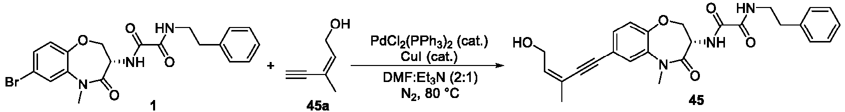

The synthesis route and method were the same as Example 2, except that cyclopropylacetylene **2a** was replaced with an equivalent amount of (Z)-3-methylpent-2-en-4-yn-1-ol **45a** to obtain the title compound **45.**

MS (ES⁻): *m*/*z* 460.1 [M-H]⁻.

¹H NMR (400 MHz, CDCl₃): *δ* 8.24 (d, *J* = 7.2 Hz, 1H), 7.32 - 7.28 (m, 5H), 7.24 - 7.21 (m, 1H), 7.18 (d, *J* = 7.0 Hz, 2H), 7.13 (d, *J* = 8.0 Hz, 1H), 5.97 (td, *J* = 6.8, 1.4 Hz, 1H), 4.82 (dt, *J =* 11.2, 7.4 Hz, 1H), 4.59 (dd, *J* = 9.7, 7.3 Hz, 1H), 4.41 (d, *J* = 6.2 Hz, 2H), 4.25 (t, *J* = 10.5 Hz, 1H), 3.59 - 3.52 (m, 2H), 3.42 (s, 3H), 2.84 (t, *J* = 7.0 Hz, 2H), 1.99 (s, 3H).

### Example 46: Synthesis of N¹-((3S)-7-(3-(3-hydroxypyrrolidin-1-yl)prop-1-yn-1-yl)-5-methyl-4-oxo-2,3,4,5-tetrahydrobenz o[b][1,4]oxazepin-3-yl)-N²-phenethyloxalamide (46)

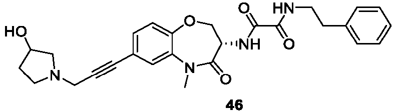

### Synthesis route:

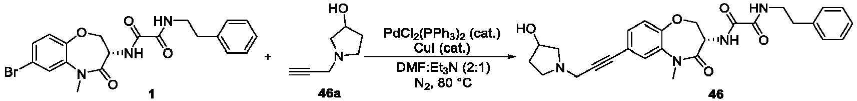

The synthesis route and method were the same as Example 2, except that cyclopropylacetylene **2a** was replaced with an equivalent amount of 1-(prop-2-yn-1-yl)pyrrolidin-3-ol **46a** to obtain the title compound **46.**

MS (ES⁺): *m*/*z* 491.3 [M+H]⁺.

¹H NMR (400 MHz, CDCl₃): *δ* 8.24 (d, *J* = 7.4 Hz, 1H), 7.36 - 7.28 (m, 5H), 7.24 - 7.20 (m, 1H), 7.17 (d, *J =* 7.1 Hz, 2H), 7.10 (d, *J* = 8.8 Hz, 1H), 4.80 (dt, *J =* 11.2, 7.4 Hz, 1H), 4.57 (dd, *J* = 9.6, 7.4 Hz, 1H), 4.47 - 4.45 (m, 1H), 4.24 (t, *J* = 10.5 Hz, 1H), 3.76 (s, 2H), 3.58 - 3.53 (m, 2H), 3.40 (s, 3H), 3.17 - 3.11 (m, 1H), 2.94 (s, 2H), 2.84 (t, *J =* 7.1 Hz, 2H), 2.78 - 2.72 (m, 1H), 2.32 - 2.24 (m, 1H), 1.93 - 1.86 (m, 1H).

### Example 47: Synthesis of (S)-N¹-(5-methyl-4-oxo-7-(3-(4-oxopiperidin-1-yl)prop-1-yn-1-yl)-2,3,4,5-tetrahydrobenzo[b][1, 4]oxazepin-3-yl)-N²-phenethyloxalamide (47)

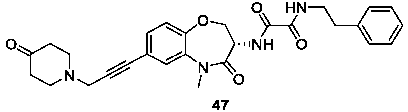

### Synthesis route:

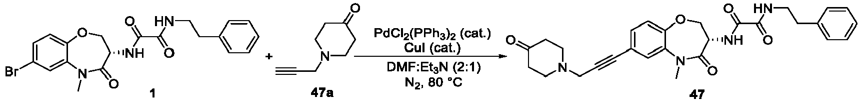

The synthesis route and method were the same as Example 2, except that cyclopropylacetylene **2a** was replaced with an equivalent amount of 1-(prop-2-yn-1-yl)piperidin-4-one **47a** to obtain the title compound **47.**

MS (ES⁺): *m*/*z* 503.1 [M+H]⁺.

¹H NMR (400 MHz, CDCl₃): *δ* 8.23 (d, *J* = 7.4 Hz, 1H), 7.32 - 7.28 (m, 5H), 7.24 - 7.21 (m, 1H), 7.17 (d, *J* = 7.1 Hz, 2H), 7.10 (d, *J =* 8.9 Hz, 1H), 4.81 (dt, *J* = 11.3, 7.4 Hz, 1H), 4.58 (dd, *J* = 9.7, 7.3 Hz, 1H), 4.24 (t, *J =* 10.5 Hz, 1H), 3.70 (s, 2H), 3.59 - 3.53 (m, 2H), 3.41 (s, 3H), 2.99 (t, *J* = 5.4 Hz, 4H), 2.84 (t, *J =* 7.1 Hz, 2H), 2.58 (t, *J* = 5.5 Hz, 4H).

### Example 48: Synthesis of (S)-N¹-(5-methyl-7-(3-morpholinoprop-1-yn-1-yl)-4-oxo-2,3,4,5-tetrahydrobenzo[b][1,4]oxazepi n-3-yl)-N²-phenethyloxalamide (48)

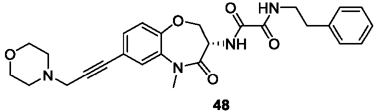

### Synthesis route:

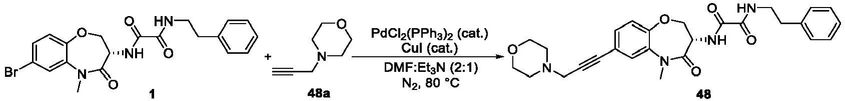

The synthesis route and method were the same as Example 2, except that cyclopropylacetylene **2a** was replaced with an equivalent amount of 4-(prop-2-yn-1-yl)morpholine **48a** to obtain the title compound **48.**

MS (ES⁺): *m*/*z* 491.0 [M+H]⁺.

¹H NMR (400 MHz, CDCl₃): *δ* 8.24 (d, *J =* 7.4 Hz, 1H), 7.32 - 7.28 (m, 5H), 7.24 - 7.20 (m, 1H), 7.17 (d, *J =* 7.1 Hz, 2H), 7.11 (d, *J =* 8.8 Hz, 1H), 4.81 (dt, *J =* 11.2, 7.4 Hz, 1H), 4.58 (dd, *J* = 9.7, 7.3 Hz, 1H), 4.24 (t, *J =* 10.5 Hz, 1H), 3.85 (t, *J =* 4.6 Hz, 4H), 3.62 (s, 2H), 3.59 - 3.53 (m, 2H), 3.41 (s, 3H), 2.84 (t, *J* = 7.1 Hz, 2H), 2.77 (t, *J* = 4.4 Hz, 4H).

### Example 49: Synthesis of (S)-N¹-(5-methyl-4-oxo-7-(3-(piperazin-1-yl)prop-1-yn-1-yl)-2,3,4,5-tetrahydrobenzo[b][1,4]oxa zepin-3-yl)-N²-phenethyloxalamide (49)

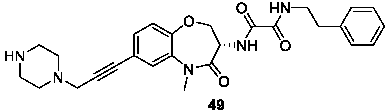

### Synthesis route:

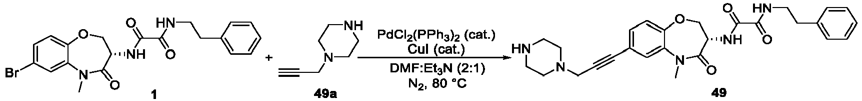

The synthesis route and method were the same as Example 2, except that cyclopropylacetylene **2a** was replaced with an equivalent amount of 1-(prop-2-yn-1-yl)piperazine **49a** to obtain the title compound **49.**

MS (ES⁺): *m*/*z* 490.1 [M+H]⁺.

¹H NMR (400 MHz, CDCl₃): *δ* 8.26 (d, *J* = 6.8 Hz, 1H), 7.39 - 7.35 (m, 2H), 7.31 - 7.27 (m, 3H), 7.23 - 7.19 (m, 1H), 7.17 (d, *J* = 7.2 Hz, 2H), 7.11 (d, *J =* 8.3 Hz, 1H), 4.79 (dt, *J* = 11.2, 6.8 Hz, 1H), 4.57 (dd, *J* = 9.6, 7.4 Hz, 1H), 4.24 (t, *J =* 10.5 Hz, 1H), 3.59 - 3.52 (m, 4H), 3.40 (s, 3H), 3.30 - 3.23 (m, 4H), 2.95 - 2.87 (m, 4H), 2.83 (t, *J =* 7.1 Hz, 2H).

### Example 50: Synthesis of (S)-N¹-(7-(4-aminobut-1-yn-1-yl)-5-methyl-4-oxo-2,3,4,5-tetrahydrobenzo[b][1,4]oxazepin-3-yl) -N²-phenethyloxalamide (50)

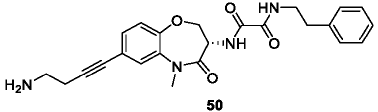

### Synthesis route:

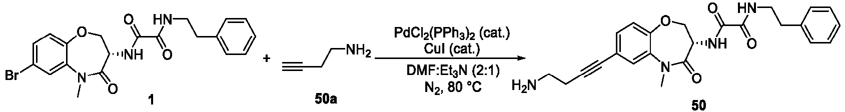

The synthesis route and method were the same as Example 2, except that cyclopropylacetylene **2a** was replaced with an equivalent amount of but-3-yn-1-amine **50a** to obtain the title compound **50.**

MS (ES⁺): *m*/*z* 435.0 [M+H]⁺.

¹H NMR (400 MHz, CDCl₃): *δ* 8.24 (d, *J* = 7.3 Hz, 1H), 7.32 - 7.28 (m, 5H), 7.24 - 7.21 (m, 1H), 7.18 (d, *J* = 7.0 Hz, 2H), 7.10 (d, *J* = 8.8 Hz, 1H), 4.81 (dt, *J* = 11.5, 7.4 Hz, 1H), 4.58 (dd, *J* = 9.8, 7.2 Hz, 1H), 4.24 (t, *J* = 10.5 Hz, 1H), 3.60 - 3.53 (m, 4H), 3.40 (s, 3H), 2.84 (t, *J* = 7.0 Hz, 2H), 2.67 (t, *J* = 6.6 Hz, 2H).

### Example 51: Synthesis of (S)-N¹-(5-methyl-7-(4-(methylsulfonamido)but-1-yn-1-yl)-4-oxo-2,3,4,5-tetrahydrobenzo[b][1,4] oxazepin-3-yl)-N²-phenethyloxalamide (51)

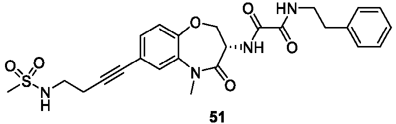

### Synthesis route:

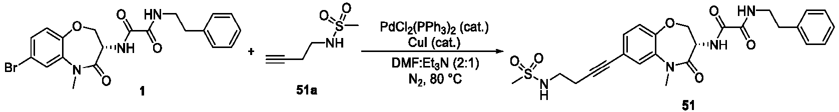

The synthesis route and method were the same as Example 2, except that cyclopropylacetylene **2a** was replaced with an equivalent amount of *N*-(but-3-yn-1-yl)methanesulfonamide **51a** to obtain the title compound **51.**

MS (ES⁺): *m*/*z* 513.0 [M+H]⁺.

¹H NMR (400 MHz, CDCl₃): *δ* 8.24 (d, *J* = 7.4 Hz, 1H), 7.32 - 7.27 (m, 5H), 7.24 - 7.21 (m, 1H), 7.18 (d, *J =* 7.2 Hz, 2H), 7.11 (d, *J =* 8.8 Hz, 1H), 4.81 (dt, *J =* 11.1, 7.4 Hz, 1H), 4.63 - 4.57 (m, 2H), 4.24 (t, *J =* 10.6 Hz, 1H), 3.59 - 3.51 (m, 2H), 3.41 - 3.35 (m, 5H), 3.02 (s, 3H), 2.84 (t, *J =* 7.0 Hz, 2H), 2.73 (t, *J =* 6.4 Hz, 2H).

### Example 52: Synthesis of (S)-N¹-(7-(3-methoxyprop-1-yn-1-yl)-5-methyl-4-oxo-2,3,4,5-tetrahydrobenzo[b][1,4]oxazepin-3 -yl)-N²-phenethyloxalamide (52)

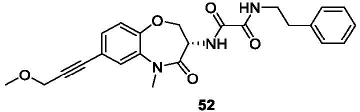

### Synthesis route:

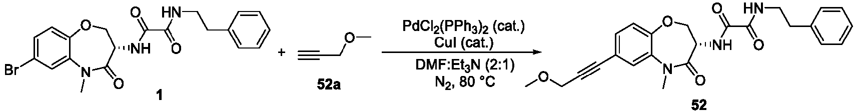

The synthesis route and method were the same as Example 2, except that cyclopropylacetylene **2a** was replaced with an equivalent amount of methyl propargyl ether **52a** to obtain the title compound **52.**

MS (ES⁺): *m*/*z* 436.1 [M+H]⁺.

¹H NMR (400 MHz, CDCl₃): *δ* 8.24 (d, *J* = 7.0 Hz, 1H), 7.32 - 7.28 (m, 5H), 7.24 - 7.20 (m, 1H), 7.18 (d, *J* = 7.2 Hz, 2H), 7.11 (d, *J* = 8.7 Hz, 1H), 4.81 (dt, *J* = 11.4, 7.1 Hz, 1H), 4.59 (dd, *J* = 9.6, 7.4 Hz, 1H), 4.32 (s, 2H), 4.24 (t, *J* = 10.5 Hz, 1H), 3.59 - 3.53 (m, 2H), 3.46 (s, 3H), 3.40 (s, 3H), 2.84 (t, *J* = 7.1 Hz, 2H).

### Example 53: Synthesis of tert-butyl (S)-2-(2-(2-(4-((5-methyl-4-oxo-3-(2-oxo-2-(phenethylamino)acetamido)-2,3,4,5-tetrahydrobenz o[b][1,4]oxazepin-7-yl)ethynyl)piperidin-1-yl)ethoxy)ethoxy)acetate (53)

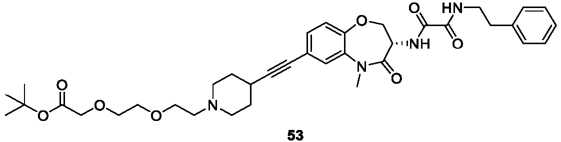

### Synthesis route:

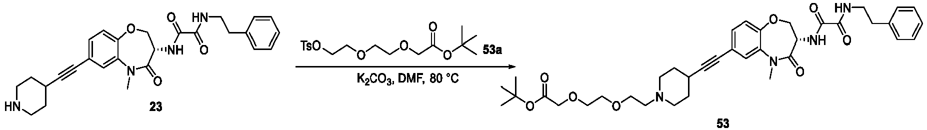

A mixture of compound **23** (60 mg, 0.13 mmol), potassium carbonate (54 mg, 0.39 mmol) and *tert*-butyl 2-(2-(2-(tosyloxy)ethoxy)ethoxy)acetate **53a** (57 mg, 0.15 mmol) was dissolved in DMF (1.0 mL) at room temperature. The reaction solution was heated to 80°C and stirred for 2 hours. TLC and LC-MS showed that the reaction was complete. The reaction mixture was poured into water (10 mL) and extracted with ethyl acetate (10 mL × 3). The combined organic layers were washed with saturated brine (10 mL), dried over anhydrous sodium sulfate, and concentrated under reduced pressure to obtain a crude product, which was then purified by preparative TLC (dichloromethane containing 5% methanol as the eluent) to obtain the title compound **53** (55 mg, 65% yield) as a colorless oil.

MS (ES⁺): *m*/*z* 677.1 [M+H]⁺.

### Example 54: Synthesis of (S)-1-(2-(2-(carboxymethoxy)ethoxy)ethyl)-4-((5-methyl-4-oxo-3-(2-oxo-2-(phenethylamino)ace tamido)-2,3,4,5-tetrahydrobenzo[b][1,4]oxazepin-7-yl)ethynyl)piperidin-1-ium 2,2,2-trifluoroacetate (54)

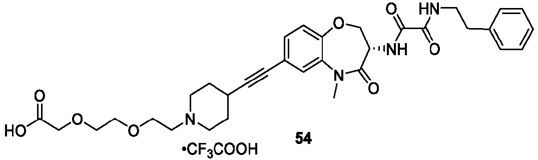

### Synthesis route:

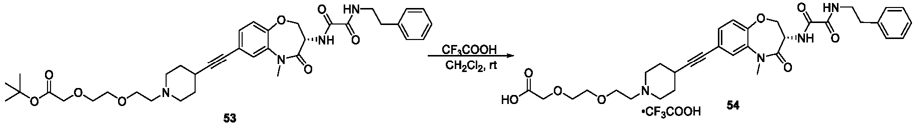

Trifluoroacetic acid (0.6 mL) was added to a solution of compound **53** (34 mg, 0.05 mmol) in dichloromethane (0.6 mL) at room temperature, and the reaction solution was stirred at room temperature for 2 hours. TLC and LC-MS showed that the reaction was complete. The reaction solution was concentrated under reduced pressure and dried in vacuo to obtain the title compound **54** (36 mg, 98% yield) as a light yellow oil.

MS (ES⁺): *m*/*z* 621.2 [M+H]⁺.

### Example 55: Synthesis of N¹-((S)-7-((1-(2-(2-(2-(((S)-1-((2S,4R)-4-hydroxy-2-((4-(4-methylthiazol-5-yl)benzyl)carbamoyl) pyrrolidin-1-yl)-3,3-dimethyl-1-oxobutan-2-yl)amino)-2-oxoethoxy)ethoxy)ethyl)piperidin-4-yl) ethynyl)-5-methyl-4-oxo-2,3,4,5-tetrahydrobenzo[b][1,4]oxazepin-3-yl)-N²-phenethyloxalamide (55)

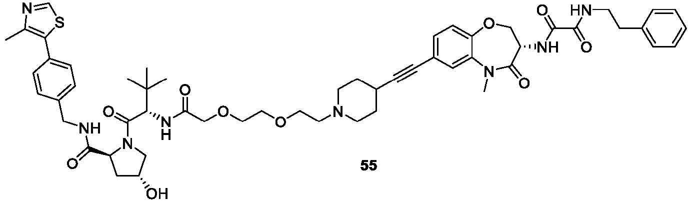

### Synthesis route:

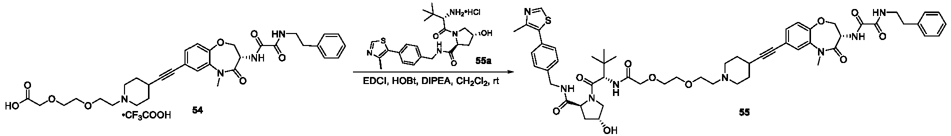

Compound **54** (35 mg, 0.05 mmol), (2*S*,4*R*)-1-((*S*)-2-amino-3,3-dimethylbutanoyl)-4-hydroxy-*N*-(4-(4-methylthiazol-5-yl)benzyl)pyr rolidine-2-carboxamide hydrochloride **55a** (25 mg, 0.05 mmol), 1-(3-dimethylaminopropyl)-3-ethylcarbodiimide hydrochloride (EDCI, 19 mg, 0.1 mmol), 1-hydroxybenzotriazole (HOBt, 14 mg, 0.1 mmol) and *N,N*-diisopropylethylamine (DIPEA, 39 mg, 0.3 mmol) were dissolved in dichloromethane (1.0 mL), and the reaction solution was stirred at room temperature for 15 h. TLC and LC-MS showed that the reaction was complete. The reaction solution was concentrated under reduced pressure and purified by Prep-HPLC (water containing 30 - 80% acetonitrile as the mobile phase) to obtain compound **55** (11 mg, 23% yield) as a white solid.

MS (ES⁺): *m*/*z* 1033.2 [M+H]⁺
¹H NMR (400 MHz, CDCl₃): *δ* 8.67 (s, 1H), 8.24 (d, *J* = 7.5 Hz, 1H), 7.80 (s, 1H), 7.39 - 7.28 (m, 9H), 7.24 - 7.21 (m, 2H), 7.17 (d, *J =* 6.9 Hz, 2H), 7.09 (d, *J* = 8.2 Hz, 1H), 4.83 - 4.77 (m, 2H), 4.63 (d, *J* = 8.9 Hz, 1H), 4.59 - 4.52 (m, 3H), 4.42 - 4.38 (m, 1H), 4.24 (t, *J* = 10.5 Hz, 1H), 4.13 - 4.09 (m, 1H), 3.97 (d, *J* = 11.0 Hz, 1H), 3.93 - 3.85 (m, 3H), 3.76 - 3.62 (m, 6H), 3.58 - 3.48 (m, 3H), 3.39 (s, 3H), 3.33 - 3.12 (m, 6H), 2.85 - 2.78 (m, 3H), 2.51 (s, 3H), 2.35 - 2.29 (m, 2H), 2.05 - 2.01 (m, 2H), 1.83 (br, 1H), 0.99 (s, 9H).

### Example 56: Synthesis of N¹-((3S)-7-((1-(2-(2-(2-(2-(2,6-dioxopiperidin-3-yl)-(1-oxoisoindolin-4-yl)amino)-2-oxoethoxy)e thoxy)ethyl)piperidin-4-yl)ethynyl)-5-methyl-4-oxo-2,3,4,5-tetrahydrobenzo[b][1,4]oxazepin-3-y 1)-N²-phenethyloxalamide (56)

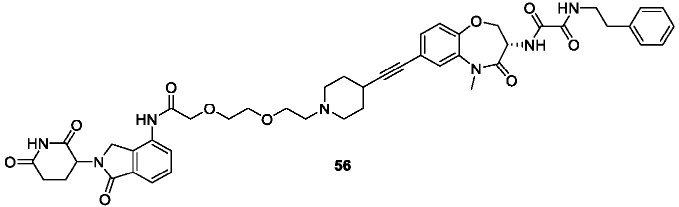

### Synthesis route:

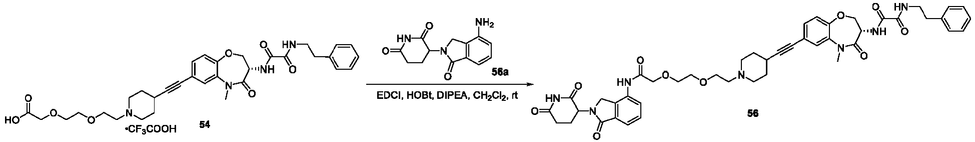

The synthesis route and method were the same as Example 55, except that (2*R*,4*S*)-1-((*R*)-2-amino-3,3-dimethylbutanoyl)-4-hydroxy-*N*-(4-(4-methylthiazol-5-yl)benzyl)pyr rolidine-2-carboxamide hydrochloride **55a** was replaced with an equivalent amount of 3-(4-amino-1-oxoisoindolin-2-yl)piperidine-2,6-dione **56a** (lenalidomide) to obtain the title compound **56.**

MS (ES⁺): *m*/*z* 862.2 [M+H]⁺
¹H NMR (400 MHz, DMSO-*d*₆): *δ* 11.01 (s, 1H), 9.68 (s, 1H), 8.81 (t, *J* = 5.9 Hz, 1H), 8.74 (d, *J =* 7.3 Hz, 1H), 7.74 (d, *J* = 7.7 Hz, 1H), 7.57 - 7.47 (m, 3H), 7.29 - 7.25 (m, 3H), 7.20 - 7.17 (m, 4H), 5.14 (dd, *J* = 13.2, 4.8 Hz, 1H), 4.67 - 4.55 (m, 2H), 4.44 - 4.32 (m, 3H), 4.14 (s, 2H), 3.70 - 3.68 (m, 2H), 3.61 - 3.59 (m, 2H), 3.54 (t, *J* = 5.4 Hz, 2H), 3.43 - 3.35 (m, 2H), 3.29 (s, 3H), 2.90 - 2.86 (m, 1H), 2.77 (t, *J* = 7.5 Hz, 2H), 2.73 - 2.67 (m, 2H), 2.61 - 2.57 (m, 2H), 2.39 - 2.33 (m, 2H), 2.18 - 2.09 (m, 2H), 2.03 - 1.97 (m, 2H), 1.81 - 1.75 (m, 2H), 1.56 - 1.48 (m, 2H).

### Example 57: Synthesis of (S)-N¹-(5-methyl-4-oxo-2,3,4,5-tetrahydrobenzo[b][1,4]oxazepin-3-yl)-N²-phenethyloxalamide (57)

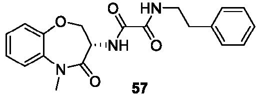

### Synthesis route:

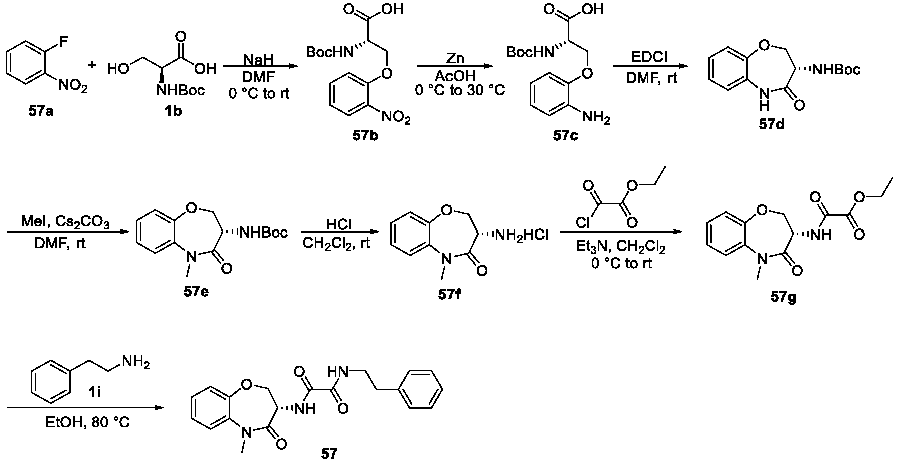

The synthesis route and method were the same as Example 1, except that 4-bromo-1-fluoro-2-nitrobenzene **1a** in step 1 was replaced with an equivalent amount of 1-fluoro-2-nitrobenzene **57a** to obtain the title compound **57.**

MS (ES⁺): *m*/*z* 368.1 [M+H]⁺.

¹H NMR (400 MHz, CDCl₃): *δ* 8.26 (d, *J* = 7.2 Hz, 1H), 7.32 - 7.28 (m, 3H), 7.25 - 7.21 (m, 4H), 7.19 - 7.17 (m, 3H), 4.83 (dt, *J* = 11.2, 7.5 Hz, 1H), 4.61 (dd, *J* = 9.6, 7.6 Hz, 1H), 4.23 (t, *J =* 10.4 Hz, 1H), 3.58 - 3.55 (m, 2H), 3.42 (s, 3H), 2.84 (t, *J* = 7.2 Hz, 2H).

### Example 58: Synthesis of (S)-N¹-benzyl-N²-(5-methyl-4-oxo-2,3,4,5-tetrahydrobenzo[b][1,4]oxazepin-3-yl)oxalamide (58)

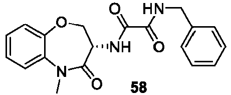

### Synthesis route:

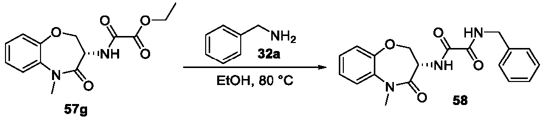

The synthesis route and method were the same as step 7 of Example 57, except that 2-phenylethylamine **1i** was replaced with an equivalent amount of benzylamine **32a** to obtain the title compound **58.**

MS (ES⁺): *m*/*z* 354.1 [M+H]⁺.

¹H NMR (400 MHz, CDCl₃): *δ* 8.31 (d, *J =* 7.6 Hz, 1H), 7.55 (t, *J* = 4.8 Hz, 1H), 7.35 - 7.27 (m, 4H), 7.24 - 7.17 (m, 5H), 4.84 (dt, *J =* 11.1, 7.6 Hz, 1H), 4.62 (dd, *J* = 9.8, 7.6 Hz, 1H), 4.48 (d, *J =* 6.0 Hz, 2H), 4.24 (t, *J* = 10.0 Hz, 1H), 3.43 (s, 3H).

### Example 59: Synthesis of (S)-N¹-(5-methyl-4-oxo-2,3,4,5-tetrahydrobenzo[b][1,4]oxazepin-3-yl)-N²-(3-phenylpropyl)oxala mide (59)

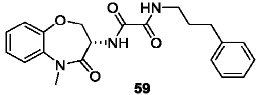

### Synthesis route:

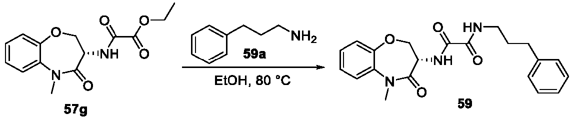

The synthesis route and method were the same as step 7 of Example 57, except that 2-phenylethylamine **1i** was replaced with an equivalent amount of 3-phenyl-1-propylamine **59a** to obtain the title compound **59.**

MS (ES⁺): *m*/*z* 382.1 [M+H]⁺.

¹H NMR (400 MHz, CDCl₃): *δ* 8.27 (d, *J* = 4.4 Hz, 1H), 7.30 - 7.28 (m, 2H), 7.25 - 7.15 (m, 8H), 4.83 (dt, *J* = 10.9, 6.8 Hz, 1H), 4.62 (dd, *J* = 9.8, 7.4 Hz, 1H), 4.24 (dd, *J* = 11.1, 9.8 Hz, 1H), 3.42 (s, 3H), 3.33 (q, *J* = 6.9 Hz, 2H), 2.65 (t, *J* = 7.6 Hz, 2H), 1.88 (qn, *J* = 7.4 Hz, 2H).

### Example 60: Synthesis of (S)-N¹-(5-methyl-4-oxo-2,3,4,5-tetrahydrobenzo[b][1,4]oxazepin-3-yl)-N²-phenyloxalamide (60)

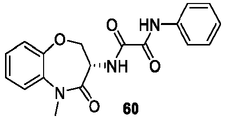

### Synthesis route:

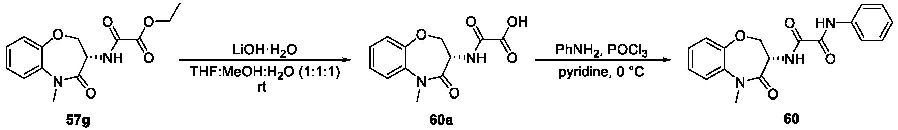

### Step 1: Synthesis of (S)-2-((5-methyl-4-oxo-2,3,4,5-tetrahydrobenzo[b][1,4]oxazepin-3-yl)amino)-2-oxoacetic acid (60a)

To a solution of compound **57g** (60 mg, 0.2 mmol) in tetrahydrofuran : methanol : water (1:1:1, 6 mL) was added lithium hydroxide monohydrate (26 mg, 0.6 mmol) at room temperature, and the reaction solution was stirred at room temperature for 1 hour. TLC and LC-MS showed that the reaction was complete. The reaction mixture was poured into water (10 mL), acidified with dilute hydrochloric acid (1 N) to pH = 6, and then extracted with ethyl acetate (20 mL × 5). The combined organic layers were washed with saturated brine (50 mL), dried over anhydrous sodium sulfate, and concentrated under reduced pressure to obtain a crude product of compound **60a** (46 mg), which was used directly in the next step without further purification.

MS (ES⁺): *m*/*z* 263.1 [M+H]⁺.

### Step 2: Synthesis of (S)-N¹-(5-methyl-4-oxo-2,3,4,5-tetrahydrobenzo[b][1,4]oxazepin-3-yl)-N²-phenyloxalamide (60)

Phosphorus oxychloride (72 mg, 0.47 mmol) was slowly added dropwise to a solution of compound **60a** (46 mg, 0.17 mmol) and aniline (20 mg, 0.2 mmol) in pyridine (0.5 mL) at 0 °C in an ice bath, and the resulting mixture was stirred at 0 °C for 0.5 hours. TLC and LC-MS showed that the reaction was complete, and the reaction mixture was poured into ice water (10 mL) and extracted with ethyl acetate (10 mL × 3). The combined organic layers were washed with saturated brine (10 mL), dried over anhydrous sodium sulfate and concentrated under reduced pressure, and then purified by Prep-HPLC (water containing 30 - 90% acetonitrile as the mobile phase) to obtain compound **60** (5 mg, 7% two-step yield) as a white solid.

MS (ES⁺): *m*/*z* 340.1 [M+H]⁺.

¹H NMR (400 MHz, CDCl₃): *δ* 9.01 (s, 1H), 8.42 (d, *J =* 7.1 Hz, 1H), 7.69 - 7.60 (m, 3H), 7.43 - 7.35 (m, 3H), 7.24 - 7.16 (m, 3H), 4.88 (dt, *J =* 11.1, 7.4 Hz, 1H), 4.68 (dd, *J* = 9.7, 7.5 Hz, 1H), 4.27 (dd, *J* = 11.0, 9.9 Hz, 1H), 3.44 (s, 3H).

### Example 61: Synthesis of N¹-(8-bromo-1-methyl-2-oxo-2,3,4,5-tetrahydro-1H-benzo[b]azepin-3-yl)-N²-phenethyloxalamid e (61)

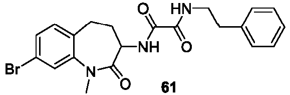

### Synthesis route:

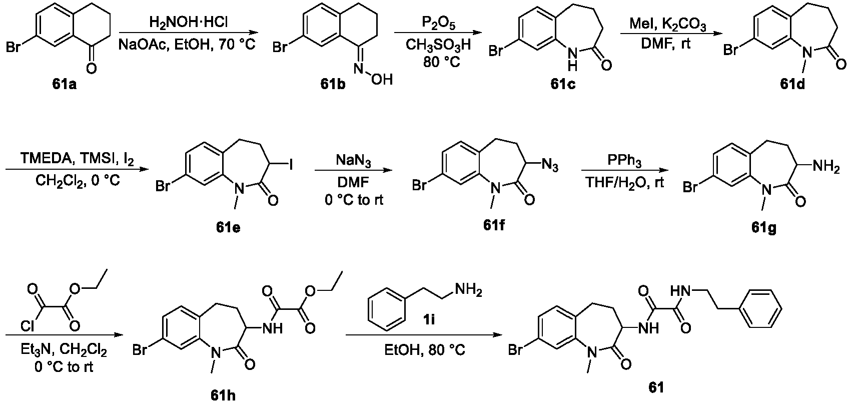

### Step 1: Synthesis of (E)-7-bromo-3,4-dihydronaphthalen-1(2H)-one oxime (61b)

Hydroxyamine hydrochloride (3.1 g, 44 mmol) and sodium acetate (3.6 g, 44 mmol) were added to a solution of 7-bromo-3,4-dihydronaphthalen-1(2*H*)-one **61a** (5.0 g, 22 mmol) in ethanol (50 mL), and the reaction solution was stirred at 70 °C for 2 hours. TLC and LC-MS showed that the reaction was complete. After the reaction solution was cooled to room temperature, it was concentrated under reduced pressure to remove most of the solvent, and the residue was poured into water (50 mL) and extracted with ethyl acetate (50 mL × 3). The combined organic layers were dried over anhydrous sodium sulfate and concentrated under reduced pressure to obtain a crude product of compound **61b** (5.3 g) as a yellow solid, which was used directly in the next step without further purification.

MS (ES⁺): *m*/*z* 283.0 [M+CH₃CN+H]⁺.

¹H NMR (400 MHz, CDCl₃): *δ* 8.24 (br, 1H), 8.03 (d, *J =* 2.0 Hz, 1H), 7.37 (dd, *J* = 8.2, 2.4 Hz, 1H), 7.03 (d, *J =* 8.0 Hz, 1H), 2.80 (t, *J =* 6.8 Hz, 2H), 2.71 (t, *J =* 6.4 Hz, 2H), 1.86 (qn, *J =* 6.4 Hz, 2H).

### Step 2: Synthesis of 8-bromo-1,3,4,5-tetrahydro-2H-benzo[b]azepin-2-one (61c)

A mixture of phosphorus pentoxide (5.4 g, 38 mmol) and methanesulfonic acid (54 mL) was stirred at 90 °C for 1 hour, then cooled to 50 °C and compound **61b** was added in five batches. The resulting mixture was stirred at 80 °C for 16 hours. TLC and LC-MS showed that the reaction was complete. The reaction mixture was slowly poured into ice water (100 mL) and extracted with ethyl acetate (100 mL × 3). The combined organic layers were dried over anhydrous sodium sulfate and concentrated under reduced pressure, and then purified by silica gel column chromatography (petroleum ether containing 30% ethyl acetate as the eluent) to obtain compound **61c** (3.8 g, 70% yield) as a light yellow solid.

MS (ES⁺): *m*/*z* 282.9 [M+CH₃CN+H]⁺.

¹H NMR (400 MHz, DMSO-*d*₆): *δ* 9.61 (s, 1H), 7.27 - 7.24 (m, 1H), 7.22 - 7.19 (m, 1H), 7.13 (d, *J* = 2.0 Hz, 1H), 2.65 (t, *J =* 7.2 Hz, 2H), 2.16 - 2.06 (m, 4H).

### Step 3: Synthesis of 8-bromo-1-methyl-1,3,4,5-tetrahydro-2H-benzo[b]azepin-2-one (61d)

To a mixture of compound **61c** (3.8 g, 16 mmol) and potassium carbonate (6.6 g, 47 mmol) in DMF (30 mL) was added iodomethane (4.5 g, 32 mmol) at room temperature, and the reaction mixture was stirred at room temperature for 15 hours. TLC and LC-MS showed that the reaction was complete, and the reaction mixture was poured into water (200 mL) and extracted with ethyl acetate (100 mL × 3). The combined organic layers were washed with saturated brine (100 mL), dried over anhydrous sodium sulfate and concentrated under reduced pressure, and then purified by silica gel column chromatography (petroleum ether containing 20% ethyl acetate as the eluent) to obtain compound **61d** (3.4 g, 84% yield) as a white solid.

MS (ES⁺): *m*/*z* 255.9 [M+H]⁺.

¹H NMR (400 MHz, CDCl₃): *δ* 7.31 (d, *J* = 2.0 Hz, 1H), 7.28 (dd, *J* = 8.0, 2.0 Hz, 1H), 7.06 (d, *J =* 8.0 Hz, 1H), 3.33 (s, 3H), 2.67 (t, *J =* 6.8 Hz, 2H), 2.30 (t, *J =* 6.8 Hz, 2H), 2.17 - 2.14 (m, 2H).

### Step 4: Synthesis of 8-bromo-3-iodo-1-methyl-1,3,4,5-tetrahydro-2H-benzo[b]azepin-2-one (61e)

To a solution of compound **61d** (1.5 g, 5.9 mmol) and *N*,*N*,*N*,*N*-tetramethylethylenediamine (2.1 g, 17.8 mmol) in dichloromethane (15 mL) was added iodotrimethylsilane (3.6 g, 17.8 mmol) dropwise at 0°C in an ice bath. After the reaction mixture was stirred at 0°C for 1 hour, iodine (2.3 g, 8.9 mmol) was added to the reaction solution, and then the reaction solution was stirred at 0°C for another 2 hours. TLC and LC-MS showed that the reaction was complete, and the reaction was quenched with 5% sodium thiosulfate aqueous solution (40 mL), and the aqueous phase was extracted with dichloromethane (40 mL × 3). The combined organic layers were dried over anhydrous sodium sulfate and concentrated under reduced pressure, and then purified by silica gel column chromatography (petroleum ether containing 5% ethyl acetate as the eluent) to obtain compound **61e** (1.5 g, 66% yield) as a light yellow solid.

MS (ES⁺): *m*/*z* 381.7 [M+H]⁺.

¹H NMR (400 MHz, DMSO-*d*₆): *δ* 7.61 (d, *J =* 2.0 Hz, 1H), 7.41 (dd, *J* = 8.0, 2.0 Hz, 1H), 7.24 (d, *J* = 8.0 Hz, 1H), 4.61 (t, *J* = 8.4 Hz, 1H), 3.28 (s, 3H), 2.72 - 2.54 (m, 4H)

### Step 5: Synthesis of 3-azido-8-bromo-1-methyl-1,3,4,5-tetrahydro-2H-benzo[b]azepin-2-one (61f)

To a solution of compound **61e** (1.5 g, 3.9 mmol) in DMF (15 mL) was added sodium azide (309 mg, 4.7 mmol) at 0 °C in an ice bath. The reaction mixture was warmed to room temperature and stirred for 3 hours. TLC and LC-MS showed that the reaction was complete. The reaction was quenched with ice water (20 mL) and filtered. The filter cake was rinsed with ice water (5 mL) and dried to obtain compound **61f** (1.1 g, 96% yield) as a white solid, which was used directly in the next step without further purification.

MS (ES⁺): *m*/*z* 296.8 [M+H]⁺.

### Step 6: Synthesis of 3-amino-8-bromo-1-methyl-1,3,4,5-tetrahydro-2H-benzo[b]azepin-2-one (61g)

Water (132 mg, 7.3 mmol) was added to a solution of compound **61e** (1.1 g, 3.7 mmol) and triphenylphosphine (1.1 g, 4.0 mmol) in tetrahydrofuran (20 mL) at room temperature, and the reaction mixture was stirred at room temperature for 15 hours. TLC and LC-MS showed that the reaction was complete. The reaction solution was directly concentrated under reduced pressure and purified by silica gel column chromatography (dichloromethane containing 1-5% methanol as the eluent) to obtain compound **61g** (790 mg, 79% yield) as a white solid.

MS (ES⁺): *m*/*z* 270.9 [M+H]⁺.

¹H NMR (400 MHz, CDCl₃): *δ* 7.30-7.27 (m, 2H), 7.08 (d, *J* = 7.6 Hz, 1H), 3.37 (s, 3H), 3.35-3.33 (m, 1H), 2.77-2.68 (m, 1H), 2.59-2.53 (m, 1H), 2.42-2.32 (m, 1H), 1.91-1.83 (m, 1H).

### Step 7: Synthesis of ethyl 2-((8-bromo-1-methyl-2-oxo-2,3,4,5-tetrahydro-1H-benzo[b]azepin-3-yl)amino)-2-oxoacetate (61h)

To a mixture of compound **61g** (790 mg, 2.9 mmol) and triethylamine (587 mg, 5.8 mmol) in dichloromethane (15 mL), a solution of monoethyl oxalyl chloride (478 mg, 3.5 mmol) in dichloromethane (2 mL) was slowly added dropwise at 0 °C in an ice bath. The reaction solution was slowly warmed to room temperature and stirred for 3 hours. TLC and LC-MS showed that the reaction was complete. The reaction mixture was poured into water (100 mL) and extracted with dichloromethane (60 mL × 3). The organic layers were combined, washed with saturated brine (60 mL), dried over anhydrous sodium sulfate and concentrated under reduced pressure, and then purified by silica gel column chromatography (petroleum ether containing 20% ethyl acetate as the eluent) to obtain compound **61h** (985 mg, 92% yield) as a white solid.

MS (ES⁺): *m*/*z* 368.9 [M+H]⁺.

¹H NMR (400 MHz, CDCl₃): *δ* 8.02 (d, *J* = 6.7 Hz, 1H), 7.37 - 7.33 (m, 2H), 7.13 (d, *J* = 8.0 Hz, 1H), 4.43 (dt, *J* = 11.0, 7.4 Hz, 1H), 4.34 (q, *J* = 7.1 Hz, 2H), 3.41 (s, 3H), 2.85 - 2.60 (m, 3H), 2.00 - 1.92 (m, 1H), 1.37 (t, *J* = 7.1 Hz, 3H).

### Step 8: Synthesis of N¹-(8-bromo-1-methyl-2-oxo-2,3,4,5-tetrahydro-1H-benzo[b]azepin-3-yl)-N²-phenethyloxalamid e (61)

To a solution of compound **61h** (985 mg, 2.7 mmol) in ethanol (20 mL), 2-phenylethylamine **1i** (787 mg, 6.0 mmol) was added, the reaction solution was heated to 80 °C and stirred for 4 hours. TLC and LC-MS showed that the reaction was complete and a large amount of white solid precipitated in the reaction solution. The reaction solution was cooled to room temperature and filtered, and the filter cake was rinsed with petroleum ether (10 mL) containing 15% ethyl acetate. After the filter cake was dried, the title compound **61** (971 mg, 81% yield) was obtained as a white solid.

MS (ES⁺): *m*/*z* 446.0 [M+H]⁺.

¹H NMR (400 MHz, CDCl₃): *δ* 8.24 (d, *J* = 7.5 Hz, 1H), 7.35 - 7.28 (m, 5H), 7.24 - 7.20 (m, 1H), 7.18 (d, *J* = 7.0 Hz, 2H), 7.11 (d, *J* = 8.6 Hz, 1H), 4.39 (dt, *J* = 11.2, 7.5 Hz, 1H), 3.62 - 3.49 (m, 2H), 3.40 (s, 3H), 2.87 - 2.76 (m, 3H), 2.64 - 2.53 (m, 2H), 2.05 - 1.97 (m, 1H).

### Example 62: Synthesis of N¹-(8-(cyclopropylethynyl)-1-methyl-2-oxo-2,3,4,5-tetrahydro-1H-benzo[b]azepin-3-yl)-N²-phen ethyloxalamide (62)

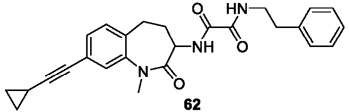

### Synthesis route:

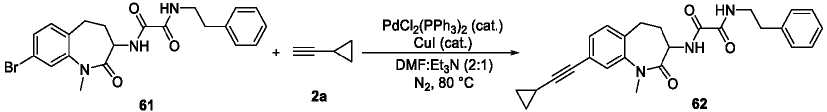

Compound **61** (44.4 mg, 0.1 mmol), bis(triphenylphosphine)palladium(II) dichloride (7.0 mg, 0.01 mmol), cuprous iodide (1.9 mg, 0.01 mmol), triethylamine (1 mL), DMF (2 mL) and cyclopropylacetylene **2a** (13.2 mg, 0.2 mmol) were added in turn to a sealed tube at room temperature. After rapidly replacing the air in the tube with nitrogen three times, the reaction solution was stirred at 80 °C for 15 hours. TLC and LC-MS showed that the reaction was complete. After the reaction solution was cooled to room temperature, water (20 mL) was added to quench it, and extraction was performed with ethyl acetate (10 mL × 3). The combined organic layers were washed with saturated brine (10 mL), dried over anhydrous sodium sulfate, and concentrated under reduced pressure to obtain a crude product, which was then purified by silica gel column chromatography (petroleum ether containing 20% ethyl acetate as the eluent) to obtain the title compound **62** (37.4 mg, 87% yield) as a white solid.

MS (ES⁺): *m*/*z* 430.2 [M+H]⁺.

¹H NMR (400 MHz, CDCl₃): *δ* 8.25 (d, *J* = 7.6 Hz, 1H), 7.35 - 7.28 (m, 3H), 7.24 - 7.17 (m, 5H), 7.12 (d, *J* = 7.7 Hz, 1H), 4.37 (dt, *J* = 11.3, 7.5 Hz, 1H), 3.61 - 3.50 (m, 2H), 3.38 (s, 3H), 2.89 - 2.79 (m, 3H), 2.63 - 2.52 (m, 2H), 2.05 - 1.95 (m, 1H), 1.51 - 1.42 (m, 1H), 0.91 - 0.85 (m, 2H), 0.83 - 0.79 (m, 2H).

### Example 63: Synthesis of N¹-(7-bromo-1-methyl-2-oxo-2,3,4,5-tetrahydro-1H-benzo[b]azepin-3-yl)-N²-phenethyloxalamid e (63)

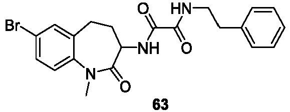

### Synthesis route:

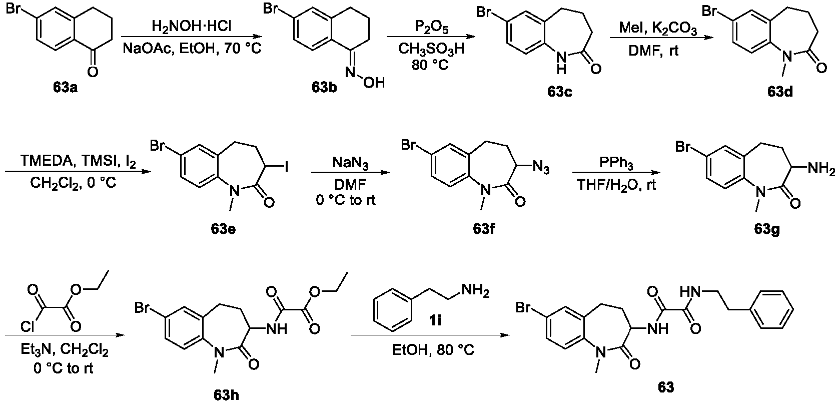

The synthesis route and method were the same as Example 61, except that compound 7-bromo-3,4-dihydronaphthalen-1(2*H*)-one **61a** in step 1 was replaced with an equivalent amount of 6-bromo-3,4-dihydronaphthalen-1(2*H*)-one **63a** to obtain the title compound **63.**

MS (ES⁺): *m*/*z* 446.0 [M+H]⁺.

¹H NMR (400 MHz, CDCl₃): *δ* 8.25 (d, *J =* 7.5 Hz, 1H), 7.45 (dd, *J* = 8.5, 2.1 Hz, 1H), 7.38 (d, *J* = 2.1 Hz, 1H), 7.34 - 7.28 (m, 3H), 7.24 - 7.20 (m, 1H), 7.18 (d, *J* = 7.2 Hz, 2H), 7.05 (d, *J=* 8.5 Hz, 1H), 4.38 (dt, *J* = 11.2, 7.5 Hz, 1H), 3.62 - 3.49 (m, 2H), 3.38 (s, 3H), 2.89 - 2.80 (m, 3H), 2.64 - 2.54 (m, 2H), 2.05 - 1.97 (m, 1H).

### Example 64: Synthesis of N¹-(7-(cyclopropylethynyl)-1-methyl-2-oxo-2,3,4,5-tetrahydro-1H-benzo[b]azepin-3-yl)-N²-phen ethyloxalamide (64)

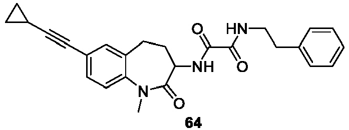

### Synthesis route:

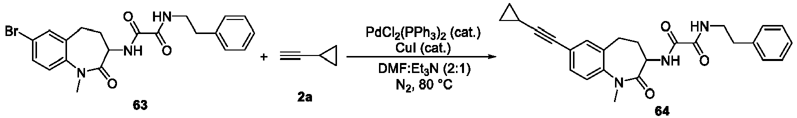

The synthesis route and method were the same as Example 62, except that compound **61** was replaced with an equivalent amount of compound **63** to obtain the title compound **64.**

MS (ES⁺): *m*/*z* 430.2 [M+H]⁺.

¹H NMR (400 MHz, CDCl₃): *δ* 8.24 (d, *J* = 7.6 Hz, 1H), 7.36 - 7.27 (m, 4H), 7.25 - 7.20 (m, 2H), 7.17 (d, *J* = 7.3 Hz, 2H), 7.07 (d, *J* = 8.2 Hz, 1H), 4.37 (dt, *J* = 11.2, 7.4 Hz, 1H), 3.61 - 3.48 (m, 2H), 3.38 (s, 3H), 2.88 - 2.77 (m, 3H), 2.62 - 2.52 (m, 2H), 2.04 - 1.95 (m, 1H), 1.49 - 1.42 (m, 1H), 0.91 - 0.85 (m, 2H), 0.83 - 0.79 (m, 2H).

### Example 65: Synthesis of N¹-(8-(4-hydroxybut-1-yn-1-yl)-1-methyl-2-oxo-2,3,4,5-tetrahydro-1H-benzo[b]azepin-3-yl)-N²-phenethyloxalamide (65)

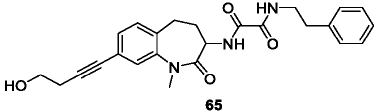

### Synthesis route:

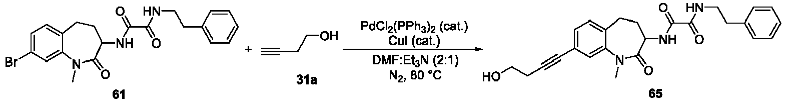

The synthesis route and method were the same as Example 62, except that cyclopropylacetylene **2a** was replaced with an equivalent amount of 3-butyn-1-ol **31a** to obtain the title compound **65.**

MS (ES⁺): *m*/*z* 434.0 [M+H]⁺.

¹H NMR (400 MHz, CDCl₃): *δ* 8.25 (d, *J =* 7.4 Hz, 1H), 7.35 - 7.28 (m, 3H), 7.23 - 7.15 (m, 6H), 4.38 (dt, *J* = 11.2, 7.6 Hz, 1H), 3.83 (t, *J* = 6.2 Hz, 2H), 3.60 - 3.50 (m, 2H), 3.39 (s, 3H), 2.89 - 2.80 (m, 3H), 2.70 (t, *J* = 6.2 Hz, 2H), 2.65 - 2.53 (m, 2H), 2.04 - 1.96 (m, 1H), 1.42 (t, *J* = 7.2 Hz, 1H).

### Example 66: Synthesis of N¹-(1-methyl-2-oxo-8-(piperidin-4-ylethynyl)-2,3,4,5-tetrahydro-1H-benzo[b]azepin-3-yl)-N²-ph enethyloxalamide (66)

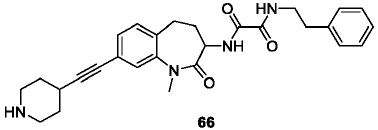

### Synthesis route:

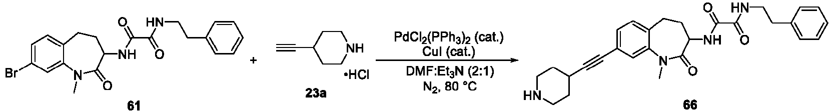

The synthesis route and method were the same as Example 62, except that cyclopropylacetylene **2a** was replaced with an equivalent amount of 4-ethynylpiperidine hydrochloride **23a** to obtain the title compound **66.**

MS (ES⁺): *m*/*z* 473.3 [M+H]⁺.

¹H NMR (400 MHz, CDCl₃): *δ* 8.25 (d, *J* = 7.3 Hz, 1H), 7.34 - 7.28 (m, 3H), 7.23 - 7.16 (m, 6H), 4.38 (dt, *J* = 11.2, 7.6 Hz, 1H), 3.58 - 3.52 (m, 2H), 3.45 - 3.40 (m, 5H), 3.29 - 3.23 (m, 2H), 3.10 - 3.06 (m, 1H), 2.88 - 2.81 (m, 3H), 2.66 - 2.54 (m, 2H), 2.32 - 2.24 (m, 2H), 2.11 - 1.96 (m, 3H).

### Example 67: Synthesis of N¹-(1-methyl-2-oxo-2,3,4,5-tetrahydro-1H-benzo[b]azepin-3-yl)-N²-phenethyloxalamide (67)

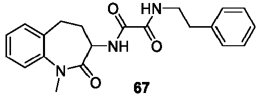

### Synthesis route:

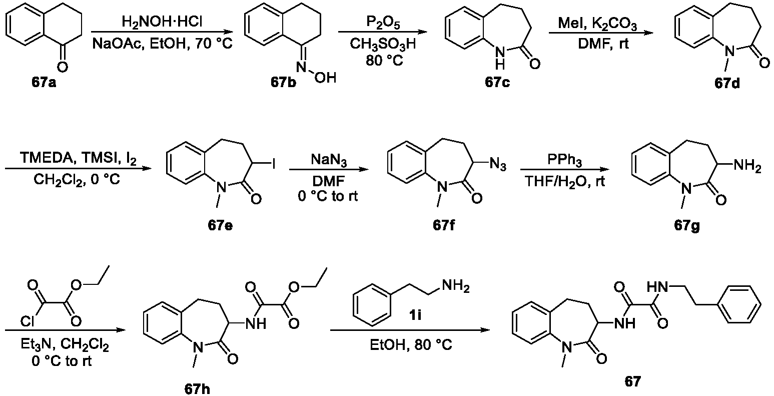

The synthesis route and method were the same as Example 61, except that compound 7-bromo-3,4-dihydronaphthalen-1(2H)-one **61a** in step 1 was replaced with an equivalent amount of 3,4-dihydronaphthalen-1(2H)-one **67a** to obtain the title compound **67.**

MS (ES⁺): *m*/*z* 366.1 [M+H]⁺.

¹H NMR (400 MHz, CDCl₃): *δ* 8.27 (d, *J* = 7.7 Hz, 1H), 7.34 - 7.27 (m, 4H), 7.24 - 7.16 (m, 6H), 4.41 (dt, *J* = 11.3, 7.6 Hz, 1H), 3.62 - 3.49 (m, 2H), 3.41 (s, 3H), 2.93 - 2.81 (m, 3H), 2.66 - 2.56 (m, 2H), 2.04 - 1.97 (m, 1H).

### Example 68: Synthesis of N¹-benzyl-N²-(1-methyl-2-oxo-2,3,4,5-tetrahydro-1H-benzo[b]azepin-3-yl)oxalamide (68)

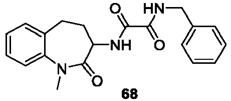

### Synthesis route:

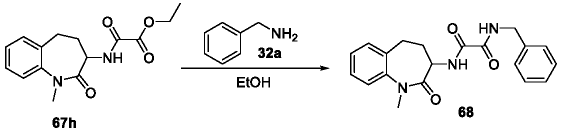

The synthesis route and method were the same as step 8 of Example 67, except that 2-phenylethylamine **1i** was replaced with an equivalent amount of benzylamine **32a** to obtain the title compound 68.

MS (ES⁺): *m*/*z* 352.1 [M+H]⁺.

¹H NMR (400 MHz, CDCl₃): *δ* 8.33 (d, *J* = 7.1 Hz, 1H), 7.57 (t, *J* = 5.4 Hz, 1H), 7.35 - 7.27 (m, 6H), 7.23 - 7.17 (m, 3H), 4.48 (d, *J* = 6.2 Hz, 2H), 4.43 (dt, *J* = 11.3, 7.5 Hz, 1H), 3.42 (s, 3H), 2.93 - 2.84 (m, 1H), 2.68 - 2.58 (m, 2H), 2.06 - 1.98 (m, 1H).

### Example 69: Synthesis of N¹-(9-methyl-8-oxo-6,7,8,9-tetrahydro-5H-pyrido[2,3-b]azepin-7-yl)-N²-phenethyloxalamide (69)

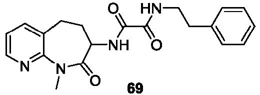

### Synthesis route:

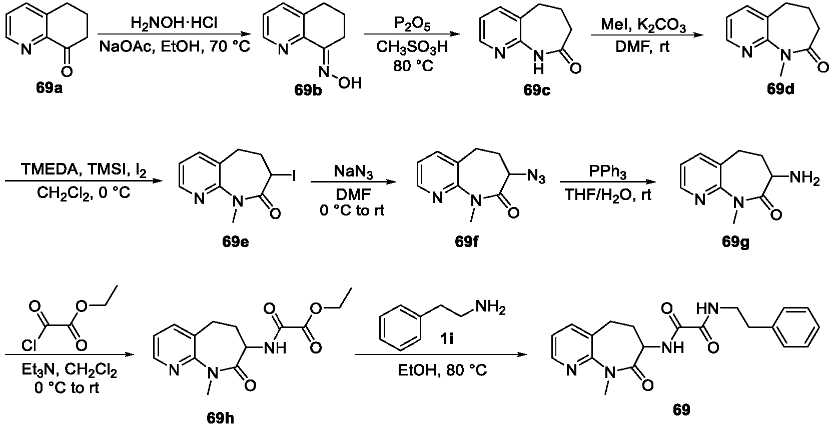

The synthesis route and method were the same as Example 61, except that compound 7-bromo-3,4-dihydronaphthalen-1(2*H*)-one **61a** in step 1 was replaced with an equivalent amount of 6,7-dihydroquinolin-8(5*H*)-one **69a** to obtain the title compound **69.**

MS (ES⁺): *m*/*z* 367.1 [M+H]⁺.

¹H NMR (400 MHz, CDCl₃): *δ* 8.41 (dd, *J* = 4.8, 1.5 Hz, 1H), 8.34 (d, *J =* 7.2 Hz, 1H), 7.58 (dd, *J* = 7.5, 1.4 Hz, 1H), 7.32 - 7.28 (m, 3H), 7.24 - 7.20 (m, 1H), 7.18 (d, *J* = 7.2 Hz, 2H), 7.13 (dd, *J* = 7.5, 4.8 Hz, 1H), 4.40 (dt, *J* = 11.3, 7.4 Hz, 1H), 3.61 - 3.52 (m, 2H), 3.51 (s, 3H), 2.87 - 2.82 (m, 3H), 2.74 - 2.62 (m, 2H), 2.10 - 2.00 (m, 1H).

### Example 70: Synthesis of N¹-(8-oxo-6,7,8,9-tetrahydro-5H-pyrido[2,3-b]azepin-7-yl)-N²-phenethyloxalamide (70)

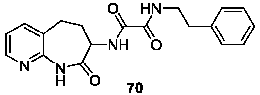

### Synthesis route:

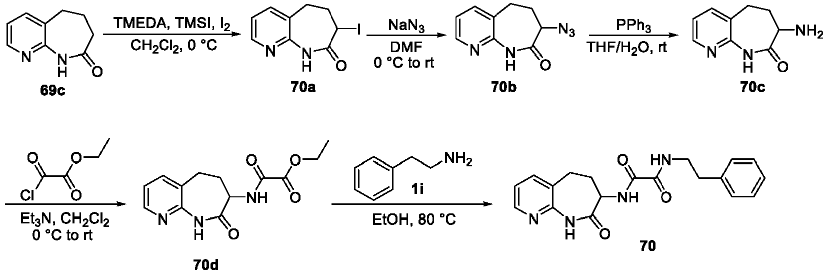

The synthesis route and method were the same as steps 4 - 8 of Example 69, except that compound 69d in step 4 was replaced with an equivalent amount of compound 69c to obtain the title compound 70.

MS (ES⁺): *m*/*z* 353.1 [M+H]⁺.

¹H NMR (400 MHz, DMSO-*d*₆): *δ* 10.42 (s, 1H), 8.81 (t, *J =* 6.0 Hz, 1H), 8.66 (d, *J* = 7.8 Hz, 1H), 8.30 (dd, *J* = 4.8, 1.2 Hz, 1H), 7.76 (d, *J =* 7.4 Hz, 1H), 7.30 - 7.26 (m, 2H), 7.21 - 7.16 (m, 4H), 4.13 (dt, *J* = 11.3, 8.1 Hz, 1H), 2.79 - 2.64 (m, 5H), 2.43 - 2.31 (m, 3H).

### Example 71: Synthesis of (R)-N¹-(7-bromo-5-methyl-4-oxo-2,3,4,5-tetrahydrobenzo[b][1,4]oxazepin-3-yl)-N²-phenethylox alamide (71)

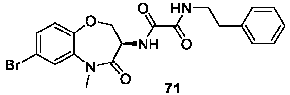

### Synthesis route:

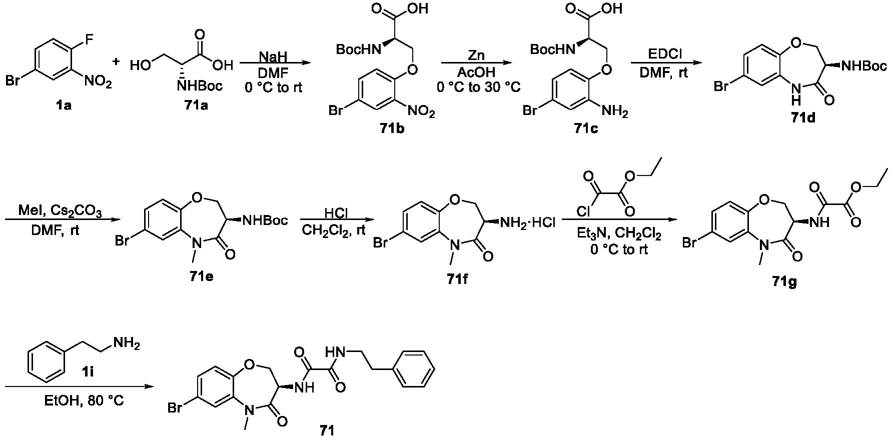

The synthesis route and method were the same as Example 1, except that (*tert*-butoxycarbonyl)-*L*-serine **1b** was replaced with an equivalent amount of (*tert*-butoxycarbonyl)-*D*-serine **71a** to obtain the title compound **71.**

MS (ES⁺): *m*/*z* 422.0 [M+H]⁺.

¹H NMR (400 MHz, CDCl₃): *δ* 8.22 (d, *J* = 7.5 Hz, 1H), 7.35 - 7.28 (m, 5H), 7.25 - 7.21 (m, 1H), 7.18 (d, *J* = 7.3 Hz, 2H), 7.06 (d, *J =* 8.7 Hz, 1H), 4.81 (dt, *J* = 11.3, 7.4 Hz, 1H), 4.58 (dd, *J* = 9.7, 7.4 Hz, 1H), 4.23 (t, *J* = 10.5 Hz, 1H), 3.59 - 3.54 (m, 2H), 3.40 (s, 3H), 2.84 (t, *J =* 7.1 Hz, 2H).

### Example 72: Synthesis of (S)-N¹-(7-(3-(1H-imidazol-1-yl)prop-1-yn-1-yl)-5-methyl-4-oxo-2,3,4,5-tetrahydrobenzo[b][1,4] oxazepin-3-yl)-N²-phenethyloxalamide (72)

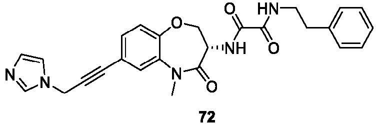

### Synthesis route:

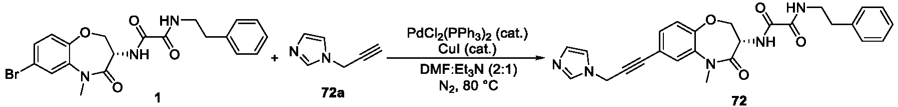

The synthesis route and method were the same as Example 2, except that cyclopropylacetylene **2a** was replaced with an equivalent amount of 1-(prop-2-yn-1-yl)-1*H*-imidazole **72a** to obtain the title compound **72.**

MS (ES⁺): *m*/*z* 472.2 [M+H]⁺.

¹H NMR (400 MHz, CDCl₃): *δ* 8.24 (d, *J* = 7.3 Hz, 1H), 7.77 (br, 1H), 7.32 - 7.28 (m, 6H), 7.24 - 7.21 (m, 2H), 7.17 (d, *J* = 7.2 Hz, 2H), 7.13 (d, *J* = 8.4 Hz, 1H), 4.96 (s, 2H), 4.81 (dt, *J* = 11.3, 7.4 Hz, 1H), 4.59 (dd, *J* = 9.7, 7.3 Hz, 1H), 4.25 (t, *J* = 10.6 Hz, 1H), 3.59 - 3.53 (m, 2H), 3.41 (s, 3H), 2.84 (t, *J* = 7.1 Hz, 2H).

### Example 73: Synthesis of (S)-N¹-(5-methyl-7-((1-methylpiperidin-4-yl)ethynyl)-4-oxo-2,3,4,5-tetrahydrobenzo[b][1,4]oxaz epin-3-yl)-N²-phenethyloxalamide (73)

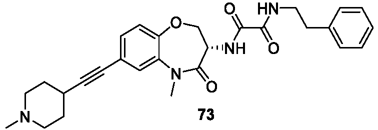

### Synthesis route:

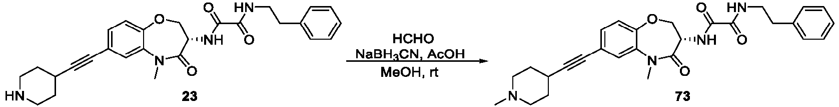

To a solution of compound **23** (47 mg, 0.1 mmol) in methanol (3.0 mL) at room temperature, paraformaldehyde (18 mg, 0.2 mmol) was added, the reaction solution was stirred for 2 minutes, sodium cyanoborohydride (13 mg, 0.2 mmol) and acetic acid (12 mg, 0.2 mmol) were added thereto, and the mixture was stirred at room temperature for 15 hours. TLC and LC-MS showed that the reaction was complete. The reaction solution was directly concentrated under reduced pressure to obtain a crude product, which was then purified by silica gel column chromatography (dichloromethane containing 5 - 10% methanol as the eluent) to obtain the title compound 73 (20 mg, 41% yield) as a white solid.

MS (ES⁺): *m*/*z* 489.3 [M+H]⁺.

¹H NMR (400 MHz, CDCl₃): *δ* 8.24 (d, *J* = 7.4 Hz, 1H), 7.35 - 7.28 (m, 3H), 7.25 - 7.21 (m, 3H), 7.17 (d, *J* = 7.2 Hz, 2H), 7.11 (d, *J* = 8.8 Hz, 1H), 4.81 (dt, *J* = 11.1, 7.4 Hz, 1H), 4.57 (dd, *J* = 9.6, 7.5 Hz, 1H), 4.24 (t, *J* = 10.6 Hz, 1H), 3.59 - 3.54 (m, 2H), 3.41 (s, 3H), 3.18 - 3.12 (m, 2H), 3.00 - 2.95 (m, 2H), 2.84 (t, *J* = 7.0 Hz, 2H), 2.69 (s, 3H), 2.33 - 2.28 (m, 2H), 2.07 - 2.02 (m, 3H).

### Example 74: Synthesis of (S)-N¹-(7-((1-(cyanomethyl)piperidin-4-yl)ethynyl)-5-methyl-4-oxo-2,3,4,5-tetrahydrobenzo[b][1 ,4]oxazepin-3-yl)-N²-phenethyloxalamide (74)

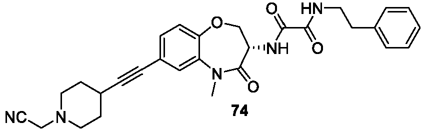

### Synthesis route:

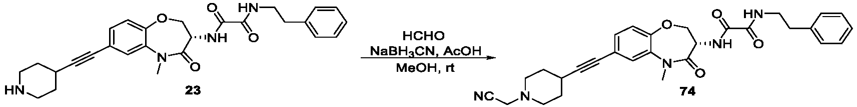

The synthesis route and method were the same as Example 73, and the title compound **74** was prepared through the same reaction.

MS (ES⁺): *m*/*z* 514.2 [M+H]⁺.

¹H NMR (400 MHz, CDCl₃): *δ* 8.24 (d, *J* = 7.2 Hz, 1H), 7.32 - 7.28 (m, 4H), 7.25 - 7.21 (m, 2H), 7.17 (d, *J =* 7.2 Hz, 2H), 7.09 (d, *J* = 8.2 Hz, 1H), 4.80 (dt, *J* = 11.1, 7.4 Hz, 1H), 4.58 (dd, *J* = 9.4, 7.6 Hz, 1H), 4.23 (t, *J* = 10.5 Hz, 1H), 3.58 - 3.54 (m, 4H), 3.40 (s, 3H), 2.85 - 2.82 (m, 4H), 2.66 (br, 1H), 2.49 (t, *J* = 8.6 Hz, 2H), 2.00 - 1.97 (m, 2H), 1.84 - 1.77 (m, 2H).

### Example 75: Synthesis of (S)-N¹-(7-((4-(dimethylamino)phenyl)ethynyl)-5-methyl-4-oxo-2,3,4,5-tetrahydrobenzo[b][1,4]o xazepin-3-yl)-N²-phenethyloxalamide (75)

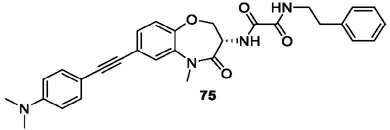

### Synthesis route:

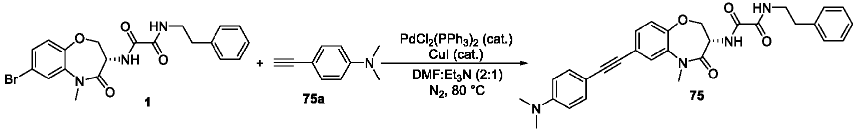

The synthesis route and method were the same as Example 2, except that cyclopropylacetylene **2a** was replaced with an equivalent amount of 4-ethynyl-*N,N*-dimethylaniline **75a** to obtain the title compound **75.**

MS (ES⁺): *m*/*z* 511.3 [M+H]⁺.

¹H NMR (400 MHz, CDCl₃): *δ* 8.25 (d, *J* = 7.6 Hz, 1H), 7.40 (d, *J* = 8.7 Hz, 2H), 7.35 - 7.28 (m, 5H), 7.24 - 7.21 (m, 1H), 7.18 (d, *J* = 7.2 Hz, 2H), 7.12 (d, *J* = 8.8 Hz, 1H), 6.66 (d, *J =* 8.8 Hz, 2H), 4.84 (dt, *J* = 11.2, 7.3 Hz, 1H), 4.61 (dd, *J* = 9.7, 7.4 Hz, 1H), 4.23 (t, *J* = 10.5 Hz, 1H), 3.59 - 3.54 (m, 2H), 3.43 (s, 3H), 3.00 (s, 6H), 2.84 (t, *J* = 7.1 Hz, 2H).

### Example 76: Synthesis of (S)-N¹-(5-methyl-7-(oxetan-3-ylethynyl)-4-oxo-2,3,4,5-tetrahydrobenzo[b][1,4]oxazepin-3-yl)-N² -phenethyloxalamide (76)

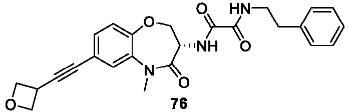

### Synthesis route:

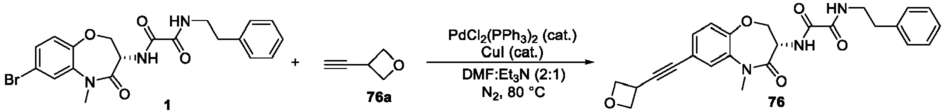

The synthesis route and method were the same as Example 2, except that cyclopropylacetylene **2a** was replaced with an equivalent amount of 3-ethynyloxetane **76a** to obtain the title compound **76.**

MS (ES⁺): *m*/*z* 448.2 [M+H]⁺.

¹H NMR (400 MHz, CDCl₃): *δ* 8.24 (d, *J* = 7.3 Hz, 1H), 7.31 - 7.28 (m, 5H), 7.24 - 7.21 (m, 1H), 7.16 (d, *J* = 6.9 Hz, 2H), 7.11 (d, *J* = 8.8 Hz, 1H), 4.91 - 4.86 (m, 2H), 4.82 - 4.78 (m, 3H), 4.59 (dd, *J* = 9.7, 7.4 Hz, 1H), 4.24 (t, *J* = 10.5 Hz, 1H), 4.10 - 4.02 (m, 1H), 3.59 - 3.52 (m, 2H), 3.41 (s, 3H), 2.84 (t, *J* = 7.1 Hz, 2H).

### Example 77: Synthesis of (R)-N¹-(7-((3-hydroxyoxetan-3-yl)ethynyl)-5-methyl-4-oxo-2,3,4,5-tetrahydrobenzo[b][1,4]oxaze pin-3-yl)-N²-phenethyloxalamide (77)

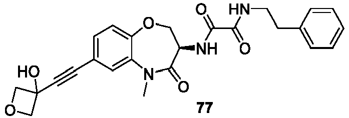

### Synthesis route:

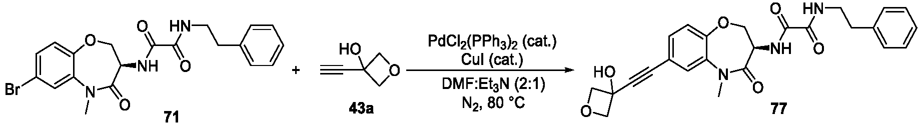

The synthesis route and method were the same as Example 43, except that compound **1** was replaced with an equivalent amount of compound **71** to obtain the title compound **77.**

MS (ES⁺): *m*/*z* 464.1 [M+H]⁺.

¹H NMR (400 MHz, CDCl₃): *δ* 8.24 (d, *J* = 7.4 Hz, 1H), 7.32 - 7.28 (m, 5H), 7.24 - 7.21 (m, 1H), 7.18 (d, *J =* 7.3 Hz, 2H), 7.14 (d, *J =* 8.8 Hz, 1H), 4.94 (d, *J =* 6.6 Hz, 2H), 4.85 - 4.78 (m, 3H), 4.60 (dd, *J* = 9.6, 7.4 Hz, 1H), 4.26 (t, *J =* 10.6 Hz, 1H), 3.59 - 3.54 (m, 2H), 3.42 (s, 3H), 2.84 (t, *J* = 7.1 Hz, 2H), 2.69 (br, 1H).

### Example 78: Synthesis of (S)-N¹-(7-((3-methoxyoxetan-3-yl)ethynyl)-5-methyl-4-oxo-2,3,4,5-tetrahydrobenzo[b][1,4]oxaz epin-3-yl)-N²-phenethyloxalamide (78)

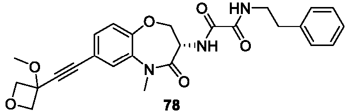

### Synthesis route:

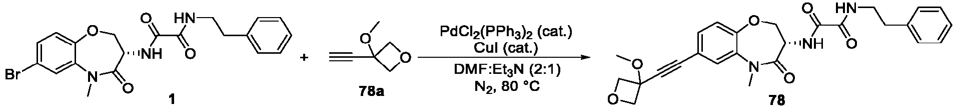

The synthesis route and method were the same as Example 2, except that cyclopropylacetylene **2a** was replaced with an equivalent amount of 3-ethynyl-3-methoxyoxetane **78a** to obtain the title compound **78.**

MS (ES⁺): *m*/*z* 478.3 [M+H]⁺.

¹H NMR (400 MHz, CDCl₃): *δ* 8.24 (d, *J =* 7.2 Hz, 1H), 7.34 - 7.28 (m, 5H), 7.24 - 7.21 (m, 1H), 7.18 (d, *J =* 7.3 Hz, 2H), 7.14 (d, *J* = 8.8 Hz, 1H), 4.85 (d, *J =* 6.6 Hz, 2H), 4.83 - 4.80 (m, 1H), 4.77 (d, *J =* 6.5 Hz, 2H), 4.59 (dd, *J* = 9.7, 7.3 Hz, 1H), 4.26 (t, *J =* 10.6 Hz, 1H), 3.59 - 3.52 (m, 2H), 3.42 (s, 3H), 3.41 (s, 3H), 2.84 (t, *J* = 7.1 Hz, 2H).

### Example 79: Synthesis of (S)-N¹-(7-(3-(3-hydroxyoxetan-3-yl)prop-1-yn-1-yl)-5-methyl-4-oxo-2,3,4,5-tetrahydrobenzo[b][ 1,4]oxazepin-3-yl)-N²-phenethyloxalamide (79)

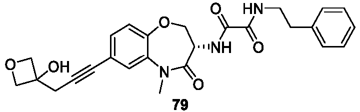

### Synthesis route:

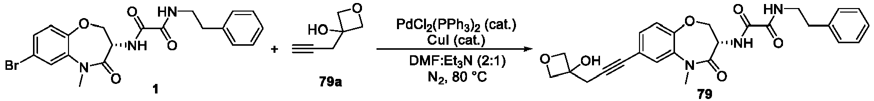

The synthesis route and method were the same as Example 2, except that cyclopropylacetylene **2a** was replaced with an equivalent amount of 3-(prop-2-yn-1-yl)oxetan-3-ol **79a** to obtain the title compound **79.**

MS (ES⁺): *m*/*z* 478.2 [M+H]⁺.

¹H NMR (400 MHz, CDCl₃): *δ* 8.23 (d, *J* = 7.3 Hz, 1H), 7.32 - 7.27 (m, 5H), 7.24 - 7.21 (m, 1H), 7.18 (d, *J* = 6.9 Hz, 2H), 7.11 (d, *J* = 8.8 Hz, 1H), 4.80 (dt, *J* = 11.3, 7.4 Hz, 1H), 4.69 (d, *J* = 7.0 Hz, 2H), 4.60 - 4.56 (m, 3H), 4.24 (t, *J* = 10.5 Hz, 1H), 3.59 - 3.52 (m, 2H), 3.41 (s, 3H), 3.01 (s, 2H), 2.84 (t, *J* = 7.2 Hz, 2H), 3.54 (s, 1H).

### Example 80: Synthesis of N¹-((3S)-7-((3-hydroxytetrahydrofuran-3-yl)ethynyl)-5-methyl-4-oxo-2,3,4,5-tetrahydrobenzo[b][ 1,4]oxazepin-3-yl)-N²-phenethyloxalamide (80)

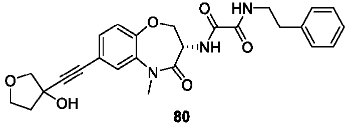

### Synthesis route:

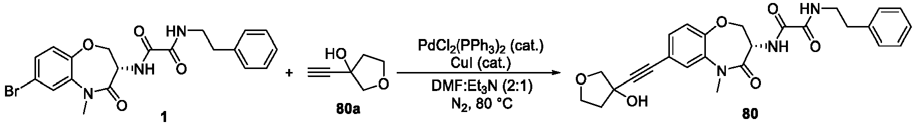

The synthesis route and method were the same as Example 2, except that cyclopropylacetylene **2a** was replaced with an equivalent amount of 3-ethynyltetrahydrofuran-3-ol **80a** to obtain the title compound **80.**

MS (ES⁻): *m*/*z* 476.2 [M-H]⁻.

¹H NMR (400 MHz, CDCl₃): *δ* 8.24 (d, *J* = 7.3 Hz, 1H), 7.32 - 7.28 (m, 5H), 7.24 - 7.21 (m, 1H), 7.18 (d, *J =* 6.8 Hz, 2H), 7.12 (d, *J* = 8.7 Hz, 1H), 4.80 (dt, *J* = 11.3, 7.3 Hz, 1H), 4.59 (dd, *J* = 9.7, 7.3 Hz, 1H), 4.25 (t, *J* = 10.5 Hz, 1H), 4.11 - 3.92 (m, 4H), 3.61 - 3.52 (m, 2H), 3.41 (s, 3H), 2.84 (t, *J* = 7.0 Hz, 2H), 2.46 - 2.30 (m, 2H), 2.24 (s, 1H).

### Example 81: Synthesis of (S)-N¹-(7-(3-(4-hydroxypiperidin-1-yl)prop-1-yn-1-yl)-5-methyl-4-oxo-2,3,4,5-tetrahydrobenzo[b ][1,4]oxazepin-3-yl)-N²-phenethyloxalamide (81)

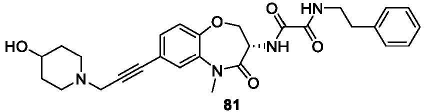

### Synthesis route:

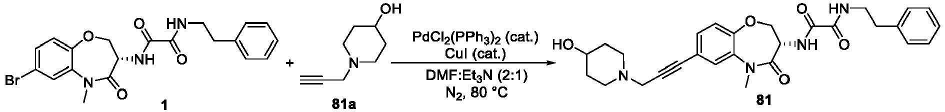

The synthesis route and method were the same as Example 2, except that cyclopropylacetylene **2a** was replaced with an equivalent amount of 1-(prop-2-yn-1-yl)piperidin-4-ol **81a** to obtain the title compound **81.**

MS (ES⁺): *m*/*z* 505.3 [M+H]⁺.

¹H NMR (400 MHz, CDCl₃): *δ* 8.23 (d, *J* = 7.5 Hz, 1H), 7.32 - 7.28 (m, 5H), 7.24 - 7.21 (m, 1H), 7.17 (d, *J* = 7.1 Hz, 2H), 7.10 (d, *J =* 8.9 Hz, 1H), 4.81 (dt, *J* = 11.2, 7.4 Hz, 1H), 4.58 (dd, *J* = 9.7, 7.4 Hz, 1H), 4.23 (dd, *J* = 11.0, 10.0 Hz, 1H), 3.75 (br, 1H), 3.59 - 3.54 (m, 2H), 3.51 (s, 2H), 3.40 (s, 3H), 2.90 - 2.86 (m, 2H), 2.84 (t, *J* = 7.2 Hz, 2H), 2.44 - 2.39 (m, 2H), 1.99 - 1.95 (m, 2H), 1.71 - 1.63 (m, 2H), 1.41 (br, 1H).

### Example 82: Synthesis of N¹-((3S)-5-methyl-7-(3-(1-methyl-2-oxopyrrolidin-3-yl)prop-1-yn-1-yl)-4-oxo-2,3,4,5-tetrahydro benzo[b][1,4]oxazepin-3-yl)-N²-phenethyloxalamide (82)

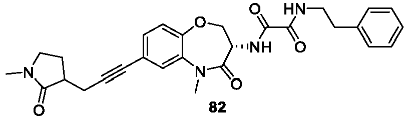

### Synthesis route:

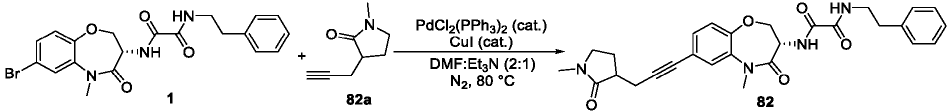

The synthesis route and method were the same as Example 2, except that cyclopropylacetylene **2a** was replaced with an equivalent amount of 1-methyl-3-(prop-2-yn-1-yl)pyrrolidin-2-one **82a** to obtain the title compound **82.**

MS (ES⁺): *m*/*z* 503.2 [M+H]⁺.

¹H NMR (400 MHz, CDCl₃): *δ* 8.23 (d, *J* = 7.4 Hz, 1H), 7.32 - 7.28 (m, 3H), 7.24 - 7.21 (m, 3H), 7.18 (d, *J* = 7.0 Hz, 2H), 7.08 (d, *J =* 8.7 Hz, 1H), 4.80 (dt, *J* = 11.3, 7.4 Hz, 1H), 4.57 (dd, *J* = 9.7, 7.4 Hz, 1H), 4.22 (t, *J* = 10.5 Hz, 1H), 3.59 - 3.53 (m, 2H), 3.42 - 3.35 (m, 5H), 2.89 (s, 3H), 2.88 - 2.79 (m, 3H), 2.74 - 2.61 (m, 2H), 2.38 - 2.30 (m, 1H), 2.07 - 1.98 (m, 1H).

### Example 83: Synthesis of (S)-N¹-(7-(3-(4-(2-hydroxyethyl)piperazin-1-yl)prop-1-yn-1-yl)-5-methyl-4-oxo-2,3,4,5-tetrahydr obenzo[b][1,4]oxazepin-3-yl)-N²-phenethyloxalamide (83)

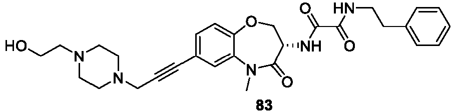

### Synthesis route:

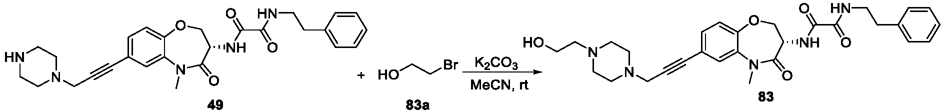

To a mixture of compound **49** (39 mg, 0.08 mmol) and potassium carbonate (22 mg, 0.16 mmol) in acetonitrile (1.0 mL), a solution of 2-bromoethanol **83a** (15 mg, 0.12 mmol) in acetonitrile (0.2 mL) was added dropwise, and the reaction solution was stirred at room temperature for 1.5 hours. TLC and LC-MS showed that the reaction was complete. The reaction solution was filtered, the filter cake was washed with a small amount of acetonitrile, and the filtrate was concentrated under reduced pressure to obtain a crude product, which was then purified by silica gel column chromatography (dichloromethane containing 5 - 10% methanol as the eluent) to obtain the title compound **83** (36 mg, 84% yield) as a white solid.

MS (ES⁺): *m*/*z* 534.3 [M+H]⁺.

¹H NMR (400 MHz, CDCl₃): *δ* 8.24 (d, *J =* 7.5 Hz, 1H), 7.32 - 7.28 (m, 5H), 7.24 - 7.21 (m, 1H), 7.18 (d, *J* = 7.4 Hz, 2H), 7.10 (d, *J* = 8.8 Hz, 1H), 4.81 (dt, *J* = 11.6, 7.5 Hz, 1H), 4.58 (dd, *J* = 9.3, 7.7 Hz, 2H), 4.23 (t, *J* = 10.5 Hz, 1H), 3.63 (t, *J =* 5.2 Hz, 2H), 3.59 - 3.54 (m, 2H), 3.52 (s, 2H), 3.41 (s, 3H), 2.84 (t, *J* = 7.0 Hz, 2H), 2.72 - 2.59 (m, 8H), 2.58 (t, *J =* 5.2 Hz, 2H).

### Example 84: Synthesis of (S)-N¹-(7-(3-(2,5-dioxopyrrolidin-1-yl)prop-1-yn-1-yl)-5-methyl-4-oxo-2,3,4,5-tetrahydrobenzo[ b][1,4]oxazepin-3-yl)-N²-phenethyloxalamide (84)

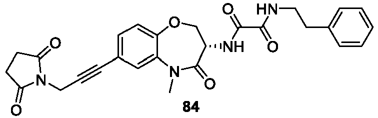

### Synthesis route:

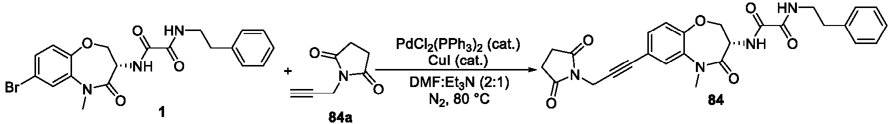

The synthesis route and method were the same as Example 2, except that cyclopropylacetylene **2a** was replaced with an equivalent amount of 1-(prop-2-yn-1-yl)pyrrolidine-2,5-dione **84a** to obtain the title compound **84.**

MS (ES⁺): *m*/*z* 503.2 [M+H]⁺.

¹H NMR (400 MHz, CDCl₃): *δ* 8.23 (d, *J* = 7.3 Hz, 1H), 7.32 - 7.27 (m, 5H), 7.24 - 7.21 (m, 1H), 7.17 (d, *J =* 7.1 Hz, 2H), 7.09 (d, *J =* 8.8 Hz, 1H), 4.78 (dt, *J =* 11.2, 7.4 Hz, 1H), 4.58 (dd, *J* = 9.7, 7.3 Hz, 1H), 4.49 (s, 2H), 4.23 (t, *J =* 10.5 Hz, 1H), 3.60 - 3.52 (m, 2H), 3.39 (s, 3H), 2.84 (t, *J* = 7.1 Hz, 2H), 2.80 (s, 4H).

### Example 85: Synthesis of (S)-N¹-(5-methyl-4-oxo-7-(3-(3-oxopiperazin-1-yl)prop-1-yn-1-yl)-2,3,4,5-tetrahydrobenzo[b][1, 4]oxazepin-3-yl)-N²-phenethyloxalamide (85)

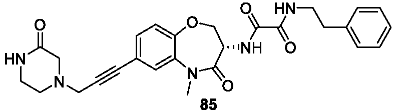

### Synthesis route:

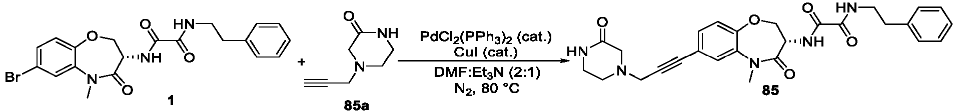

The synthesis route and method were the same as Example 2, except that cyclopropylacetylene **2a** was replaced with an equivalent amount of 4-(prop-2-yn-1-yl)piperazin-2-one **85a** to obtain the title compound **85.**

MS (ES⁺): *m*/*z* 504.2 [M+H]⁺.

¹H NMR (400 MHz, CDCl₃): *δ* 8.24 (d, *J* = 7.5 Hz, 1H), 7.32 - 7.28 (m, 5H), 7.24 - 7.21 (m, 1H), 7.17 (d, *J* = 7.2 Hz, 2H), 7.11 (d, *J =* 8.8 Hz, 1H), 5.96 (s, 1H), 4.81 (dt, *J* = 11.3, 7.4 Hz, 1H), 4.58 (dd, *J* = 9.7, 7.3 Hz, 1H), 4.24 (t, *J* = 10.5 Hz, 1H), 3.63 (s, 2H), 3.59 - 3.53 (m, 2H), 3.46 - 3.43 (m, 2H), 3.41 (s, 3H), 3.36 (s, 2H), 2.86 - 2.82 (m, 4H).

### Example 86: Synthesis of tert-butyl (S)-(3-(5-methyl-4-oxo-3-(2-oxo-2-(phenethylamino)acetamido)-2,3,4,5-tetrahydrobenzo[b][1,4] oxazepin-7-yl)prop-2-yn-1-yl)carbamate (86)

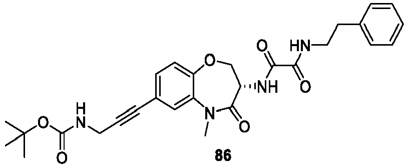

### Synthesis route:

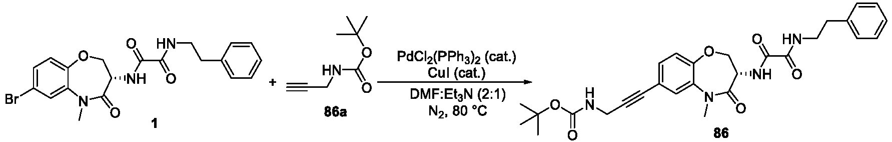

The synthesis route and method were the same as Example 2, except that cyclopropylacetylene **2a** was replaced with an equivalent amount of *tert*-butyl prop-2-yn-1-ylcarbamate **86a** to obtain compound **86.**

MS (ES⁺): *m*/*z* 521.2 [M+H]⁺.

¹H NMR (400 MHz, CDCl₃): *δ* 8.24 (d, *J* = 7.5 Hz, 1H), 7.32 - 7.27 (m, 5H), 7.24 - 7.21 (m, 1H), 7.17 (d, *J* = 7.1 Hz, 2H), 7.10 (d, *J* = 8.7 Hz, 1H), 4.84 - 4.77 (m, 2H), 4.59 (dd, *J* = 9.7, 7.3 Hz, 1H), 4.23 (t, *J* = 10.5 Hz, 1H), 4.15 (d, *J* = 4.8 Hz, 2H), 3.61 - 3.52 (m, 2H), 3.40 (s, 3H), 2.84 (t, *J* = 7.1 Hz, 2H), 1.47 (s, 9H).

### Example 87: Synthesis of (S)-N¹-(7-(3-aminoprop-1-yn-1-yl)-5-methyl-4-oxo-2,3,4,5-tetrahydrobenzo[b][1,4]oxazepin-3-yl )-N²-phenethyloxalamide hydrochloride (87)

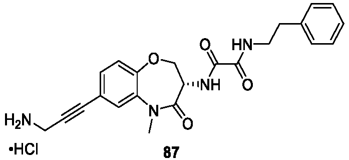

### Synthesis route:

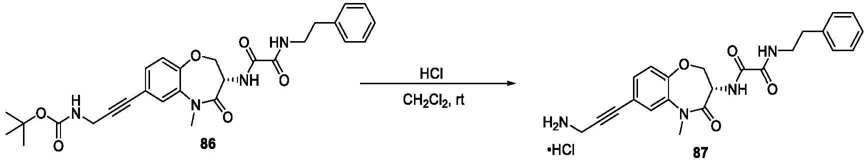

To a solution of compound **86** (26 mg, 0.05 mmol) in dichloromethane (1 mL) at room temperature, a solution of hydrogen chloride in 1,4-dioxane (4 N, 0.2 mL) was added dropwise, and the reaction mixture was stirred at room temperature for 3 hours. TLC and LC-MS showed that the reaction was complete, and the solvent and other volatiles were removed by concentration under reduced pressure to obtain the title compound **87** (22 mg, 96% yield) as a white solid.

MS (ES⁺): *m*/*z* 421.2 [M+H]⁺.

¹H NMR (400 MHz, DMSO-*d*₆): *δ* 8.81 - 8.77 (m, 2H), 8.44 (br, 3H), 7.56 (d, *J* = 1.5 Hz, 1H), 7.37 (dd, *J* = 8.3, 1.6 Hz, 1H), 7.30 - 7.26 (m, 3H), 7.21 - 7.18 (m, 3H), 4.69 - 4.60 (m, 2H), 4.42 - 4.35 (m, 1H), 4.00 (s, 2H), 3.39 - 3.36 (m, 2H), 3.30 (s, 3H), 2.77 (t, *J* = 7.4 Hz, 2H).

### Example 88: Synthesis of (S)-N¹-(7-(3-(2-hydroxyacetamido)prop-1-yn-1-yl)-5-methyl-4-oxo-2,3,4,5-tetrahydrobenzo[b][1, 4]oxazepin-3-yl)-N²-phenethyloxalamide (88)

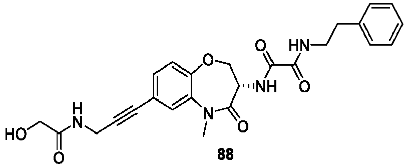

### Synthesis route:

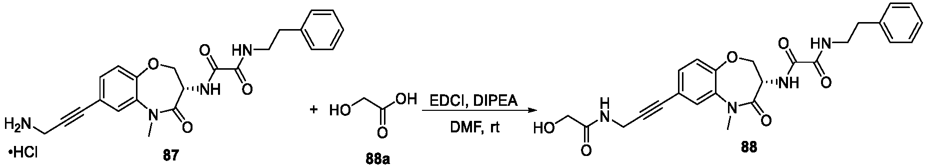

1-Ethyl-3-(3-dimethylaminopropyl)carbodiimide hydrochloride (EDCI, 12 mg, 0.06 mmol), *N,N*-diisopropylethylamine (DIPEA, 16 mg, 0.12 mmol) and glycolic acid (5 mg, 0.06 mmol) were added to a solution of compound **87** (23 mg, 0.05 mmol) in DMF (2 mL) at room temperature, and the reaction solution was stirred at room temperature for 6 hours. TLC and LC-MS showed that the reaction was complete, and water (5 mL) was added to quench the reaction, and the mixture was extracted with ethyl acetate (5 mL × 3). The combined organic layers were washed with saturated brine (5 mL), dried over anhydrous sodium sulfate, and concentrated under reduced pressure to obtain a crude product, which was then purified by silica gel column chromatography (using dichloromethane containing 5 - 10% methanol as the eluent) to obtain the title compound **88** (15 mg, 63% yield) as a white solid.

MS (ES⁺): *m*/*z* 479.2 [M+H]⁺.

¹H NMR (400 MHz, DMSO-*d*₆): *δ* 8.82 (t, *J* = 5.9 Hz, 1H), 8.75 (d, *J* = 7.4 Hz, 1H), 8.26 (t, *J* = 5.7 Hz, 1H), 7.53 (d, *J* = 1.2 Hz, 1H), 7.32 - 7.26 (m, 3H), 7.21 - 7.18 (m, 4H), 5.54 (t, *J* = 5.8 Hz, 1H), 4.69 - 4.55 (m, 2H), 4.41 - 4.32 (m, 1H), 4.15 (d, *J* = 4.2 Hz, 2H), 3.85 (d, *J* = 5.8 Hz, 2H), 3.39 - 3.35 (m, 2H), 3.29 (s, 3H), 2.77 (t, *J* = 7.4 Hz, 2H).

### Example 89: Synthesis of (S)-N¹-(7-(3-acrylamidoprop-1-yn-1-yl)-5-methyl-4-oxo-2,3,4,5-tetrahydrobenzo[b][1,4]oxazepin -3-yl)-N²-phenethyloxalamide (89)

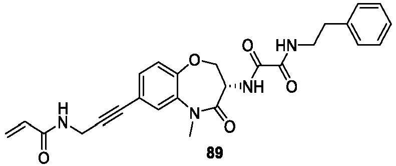

### Synthesis route:

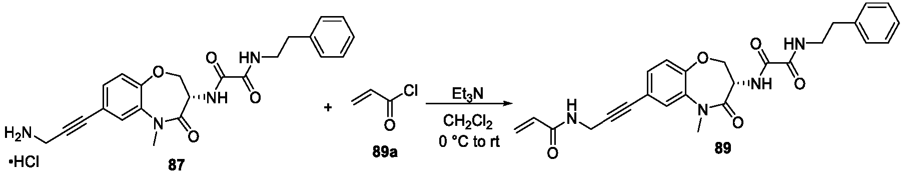

Acryloyl chloride (6 mg, 0.06 mmol) was added to a solution of compound **87** (23 mg, 0.05 mmol) and triethylamine (15 mg, 0.15 mmol) in dichloromethane (2 mL) at 0 °C in an ice bath, and the reaction solution was slowly warmed to room temperature and stirred at room temperature for another 3 hours. TLC and LC-MS showed that the reaction was complete, and water (5 mL) was added to quench the reaction, and the mixture was extracted with dichloromethane (5 mL × 3). The combined organic layers were washed with saturated brine (5 mL), dried over anhydrous sodium sulfate, and concentrated under reduced pressure to obtain a crude product, which was then purified by silica gel column chromatography (dichloromethane containing 5 - 10% methanol as the eluent) to obtain the title compound **89** (20 mg, 84% yield) as a white solid.

MS (ES⁺): *m*/*z* 475.2 [M+H]⁺.

¹H NMR (400 MHz, DMSO-*d*₆): *δ* 8.82 (t, *J* = 5.9 Hz, 1H), 8.76 (d, *J* = 7.4 Hz, 1H), 8.66 (t, *J* = 5.2 Hz, 1H), 7.55 (d, *J* = 1.5 Hz, 1H), 7.33 - 7.26 (m, 3H), 7.22 - 7.17 (m, 4H), 6.25 (dd, *J* = 17.1, 10.0 Hz, 1H), 6.14 (dd, *J* = 17.1, 2.1 Hz, 1H), 5.65 (dd, *J* = 10.0, 2.2 Hz, 1H), 4.68 - 4.57 (m, 2H), 4.41 - 4.33 (m, 1H), 4.22 (d, *J* = 5.5 Hz, 2H), 3.39 - 3.35 (m, 2H), 3.29 (s, 3H), 2.77 (t, *J* = 7.4 Hz, 2H).

### Example 90: Synthesis of (S)-N¹-(7-(azetidin-3-ylethynyl)-5-methyl-4-oxo-2,3,4,5-tetrahydrobenzo[b][1,4]oxazepin-3-yl)-N²-phenethyloxalamide (90)

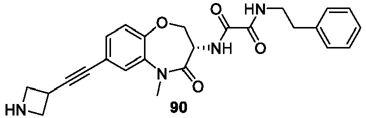

### Synthesis route:

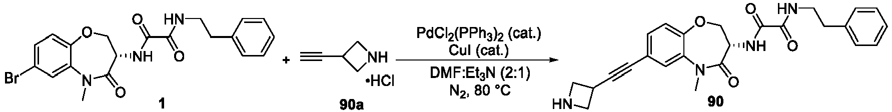

The synthesis route and method were the same as Example 2, except that cyclopropylacetylene **2a** was replaced with an equivalent amount of 3-ethynylazetidine hydrochloride **90a** to obtain the title compound **90.**

MS (ES⁺): *m*/*z* 447.2 [M+H]⁺.

¹H NMR (400 MHz, CDCl₃): *δ* 8.26 (d, *J* = 5.3 Hz, 1H), 7.34 - 7.28 (m, 5H), 7.24 - 7.20 (m, 1H), 7.17 (d, *J* = 7.3 Hz, 2H), 7.12 (d, *J* = 8.3 Hz, 1H), 4.84 - 4.78 (m, 1H), 4.61 - 4.56 (m, 1H), 4.30 - 4.19 (m, 5H), 4.01 - 3.93 (m, 1H), 3.59 - 3.53 (m, 2H), 3.41 (s, 3H), 2.84 (t, *J* = 7.0 Hz, 2H), 2.41 (br, 1H).

### Example 91: Synthesis of (S)-N¹-(7-((1-formylazetidin-3-yl)ethynyl)-5-methyl-4-oxo-2,3,4,5-tetrahydrobenzo[b][1,4]oxaze pin-3-yl)-N²-phenethyloxalamide (91)

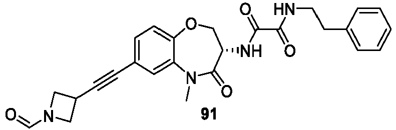

### Synthesis route:

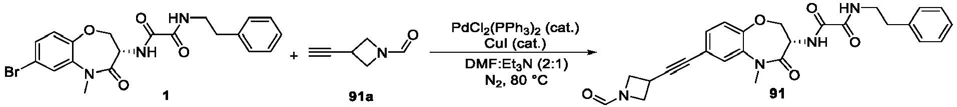

The synthesis route and method were the same as Example 2, except that cyclopropylacetylene **2a** was replaced with an equivalent amount of 3-ethynylazetidine-1-carbaldehyde **91a** to obtain the title compound **91.**

MS (ES⁺): *m*/*z* 475.2 [M+H]⁺.

¹H NMR (400 MHz, CDCl₃): *δ* 8.24 (d, *J* = 7.3 Hz, 1H), 8.02 (s, 1H), 7.32 - 7.28 (m, 5H), 7.25 - 7.21 (m, 1H), 7.18 (d, *J* = 7.1 Hz, 2H), 7.12 (d, *J* = 8.7 Hz, 1H), 4.81 (dt, *J* = 11.3, 7.4 Hz, 1H), 4.59 (dd, *J* = 9.7, 7.3 Hz, 1H), 4.48 (t, *J* = 8.5 Hz, 1H), 4.36 (t, *J* = 9.3 Hz, 1H), 4.27 - 4.22 (m, 2H), 4.14 - 4.10 (m, 1H), 3.77 - 3.70 (m, 1H), 3.61 - 3.52 (m, 2H), 3.41 (s, 3H), 2.84 (t, *J =* 7.1 Hz, 2H).

### Example 92: Synthesis of (S)-N¹-(7-((1-formyl-3-hydroxyazetidin-3-yl)ethynyl)-5-methyl-4-oxo-2,3,4,5-tetrahydrobenzo[b] [1,4]oxazepin-3-yl)-N²-phenethyloxalamide (92)

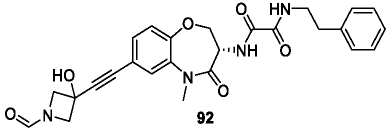

### Synthesis route:

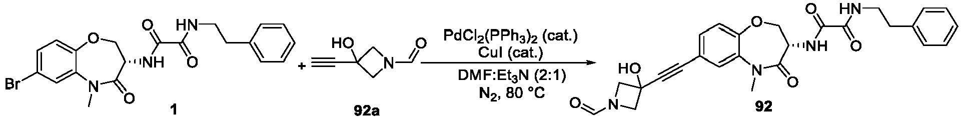

The synthesis route and method were the same as Example 2, except that cyclopropylacetylene **2a** was replaced with an equivalent amount of 3-ethynyl-3-hydroxyazetidine-1-carbaldehyde **92a** to obtain the title compound **92.**

MS (ES⁺): *m*/*z* 491.1 [M+H]⁺.

¹H NMR (400 MHz, CDCl₃): *δ* 8.24 (d, *J* = 7.3 Hz, 1H), 8.11 (s, 1H), 7.34 - 7.28 (m, 5H), 7.25 - 7.21 (m, 1H), 7.19 - 7.14 (m, 3H), 4.81 (dt, *J* = 11.3, 7.2 Hz, 1H), 4.59 (dd, *J* = 9.5, 7.5 Hz, 1H), 4.53 (d, *J* = 8.7 Hz, 1H), 4.42 (d, *J* = 10.5 Hz, 1H), 4.35 (d, *J* = 8.5 Hz, 1H), 4.25 (t, *J* = 10.6 Hz, 1H), 4.21 (d, *J* = 10.2 Hz, 1H), 3.59 - 3.52 (m, 2H), 3.42 (s, 3H), 2.84 (t, *J* = 7.0 Hz, 2H).2

### Example 93: Synthesis of (S)-N¹-(7-((1-acetyl-3-hydroxyazetidin-3-yl)ethynyl)-5-methyl-4-oxo-2,3,4,5-tetrahydrobenzo[b][ 1,4]oxazepin-3-yl)-N²-phenethyloxalamide (93)

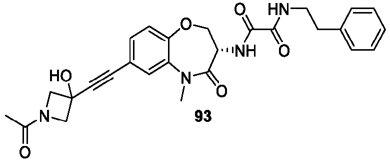

### Synthesis route:

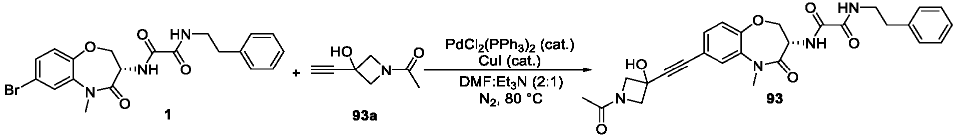

The synthesis route and method were the same as Example 2, except that cyclopropylacetylene **2a** was replaced with an equivalent amount of 1-(3-ethynyl-3-hydroxyazetidin-1-yl)ethan-1-one **93a** to obtain the title compound **93.**

MS (ES⁺): *m*/*z* 505.1 [M+H]⁺.

¹H NMR (400 MHz, CD₃OD): *δ* 7.54 (d, *J* = 1.6 Hz, 1H), 7.39 (dd, *J* = 8.3, 1.9 Hz, 1H), 7.29 - 7.25 (m, 3H), 7.22 - 7.18 (m, 5H), 4.84 - 4.78 (m, 1H), 4.60 - 4.50 (m, 3H), 4.41 (t, *J* = 10.7 Hz, 1H), 4.29 (d, *J* = 10.0 Hz, 1H), 4.05 (d, *J* = 10.3 Hz, 1H), 3.48 - 3.44 (m, 2H), 3.39 (s, 3H), 2.82 (t, *J* = 7.6 Hz, 2H), 1.92 (s, 3H).

### Example 94: Synthesis of ethyl (S)-5-(5-methyl-4-oxo-3-(2-oxo-2-(phenethylamino)acetamido)-2,3,4,5-tetrahydrobenzo[b][1,4]o xazepin-7-yl)pent-4-ynoate (94)

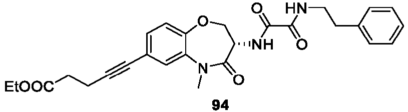

### Synthesis route:

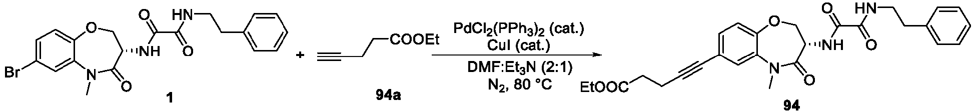

The synthesis route and method were the same as Example 2, except that cyclopropylacetylene **2a** was replaced with an equivalent amount of ethyl 4-pentynoate **94a** to obtain the title compound **94.**

MS (ES⁺): *m*/*z* 492.2 [M+H]⁺.

¹H NMR (400 MHz, CDCl₃): *δ* 8.24 (d, *J* = 7.4 Hz, 1H), 7.32 - 7.28 (m, 3H), 7.24 - 7.21 (m, 3H), 7.17 (d, *J* = 7.1 Hz, 2H), 7.08 (d, *J =* 8.8 Hz, 1H), 4.80 (dt, *J* = 11.3, 7.4 Hz, 1H), 4.58 (dd, *J* = 9.7, 7.4 Hz, 1H), 4.25 - 4.16 (m, 3H), 3.59 - 3.53 (m, 2H), 3.39 (s, 3H), 2.84 (t, *J* = 7.1 Hz, 2H), 2.73 (t, *J* = 7.0 Hz, 2H), 2.62 (t, *J* = 7.1 Hz, 2H), 1.28 (t, *J* = 7.1 Hz, 3H).

### Example 95: Synthesis of (S)-5-(5-methyl-4-oxo-3-(2-oxo-2-(phenethylamino)acetamido)-2,3,4,5-tetrahydrobenzo[b][1,4]o xazepin-7-yl)pent-4-ynoic acid (95)

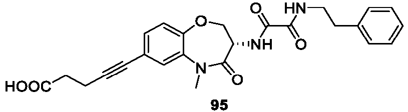

### Synthesis route:

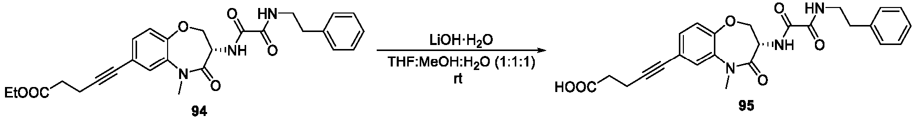

To a solution of compound **94** (59 mg, 0.12 mmol) in tetrahydrofuran : methanol : water (1:1:1, 6 mL) was added lithium hydroxide monohydrate (15 mg, 0.36 mmol) at room temperature, and the reaction solution was stirred at room temperature for 3 hours. TLC and LC-MS showed that the reaction was complete. The reaction mixture was poured into water (10 mL), acidified with dilute hydrochloric acid (1 N) to pH = 5, and then extracted with ethyl acetate (20 mL × 5). The combined organic layers were washed with saturated brine (50 mL), dried over anhydrous sodium sulfate, and concentrated under reduced pressure to obtain a crude product, which was then purified by silica gel column chromatography (dichloromethane containing 5 - 10% methanol as the eluent) to obtain the title compound **95** (26 mg, 47% yield) as a white solid.

MS (ES⁺): *m*/*z* 464.1 [M+H]⁺.

¹H NMR (400 MHz, DMSO-*d*₆): *δ* 12.31 (br, 1H), 8.82 (t, *J =* 5.9 Hz, 1H), 8.74 (d, *J* = 7.4 Hz, 1H), 7.49 (s, 1H), 7.30 - 7.26 (m, 3H), 7.21 - 7.16 (m, 4H), 4.67 - 4.56 (m, 2H), 4.35 (t, *J =* 7.2 Hz, 1H), 3.33 - 3.27 (m, 5H), 2.77 (t, *J* = 7.7 Hz, 2H), 2.67 - 2.59 (m, 4H).

### Example 96: Synthesis of methyl (S)-1-((5-methyl-4-oxo-3-(2-oxo-2-(phenethylamino)acetamido)-2,3,4,5-tetrahydrobenzo[b][1,4] oxazepin-7-yl)ethynyl)cyclopropane-1-carboxylate (96)

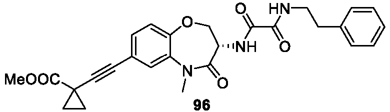

### Synthetic route:

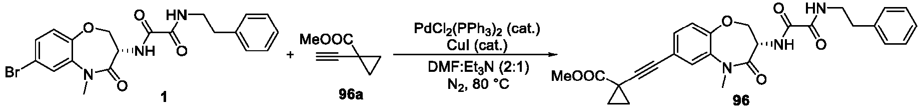

The synthetic route and method were the same as Example 2, except that cyclopropaneacetylene **2a** was replaced with an equivalent amount of methyl 1-ethynylcyclopropane-1-carboxylate **96a** to obtain the title compound **96.**

MS (ES⁺): *m*/*z* 490.2 [M+H]⁺.

¹H NMR (400 MHz, CDCl₃): *δ* 8.23 (d, *J* = 7.3 Hz, 1H), 7.32 - 7.28 (m, 5H), 7.24 - 7.21 (m, 1H), 7.18 (d, *J* = 7.2 Hz, 2H), 7.09 (d, *J* = 8.7 Hz, 1H), 4.79 (dt, *J* = 11.1, 7.3 Hz, 1H), 4.58 (dd, *J* = 9.5, 7.5 Hz, 1H), 4.23 (t, *J* = 10.5 Hz, 1H), 3.78 (s, 3H), 3.59 - 3.54 (m, 2H), 3.40 (s, 3H), 2.84 (t, *J* = 7.1 Hz, 2H), 1.68 - 1.63 (m, 2H), 1.45 - 1.42 (m, 2H).

### Example 97: Synthesis of (S)-N¹-(5-methyl-4-oxo-2,3,4,5-tetrahydrobenzo[b][1,4]oxazepin-3-yl)-N²-(2-(thiophen-2-yl)ethy l)oxalamide (97)

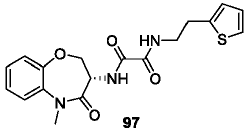

### Synthesis route:

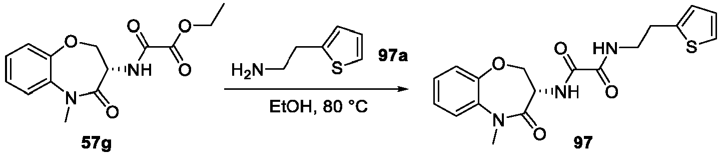

The synthesis route and method were the same as step 7 of Example 57, except that 2-phenylethylamine **1i** was replaced with an equivalent amount of 2-(2-thienyl)ethylamine **97a** to obtain the title compound **97.**

MS (ES⁺): *m*/*z* 374.1 [M+H]⁺.

¹H NMR (400 MHz, CDCl₃): *δ* 8.26 (d, *J* = 7.4 Hz, 1H), 7.39 (t, *J* = 6.4 Hz, 1H), 7.25 - 7.14 (m, 5H), 6.93 (dd, *J* = 5.1, 3.4 Hz, 1H), 6.83 - 6.82 (m, 1H), 4.83 (dt, *J* = 11.2, 7.5 Hz, 1H), 4.62 (dd, *J* = 9.7, 7.4 Hz, 1H), 4.23 (dd, *J =* 11.1, 9.8 Hz, 1H), 3.63 - 3.54 (m, 2H), 3.42 (s, 3H), 3.06 (t, *J* = 6.8 Hz, 2H).

### Example 98: Synthesis of (S)-N¹-(5-methyl-4-oxo-2,3,4,5-tetrahydrobenzo[b][1,4]oxazepin-3-yl)-N²-(2-(thiophen-3-yl)ethy l)oxalamide (98)

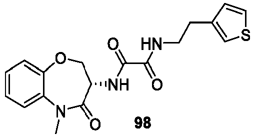

### Synthesis route:

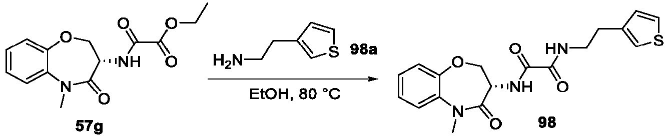

The synthesis route and method were the same as step 7 of Example 57, except that 2-phenylethylamine **1i** was replaced with an equivalent amount of 2-(3-thienyl)ethylamine **98a** to obtain the title compound **98.**

MS (ES⁺): *m*/*z* 374.1 [M+H]⁺.

¹H NMR (400 MHz, CDCl₃): *δ* 8.26 (d, *J* = 7.7 Hz, 1H), 7.32 - 7.17 (m, 6H), 7.01 - 6.99 (m, 1H), 6.93 (dd, *J* = 4.9, 1.2 Hz, 1H), 4.83 (dt, *J =* 11.2, 7.4 Hz, 1H), 4.61 (dd, *J* = 9.8, 7.4 Hz, 1H), 4.23 (dd, *J =* 11.1, 9.8 Hz, 1H), 3.59 - 3.53 (m, 2H), 3.42 (s, 3H), 2.88 (t, *J =* 7.0 Hz, 2H).

### Example 99: Synthesis of (S)-N¹-(2-fluorophenethyl)-N²-(5-methyl-4-oxo-2,3,4,5-tetrahydrobenzo[b][1,4]oxazepin-3-yl)ox alamide (99)

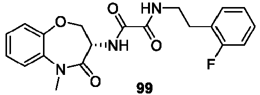

### Synthesis route:

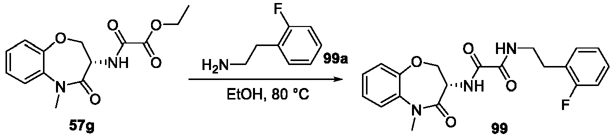

The synthesis route and method were the same as step 7 of Example 57, except that 2-phenylethylamine **1i** was replaced with an equivalent amount of 2-(2-fluorophenyl)ethylamine **99a** to obtain the title compound **99.**

MS (ES⁺): *m*/*z* 386.0 [M+H]⁺.

¹H NMR (400 MHz, CDCl₃): *δ* 8.25 (d, *J* = 6.9 Hz, 1H), 7.34 - 7.14 (m, 7H), 7.08 - 7.00 (m, 2H), 4.83 (dt, *J* = 11.2, 7.5 Hz, 1H), 4.61 (dd, *J* = 9.7, 7.5 Hz, 1H), 4.23 (dd, *J* = 11.0, 10.0 Hz, 1H), 3.59 - 3.54 (m, 2H), 3.42 (s, 3H), 2.89 (t, *J* = 7.0 Hz, 2H).

### Example 100: Synthesis of (S)-N¹-(3-fluorophenethyl)-N²-(5-methyl-4-oxo-2,3,4,5-tetrahydrobenzo[b][1,4]oxazepin-3-yl)ox alamide (100)

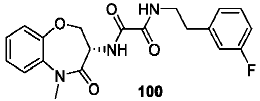

### Synthesis route:

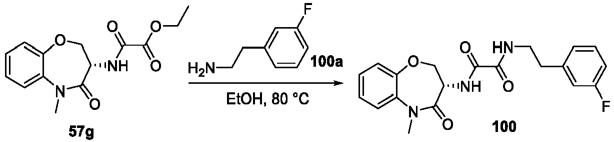

The synthesis route and method were the same as step 7 of Example 57, except that 2-phenylethylamine **1i** was replaced with an equivalent amount of 2-(3-fluorophenyl)ethylamine **100a** to obtain the title compound **100.**

MS (ES⁺): *m*/*z* 386.0 [M+H]⁺.

¹H NMR (400 MHz, CDCl₃): *δ* 8.25 (d, *J =* 7.4 Hz, 1H), 7.31 - 7.16 (m, 6H), 6.96 - 6.87 (m, 3H), 4.83 (dt, *J* = 11.2, 7.5 Hz, 1H), 4.61 (dd, *J* = 9.7, 7.5 Hz, 1H), 4.23 (t, *J* = 10.5 Hz, 1H), 3.59 - 3.53 (m, 2H), 3.42 (s, 3H), 2.84 (t, *J* = 7.1 Hz, 2H).

### Example 101: Synthesis of (S)-N¹-(3-chlorophenethyl)-N²-(5-methyl-4-oxo-2,3,4,5-tetrahydrobenzo[b][1,4]oxazepin-3-yl)ox alamide (101)

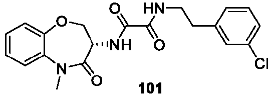

### Synthesis route:

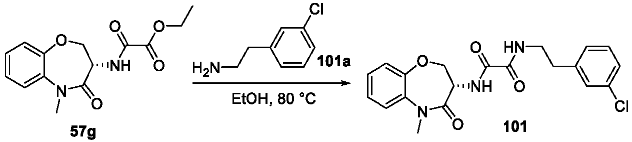

The synthesis route and method were the same as step 7 of Example 57, except that 2-phenylethylamine **1i** was replaced with an equivalent amount of 2-(3-chlorophenyl)ethylamine **101a** to obtain the title compound **101.**

MS (ES⁺): *m*/*z* 402.0 [M+H]⁺.

¹H NMR (400 MHz, CDCl₃): *δ* 8.25 (d, *J* = 7.7 Hz, 1H), 7.30 - 7.18 (m, 8H), 7.06 (d, *J* = 6.3 Hz, 1H), 4.83 (dt, *J =* 11.1, 7.5 Hz, 1H), 4.62 (dd, *J* = 9.7, 7.4 Hz, 1H), 4.23 (t, *J* = 10.5 Hz, 1H), 3.58 - 3.52 (m, 2H), 3.42 (s, 3H), 2.82 (t, *J* = 7.2 Hz, 2H).

### Example 102: Synthesis of (S)-N¹-(3,5-difluorobenzyl)-N²-(5-methyl-4-oxo-2,3,4,5-tetrahydrobenzo[b][1,4]oxazepin-3-yl)ox alamide (102)

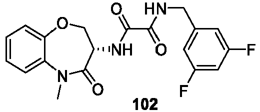

### Synthesis route:

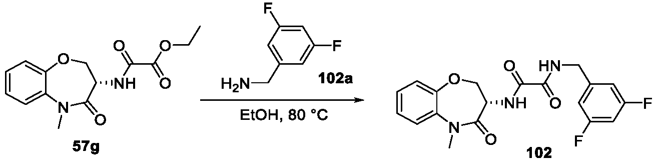

The synthesis route and method were the same as step 7 of Example 57, except that 2-phenylethylamine **1i** was replaced with an equivalent amount of 3,5-difluorobenzylamine **102a** to obtain the title compound **102.**

MS (ES⁺): *m*/*z* 390.0 [M+H]⁺.

¹H NMR (400 MHz, DMSO-*d*₆): *δ* 9.39 (t, *J* = 6.4 Hz, 1H), 8.80 (d, *J* = 7.7 Hz, 1H), 7.49 (dd, *J* = 7.5, 1.9 Hz, 1H), 7.34 - 7.22 (m, 3H), 7.13 - 7.07 (m, 1H), 6.98 - 6.93 (m, 2H), 4.69 - 4.56 (m, 2H), 4.39 - 4.32 (m, 3H), 3.30 (s, 3H).

### Example 103: Synthesis of (S)-N¹-(5-methyl-4-oxo-2,3,4,5-tetrahydrobenzo[b][1,4]oxazepin-3-yl)-N²-(2-phenoxyethyl)oxala mide (103)

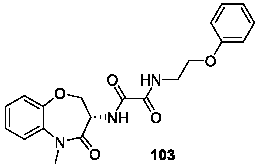

### Synthesis route:

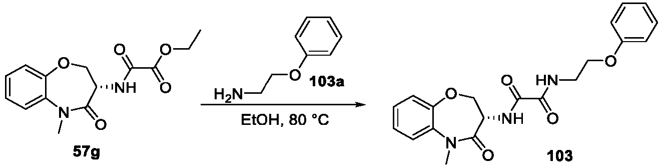

The synthesis route and method were the same as step 7 of Example 57, except that 2-phenylethylamine **1i** was replaced with an equivalent amount of 2-phenoxyethylamine **103a** to obtain the title compound **103.**

MS (ES⁺): *m*/*z* 384.0 [M+H]⁺.

¹H NMR (400 MHz, CDCl₃): *δ* 8.27 (d, *J* = 7.2 Hz, 1H), 7.66 (t, *J* = 6.3 Hz, 1H), 7.30 - 7.17 (m, 6H), 6.96 (t, *J =* 7.3 Hz, 1H), 6.87 (d, *J =* 8.2 Hz, 2H), 4.84 (dt, *J =* 11.2, 7.4 Hz, 1H), 4.63 (dd, *J* = 9.7, 7.4 Hz, 1H), 4.23 (t, *J =* 10.5 Hz, 1H), 4.06 (t, *J =* 5.2 Hz, 2H), 3.77 - 3.67 (m, 2H), 3.42 (s, 3H).

### Example 104: Synthesis of (S)-N¹-(7-(3-(1H-imidazol-1-yl)prop-1-yn-1-yl)-5-methyl-4-oxo-2,3,4,5-tetrahydrobenzo[b][1,4] oxazepin-3-yl)-N²-benzyloxalamide (104)

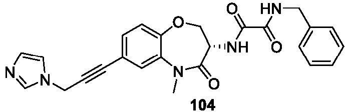

### Synthesis route:

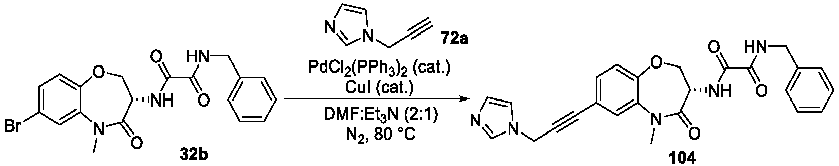

The synthesis route and method were the same as step 2 of Example 32, except that 3-butyn-1-ol **31a** was replaced with an equivalent amount of 1-(prop-2-yn-1-yl)-1H-imidazole **72a** to obtain the title compound **104.**

MS (ES⁺): *m*/*z* 458.2 [M+H]⁺.

¹H NMR (400 MHz, CDCl₃): *δ* 8.29 (d, *J* = 7.4 Hz, 1H), 7.72 (s, 1H), 7.55 (t, *J* = 5.8 Hz, 1H), 7.35 - 7.26 (m, 8H), 7.14 (d, *J* = 8.3 Hz, 2H), 4.97 (s, 2H), 4.82 (dt, *J* = 11.4, 7.5 Hz, 1H), 4.60 (dd, *J* = 9.6, 7.5 Hz, 1H), 4.48 (d, *J* = 6.2 Hz, 2H), 4.26 (t, *J* = 10.5 Hz, 1H), 3.41 (s, 3H).

### Example 105: Synthesis of (S)-N¹-benzyl-N²-(7-(3-(4-hydroxypiperidin-1-yl)prop-1-yn-1-yl)-5-methyl-4-oxo-2,3,4,5-tetrahy drobenzo[b][1,4]oxazepin-3-yl)oxalamide (105)

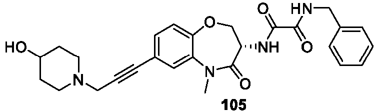

### Synthesis route:

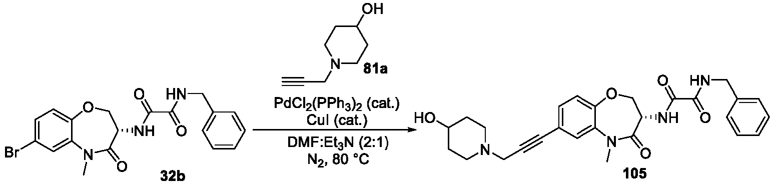

The synthesis route and method were the same as step 2 of Example 32, except that 3-butyn-1-ol **31a** was replaced with an equivalent amount of 1-(prop-2-yn-1-yl)piperidin-4-ol **81a** to obtain the title compound **105.**

MS (ES⁺): *m*/*z* 491.3 [M+H]⁺.

¹H NMR (400 MHz, CDCl₃): *δ* 8.28 (d, *J* = 7.5 Hz, 1H), 7.54 (t, *J* = 5.8 Hz, 1H), 7.36 - 7.25 (m, 7H), 7.11 (d, *J =* 8.9 Hz, 1H), 4.82 (dt, *J* = 11.3, 7.4 Hz, 1H), 4.60 (dd, *J* = 9.7, 7.3 Hz, 1H), 4.48 (d, *J* = 6.3 Hz, 2H), 4.25 (dd, *J* = 11.1, 9.9 Hz, 1H), 3.76 (br, 1H), 3.52 (s, 2H), 3.41 (s, 3H), 2.92 - 2.87 (m, 2H), 2.47 - 2.40 (m, 2H), 2.00 - 1.95 (m, 2H), 1.72 - 1.63 (m, 2H), 1.42 (br, 1H).

### Example 106: Synthesis of (S)-N¹-benzyl-N²-(5-methyl-7-(3-morpholinoprop-1-yn-1-yl)-4-oxo-2,3,4,5-tetrahydrobenzo[b][1 ,4]oxazepin-3-yl)oxalamide (106)

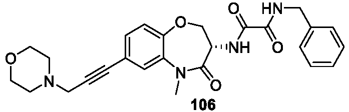

### Synthesis route:

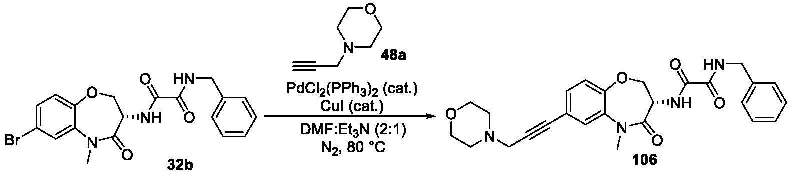

The synthesis route and method were the same as step 2 of Example 32, except that 3-butyn-1-ol 31a was replaced with an equivalent amount of 4-(prop-2-yn-1-yl)morpholine **48a** to obtain the title compound **106.**

MS (ES⁺): *m*/*z* 477.2 [M+H]⁺.

¹H NMR (400 MHz, CDCl₃): *δ* 8.29 (d, *J* = 7.4 Hz, 1H), 7.55 (t, *J* = 5.9 Hz, 1H), 7.36 - 7.25 (m, 7H), 7.11 (d, *J =* 8.9 Hz, 1H), 4.82 (dt, *J* = 11.4, 7.3 Hz, 1H), 4.60 (dd, *J* = 9.7, 7.4 Hz, 1H), 4.48 (d, *J =* 6.2 Hz, 2H), 4.25 (dd, *J =* 11.0, 10.0 Hz, 1H), 3.78 (t, *J* = 4.6 Hz, 4H), 3.50 (s, 2H), 3.41 (s, 3H), 2.64 (t, *J* = 4.5 Hz, 4H).

### Example 107: Synthesis of (S)-N¹-benzyl-N²-(5-methyl-4-oxo-7-(3-(3-oxopiperazin-1-yl)prop-1-yn-1-yl)-2,3,4,5-tetrahydrob enzo[b][1,4]oxazepin-3-yl)oxalamide (107)

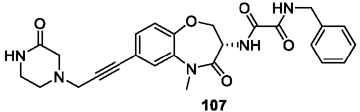

### Synthesis route:

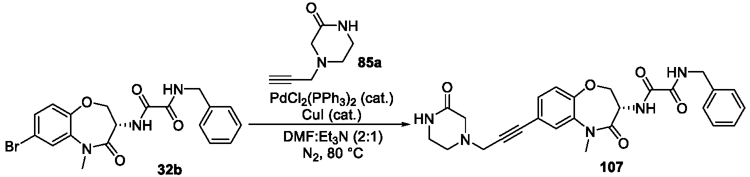

The synthesis route and method were the same as step 2 of Example 32, except that 3-butyn-1-ol **31a** was replaced with an equivalent amount of 4-(prop-2-yn-1-yl)piperazin-2-one 85a to obtain the title compound **107.**

MS (ES⁺): *m*/*z* 490.3 [M+H]⁺.

¹H NMR (400 MHz, CDCl₃): *δ* 8.29 (d, *J* = 7.5 Hz, 1H), 7.55 (t, *J* = 6.0 Hz, 1H), 7.36 - 7.25 (m, 7H), 7.12 (d, *J* = 8.9 Hz, 1H), 5.88 (s, 1H), 4.83 (dt, *J* = 11.2, 7.4 Hz, 1H), 4.60 (dd, *J* = 9.7, 7.4 Hz, 1H), 4.48 (d, *J* = 6.2 Hz, 2H), 4.26 (t, *J* = 10.5 Hz, 1H), 3.63 (s, 2H), 3.46 - 3.43 (m, 2H), 3.42 (s, 3H), 3.36 (s, 2H), 2.83 (t, *J* = 5.4 Hz, 2H).

### Example 108: Synthesis of (S)-N¹-(5-methyl-4-oxo-7-(3-(3-oxopiperazin-1-yl)prop-1-yn-1-yl)-2,3,4,5-tetrahydrobenzo[b][1, 4]oxazepin-3-yl)-N²-(2-methylbenzyl)oxalamide (108)

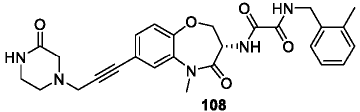

### Synthesis route:

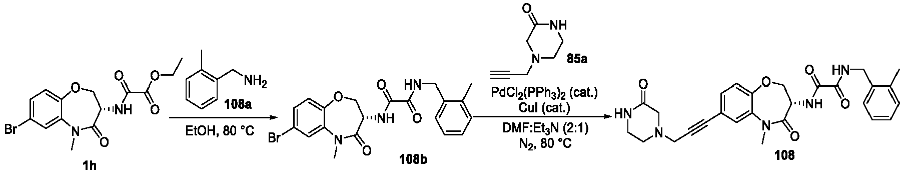

The synthesis route and method were the same as Example 32, except that the benzylamine **32a** in step 1 and the 3-butyn-1-ol **31a** in step 2 were replaced with equivalent amounts of 2-methylbenzylamine **108a** and 4-(prop-2-yn-1-yl)piperazin-2-one **85a,** respectively, to obtain the title compound **108.**

MS (ES⁺): *m*/*z* 504.3 [M+H]⁺.

¹H NMR (400 MHz, CDCl₃): *δ* 8.29 (d, *J* = 7.5 Hz, 1H), 7.38 (t, *J* = 5.7 Hz, 1H), 7.30 - 7.28 (m, 2H), 7.22 - 7.17 (m, 4H), 7.12 (d, *J* = 8.9 Hz, 1H), 5.89 (s, 1H), 4.82 (dt, *J* = 11.2, 7.4 Hz, 1H), 4.60 (dd, *J* = 9.7, 7.3 Hz, 1H), 4.48 (d, *J* = 5.9 Hz, 2H), 4.26 (t, *J* = 10.5 Hz, 1H), 3.63 (s, 2H), 3.45 - 3.43 (m, 2H), 3.42 (s, 3H), 3.36 (s, 2H), 2.83 (t, *J* = 5.4 Hz, 2H), 2.31 (s, 3H).

### Example 109: Synthesis of (R)-N¹-(3-fluorophenethyl)-N²-(7-(4-hydroxybut-1-yn-1-yl)-5-methyl-4-oxo-2,3,4,5-tetrahydrobe nzo[b][1,4]oxazepin-3-yl)oxalamide (109)

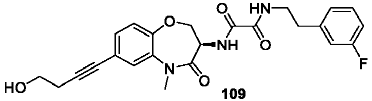

### Synthesis route:

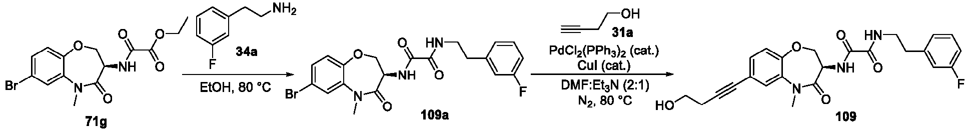

The synthesis route and method were the same as Example 34, except that compound **1h** in step 1 was replaced with an equivalent amount of compound **71g** to obtain the title compound **109.**

MS (ES⁺): *m*/*z* 454.2 [M+H]⁺.

¹H NMR (400 MHz, CDCl₃): *δ* 8.24 (d, *J =* 7.4 Hz, 1H), 7.33 - 7.23 (m, 4H), 7.09 (d, *J* = 8.7 Hz, 1H), 6.96 - 6.87 (m, 3H), 4.80 (dt, *J =* 11.2, 7.4 Hz, 1H), 4.58 (dd, *J* = 9.7, 7.4 Hz, 1H), 4.23 (dd, *J =* 11.0, 10.0 Hz, 1H), 3.83 (q, *J =* 6.2 Hz, 2H), 3.58 - 3.53 (m, 2H), 3.40 (s, 3H), 2.84 (t, *J* = 7.1 Hz, 2H), 2.70 (t, *J* = 6.3 Hz, 2H), 1.85 (t, *J* = 6.2 Hz, 1H).

### Example 110: Synthesis of (S)-N¹-(3-fluorophenethyl)-N²-(7-((3-hydroxyoxetan-3-yl)ethynyl)-5-methyl-4-oxo-2,3,4,5-tetrah ydrobenzo[b][1,4]oxazepin-3-yl)oxalamide (110)

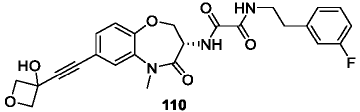

### Synthesis route:

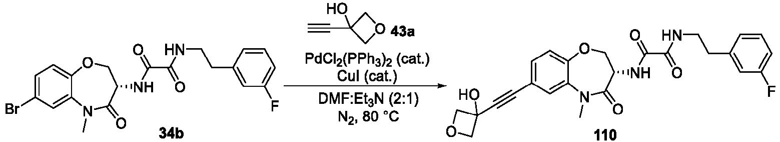

The synthesis route and method were the same as step 2 of Example 34, except that 3-butyn-1-ol **31a** was replaced with an equivalent amount of 3-ethynyloxetan-3-ol **43a** to obtain the title compound **110.**

MS (ES⁻): *m*/*z* 480.0 [M-H]⁻.

¹H NMR (400 MHz, CDCl₃): *δ* 8.24 (d, *J* = 7.3 Hz, 1H), 7.33 - 7.24 (m, 4H), 7.15 (d, *J* = 8.8 Hz, 1H), 6.94 - 6.87 (m, 3H), 4.95 - 4.92 (m, 2H), 4.83 - 4.79 (m, 3H), 4.60 (dd, *J* = 9.8, 7.3 Hz, 1H), 4.26 (t, *J* = 10.6 Hz, 1H), 3.59 - 3.51 (m, 2H), 3.42 (s, 3H), 2.84 (t, *J* = 6.9 Hz, 2H), 2.58 (s, 1H).

### Example 111: Synthesis of (S)-N¹-(5-(cyclopropylmethyl)-7-((3-hydroxyoxetan-3-yl)ethynyl)-4-oxo-2,3,4,5-tetrahydrobenzo [b][1,4]oxazepin-3-yl)-N²-(3-fluorophenethyl)oxalamide (111)

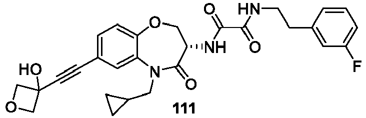

### Synthesis route:

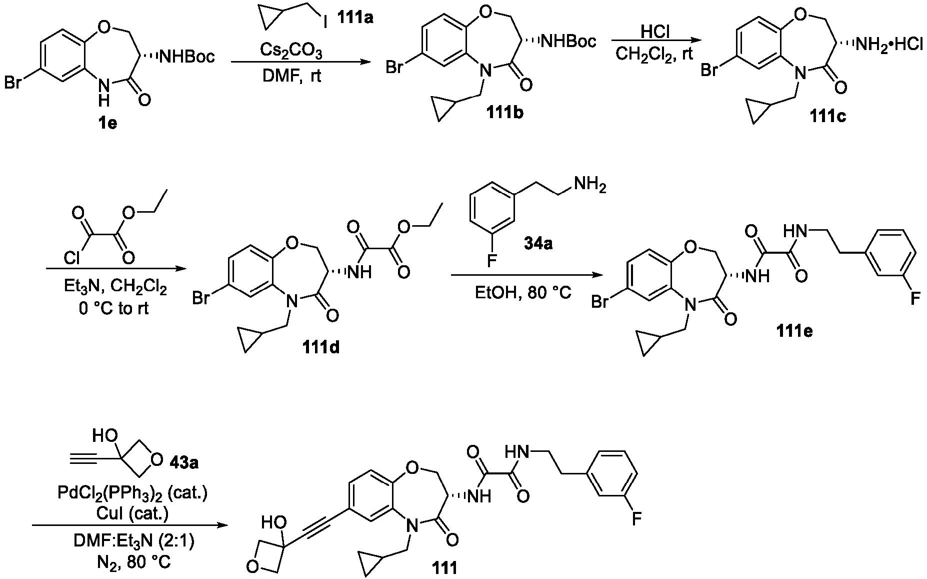

The synthesis route and method of steps 1-3 were the same as steps 4-6 of Example 1, except that iodomethane in step 4 of Example 1 was replaced with an equivalent amount of (iodomethyl)cyclopropane **111a;** and the synthesis route and method of steps 4-5 were the same as Example 34, except that compound **1h** in step 1 of Example 34 and 3-butyn-1-ol **31a** in step 2 of Example 34 were replaced with equivalent amounts of compound **111d** and 3-ethynyloxetan-3-ol **43a,** respectively, to obtain the title compound **111.**

MS (ES⁻): *m*/*z* 520.2 [M-H]⁻.

¹H NMR (400 MHz, CDCl₃): *δ* 8.25 (d, *J =* 7.4 Hz, 1H), 7.36 - 7.23 (m, 4H), 7.16 (d, *J* = 8.2 Hz, 1H), 6.96 - 6.87 (m, 3H), 4.94 (d, *J =* 6.6 Hz, 2H), 4.83 - 4.76 (m, 3H), 4.58 (dd, *J =* 9.6, 7.4 Hz, 1H), 4.26 (t, *J* = 10.5 Hz, 1H), 4.11 - 4.05 (m, 1H), 3.59 - 3.51 (m, 2H), 3.48 - 3.43 (m, 1H), 2.84 (t, *J =* 7.1 Hz, 2H), 2.73 (s, 1H), 1.07 - 0.99 (m, 1H), 0.49 - 0.41 (m, 2H), 0.31 - 0.26 (m, 1H), 0.18 - 0.12 (m, 1H).

### Example 112: Synthesis of (S)-N¹-(3-fluorophenethyl)-N²-(7-((1-hydroxycyclohexyl)ethynyl)-5-methyl-4-oxo-2,3,4,5-tetrah ydrobenzo[b][1,4]oxazepin-3-yl)oxalamide (112)

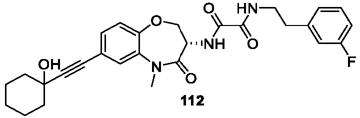

### Synthesis route:

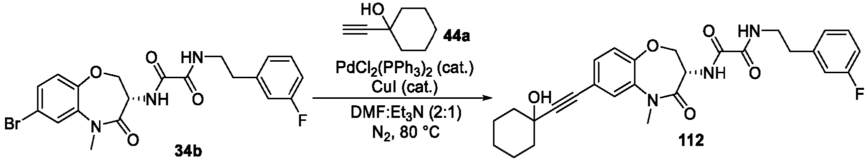

The synthesis route and method were the same as step 2 of Example 34, except that 3-butyn-1-ol **31a** was replaced with an equivalent amount of 1-ethynyl-1-cyclohexanol **44a** to obtain the title compound **112.**

MS (ES⁺): *m*/*z* 508.2 [M+H]⁺.

¹H NMR (400 MHz, CDCl₃): *δ* 8.23 (d, *J* = 7.5 Hz, 1H), 7.30 - 7.23 (m, 4H), 7.11 (d, *J* = 8.0 Hz, 1H), 6.96 - 6.87 (m, 3H), 4.80 (dt, *J* = 11.3, 7.4 Hz, 1H), 4.59 (dd, *J* = 9.6, 7.5 Hz, 1H), 4.24 (t, *J* = 10.5 Hz, 1H), 3.59 - 3.52 (m, 2H), 3.41 (s, 3H), 2.84 (t, *J* = 7.1 Hz, 2H), 2.04 - 2.00 (m, 3H), 1.79 - 1.59 (m, 8H).

### Example 113: Synthesis of N¹-(3-fluorophenethyl)-N²-((3S)-7-(3-(3-hydroxypyrrolidin-1-yl)prop-1-yn-1-yl)-5-methyl-4-oxo -2,3,4,5-tetrahydrobenzo[b][1,4]oxazepin-3-yl)oxalamide (113)

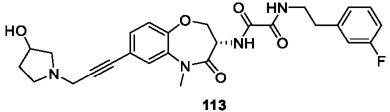

### Synthesis route:

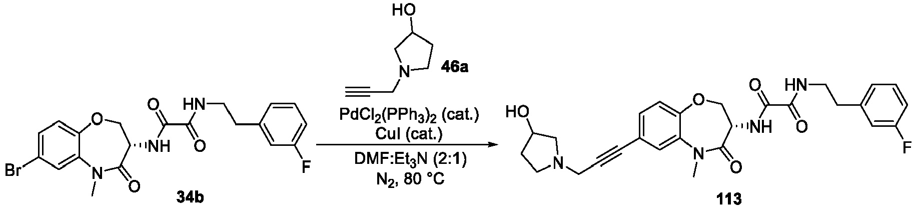

The synthesis route and method were the same as step 2 of Example 34, except that 3-butyn-1-ol **31a** was replaced with an equivalent amount of 1-(prop-2-yn-1-yl)pyrrolidin-3-ol **46a** to obtain the title compound **113.**

MS (ES⁺): *m*/*z* 509.2 [M+H]⁺.

¹H NMR (400 MHz, CDCl₃): *δ* 8.23 (d, *J* = 7.4 Hz, 1H), 7.31 - 7.23 (m, 4H), 7.10 (d, *J* = 8.8 Hz, 1H), 6.96 - 6.87 (m, 3H), 4.80 (dt, *J* = 11.2, 7.4 Hz, 1H), 4.58 (dd, *J* = 9.7, 7.4 Hz, 1H), 4.43 - 4.39 (m, 1H), 4.24 (t, *J* = 10.5 Hz, 1H), 3.65 (s, 2H), 3.58 - 3.53 (m, 2H), 3.40 (s, 3H), 3.03 - 2.97 (m, 1H), 2.84 (t, *J* = 7.1 Hz, 2H), 2.79 (d, *J* = 3.6 Hz, 2H), 2.60 - 2.54 (m, 1H), 2.32 - 2.20 (m, 1H), 1.85 - 1.77 (m, 1H).

### Example 114: Synthesis of N¹-(3-fluorophenethyl)-N²-((S)-7-(3-((R)-3-hydroxypyrrolidin-1-yl)prop-1-yn-1-yl)-5-methyl-4-o xo-2,3,4,5-tetrahydrobenzo[b][1,4]oxazepin-3-yl)oxalamide (114)

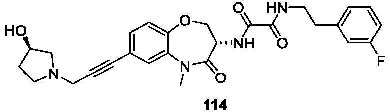

### Synthesis route:

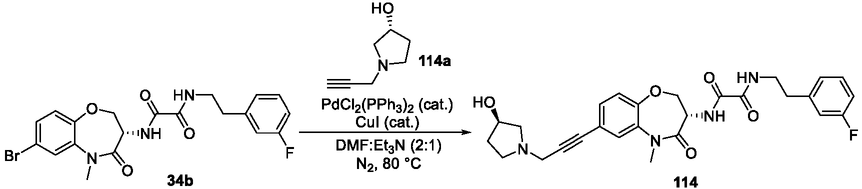

The synthesis route and method were the same as step 2 of Example 34, except that 3-butyn-1-ol **31a** was replaced with an equivalent amount of (*R*)-1-(prop-2-yn-1-yl)pyrrolidin-3-ol **114a** to obtain the title compound **114.**

MS (ES⁺): *m*/*z* 509.2 [M+H]⁺.

¹H NMR (400 MHz, CDCl₃): *δ* 8.23 (d, *J* = 7.4 Hz, 1H), 7.31 - 7.23 (m, 4H), 7.10 (d, *J* = 8.9 Hz, 1H), 6.96 - 6.87 (m, 3H), 4.80 (dt, *J* = 11.3, 7.4 Hz, 1H), 4.58 (dd, *J* = 9.7, 7.4 Hz, 1H), 4.43 - 4.39 (m, 1H), 4.24 (t, *J* = 10.5 Hz, 1H), 3.65 (s, 2H), 3.59 - 3.53 (m, 2H), 3.40 (s, 3H), 3.03 - 2.97 (m, 1H), 2.84 (t, *J* = 7.1 Hz, 2H), 2.80 (d, *J* = 3.7 Hz, 2H), 2.61 - 2.55 (m, 1H), 2.31 - 2.20 (m, 1H), 1.85 - 1.77 (m, 1H).

### Example 115: Synthesis of N¹-(3-fluorophenethyl)-N²-((S)-7-(3-((S)-3-hydroxypyrrolidin-1-yl)prop-1-yn-1-yl)-5-methyl-4-o xo-2,3,4,5-tetrahydrobenzo[b][1,4]oxazepin-3-yl)oxalamide (115)

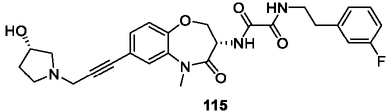

### Synthesis route:

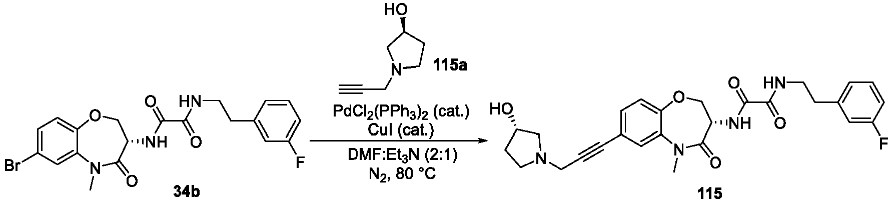

The synthesis route and method were the same as step 2 of Example 34, except that 3-butyn-1-ol **31a** was replaced with an equivalent amount of (S)-1-(prop-2-yn-1-yl)pyrrolidin-3-ol **115a** to obtain the title compound **115.**

MS (ES⁺): *m*/*z* 509.2 [M+H]⁺.

¹H NMR (400 MHz, CDCl₃): *δ* 8.23 (d, *J* = 7.5 Hz, 1H), 7.31 - 7.23 (m, 4H), 7.10 (d, *J* = 8.9 Hz, 1H), 6.96 - 6.87 (m, 3H), 4.81 (dt, *J* = 11.3, 7.4 Hz, 1H), 4.58 (dd, *J* = 9.7, 7.4 Hz, 1H), 4.43 - 4.39 (m, 1H), 4.24 (t, *J* = 10.5 Hz, 1H), 3.65 (s, 2H), 3.59 - 3.53 (m, 2H), 3.40 (s, 3H), 3.02 - 2.97 (m, 1H), 2.84 (t, *J* = 7.1 Hz, 2H), 2.79 (d, *J* = 3.7 Hz, 2H), 2.61 - 2.55 (m, 1H), 2.30 - 2.20 (m, 1H), 1.85 - 1.77 (m, 1H).

### Example 116: Synthesis of N¹-(3-fluorophenethyl)-N²-((3S)-5-methyl-4-oxo-7-(piperidin-3-ylethynyl)-2,3,4,5-tetrahydroben zo[b][1,4]oxazepin-3-yl)oxalamide (116)

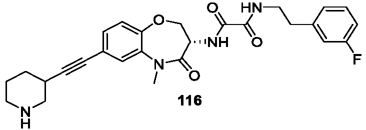

### Synthesis route:

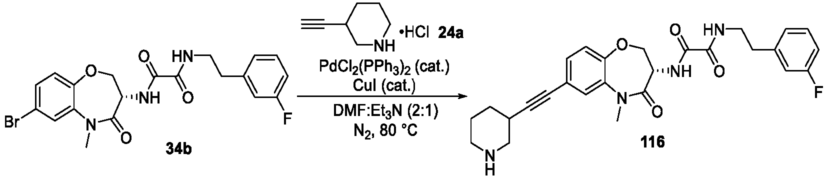

The synthesis route and method were the same as step 2 of Example 34, except that 3-butyn-1-ol **31a** was replaced with an equivalent amount of 3-ethynylpiperidine hydrochloride **24a** to obtain the title compound **116.**

MS (ES⁺): *m*/*z* 493.4 [M+H]⁺.

¹H NMR (400 MHz, CDCl₃): *δ* 8.24 (d, *J* = 7.2 Hz, 1H), 7.39 - 7.34 (m, 2H), 7.29 - 7.22 (m, 2H), 7.09 (d, *J* = 8.2 Hz, 1H), 6.96 - 6.87 (m, 3H), 4.79 (dt, *J* = 11.0, 7.3 Hz, 1H), 4.58 (dd, *J* = 9.5, 7.5 Hz, 1H), 4.24 (t, *J* = 10.5 Hz, 1H), 3.58 - 3.53 (m, 3H), 3.41 (s, 3H), 3.36 - 3.33 (m, 1H), 3.28 - 3.22 (m, 1H), 3.04 (t, *J* = 9.9 Hz, 2H), 2.84 (t, *J* = 7.0 Hz, 2H), 2.15 - 2.10 (m, 1H), 2.05 - 1.96 (m, 2H), 1.79 - 1.70 (m, 1H).

### Example 117: Synthesis of (S)-N¹-(3-fluorophenethyl)-N²-(5-methyl-4-oxo-7-(pyridin-3-ylethynyl)-2,3,4,5-tetrahydrobenzo[ b][1,4]oxazepin-3-yl)oxalamide (117)

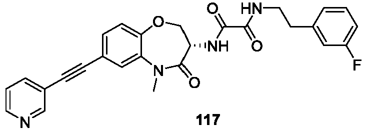

### Synthesis route:

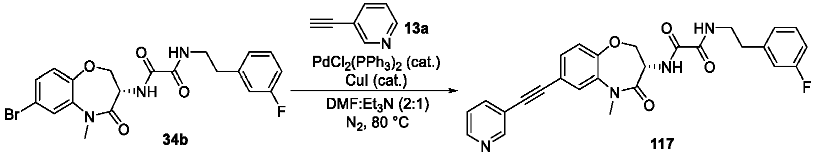

The synthesis route and method were the same as step 2 of Example 34, except that 3-butyn-1-ol **31a** was replaced with an equivalent amount of 3-ethynylpyridine **13a** to obtain the title compound **117.**

MS (ES⁺): *m*/*z* 487.2 [M+H]⁺.

¹H NMR (400 MHz, CDCl₃): *δ* 8.80 (br, 1H), 8.60 (br, 1H), 8.25 (d, *J* = 7.3 Hz, 1H), 7.82 (d, *J* = 7.3 Hz, 1H), 7.42 - 7.40 (m, 2H), 7.33 - 7.29 (m, 2H), 7.27 - 7.25 (m, 1H), 7.18 (d, *J* = 8.8 Hz, 1H), 6.96 - 6.87 (m, 3H), 4.84 (dt, *J* = 11.2, 7.4 Hz, 1H), 4.62 (dd, *J* = 9.7, 7.4 Hz, 1H), 4.27 (t, *J* = 10.5 Hz, 1H), 3.59 - 3.54 (m, 2H), 3.45 (s, 3H), 2.84 (t, *J* = 7.1 Hz, 2H).

### Example 118: Synthesis of (S)-N¹-(3-fluorophenethyl)-N²-(5-methyl-4-oxo-7-(3-(4-oxopiperidin-1-yl)prop-1-yn-1-yl)-2,3,4, 5-tetrahydrobenzo[b][1,4]oxazepin-3-yl)oxalamide (118)

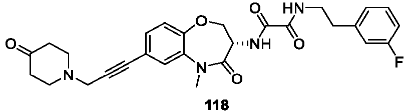

### Synthesis route:

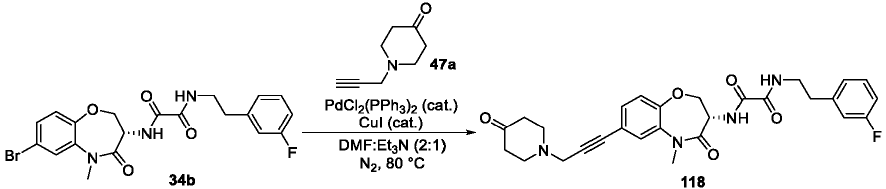

The synthesis route and method were the same as step 2 of Example 34, except that 3-butyn-1-ol **31a** was replaced with an equivalent amount of 1-(prop-2-yn-1-yl)piperidin-4-one **47a** to obtain the title compound **118.**

MS (ES⁺): *m*/*z* 521.1 [M+H]⁺.

¹H NMR (400 MHz, CDCl₃): *δ* 8.23 (d, *J* = 7.5 Hz, 1H), 7.32 - 7.23 (m, 4H), 7.11 (d, *J* = 8.9 Hz, 1H), 6.96 - 6.87 (m, 3H), 4.81 (dt, *J* = 11.3, 7.4 Hz, 1H), 4.58 (dd, *J* = 9.8, 7.3 Hz, 1H), 4.24 (dd, *J* = 11.2, 9.9 Hz, 1H), 3.65 (s, 2H), 3.59 - 3.53 (m, 2H), 3.41 (s, 3H), 2.94 (t, *J* = 6.1 Hz, 4H), 2.84 (t, *J* = 7.1 Hz, 2H), 2.54 (t, *J* = 6.1 Hz, 4H).

### Example 119: Synthesis of (S)-N¹-(3-fluorophenethyl)-N²-(5-methyl-4-oxo-7-(3-(3-oxopyrrolidin-1-yl)prop-1-yn-1-yl)-2,3,4, 5-tetrahydrobenzo[b][1,4]oxazepin-3-yl)oxalamide (119)

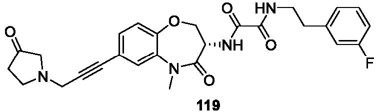

### Synthesis route:

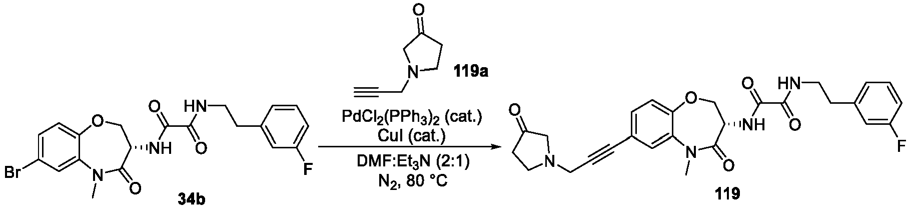

The synthesis route and method were the same as step 2 of Example 34, except that 3-butyn-1-ol **31a** was replaced with an equivalent amount of 1-(prop-2-yn-1-yl)pyrrolidin-3-one **119a** to obtain the title compound **119.**

MS (ES⁺): *m*/*z* 507.1 [M+H]⁺.

¹H NMR (400 MHz, CDCl₃): *δ* 8.23 (d, *J* = 7.4 Hz, 1H), 7.30 - 7.23 (m, 4H), 7.12 (d, *J* = 8.4 Hz, 1H), 6.96 - 6.87 (m, 3H), 4.81 (dt, *J* = 11.3, 7.4 Hz, 1H), 4.59 (dd, *J* = 9.7, 7.4 Hz, 1H), 4.24 (t, *J* = 10.5 Hz, 1H), 3.76 (s, 2H), 3.59 - 3.53 (m, 2H), 3.41 (s, 3H), 3.16 (s, 2H), 3.10 (t, *J*= 7.0 Hz, 2H), 2.84 (t, *J* = 7.1 Hz, 2H), 2.48 (t, *J* = 7.0 Hz, 2H).

### Example 120: Synthesis of (S)-N¹-(3-fluorophenethyl)-N²-(5-methyl-7-(3-morpholinoprop-1-yn-1-yl)-4-oxo-2,3,4,5-tetrahyd robenzo[b][1,4]oxazepin-3-yl)oxalamide (120)

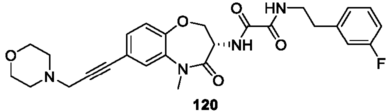

### Synthesis route:

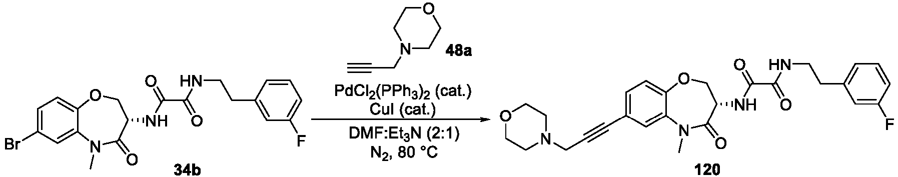

The synthesis route and method were the same as step 2 of Example 34, except that 3-butyn-1-ol **31a** was replaced with an equivalent amount of 4-(prop-2-yn-1-yl)morpholine **48a** to obtain the title compound **120.**

MS (ES⁺): *m*/*z* 509.0 [M+H]⁺.

¹H NMR (400 MHz, CDCl₃): *δ* 8.23 (d, *J* = 7.4 Hz, 1H), 7.31 - 7.23 (m, 4H), 7.11 (d, *J* = 8.8 Hz, 1H), 6.96 - 6.87 (m, 3H), 4.81 (dt, *J* = 11.3, 7.4 Hz, 1H), 4.59 (dd, *J* = 9.7, 7.4 Hz, 1H), 4.24 (dd, *J* = 11.0, 10.0 Hz, 1H), 3.78 (t, *J* = 4.6 Hz, 4H), 3.59 - 3.53 (m, 2H), 3.50 (s, 2H), 3.41 (s, 3H), 2.84 (t, *J* = 7.1 Hz, 2H), 2.64 (t, *J* = 4.5 Hz, 4H).

### Example 121: Synthesis of (S)-N¹-(3-fluorophenethyl)-N²-(7-(3-(4-(2-hydroxyethyl)piperazin-1-yl)prop-1-yn-1-yl)-5-methyl -4-oxo-2,3,4,5-tetrahydrobenzo[b][1,4]oxazepin-3-yl)oxalamide (121)

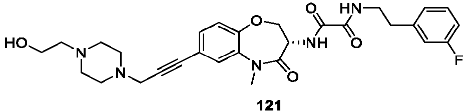

### Synthesis route:

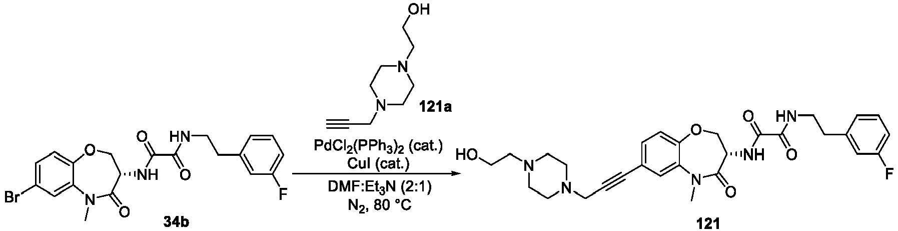

The synthesis route and method were the same as step 2 of Example 34, except that 3-butyn-1-ol **31a** was replaced with an equivalent amount of 2-(4-(prop-2-yn-1-yl)piperazin-1-yl)ethan-1-ol **121a** to obtain the title compound **121.**

MS (ES⁺): *m*/*z* 552.3 [M+H]⁺.

¹H NMR (400 MHz, CDCl₃): *δ* 8.23 (d, *J* = 7.3 Hz, 1H), 7.32 - 7.23 (m, 4H), 7.11 (d, *J* = 8.8 Hz, 1H), 6.96 - 6.87 (m, 3H), 4.81 (dt, *J* = 11.1, 7.4 Hz, 1H), 4.59 (dd, *J* = 9.6, 7.5 Hz, 1H), 4.24 (t, *J* = 10.5 Hz, 1H), 3.62 (t, *J* = 5.3 Hz, 2H), 3.58 - 3.53 (m, 2H), 3.52 (s, 2H), 3.41 (s, 3H), 2.84 (t, *J* = 7.1 Hz, 2H), 2.68 - 2.59 (m, 8H), 2.57 (t, *J* = 5.3 Hz, 2H).

### Example 122: Synthesis of (S)-N¹-(7-(3-(2,5-dioxopyrrolidin-1-yl)prop-1-yn-1-yl)-5-methyl-4-oxo-2,3,4,5-tetrahydrobenzo[ b][1,4]oxazepin-3-yl)-N²-(3-fluorophenethyl)oxalamide (122)

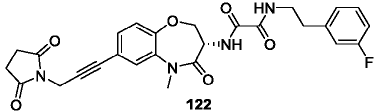

### Synthesis route:

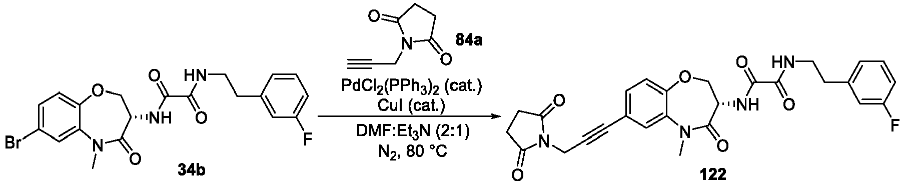

The synthesis route and method were the same as step 2 of Example 34, except that 3-butyn-1-ol **31a** was replaced with an equivalent amount of 1-(prop-2-yn-1-yl)pyrrolidine-2,5-dione **84a** to obtain the title compound **122.**

MS (ES⁺): *m*/*z* 521.2 [M+H]⁺.

¹H NMR (400 MHz, CDCl₃): *δ* 8.22 (d, *J* = 7.2 Hz, 1H), 7.31 - 7.23 (m, 4H), 7.09 (d, *J* = 9.0 Hz, 1H), 6.96 - 6.87 (m, 3H), 4.78 (dt, *J* = 11.3, 7.4 Hz, 1H), 4.58 (dd, *J* = 9.6, 7.4 Hz, 1H), 4.49 (s, 2H), 4.23 (t, *J* = 10.5 Hz, 1H), 3.58 - 3.53 (m, 2H), 3.39 (s, 3H), 2.84 (t, *J* = 7.1 Hz, 2H), 2.79 (s, 4H).

### Example 123: Synthesis of (S)-N¹-(3-fluorophenethyl)-N²-(5-methyl-4-oxo-7-(3-(3-oxopiperazin-1-yl)prop-1-yn-1-yl)-2,3,4, 5-tetrahydrobenzo[b][1,4]oxazepin-3-yl)oxalamide (123)

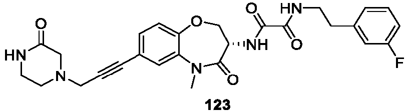

### Synthesis route:

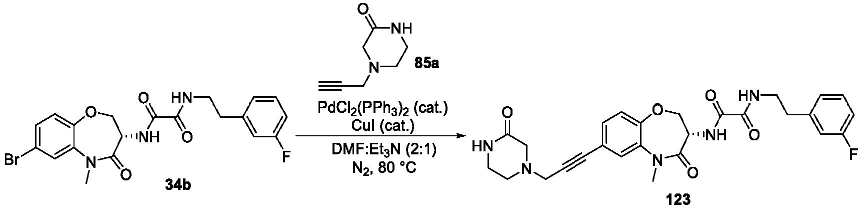

The synthesis route and method were the same as step 2 of Example 34, except that 3-butyn-1-ol **31a** was replaced with an equivalent amount of 4-(prop-2-yn-1-yl)piperazin-2-one **85a** to obtain the title compound **123.**

MS (ES⁺): *m*/*z* 522.3 [M+H]⁺.

¹H NMR (400 MHz, CDCl₃): *δ* 8.24 (d, *J* = 7.5 Hz, 1H), 7.32 - 7.23 (m, 4H), 7.12 (d, *J* = 8.9 Hz, 1H), 6.96 - 6.87 (m, 3H), 5.94 (s, 1H), 4.81 (dt, *J* = 11.3, 7.4 Hz, 1H), 4.59 (dd, *J* = 9.7, 7.4 Hz, 1H), 4.25 (t, *J* = 10.5 Hz, 1H), 3.63 (s, 2H), 3.59 - 3.53 (m, 2H), 3.46 - 3.43 (m, 2H), 3.41 (s, 3H), 3.36 (s, 2H), 2.86 - 2.82 (m, 4H).

### Example 124: Synthesis of N¹-((S)-7-( 4-hydroxybut-1-yn-1-yl)-5-methyl-4-oxo-2,3,4,5-tetrahydrobenzo[b][1,4]oxazepin-3-y l)-N²-((S)-1-phenylethyl)oxalamide (124)

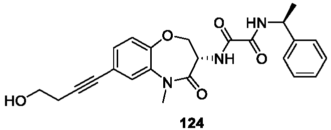

### Synthesis route:

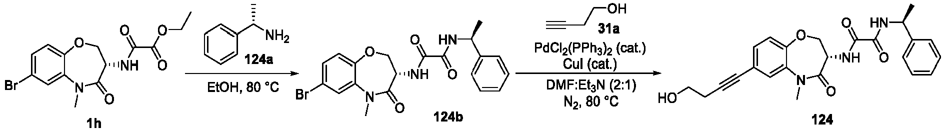

The synthesis route and method were the same as Example 32, except that the benzylamine **32a** in step 1 was replaced with an equivalent amount of (*S*)-1-phenylethylamine **124a** to obtain the title compound **124.**

MS (ES⁻): *m*/*z* 434.2 [M-H]⁻.

¹H NMR (400 MHz, CDCl₃): *δ* 8.29 (d, *J* = 7.4 Hz, 1H), 7.55 (d, *J* = 8.3 Hz, 1H), 7.35 - 7.26 (m, 7H), 7.11 (d, *J* = 8.0 Hz, 1H), 5.08 (qn, *J* = 7.3 Hz, 1H), 4.80 (dt, *J* = 11.2, 7.4 Hz, 1H), 4.60 (dd, *J* = 9.7, 7.4 Hz, 1H), 4.25 (t, *J =* 10.5 Hz, 1H), 3.83 (t, *J =* 6.2 Hz, 2H), 3.39 (s, 3H), 2.70 (t, *J* = 6.3 Hz, 2H), 1.96 (br, 1H), 1.54 (d, *J* = 6.9 Hz, 3H).

### Example 125: Synthesis of N¹-((S)-7-(4-hydroxybut-1-yn-1-yl)-5-methyl-4-oxo-2,3,4,5-tetrahydrobenzo[b][1,4]oxazepin-3-y l)-N²-((R)-1-phenylethyl)oxalamide (125)

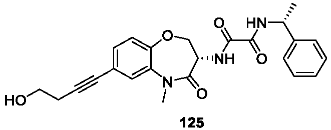

### Synthesis route:

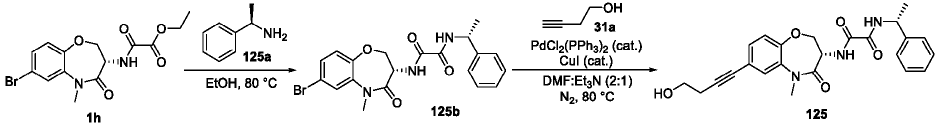

The synthesis route and method were the same as Example 32, except that the benzylamine **32a** in step 1 was replaced with an equivalent amount of (*R*)-1-phenylethylamine **125a** to obtain the title compound **125.**

MS (ES⁻): *m*/*z* 434.2 [M-H]⁻.

¹H NMR (400 MHz, CDCl₃): *δ* 8.25 (d, *J* = 7.4 Hz, 1H), 7.48 (d, *J* = 8.3 Hz, 1H), 7.36 - 7.26 (m, 7H), 7.10 (d, *J* = 8.7 Hz, 1H), 5.07 (qn, *J* = 7.3 Hz, 1H), 4.80 (dt, *J* = 11.2, 7.4 Hz, 1H), 4.56 (dd, *J* = 9.7, 7.3 Hz, 1H), 4.21 (t, *J* = 10.5 Hz, 1H), 3.83 (q, *J* = 6.2 Hz, 2H), 3.40 (s, 3H), 2.70 (t, *J* = 6.3 Hz, 2H), 1.80 (t, *J* = 6.2 Hz, 1H), 1.53 (d, *J* = 6.9 Hz, 3H).

### Example 126: Synthesis of N¹-((3S)-7-(3-(3-hydroxypyrrolidin-1-yl)prop-1-yn-1-yl)-5-methyl-4-oxo-2,3,4,5-tetrahydrobenz o[b][1,4]oxazepin-3-yl)-N²-((R)-1-phenylethyl)oxalamide (126)

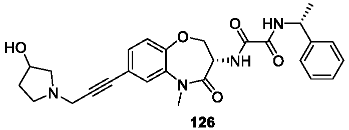

### Synthesis route:

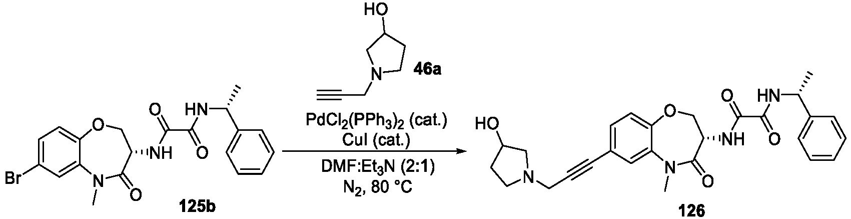

The synthesis route and method were the same as step 2 of Example 125, except that 3-butyn-1-ol **31a** was replaced with an equivalent amount of 1-(prop-2-yn-1-yl)pyrrolidin-3-ol **46a** to obtain the title compound **126.**

MS (ES⁺): *m*/*z* 491.3 [M+H]⁺.

¹H NMR (400 MHz, CDCl₃): *δ* 8.25 (d, *J* = 7.4 Hz, 1H), 7.49 (d, *J* = 8.3 Hz, 1H), 7.36 - 7.28 (m, 7H), 7.10 (d, *J =* 8.7 Hz, 1H), 5.07 (qn, *J* = 7.3 Hz, 1H), 4.81 (dt, *J =* 11.2, 7.4 Hz, 1H), 4.56 (dd, *J* = 9.7, 7.4 Hz, 1H), 4.43 - 4.39 (m, 1H), 4.21 (t, *J =* 10.6 Hz, 1H), 3.66 (s, 2H), 3.40 (s, 3H), 3.03 - 2.97 (m, 1H), 2.80 (d, *J =* 3.6 Hz, 2H), 2.61 - 2.55 (m, 1H), 2.30 - 2.21 (m, 1H), 1.85 - 1.78 (m, 1H), 1.53 (d, *J* = 6.9 Hz, 3H).

### Example 127: Synthesis of N¹-((S)-7-(3-((R)-3-hydroxypyrrolidin-1-yl)prop-1-yn-1-yl)-5-methyl-4-oxo-2,3,4,5-tetrahydrobe nzo[b][1,4]oxazepin-3-yl)-N²-((S)-1-phenylethyl)oxalamide (127)

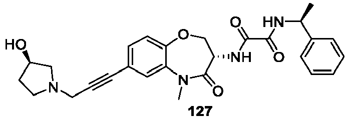

### Synthesis route:

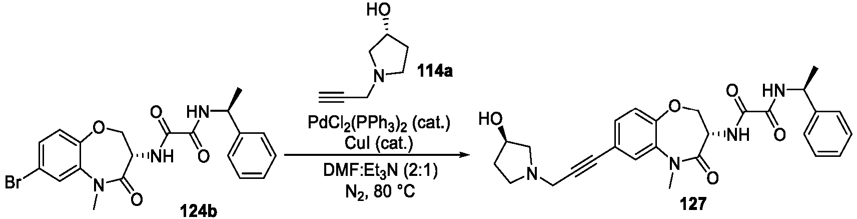

The synthesis route and method were the same as step 2 of Example 124, except that 3-butyn-1-ol **31a** was replaced with an equivalent amount of (R)-1-(prop-2-yn-1-yl)pyrrolidin-3-ol **114a** to obtain the title compound **127.**

MS (ES⁺): *m*/*z* 491.3 [M+H]⁺.

¹H NMR (400 MHz, CDCl₃): *δ* 8.26 (d, *J* = 7.4 Hz, 1H), 7.48 (d, *J* = 8.4 Hz, 1H), 7.35 - 7.27 (m, 7H), 7.11 (d, *J* = 8.2 Hz, 1H), 5.07 (qn, *J* = 7.3 Hz, 1H), 4.80 (dt, *J* = 11.2, 7.4 Hz, 1H), 4.60 (dd, *J* = 9.7, 7.4 Hz, 1H), 4.44 - 4.40 (m, 1H), 4.25 (t, *J* = 10.5 Hz, 1H), 3.67 (s, 2H), 3.39 (s, 3H), 3.05 - 3.00 (m, 1H), 2.82 (d, *J* = 3.6 Hz, 2H), 2.64 - 2.58 (m, 1H), 2.30 - 2.20 (m, 1H), 1.86 - 1.79 (m, 1H), 1.54 (d, *J* = 6.9 Hz, 3H).

### Example 128: Synthesis of N¹-((S)-7-(3-((R)-3-hydroxypyrrolidin-1-yl)prop-1-yn-1-yl)-5-methyl-4-oxo-2,3,4,5-tetrahydrobe nzo[b][1,4]oxazepin-3-yl)-N²-((R)-1-phenylethyl)oxalamide (128)

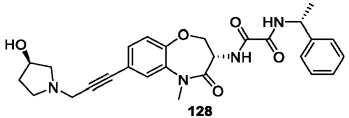

### Synthesis route:

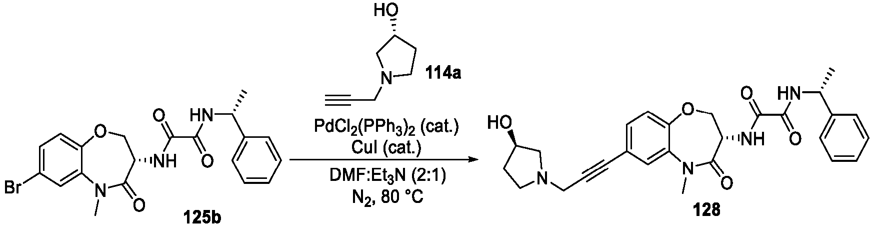

The synthesis route and method were the same as step 2 of Example 125, except that 3-butyn-1-ol 31a was replaced with an equivalent amount of (R)-1-(prop-2-yn-1-yl)pyrrolidin-3-ol 114a to obtain the title compound 128.

MS (ES⁺): *m*/*z* 491.3 [M+H]⁺.

¹H NMR (400 MHz, CDCl₃): *δ* 8.25 (d, *J* = 7.3 Hz, 1H), 7.49 (d, *J* = 8.2 Hz, 1H), 7.36 - 7.28 (m, 7H), 7.10 (d, *J =* 8.8 Hz, 1H), 5.07 (qn, J = 7.3 Hz, 1H), 4.81 (dt, *J =* 11.2, 7.4 Hz, 1H), 4.56 (dd, J = 9.7, 7.4 Hz, 1H), 4.44 - 4.39 (m, 1H), 4.21 (t, *J =* 10.5 Hz, 1H), 3.66 (s, 2H), 3.40 (s, 3H), 3.04 - 2.98 (m, 1H), 2.81 (d, *J* = 3.6 Hz, 2H), 2.62 - 2.56 (m, 1H), 2.30 - 2.20 (m, 1H), 1.85 - 1.78 (m, 1H), 1.53 (d, *J* = 6.9 Hz, 3H).

### Example 129: Synthesis of N¹-((S)-7-(3-((S)-3-hydroxypyrrolidin-1-yl)prop-1-yn-1-yl)-5-methyl-4-oxo-2,3,4,5-tetrahydrobe nzo[b][1,4]oxazepin-3-yl)-N²-((S)-1-phenylethyl)oxalamide (129)

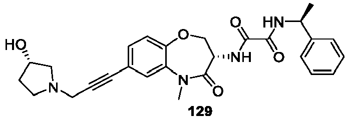

### Synthesis route:

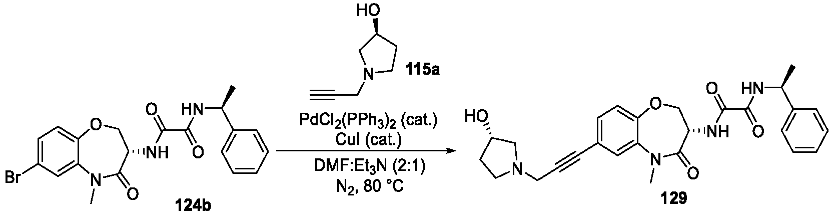

The synthesis route and method were the same as step 2 of Example 124, except that 3-butyn-1-ol **31a** was replaced with an equivalent amount of (S)-1-(prop-2-yn-1-yl)pyrrolidin-3-ol **115a** to obtain the title compound **129.**

MS (ES⁺): *m*/*z* 491.3 [M+H]⁺.

¹H NMR (400 MHz, CDCl₃): *δ* 8.26 (d, *J* = 7.4 Hz, 1H), 7.48 (d, *J* = 8.4 Hz, 1H), 7.35 - 7.27 (m, 7H), 7.12 (d, *J* = 8.2 Hz, 1H), 5.07 (qn, *J* = 7.3 Hz, 1H), 4.80 (dt, *J* = 11.2, 7.4 Hz, 1H), 4.60 (dd, *J* = 9.7, 7.4 Hz, 1H), 4.44 - 4.40 (m, 1H), 4.25 (t, *J* = 10.5 Hz, 1H), 3.68 (s, 2H), 3.39 (s, 3H), 3.07 - 3.02 (m, 1H), 2.84 (d, *J* = 3.4 Hz, 2H), 2.66 - 2.60 (m, 1H), 2.30 - 2.21 (m, 1H), 1.87 - 1.80 (m, 1H), 1.54 (d, *J* = 6.9 Hz, 3H).

### Example 130: Synthesis of N¹-((S)-7-(3-((S)-3-hydroxypyrrolidin-1-yl)prop-1-yn-1-yl)-5-methyl-4-oxo-2,3,4,5-tetrahydrobe nzo[b][1,4]oxazepin-3-yl)-N²-((R)-1-phenylethyl)oxalamide (130)

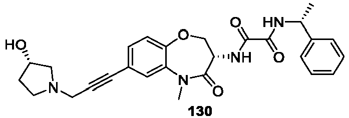

### Synthesis route:

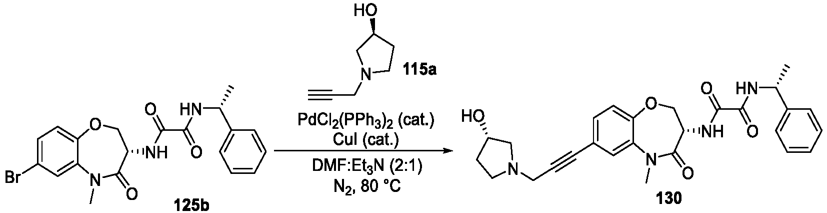

The synthesis route and method were the same as step 2 of Example 125, except that 3-butyn-1-ol **31a** was replaced with an equivalent amount of (S)-1-(prop-2-yn-1-yl)pyrrolidin-3-ol **115a** to obtain the title compound **130.**

MS (ES⁺): *m*/*z* 491.3 [M+H]⁺.

¹H NMR (400 MHz, CDCl₃): *δ* 8.25 (d, *J* = 7.4 Hz, 1H), 7.49 (d, *J* = 8.3 Hz, 1H), 7.36 - 7.27 (m, 7H), 7.10 (d, *J =* 8.8 Hz, 1H), 5.07 (qn, *J* = 7.3 Hz, 1H), 4.80 (dt, *J =* 11.3, 7.5 Hz, 1H), 4.56 (dd, *J* = 9.8, 7.4 Hz, 1H), 4.44 - 4.40 (m, 1H), 4.21 (t, *J =* 10.5 Hz, 1H), 3.67 (s, 2H), 3.40 (s, 3H), 3.06 - 3.00 (m, 1H), 2.83 (d, *J* = 3.2 Hz, 2H), 2.65 - 2.59 (m, 1H), 2.30 - 2.21 (m, 1H), 1.87 - 1.79 (m, 1H), 1.53 (d, *J* = 6.9 Hz, 3H).

### Example 131: Synthesis of (S)-N¹-(5-methyl-7-(oxetan-3-ylethynyl)-4-oxo-2,3,4,5-tetrahydrobenzo[b][1,4]oxazepin-3-yl)-N² -((R)-1-phenylethyl)oxalamide (131)

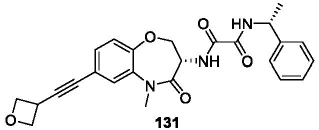

### Synthesis route:

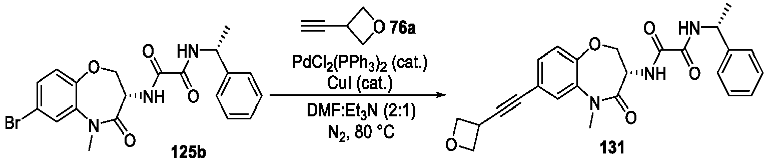

The synthesis route and method were the same as step 2 of Example 125, except that 3-butyn-1-ol **31a** was replaced with an equivalent amount of 3-ethynyloxetane **76a** to obtain the title compound **131.**

MS (ES⁺): *m*/*z* 448.2 [M+H]⁺.

¹H NMR (400 MHz, CDCl₃): *δ* 8.25 (d, *J* = 7.4 Hz, 1H), 7.47 (d, *J* = 8.3 Hz, 1H), 7.35 - 7.27 (m, 7H), 7.12 (d, *J =* 8.8 Hz, 1H), 5.01 (qn, *J* = 7.2 Hz, 1H), 4.92 - 4.86 (m, 2H), 4.83 - 4.76 (m, 3H), 4.57 (dd, *J* = 9.8, 7.4 Hz, 1H), 4.22 (t, *J* = 10.5 Hz, 1H), 4.09 - 4.01 (m, 1H), 3.42 (s, 3H), 1.53 (d, *J* = 6.9 Hz, 3H).

### Example 132: Synthesis of N¹-((S)-7-((3-hydroxyoxetan-3-yl)ethynyl)-5-methyl-4-oxo-2,3,4,5-tetrahydrobenzo[b][1,4]oxaze pin-3-yl)-N²-((R)-1-phenylethyl)oxalamide (132)

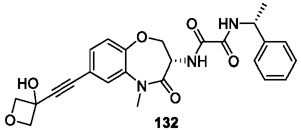

### Synthesis route:

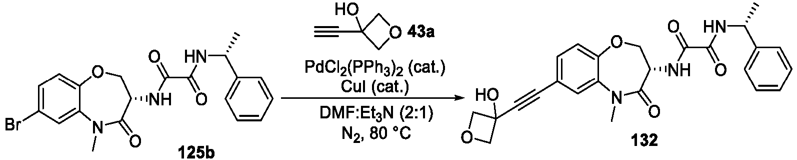

The synthesis route and method were the same as step 2 of Example 125, except that 3-butyn-1-ol **31a** was replaced with an equivalent amount of 3-ethynyloxetan-3-ol 43a to obtain the title compound **132.**

MS (ES⁺): *m*/*z* 464.2 [M+H]⁺.

¹H NMR (400 MHz, CDCl₃): *δ* 8.25 (d, *J =* 7.3 Hz, 1H), 7.47 (d, *J* = 8.4 Hz, 1H), 7.37 - 7.28 (m, 7H), 7.15 (d, *J* = 8.7 Hz, 1H), 5.07 (qn, *J* = 7.5 Hz, 1H), 4.94 (d, *J* = 7.0 Hz, 2H), 4.85 - 4.78 (m, 3H), 4.58 (dd, *J* = 9.8, 7.3 Hz, 1H), 4.23 (dd, *J* = 11.2, 9.9 Hz, 1H), 3.42 (s, 3H), 1.53 (d, *J* = 6.9 Hz, 3H).

### Example 133: Synthesis of N¹-((S)-7-((3-hydroxyoxetan-3-yl)ethynyl)-5-methyl-4-oxo-2,3,4,5-tetrahydrobenzo[b][1,4]oxaze pin-3-yl)-N²-((S)-1-phenylethyl)oxalamide (133)

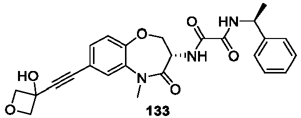

### Synthesis route:

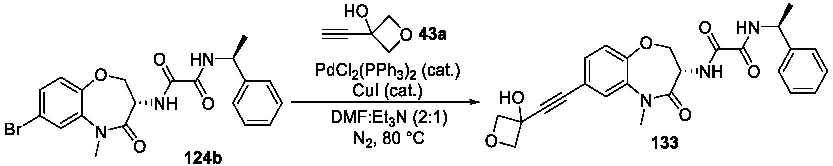

The synthesis route and method were the same as step 2 of Example 124, except that 3-butyn-1-ol **31a** was replaced with an equivalent amount of 3-ethynyloxetan-3-ol **43a** to obtain the title compound **133.**

MS (ES⁺): *m*/*z* 464.1 [M+H]⁺.

¹H NMR (400 MHz, CDCl₃): *δ* 8.26 (d, *J =* 7.3 Hz, 1H), 7.47 (d, *J* = 8.4 Hz, 1H), 7.37 - 7.28 (m, 7H), 7.15 (d, *J* = 8.7 Hz, 1H), 5.08 (qn, *J* = 7.5 Hz, 1H), 4.93 (d, *J* = 6.9 Hz, 2H), 4.84 - 4.77 (m, 3H), 4.62 (dd, *J* = 9.5, 7.4 Hz, 1H), 4.27 (dd, *J* = 11.2, 9.8 Hz, 1H), 3.41 (s, 3H), 1.53 (d, *J* = 6.9 Hz, 3H).

### Example 134: Synthesis of N¹-((S)-7-(3-(1H-imidazol-1-yl)prop-1-yn-1-yl)-5-methyl-4-oxo-2,3,4,5-tetrahydrobenzo[b][1,4] oxazepin-3-yl)-N²-((R)-1-phenylethyl)oxalamide (134)

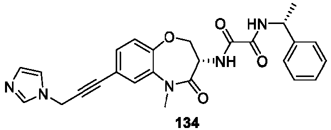

### Synthesis route:

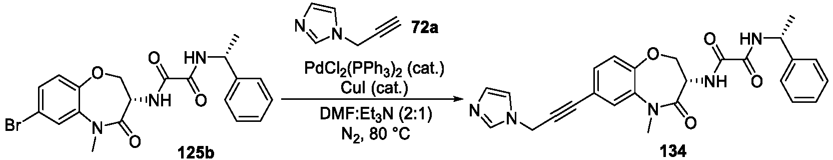

The synthesis route and method were the same as step 2 of Example 125, except that 3-butyn-1-ol **31a** was replaced with an equivalent amount of 1-(prop-2-yn-1-yl)-1H-imidazole **72a** to obtain the title compound **134.**

MS (ES⁺): *m*/*z* 472.2 [M+H]⁺.

¹H NMR (400 MHz, CDCl₃): *δ* 8.25 (d, *J* = 7.5 Hz, 1H), 7.77 (s, 1H), 7.47 (d, *J* = 8.3 Hz, 1H), 7.36 - 7.28 (m, 7H), 7.17 - 7.13 (m, 3H), 5.07 (qn, *J* = 7.3 Hz, 1H), 4.98 (s, 2H), 4.81 (dt, *J* = 11.2, 7.4 Hz, 1H), 4.57 (dd, *J* = 9.8, 7.3 Hz, 1H), 4.23 (t, *J* = 10.6 Hz, 1H), 3.41 (s, 3H), 1.53 (d, *J* = 6.9 Hz, 3H).

### Example 135: Synthesis of N¹-((S)-5-methyl-4-oxo-7-(piperidin-4-ylethynyl)-2,3,4,5-tetrahydrobenzo[b][1,4]oxazepin-3-yl)-N²-((R)-1-phenylethyl)oxalamide (135)

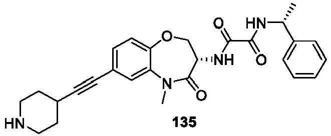

### Synthesis route:

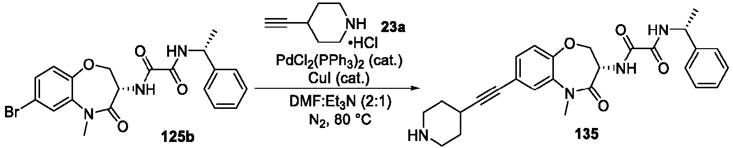

The synthesis route and method were the same as step 2 of Example 125, except that 3-butyn-1-ol 31a was replaced with an equivalent amount of 4-ethynylpiperidine hydrochloride 23a to obtain the title compound **135.**

MS (ES⁺): *m*/*z* 475.3 [M+H]⁺.

¹H NMR (400 MHz, CDCl₃): *δ* 8.27 (d, *J* = 7.4 Hz, 1H), 7.49 (d, *J* = 8.3 Hz, 1H), 7.36 - 7.25 (m, 7H), 7.11 (d, *J* = 8.7 Hz, 1H), 5.07 (qn, *J* = 7.2 Hz, 1H), 4.80 (dt, *J* = 11.2, 7.4 Hz, 1H), 4.56 (dd, *J* = 9.8, 7.4 Hz, 1H), 4.22 (t, *J* = 10.7 Hz, 1H), 3.42 (s, 3H), 3.38 - 3.32 (m, 2H), 3.17 - 3.09 (m, 2H), 3.02 - 2.97 (m, 1H), 2.24 - 2.16 (m, 2H), 2.05 - 1.97 (m, 2H), 1.53 (d, *J* = 6.9 Hz, 3H).

### Example 136: Synthesis of N¹-((S)-5-methyl-7-((1-methylpiperidin-4-yl)ethynyl)-4-oxo-2,3,4,5-tetrahydrobenzo[b][1,4]oxaz epin-3-yl)-N²-((R)-1-phenylethyl)oxalamide (136)

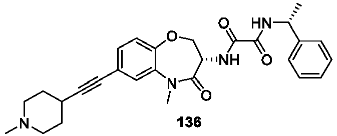

### Synthesis route:

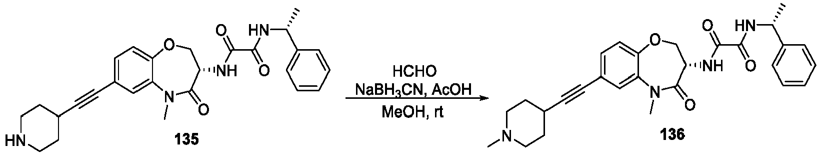

The synthesis route and method were the same as Example 73, except that compound 23 was replaced with an equivalent amount of compound 135 to obtain the title compound 136.

MS (ES⁺): *m*/*z* 489.3 [M+H]⁺.

¹H NMR (400 MHz, DMSO-*d*₆): *δ* 9.19 (d, *J* = 8.6 Hz, 1H), 8.75 (t, *J* = 6.4 Hz, 1H), 7.53 (s, 1H), 7.35 - 7.28 (m, 5H), 7.24 - 7.18 (m, 2H), 5.00 - 4.93 (m, 1H), 4.67 - 4.55 (m, 2H), 4.38 - 4.32 (m, 1H), 3.29 (s, 3H), 3.07 - 2.93 (m, 3H), 2.83 - 2.72 (m, 2H), 2.67 (s, 3H), 2.02 - 1.94 (m, 2H), 1.84 - 1.72 (m, 2H), 1.43 (d, *J* = 6.9 Hz, 3H).

### Example 137: Synthesis of N¹-((S)-7-((1-(cyanomethyl)piperidin-4-yl)ethynyl)-5-methyl-4-oxo-2,3,4,5-tetrahydrobenzo[b][1 ,4]oxazepin-3-yl)-N²-((R)-1-phenylethyl)oxalamide (137)

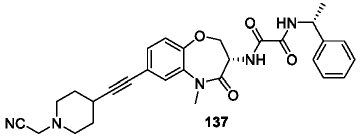

### Synthesis route:

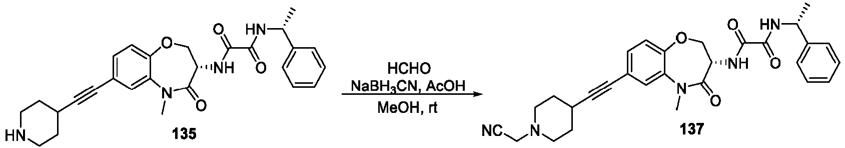

The synthesis route and method were the same as Example 136, and the title compound **137** was prepared through the same reaction.

MS (ES⁺): *m*/*z* 514.3 [M+H]⁺.

¹H NMR (400 MHz, CDCl₃): *δ* 8.25 (d, *J* = 7.5 Hz, 1H), 7.49 (d, *J* = 8.4 Hz, 1H), 7.36 - 7.25 (m, 7H), 7.10 (d, *J =* 8.5 Hz, 1H), 5.07 (qn, *J* = 7.3 Hz, 1H), 4.80 (dt, *J* = 11.3, 7.5 Hz, 1H), 4.56 (dd, *J* = 9.8, 7.4 Hz, 1H), 4.20 (dd, *J =* 11.1, 10.0 Hz, 1H), 3.54 (s, 2H), 3.40 (s, 3H), 2.86 - 2.81 (m, 2H), 2.70 - 2.63 (m, 1H), 2.52 - 2.46 (m, 2H), 2.01 - 1.96 (m, 2H), 1.85 - 1.77 (m, 2H), 1.53 (d, *J* = 6.9 Hz, 3H).

### Example 138: Synthesis of N¹-((S)-7-(3-(4-hydroxypiperidin-1-yl)prop-1-yn-1-yl)-5-methyl-4-oxo-2,3,4,5-tetrahydrobenzo[b ][1,4]oxazepin-3-yl)-N²-((R)-1-phenylethyl)oxalamide (138)

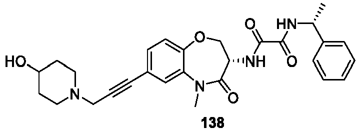

### Synthesis route:

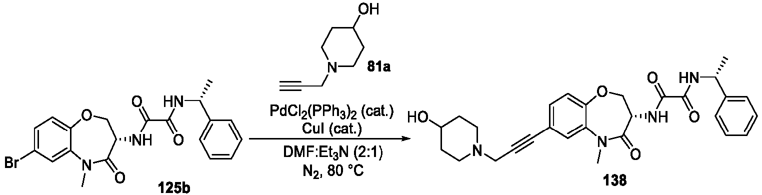

The synthesis route and method were the same as step 2 of Example 125, except that 3-butyn-1-ol **31a** was replaced with an equivalent amount of 1-(prop-2-yn-1-yl)piperidin-4-ol **81a** to obtain the title compound **138.**

MS (ES⁺): *m*/*z* 505.3 [M+H]⁺.

¹H NMR (400 MHz, CDCl₃): *δ* 8.25 (d, *J* = 7.4 Hz, 1H), 7.47 (d, *J* = 8.4 Hz, 1H), 7.36 - 7.28 (m, 7H), 7.10 (d, *J* = 8.8 Hz, 1H), 5.07 (qn, *J* = 7.2 Hz, 1H), 4.81 (dt, *J* = 11.2, 7.4 Hz, 1H), 4.56 (dd, *J* = 9.7, 7.4 Hz, 1H), 4.21 (t, *J* = 10.5 Hz, 1H), 3.78 - 3.72 (m, 1H), 3.50 (s, 2H), 3.40 (s, 3H), 2.91 - 2.86 (m, 2H), 2.44 - 2.39 (m, 2H), 1.99 - 1.95 (m, 2H), 1.71 - 1.62 (m, 2H), 1.53 (d, *J=* 6.9 Hz, 3H), 1.41 (d, *J* = 4.1 Hz, 1H).

### Example 139: Synthesis of N¹-((S)-5-methyl-4-oxo-7-(3-(4-oxopiperidin-1-yl)prop-1-yn-1-yl)-2,3,4,5-tetrahydrobenzo[b][1, 4]oxazepin-3-yl)-N²-((R)-1-phenylethyl)oxalamide (139)

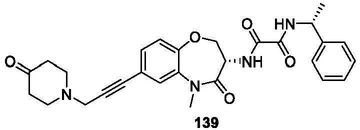

### Synthesis route:

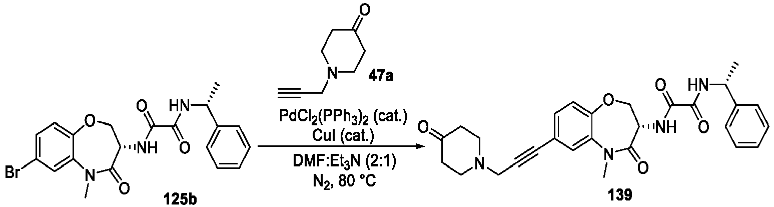

The synthesis route and method were the same as step 2 of Example 125, except that 3-butyn-1-ol **31a** was replaced with an equivalent amount of 1-(prop-2-yn-1-yl)piperidin-4-one **47a** to obtain the title compound **139.**

MS (ES⁺): *m*/*z* 503.2 [M+H]⁺.

¹H NMR (400 MHz, CDCl₃): *δ* 8.25 (d, *J* = 7.5 Hz, 1H), 7.48 (d, *J* = 8.5 Hz, 1H), 7.36 - 7.28 (m, 7H), 7.11 (d, *J =* 8.9 Hz, 1H), 5.07 (qn, *J* = 7.3 Hz, 1H), 4.81 (dt, *J =* 11.2, 7.5 Hz, 1H), 4.56 (dd, *J* = 9.7, 7.4 Hz, 1H), 4.22 (t, *J* = 10.5 Hz, 1H), 3.66 (s, 2H), 3.41 (s, 3H), 2.94 (t, *J* = 5.9 Hz, 4H), 2.54 (t, *J* = 6.1 Hz, 4H), 1.53 (d, *J* = 6.9 Hz, 3H).

### Example 140: Synthesis of N¹-((S)-5-methyl-7-(3-morpholinoprop-1-yn-1-yl)-4-oxo-2,3,4,5-tetrahydrobenzo[b][1,4]oxazepi n-3-yl)-N²-((R)-1-phenylethyl)oxalamide (140)

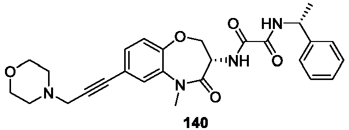

### Synthesis route:

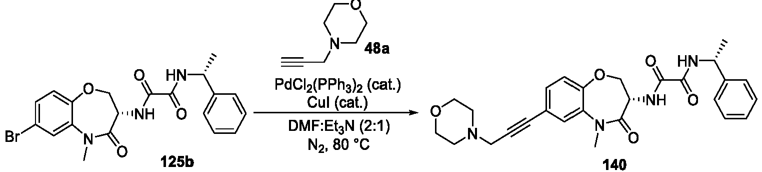

The synthesis route and method were the same as step 2 of Example 125, except that 3-butyn-1-ol **31a** was replaced with an equivalent amount of 4-(prop-2-yn-1-yl)morpholine **48a** to obtain the title compound **140.**

MS (ES⁺): *m*/*z* 491.2 [M+H]⁺.

¹H NMR (400 MHz, CDCl₃): *δ* 8.25 (d, *J* = 7.5 Hz, 1H), 7.49 (d, *J* = 8.2 Hz, 1H), 7.36 - 7.28 (m, 7H), 7.11 (d, *J =* 8.8 Hz, 1H), 5.07 (qn, *J* = 7.3 Hz, 1H), 4.81 (dt, *J =* 11.2, 7.4 Hz, 1H), 4.56 (dd, *J* = 9.7, 7.4 Hz, 1H), 4.21 (t, *J =* 10.5 Hz, 1H), 3.78 (t, *J =* 4.4 Hz, 4H), 3.51 (s, 2H), 3.40 (s, 3H), 2.64 (t, *J* = 4.2 Hz, 4H), 1.53 (d, *J* = 6.9 Hz, 3H).

### Example 141: Synthesis of N¹-((S)-5-methyl-7-(3-morpholinoprop-1-yn-1-yl)-4-oxo-2,3,4,5-tetrahydrobenzo[b][1,4]oxazepi n-3-yl)-N²-((S)-1-phenylethyl)oxalamide (141)

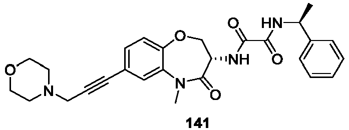

### Synthesis route:

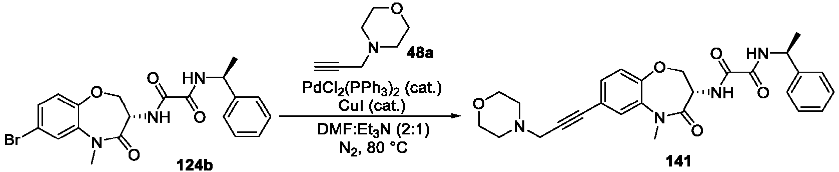

The synthesis route and method were the same as step 2 of Example 124, except that 3-butyn-1-ol **31a** was replaced with an equivalent amount of 4-(prop-2-yn-1-yl)morpholine **48a** to obtain the title compound **141.**

MS (ES⁺): *m*/*z* 491.2 [M+H]⁺.

¹H NMR (400 MHz, CDCl₃): *δ* 8.26 (d, *J* = 7.3 Hz, 1H), 7.47 (d, *J =* 8.3 Hz, 1H), 7.35 - 7.28 (m, 7H), 7.12 (d, *J =* 8.2 Hz, 1H), 5.08 (qn, *J* = 7.3 Hz, 1H), 4.80 (dt, *J =* 11.2, 7.4 Hz, 1H), 4.60 (dd, J = 9.7, 7.4 Hz, 1H), 4.25 (t, *J* = 10.5 Hz, 1H), 3.78 (t, *J* = 4.6 Hz, 4H), 3.50 (s, 2H), 3.39 (s, 3H), 2.64 (t, *J* = 4.4 Hz, 4H), 1.54 (d, *J =* 6.9 Hz, 3H).

### Example 142: Synthesis of N¹-((S)-5-methyl-4-oxo-7-(3-(piperazin-1-yl)prop-1-yn-1-yl)-2,3,4,5-tetrahydrobenzo[b][1,4]oxa zepin-3-yl)-N²-((R)-1-phenylethyl)oxalamide (142)

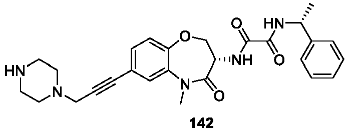

### Synthesis route:

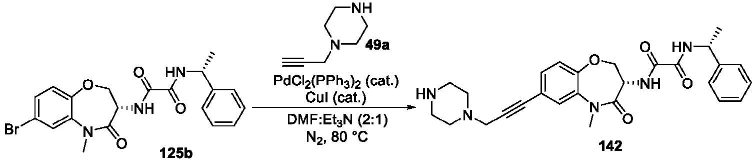

The synthesis route and method were the same as step 2 of Example 125, except that 3-butyn-1-ol **31a** was replaced with an equivalent amount of 1-(prop-2-yn-1-yl)piperazine **49a** to obtain the title compound **142.**

MS (ES⁺): *m*/*z* 490.3 [M+H]⁺.

¹H NMR (400 MHz, DMSO-*d*₆): *δ* 9.17 (d, *J* = 8.7 Hz, 1H), 8.82 - 8.80 (m, 1H), 7.69 (s, 1H), 7.40 (dd, *J* = 8.3, 1.8 Hz, 1H), 7.35 - 7.20 (m, 6H), 4.97 (qn, *J* = 7.4 Hz, 1H), 4.68 - 4.58 (m, 2H), 4.40 - 4.34 (m, 1H), 3.98 (s, 2H), 3.31 (s, 3H), 3.30 - 3.26 (m, 4H), 3.16 - 3.08 (m, 4H), 1.44 (d, *J* = 7.1 Hz, 3H).

### Example 143: Synthesis of N¹-((S)-7-(3-(4-acetylpiperazin-1-yl)prop-1-yn-1-yl)-5-methyl-4-oxo-2,3,4,5-tetrahydrobenzo[b][ 1,4]oxazepin-3-yl)-N²-((R)-1-phenylethyl)oxalamide (143)

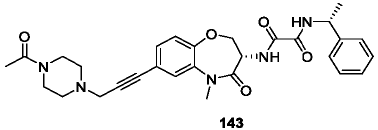

### Synthesis route:

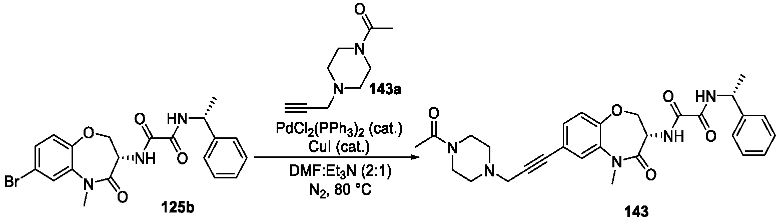

The synthesis route and method were the same as step 2 of Example 125, except that 3-butyn-1-ol **31a** was replaced with an equivalent amount of 1-(4-(prop-2-yn-1-yl)piperazin-1-yl)ethan-1-one **143a** to obtain the title compound **143.**

MS (ES⁺): *m*/*z* 532.3 [M+H]⁺.

¹H NMR (400 MHz, CDCl₃): *δ* 8.25 (d, *J* = 7.5 Hz, 1H), 7.47 (d, *J* = 8.4 Hz, 1H), 7.36 - 7.27 (m, 7H), 7.11 (d, *J =* 8.8 Hz, 1H), 5.07 (qn, *J* = 7.2 Hz, 1H), 4.81 (dt, *J =* 11.2, 7.4 Hz, 1H), 4.56 (dd, *J* = 9.8, 7.4 Hz, 1H), 4.22 (dd, *J =* 11.1, 9.9 Hz, 1H), 3.71 - 3.65 (m, 2H), 3.56 (s, 2H), 3.55 - 3.49 (m, 2H), 3.41 (s, 3H), 2.64 - 2.52 (m, 4H), 2.11 (s, 3H), 1.53 (d, *J* = 6.9 Hz, 3H).

### Example 144: Synthesis of N¹-((S)-7-(3-(2,5-dioxopyrrolidin-1-yl)prop-1-yn-1-yl)-5-methyl-4-oxo-2,3,4,5-tetrahydrobenzo[ b][1,4]oxazepin-3-yl)-N²-((R)-1-phenylethyl)oxalamide (144)

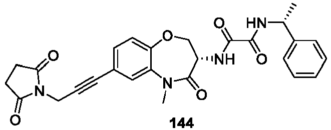

### Synthesis route:

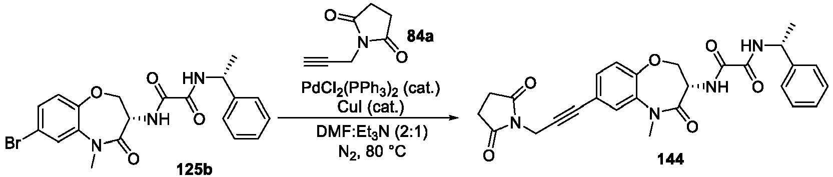

The synthesis route and method were the same as step 2 of Example 125, except that 3-butyn-1-ol **31a** was replaced with an equivalent amount of 1-(prop-2-yn-1-yl)pyrrolidine-2,5-dione **84a** to obtain the title compound **144.**

MS (ES⁺): *m*/*z* 525.2 [M+Na]⁺.

¹H NMR (400 MHz, CDCl₃): *δ* 8.25 (br, 1H), 7.49 (d, *J* = 8.2 Hz, 1H), 7.36 - 7.27 (m, 7H), 7.09 (d, *J* = 8.7 Hz, 1H), 5.06 (qn, *J* = 7.3 Hz, 1H), 4.81 - 4.75 (m, 1H), 4.55 (dd, *J* = 9.6, 7.4 Hz, 1H), 4.49 (s, 2H), 4.21 (t, *J* = 10.6 Hz, 1H), 3.39 (s, 3H), 2.79 (s, 4H), 1.53 (d, *J* = 6.9 Hz, 3H).

### Example 145: Synthesis of N¹-((S)-5-methyl-4-oxo-7-(3-(3-oxopiperazin-1-yl)prop-1-yn-1-yl)-2,3,4,5-tetrahydrobenzo[b][1, 4]oxazepin-3-yl)-N²-((R)-1-phenylethyl)oxalamide (145)

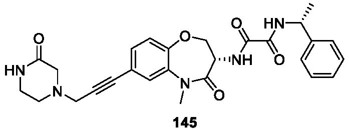

### Synthesis route:

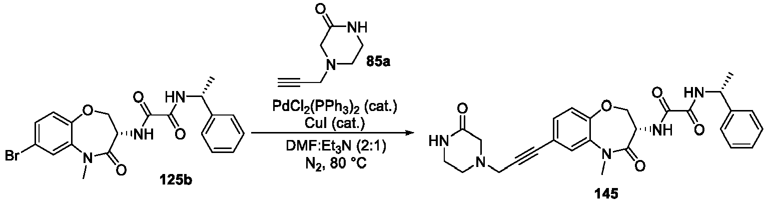

The synthesis route and method were the same as step 2 of Example 125, except that 3-butyn-1-ol **31a** was replaced with an equivalent amount of 4-(prop-2-yn-1-yl)piperazin-2-one **85a** to obtain the title compound **145.**

MS (ES⁺): *m*/*z* 526.2 [M+Na]⁺.

¹H NMR (400 MHz, CDCl₃): *δ* 8.26 (d, *J* = 7.2 Hz, 1H), 7.51 (d, *J =* 8.4 Hz, 1H), 7.36 - 7.27 (m, 7H), 7.11 (d, *J =* 8.8 Hz, 1H), 6.11 (s, 1H), 5.06 (qn, J = 7.3 Hz, 1H), 4.81 (dt, *J =* 11.2, 7.2 Hz, 1H), 4.56 (dd, *J* = 9.7, 7.4 Hz, 1H), 4.22 (t, *J =* 10.5 Hz, 1H), 3.63 (s, 2H), 3.45 - 3.42 (m, 2H), 3.41 (s, 3H), 3.36 (s, 2H), 2.83 (t, *J* = 5.4 Hz, 2H), 1.53 (d, *J =* 6.9 Hz, 3H).

### Example 146: Synthesis of N¹-((S)-7-(3-(dimethylamino)prop-1-yn-1-yl)-5-methyl-4-oxo-2,3,4,5-tetrahydrobenzo[b][1,4]ox azepin-3-yl)-N²-((R)-1-phenylethyl)oxalamide (146)

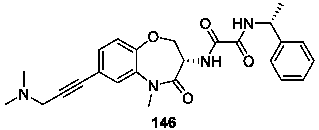

### Synthesis route:

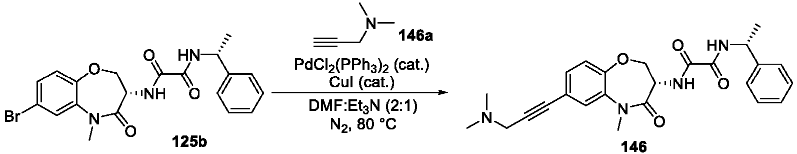

The synthesis route and method were the same as step 2 of Example 125, except that 3-butyn-1-ol **31a** was replaced with an equivalent amount of N,N-dimethylpropargylamine **146a** to obtain the title compound **146.**

MS (ES⁺): *m*/*z* 449.2 [M+H]⁺.

¹H NMR (400 MHz, CD₃OD): *δ* 7.64 (br, 1H), 7.48 (d, *J* = 8.2 Hz, 1H), 7.37 - 7.32 (m, 5H), 7.31 - 7.24 (m, 3H), 5.04 (qn, *J* = 7.0 Hz, 1H), 4.86 - 4.82 (m, 1H), 4.59 - 4.43 (m, 2H), 4.34 (s, 2H), 3.42 (s, 3H), 3.04 (s, 6H), 1.52 (d, *J* = 6.9 Hz, 3H).

### Example 147: Synthesis of N¹-((S)-5-methyl-7-((1-methylazetidin-3-yl)ethynyl)-4-oxo-2,3,4,5-tetrahydrobenzo[b][1,4]oxaze pin-3-yl)-N²-((R)-1-phenylethyl)oxalamide (147)

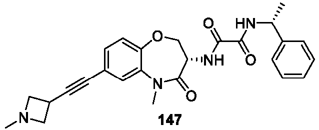

### Synthesis route:

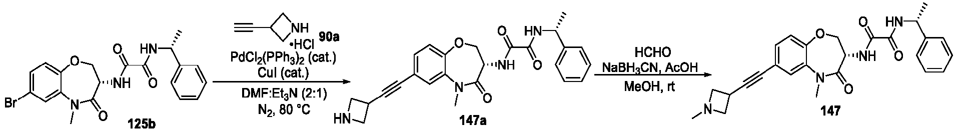

The synthesis route and method of step 1 were the same as Example 90, except that compound 1 was replaced with an equivalent amount of compound **125b;** and the synthesis route and method of step 2 were the same as Example 73, except that compound **23** was replaced with an equivalent amount of compound **147a** to obtain the title compound **147.**

MS (ES⁺): *m*/*z* 461.1 [M+H]⁺.

¹H NMR (400 MHz, DMSO-*d*₆): *δ* 9.19 (d, *J* = 9.0 Hz, 1H), 8.75 (d, *J* = 7.3 Hz, 1H), 7.54 (d, *J* = 1.7 Hz, 1H), 7.35 - 7.28 (m, 5H), 7.23 - 7.17 (m, 2H), 4.97 (qn, J = 7.7 Hz, 1H), 4.68 - 4.55 (m, 2H), 4.35 (dd, *J* = 8.9, 7.9 Hz, 1H), 3.55 (t, *J =* 7.0 Hz, 2H), 3.39 - 3.36 (m, 1H), 3.29 (s, 3H), 3.01 (t, *J* = 6.9 Hz, 2H), 2.54 (s, 3H), 1.44 (d, *J* = 7.1 Hz, 3H).

### Example 148: Synthesis of N¹-((S)-7-((1-(cyanomethyl)azetidin-3-yl)ethynyl)-5-methyl-4-oxo-2,3,4,5-tetrahydrobenzo[b][1, 4]oxazepin-3-yl)-N²-((R)-1-phenylethyl)oxalamide (148)

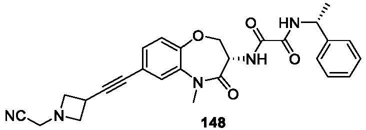

### Synthesis route:

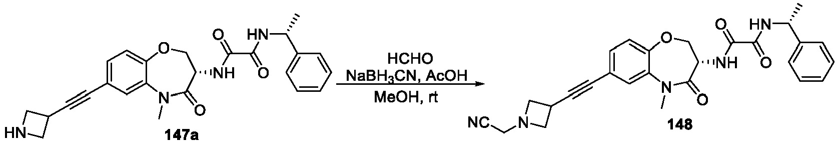

The synthesis route and method were the same as step 2 of Example 147. The title compound **148** was prepared through the same reaction.

MS (ES⁺): *m*/*z* 486.2 [M+H]⁺.

¹H NMR (400 MHz, DMSO-*d*₆): *δ* 9.19 (d, *J* = 8.6 Hz, 1H), 8.75 (d, *J* = 7.6 Hz, 1H), 7.55 (d, *J* = 1.7 Hz, 1H), 7.35 - 7.28 (m, 5H), 7.23 - 7.18 (m, 2H), 4.97 (qn, *J* = 7.3 Hz, 1H), 4.68 - 4.55 (m, 2H), 4.35 (dd, *J* = 9.0, 7.0 Hz, 1H), 3.67 (s, 2H), 3.62 (t, *J* = 7.1 Hz, 2H), 3.54 - 3.47 (m, 1H), 3.30 (s, 3H), 3.26 (t, *J* = 6.8 Hz, 2H), 1.44 (d, *J* = 7.0 Hz, 3H).

### Example 149: Synthesis of N¹-((S)-7-((1-acryloylazetidin-3-yl)ethynyl)-5-methyl-4-oxo-2,3,4,5-tetrahydrobenzo[b][1,4]oxaz epin-3-yl)-N²-((R)-1-phenylethyl)oxalamide (149)

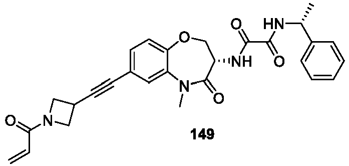

### Synthesis route:

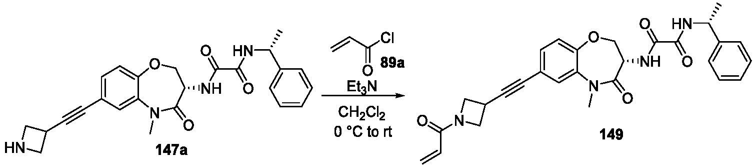

The synthesis route and method were the same as Example 89, except that compound 87 was replaced with an equivalent amount of compound **147a** to obtain the title compound 149.

MS (ES⁺): *m*/*z* 501.2 [M+H]⁺.

¹H NMR (400 MHz, DMSO-*d*₆): *δ* 9.19 (d, *J* = 8.7 Hz, 1H), 8.77 (d, *J* = 7.5 Hz, 1H), 7.61 (s, 1H), 7.35 - 7.28 (m, 5H), 7.23 - 7.19 (m, 2H), 3.32 (dd, *J* = 17.0, 10.3 Hz, 1H), 6.12 (dd, *J =* 17.0, 1.8 Hz, 1H), 5.70 (dd, *J =* 10.3, 1.8 Hz, 1H), 4.97 (qn, *J* = 7.4 Hz, 1H), 4.68 - 4.54 (m, 2H), 4.35 (t, *J* = 7.7 Hz, 1H), 4.30 - 4.23 (m, 2H), 4.13 - 4.09 (m, 2H), 3.81 - 3.74 (m, 1H), 3.29 (s, 3H), 1.43 (d, *J* = 7.0 Hz, 3H).

### Example 150: Synthesis of N¹-((S)-7-((1-(2-hydroxyethyl)azetidin-3-yl)ethynyl)-5-methyl-4-oxo-2,3,4,5-tetrahydrobenzo[b][ 1,4]oxazepin-3-yl)-N²-((R)-1-phenylethyl)oxalamide (150)

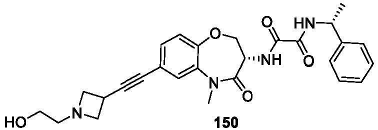

### Synthesis route:

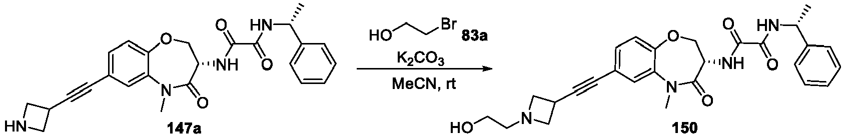

The synthesis route and method were the same as Example 83, except that compound 49 was replaced with an equivalent amount of compound **147a** to obtain the title compound 150.

MS (ES⁺): *m*/*z* 491.2 [M+H]⁺.

¹H NMR (400 MHz, CDCl₃): *δ* 8.25 (d, *J* = 7.7 Hz, 1H), 7.47 (d, *J* = 8.2 Hz, 1H), 7.37 - 7.27 (m, 7H), 7.10 (d, *J =* 8.6 Hz, 1H), 5.07 (qn, *J* = 7.5 Hz, 1H), 4.84 - 4.77 (m, 1H), 4.58 (dd, J = 9.7, 7.4 Hz, 1H), 4.21 (t, *J =* 10.7 Hz, 1H), 3.78 (t, *J =* 7.5 Hz, 2H), 3.58 (t, *J =* 5.1 Hz, 2H), 3.52 - 3.48 (m, 1H), 3.40 (s, 3H), 3.27 (t, *J =* 7.2 Hz, 2H), 2.69 (t, *J =* 5.1 Hz, 2H), 1.53 (d, *J* = 7.0 Hz, 3H).

### Example 151: Synthesis of N¹-((S)-5-methyl-7-((1-nitrosoazetidin-3-yl)ethynyl)-4-oxo-2,3,4,5-tetrahydrobenzo[b][1,4]oxaze pin-3-yl)-N²-((R)-1-phenylethyl)oxalamide (151)

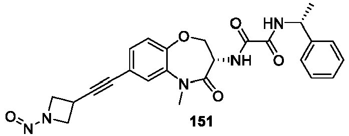

### Synthesis route:

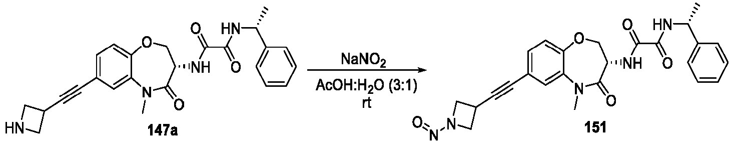

Sodium nitrite (7 mg, 0.1 mmol) was added to a solution of compound **147a** (22 mg, 0.05 mmol) in acetic acid (0.75 mL) and water (0.25 mL) at room temperature, and the reaction solution was stirred at room temperature for 6 hours. TLC and LC-MS showed that the reaction was complete, and then it was neutralized with saturated aqueous solution of sodium bicarbonate to pH = 7, directly concentrated under reduced pressure, and then purified by silica gel column chromatography (dichloromethane containing 5 - 10% methanol as the eluent) to obtain the title compound **151** (16 mg, 67% yield) as a light yellow solid.

MS (ES⁺): *m*/*z* 476.2 [M+H]⁺.

¹H NMR (400 MHz, DMSO-*d*₆): *δ* 9.19 (d, *J =* 8.6 Hz, 1H), 8.77 (d, *J =* 7.4 Hz, 1H), 7.62 (s, 1H), 7.36 - 7.27 (m, 5H), 7.23 - 7.19 (m, 2H), 5.23 (t, *J =* 9.7 Hz, 1H), 4.96 (qn, *J* = 7.3 Hz, 1H), 4.90 - 4.86 (m, 1H), 4.68 - 4.56 (m, 2H), 4.48 (t, *J* = 10.4 Hz, 1H), 4.35 (t, *J =* 7.4 Hz, 1H), 4.13 - 4.08 (m, 1H), 3.97 - 3.90 (m, 1H), 3.29 (s, 3H), 1.43 (d, *J* = 7.0 Hz, 3H).

### Example 152: Synthesis of N¹-((S)-7-(4-hydroxybut-1-yn-1-yl)-5-methyl-4-oxo-2,3,4,5-tetrahydrobenzo[b][1,4]oxazepin-3-y 1)-N²-((S)-1-phenylpropan-2-yl)oxalamide (152)

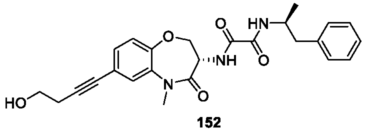

### Synthesis route:

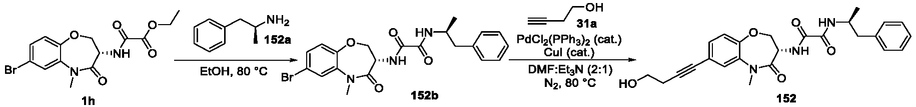

The synthesis route and method were the same as Example 32, except that the benzylamine **32a** in step 1 was replaced with an equivalent amount of (S)-1-phenylpropan-2-amine **152a** to obtain the title compound 152.

MS (ES⁺): *m*/*z* 450.2 [M+H]⁺.

¹H NMR (400 MHz, CDCl₃): *δ* 8.24 (d, *J* = 7.4 Hz, 1H), 7.30 - 7.19 (m, 5H), 7.15 - 7.13 (m, 3H), 7.10 (d, *J =* 8.9 Hz, 1H), 4.80 (dt, *J =* 11.2, 7.4 Hz, 1H), 4.58 (dd, J = 9.7, 7.3 Hz, 1H), 4.24 - 4.17 (m, 2H), 3.84 (t, *J* = 6.2 Hz, 2H), 3.40 (s, 3H), 2.87 - 2.82 (m, 1H), 2.76 - 2.69 (m, 3H), 1.77 (br, 1H), 1.16 (d, *J* = 6.7 Hz, 3H).

### Example 153: Synthesis of N¹-((S)-7-(4-hydroxybut-1-yn-1-yl)-5-methyl-4-oxo-2,3,4,5-tetrahydrobenzo[b][1,4]oxazepin-3-y 1)-N²-((R)-1-phenylpropan-2-yl)oxalamide (153)

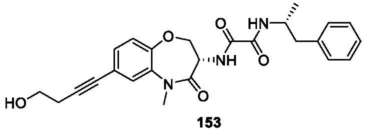

### Synthesis route:

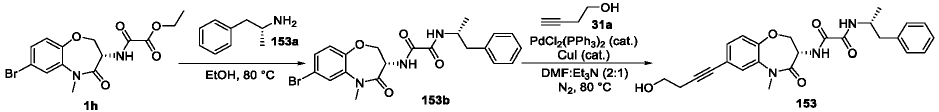

The synthesis route and method were the same as Example 32, except that the benzylamine **32a** in step 1 was replaced with an equivalent amount of (*R*)-1-phenylpropan-2-amine **153a** to obtain the title compound **153.**

MS (ES⁺): *m*/*z* 450.2 [M+H]⁺.

¹H NMR (400 MHz, CDCl₃): *δ* 8.21 (d, *J* = 7.5 Hz, 1H), 7.31 - 7.20 (m, 5H), 7.17 - 7.13 (m, 3H), 7.10 (d, *J =* 8.8 Hz, 1H), 4.80 (dt, *J* = 11.3, 7.4 Hz, 1H), 4.58 (dd, *J* = 9.8, 7.8 Hz, 1H), 4.25 - 4.15 (m, 2H), 3.83 (t, *J* = 6.0 Hz, 2H), 3.40 (s, 3H), 2.89 - 2.82 (m, 1H), 2.76 - 2.68 (m, 3H), 1.78 (br, 1H), 1.15 (d, *J* = 6.6 Hz, 3H).

### Example 154: Synthesis of N¹-((S)-7-(4-hydroxybut-1-yn-1-yl)-5-methyl-4-oxo-2,3,4,5-tetrahydrobenzo[b][1,4]oxazepin-3-y l)-N²-(2-phenylpropyl)oxalamide (154)

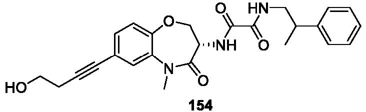

### Synthesis route:

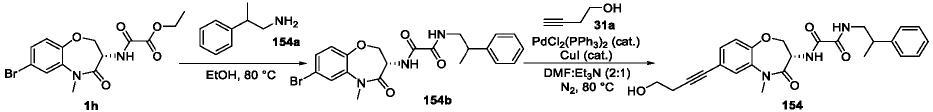

The synthesis route and method were the same as Example 32, except that the benzylamine **32a** in step 1 was replaced with an equivalent amount of 2-phenylpropan-1-amine **154a** to obtain the title compound **154** (dr = 1:1).

MS (ES⁺): *m*/*z* 450.2 [M+H]⁺.

¹H NMR (400 MHz, CDCl₃): *δ* 8.21 (d, *J* = 7.7 Hz, 1H), 7.33 - 7.15 (m, 8H), 7.08 (d, *J* = 8.7 Hz, 1H), 4.81 - 4.75 (m, 1H), 4.60 - 4.53 (m, 1H), 4.25 - 4.18 (m, 1H), 3.85 - 3.81 (m, 2H), 3.62 - 3.53 (m, 1H), 3.40, 3.39 (1:1, s, 3H), 3.37 - 3.31 (m, 1H), 2.99 - 2.92 (m, 1H), 2.70 (t, *J* = 6.2 Hz, 2H), 1.78 (t, *J* = 6.1 Hz, 1H), 1.29 (d, *J* = 7.0 Hz, 3H).

### Example 155: Synthesis of N¹-((S)-7-(4-hydroxybut-1-yn-1-yl)-5-methyl-4-oxo-2,3,4,5-tetrahydrobenzo[b][1,4]oxazepin-3-y l)-N²-((R)-2-phenylpropyl)oxalamide (155)

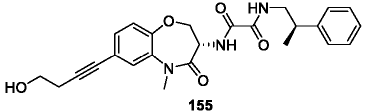

### Synthesis route:

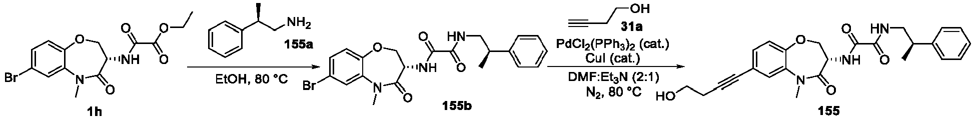

The synthesis route and method were the same as Example 32, except that the benzylamine **32a** in step 1 was replaced with an equivalent amount of (R)-2-phenylpropan-1-amine **155a** to obtain the title compound **155.**

MS (ES⁺): *m*/*z* 450.2 [M+H]⁺.

¹H NMR (400 MHz, CDCl₃): *δ* 8.21 (d, *J* = 7.4 Hz, 1H), 7.33 - 7.15 (m, 8H), 7.08 (d, *J* = 8.9 Hz, 1H), 4.82 - 4.75 (m, 1H), 4.60 - 4.54 (m, 1H), 4.25 - 4.18 (m, 1H), 3.85 - 3.81 (m, 2H), 3.62 - 3.52 (m, 1H), 3.39 (s, 3H), 3.37 - 3.31 (m, 1H), 2.99 - 2.92 (m, 1H), 2.70 (t, *J* = 6.2 Hz, 2H), 1.77 (t, *J* = 5.9 Hz, 1H), 1.29 (d, *J* = 6.9 Hz, 3H).

### Example 156: Synthesis of N¹-((S)-7-(4-hydroxybut-1-yn-1-yl)-5-methyl-4-oxo-2,3,4,5-tetrahydrobenzo[b][1,4]oxazepin-3-y l)-N²-((S)-2-phenylpropyl)oxalamide (156)

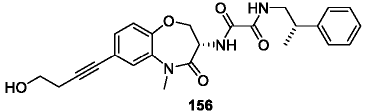

### Synthesis route:

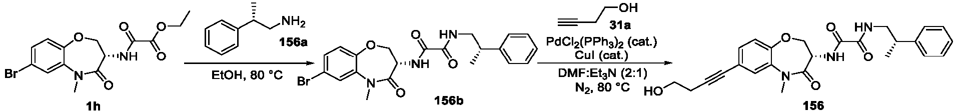

The synthesis route and method were the same as Example 32, except that the benzylamine **32a** in step 1 was replaced with an equivalent amount of (S)-2-phenylpropan-1-amine **156a** to obtain the title compound **156.**

MS (ES⁺): *m*/*z* 450.2 [M+H]⁺.

¹H NMR (400 MHz, CDCl₃): *δ* 8.21 (d, *J* = 7.1 Hz, 1H), 7.33 - 7.15 (m, 8H), 7.08 (d, *J* = 8.7 Hz, 1H), 4.82 - 4.75 (m, 1H), 4.60 - 4.54 (m, 1H), 4.25 - 4.18 (m, 1H), 3.85 - 3.81 (m, 2H), 3.62 - 3.53 (m, 1H), 3.40 (s, 3H), 3.37 - 3.31 (m, 1H), 2.99 - 2.92 (m, 1H), 2.70 (t, *J* = 6.2 Hz, 2H), 1.77 (t, *J* = 6.2 Hz, 1H), 1.29 (d, *J* = 7.0 Hz, 3H).

### Example 157: Synthesis of N¹-((S)-7-(4-hydroxybut-1-yn-1-yl)-5-isopropyl-4-oxo-2,3,4,5-tetrahydrobenzo[b][1,4]oxazepin-3-yl)-N²-(2-phenylpropyl)oxalamide (157)

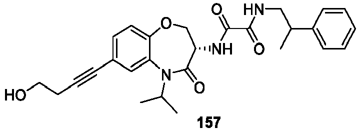

### Synthesis route:

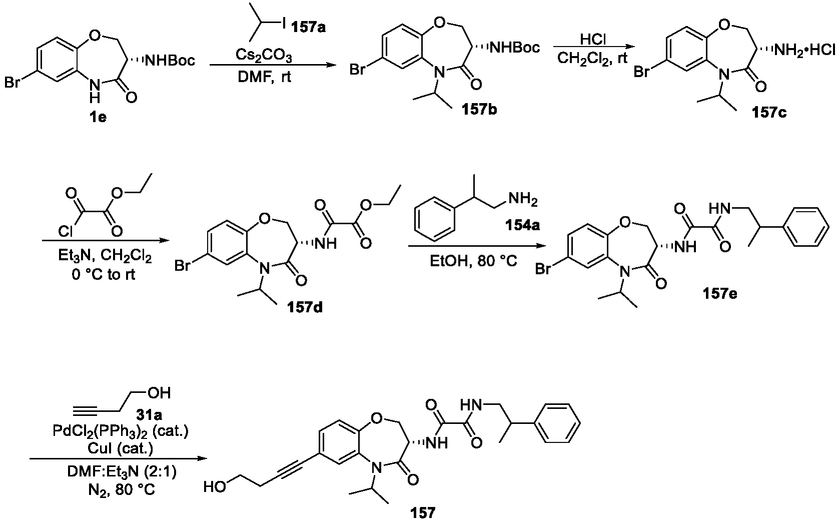

The synthesis route and method of steps 1-3 were the same as steps 4-6 in Example 1, except that the iodomethane in step 4 of Example 1 was replaced with an equivalent amount of 2-iodopropane **157a;** and the synthesis route and method of steps 4-5 were the same as Example 154, except that compound **1h** was replaced with an equivalent amount of compound **157d** to obtain the title compound **157.**

MS (ES⁺): *m*/*z* 478.2 [M+H]⁺.

¹H NMR (400 MHz, CDCl₃): *δ* 8.22 (d, *J* = 7.7 Hz, 1H), 7.33 - 7.15 (m, 8H), 7.09 (d, *J* = 8.5 Hz, 1H), 4.75 - 4.63 (m, 2H), 4.49 - 4.43 (m, 1H), 4.17 - 4.10 (m, 1H), 3.84 (t, *J* = 6.1 Hz, 2H), 3.62 - 3.53 (m, 1H), 3.40 - 3.29 (m, 1H), 2.98 - 2.90 (m, 1H), 2.70 (t, *J* = 6.3 Hz, 2H), 1.77 (br, 1H), 1.46 (dd, *J* = 6.8, 2.0 Hz, 3H), 1.28 (d, *J* = 7.0 Hz, 3H), 1.17 (d, *J* = 6.8 Hz, 3H).

### Example 158: Synthesis of N¹-((S)-7-((3-hydroxyoxetan-3-yl)ethynyl)-5-methyl-4-oxo-2,3,4,5-tetrahydrobenzo[b][1,4]oxaze pin-3-yl)-N²-((R)-2-phenylpropyl)oxalamide (158)

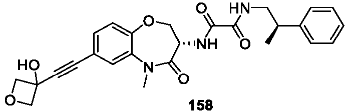

### Synthesis route:

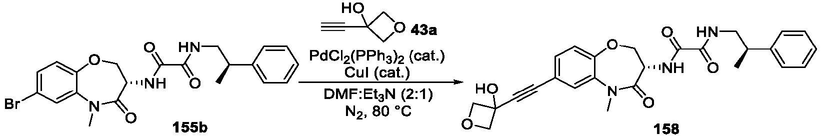

The synthesis route and method were the same as step 2 of Example 155, except that 3-butyn-1-ol **31a** was replaced with an equivalent amount of 3-ethynyloxetan-3-ol **43a** to obtain the title compound **158.**

MS (ES⁺): *m*/*z* 478.2 [M+H]+.

¹H NMR (400 MHz, CDCl₃): *δ* 8.22 (d, *J* = 7.3 Hz, 1H), 7.33 - 7.29 (m, 4H), 7.24 - 7.17 (m, 4H), 7.13 (d, *J =* 8.9 Hz, 1H), 4.93 (d, *J =* 6.7 Hz, 2H), 4.82 - 4.76 (m, 3H), 4.59 (dd, J = 9.7, 7.4 Hz, 1H), 4.25 (t, *J =* 10.6 Hz, 1H), 3.60 - 3.54 (m, 1H), 3.41 (s, 3H), 3.39 - 3.34 (m, 1H), 2.99 - 2.92 (m, 1H), 2.70 (br, 1H), 1.29 (d, *J* = 6.9 Hz, 3H).

### Example 159: Synthesis of N¹-((S)-5-methyl-7-(3-morpholinoprop-1-yn-1-yl)-4-oxo-2,3,4,5-tetrahydrobenzo[b][1,4]oxazepi n-3-yl)-N²-((R)-2-phenylpropyl)oxalamide (159)

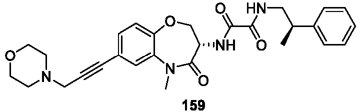

### Synthesis route:

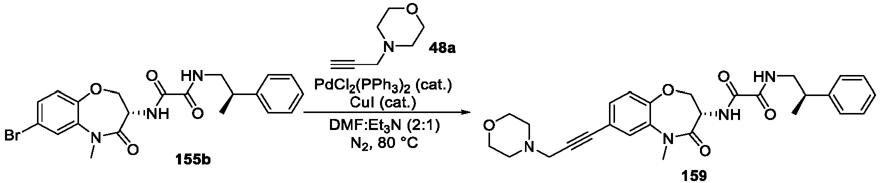

The synthesis route and method were the same as step 2 of Example 155, except that 3-butyn-1-ol **31a** was replaced with an equivalent amount of 4-(prop-2-yn-1-yl)morpholine **48a** to obtain the title compound **159.**

MS (ES⁺): *m*/*z* 505.2 [M+H]⁺.

¹H NMR (400 MHz, DMSO-*d*₆): *δ* 8.75 (d, *J* = 7.6 Hz, 1H), 8.69 (t, *J* = 6.1 Hz, 1H), 7.56 (d, *J* = 1.8 Hz, 1H), 7.33 (dd, *J* = 8.3, 1.9 Hz, 1H), 7.30 - 7.26 (m, 2H), 7.22 - 7.17 (m, 4H), 4.67 - 4.57 (m, 2H), 4.40 - 4.32 (m, 1H), 3.61 (t, *J* = 4.5 Hz, 4H), 3.51 (s, 2H), 3.38 - 3.22 (m, 9H), 3.08 - 2.97 (m, 1H), 1.14 (d, *J* = 7.0 Hz, 3H).

### Example 160: Synthesis of (S)-N¹-(5-methyl-7-(3-morpholinoprop-1-yn-1-yl)-4-oxo-2,3,4,5-tetrahydrobenzo[b][1,4]oxazepi n-3-yl)-N²-(2-phenylpropan-2-yl)oxalamide (160)

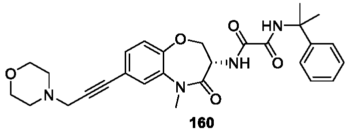

### Synthesis route:

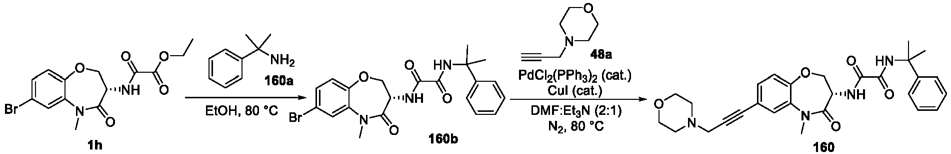

The synthesis route and method were the same as Example 32, except that the benzylamine **32a** in step 1 and the 3-butyn-1-ol **31a** in step 2 were replaced with equivalent amounts of 2-phenylpropan-2-amine **160a** and 4-(prop-2-yn-1-yl)morpholine **48a,** respectively, to obtain the title compound **160.**

MS (ES⁺): *m*/*z* 505.3 [M+H]⁺.

¹H NMR (400 MHz, DMSO-*d*₆): *δ* 8.22 (d, *J* = 7.5 Hz, 1H), 8.22 (s, 1H), 7.38 - 7.23 (m, 7H), 7.12 (d, *J* = 8.1 Hz, 1H), 4.79 (dt, *J* = 11.3, 7.4 Hz, 1H), 4.58 (dd, *J* = 9.7, 7.4 Hz, 1H), 4.22 (dd, *J=* 11.1, 9.9 Hz, 1H), 3.79 (t, *J* = 4.5 Hz, 4H), 3.52 (s, 2H), 3.39 (s, 3H), 2.66 (t, *J* = 4.6 Hz, 4H), 1.73 (s, 6H).

### Example 161: Synthesis of 4-(3-((S)-5-methyl-4-oxo-3-(2-oxo-2-(((R)-1-phenylethyl)amino)acetamido)-2,3,4,5-tetrahydrobe nzo[b][1,4]oxazepin-7-yl)prop-2-yn-1-yl)morpholine 4-oxide (161)

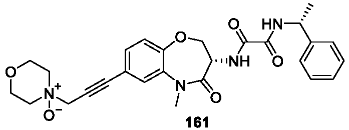

### Synthesis route:

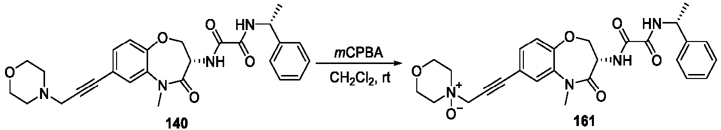

To a solution of compound **140** (25 mg, 0.05 mmol) in dichloromethane (2 mL) at room temperature, m-chloroperoxybenzoic acid (*m*CPBA, 17 mg, 0.1 mmol) was added, and the reaction solution was stirred at room temperature for 2 hours. TLC and LC-MS showed that the reaction was complete, and the product was directly concentrated under reduced pressure and purified by silica gel column chromatography (dichloromethane containing 5 - 10% methanol as the eluent) to obtain the title compound **161** (21 mg, 83% yield) as a white solid.

MS (ES⁺): *m*/*z* 507.2 [M+H]⁺.

¹H NMR (400 MHz, CDCl₃): *δ* 8.27 (d, *J* = 7.5 Hz, 1H), 7.48 (d, *J* = 8.4 Hz, 1H), 7.39 - 7.27 (m, 7H), 7.17 (d, *J* = 8.8 Hz, 1H), 5.07 (qn, *J* = 7.3 Hz, 1H), 4.81 (dt, *J =* 11.2, 7.4 Hz, 1H), 4.68 (s, 1H), 4.57 (dd, *J* = 9.7, 7.3 Hz, 1H), 4.43 (t, *J =* 14.7 Hz, 1H), 4.26 (t, *J =* 10.7 Hz, 1H), 3.92 (dd, *J* = 12.6, 3.2 Hz, 1H), 3.82 (td, *J* = 11.5, 3.3 Hz, 1H), 3.49 - 3.39 (m, 4H), 1.96 (br, 5H), 1.53 (d, *J* = 6.9 Hz, 3H).

### Example 162: Synthesis of N¹-((S)-7-(3-(4-(2-(2-(2-(((S)-1-((2S,4R)-4-hydroxy-2-((4-(4-methylthiazol-5-yl)benzyl)carbamo yl)pyrrolidin-1-yl)-3,3-dimethyl-1-oxobutan-2-yl)amino)-2-oxoethoxy)ethoxy)ethyl)piperazin-1-yl)prop-1-yn-1-yl)-5-methyl-4-oxo-2,3,4,5-tetrahydrobenzo[b][1,4]oxazepin-3-yl)-N²-((R)-1-phe nylethyl)oxalamide (162)

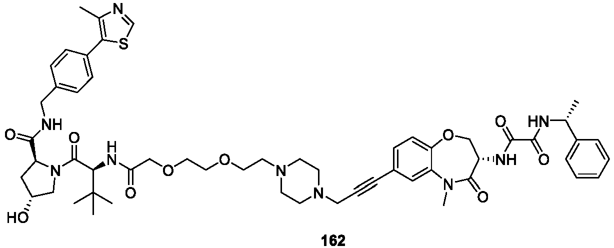

### Synthesis route:

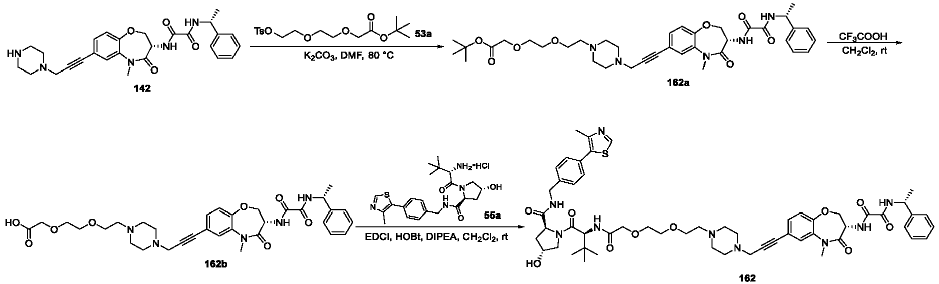

The synthesis routes and methods of steps 1, 2 and 3 were the same as Examples 53, 54 and 55, respectively, except that compound 23 was replaced with an equivalent amount of compound **142** to obtain the title compound 162.

MS (ES⁻): *m*/*z* 1046.5 [M-H]⁻.

¹H NMR (400 MHz, CDCl₃): *δ* 8.68 (s, 1H), 8.25 (d, *J* = 7.4 Hz, 1H), 7.67 - 7.51 (m, 2H), 7.38 - 7.27 (m, 11H), 7.09 (d, *J* = 8.1 Hz, 1H), 5.38 - 5.31 (m, 3H), 5.06 (qn, *J* = 7.3 Hz, 1H), 4.83 - 4.71 (m, 2H), 4.61 - 4.51 (m, 3H), 4.35 (dd, *J* = 14.9, 5.2 Hz, 1H), 4.21 (t, *J* = 10.6 Hz, 1H), 4.07 - 3.94 (m, 3H), 3.70 - 3.59 (m, 7H), 3.49 (s, 2H), 3.39 (s, 3H), 2.69 (br, 8H), 2.52 (s, 3H), 2.22 (t, *J* = 7.6 Hz, 2H), 2.04 - 1.98 (m, 2H), 1.53 (d, *J* = 6.9 Hz, 3H), 0.95 (s, 9H).

### Example 163: Synthesis of N¹-((S)-7-(3-(4-((S)-14-((2S,4R)-4-hydroxy-2-((4-(4-methylthiazol-5-yl)benzyl)carbamoyl)pyrrol idine-1-carbonyl)-15,15-dimethyl-12-oxo-3,6,9-trioxa-13-azahexadecyl)piperazin-1-yl)prop-1-yn -1-yl)-5-methyl-4-oxo-2,3,4,5-tetrahydrobenzo[b][1,4]oxazepin-3-yl)-N²-((R)-1-phenylethyl)oxal amide (163)

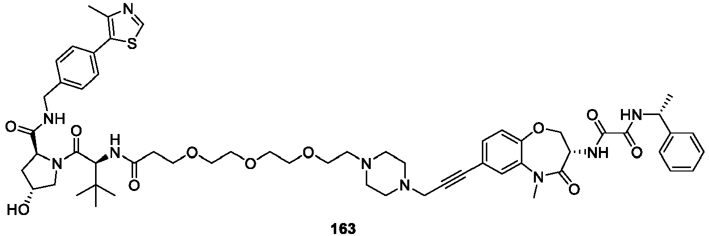

### Synthesis route:

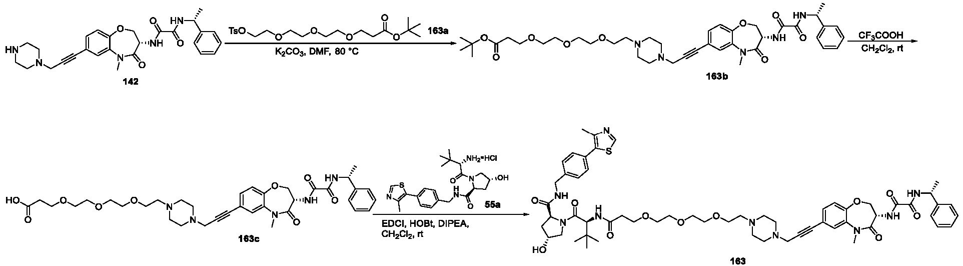

The synthesis routes and methods of steps 1, 2 and 3 were the same as Examples 53, 54 and 55, respectively, except that compound 23 and tert-butyl 2-(2-(2-(tosyloxy)ethoxy)ethoxy)acetate 53a were replaced with equivalent amounts of compound 142 and tert-butyl 3-(2-(2-(2-(tosyloxy)ethoxy)ethoxy)ethoxy)propanoate 163a, respectively, to obtain the title compound **163.**

MS (ES⁻): *m*/*z* 1104.5 [M-H]⁻.

¹H NMR (400 MHz, CDCl₃): *δ* 8.67 (s, 1H), 8.27 (d, *J* = 7.3 Hz, 1H), 7.68 - 7.41 (m, 2H), 7.37 - 7.27 (m, 11H), 7.11 - 7.02 (m, 2H), 5.06 (qn, *J* = 7.2 Hz, 1H), 4.83 - 4.70 (m, 2H), 4.61 - 4.48 (m, 4H), 4.33 (dd, *J* = 15.0, 5.2 Hz, 1H), 4.22 (t, *J* = 10.6 Hz, 1H), 4.10 (d, *J* = 11.3 Hz, 1H), 3.75 - 3.56 (m, 14H), 3.50 (s, 2H), 3.39 (s, 3H), 2.71 - 2.61 (m, 8H), 2.51 (s, 3H), 2.49 - 2.44 (m, 2H), 2.23 - 2.10 (m, 4H), 1.52 (d, *J* = 6.9 Hz, 3H), 0.93 (s, 9H).

### Example 164: N¹-((3S)-7-(3-(4-(2-(2-(2-(3-((2-(2,6-dioxopiperidin-3-yl)-1-oxoisoindolin-4-yl)amino)-3-oxopro poxy)ethoxy)ethoxy)ethyl)piperazin-1-yl)prop-1-yn-1-yl)-5-methyl-4-oxo-2,3,4,5-tetrahydrobenz o[b],4]oxazepin-3-yl)-N²-((R)-1-phenylethyl)oxalamide (164)

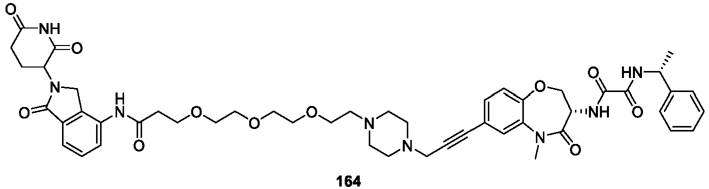

### Synthesis route:

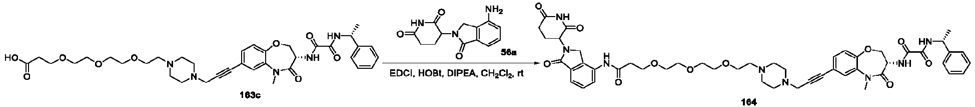

The synthesis route and method were the same as step 3 of Example 163, except that (2*R*,4*S*)-1-((*R*)-2-amino-3,3-dimethylbutanoyl)-4-hydroxy-*N*-(4-(4-methylthiazol-5-yl)benzyl)pyr rolidine-2-carboxamide hydrochloride **55a** was replaced with an equivalent amount of 3-(4-amino-1-oxoisoindolin-2-yl)piperidine-2,6-dione **56a** (lenalidomide) to obtain the title compound **164.**

MS (ES⁺): *m*/*z* 935.3 [M+H]⁺.

¹H NMR (400 MHz, CDCl₃): *δ* 9.66 (br, 1H), 9.24 (s, 1H), 8.25 (d, *J* = 7.5 Hz, 1H), 7.92 (d, *J* = 7.9 Hz, 1H), 7.66 (d, *J* = 7.4 Hz, 1H), 7.60 - 7.54 (m, 1H), 7.45 (t, *J =* 7.7 Hz, 1H), 7.38 (s, 1H), 7.35 - 7.28 (m, 5H), 7.09 (d, *J* = 8.2 Hz, 1H), 5.20 - 5.12 (m, 1H), 5.09 - 5.00 (m, 1H), 4.82 - 4.76 (m, 1H), 4.57 - 4.43 (m, 3H), 4.21 (t, *J* = 10.6 Hz, 1H), 3.83 (t, *J* = 5.4 Hz, 2H), 3.68 - 3.61 (m, 6H), 3.60 - 3.55 (m, 2H), 3.51 - 3.48 (m, 3H), 3.47 (s, 2H), 3.40 (s, 3H), 2.89 - 2.68 (m, 10H), 2.41 - 2.28 (m, 2H), 2.23 - 2.15 (m, 2H), 2.04 - 1.97 (m, 2H), 1.51 (d, *J* = 6.9 Hz, 3H).

### Example 165: Synthesis of N¹-((S)-1-methyl-8-(3-morpholinoprop-1-yn-1-yl)-2-oxo-2,3,4,5-tetrahydro-1H-benzo[b][1,4]dia zepin-3-yl)-N²-((R)-1-phenylethyl)oxalamide (165)

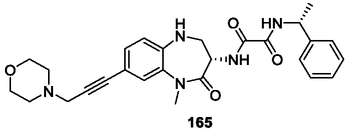

### Synthesis route:

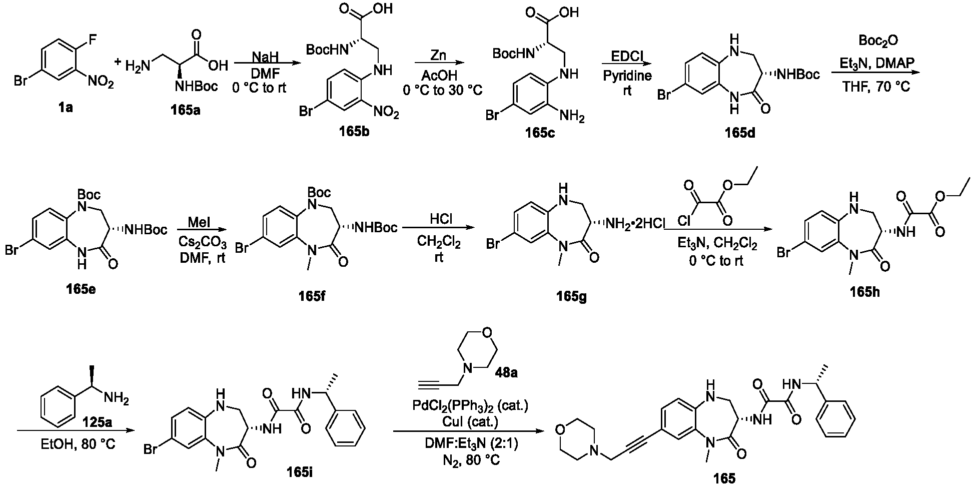

### Step 1: Synthesis of (S)-3-((4-bromo-2-nitrophenyl)amino)-2-((tert-butoxycarbonyl)amino)propanoic acid (165b)

A solution of (S)-3-amino-2-((tert-butoxycarbonyl)amino)propanoic acid **165a** (4.4 g, 21.54 mmol) in DMF (10 mL) was added to the suspension of sodium hydride (60% w/w dispersed in mineral oil, 1.8 g, 45 mmol) in DMF (N,N-dimethylformamide, 50mL) at 0 °C in an ice bath. After the reaction solution was stirred for 1 hour, a solution of 4-bromo-1-fluoro-2-nitrobenzene **1a** (5 g, 22.73 mmol) in DMF (20 mL) was added dropwise to the reaction solution at 0 °C in an ice bath, and then the reaction solution was slowly warmed to room temperature and stirred for another 15 hours. TLC and LC-MS showed that the reaction was complete, and the reaction mixture was poured into ice water (200 mL), and the aqueous phase was acidified to pH = 4 with dilute hydrochloric acid (1 N), and then extracted with ethyl acetate (100 mL × 5). The combined organic layers were washed with water (100 mL × 2) and saturated brine (100 mL) in sequence, dried over anhydrous sodium sulfate, and concentrated under reduced pressure to obtain a crude product, which was then purified by silica gel column chromatography (dichloromethane containing 1% methanol as the eluent) to obtain compound **165b** (6 g, 69% yield) as a yellow solid.

MS (ES⁺): *m*/*z* 403.9 [M+H]⁺.

### Step 2: Synthesis of (S)-3-((2-amino-4-bromophenyl)amino)-2-((tert-butoxycarbonyl)amino)propanoic acid (165c)

Zinc powder (5.7 g, 87 mmol) was added to a solution of compound **165b** (6 g, 14.84 mmol) in acetic acid (60 mL) at 0 °C in an ice bath, and the resulting mixture was stirred at 30 °C for 48 hours. TLC and LC-MS showed that the reaction was complete. After the reaction mixture was filtered through celite, the filter cake was rinsed with dichloromethane (300 mL) and the filtrate was concentrated under reduced pressure. The residue after concentration was then dissolved in water (50 mL), neutralized with saturated aqueous solution of sodium bicarbonate to pH = 7, and then extracted with dichloromethane (100 mL × 3). The organic layers were combined, washed with saturated brine (100 mL), dried over anhydrous sodium sulfate and concentrated under reduced pressure to obtain a crude product of compound **165c** (6.6 g) as a black solid, which was used directly in the next step without further purification.

MS (ES⁺): *m*/*z* 373.9 [M+H]⁺.

### Step 3: Synthesis of tert-butyl (S)-(8-bromo-2-oxo-2,3,4,5-tetrahydro-1H-benzo[b][1,4]diazepin-3-yl)carbamate (165d)

1-Ethyl-3-(3-dimethylaminopropyl)carbodiimide hydrochloride (EDCI, 3.38 g, 17.63 mmol) was added to a solution of compound **165c** (6.6 g, 17.64 mmol) in pyridine (200 mL) at room temperature, and the reaction solution was stirred at room temperature for 15 hours. TLC and LC-MS showed that the reaction was complete. After the reaction solution was concentrated under reduced pressure to remove most of the pyridine solvent, the reaction mixture was poured into saturated aqueous solution of citric acid (100 mL) and extracted with ethyl acetate (100 mL × 3). The organic layers were combined, washed with saturated brine (100 mL), dried over anhydrous sodium sulfate and concentrated under reduced pressure, and then purified by silica gel column chromatography (petroleum ether containing 30% ethyl acetate as the eluent) to obtain compound **165d** (3 g, 57% two-step yield) as a white solid.

MS (ES⁺): *m*/*z* 355.9 [M+H]⁺.

¹H NMR (400 MHz, DMSO-*d*₆): *δ* 9.79 (s, 1H), 7.07 - 7.05 (m, 2H), 6.91 (d, *J* = 8.0 Hz, 1H), 6.77 (d, *J =* 8.4 Hz, 1H), 5.90 (d, *J =* 5.6 Hz, 1H), 4.17 - 4.11 (m, 1H), 3.46 - 3.43 (m, 1H), 3.31 - 3.26 (m, 1H), 1.37 (s, 9H).

### Step 4: Synthesis of tert-butyl (S)-7-bromo-3-((tert-butoxycarbonyl)amino)-4-oxo-2,3,4,5-tetrahydro-1H-benzo[b][1,4]diazepin e-1-carboxylate (165e)

Compound **165d** (1.0 g, 2.8 mmol), di-tert-butyl dicarbonate (808 mg, 3.7 mmol), 4-dimethylaminopyridine (37 mg, 0.3 mmol), triethylamine (708 mg, 7.0 mmol) and tetrahydrofuran (10 mL) were added to a reaction flask at room temperature. The reaction mixture was heated to 70 °C and stirred for 15 hours. TLC and LC-MS showed that the reaction was complete. The reaction mixture was poured into water (20 mL) and extracted with ethyl acetate (30 mL × 3). The combined organic layers were washed with saturated brine (30 mL), dried over anhydrous sodium sulfate and concentrated under reduced pressure, and then purified by silica gel column chromatography (petroleum ether containing 50% dichloromethane as the eluent) to obtain compound **165e** (640 mg, 50% yield) as a white solid.

MS (ES⁺): *m*/*z* 456.0 [M+H]⁺.

¹H NMR (400 MHz, DMSO-*d*₆): *δ* 9.16 (s, 1H), 7.94 (s, 1H), 6.67 (d, *J* = 8.4 Hz, 1H), 7.32 - 7.79 (m, 1H), 7.22 (d, *J =* 8.4 Hz, 1H), 4.80 - 4.76 (m, 1H), 3.93 (t, *J =* 5.6 Hz, 1H), 3.69 - 3.67 (m, 1H), 1.46 (s, 9H), 1.40 (s, 9H).

### Step 5: Synthesis of tert-butyl (S)-7-bromo-3-((tert-butoxycarbonyl)amino)-5-methyl-4-oxo-2,3,4,5-tetrahydro-1H-benzo[b][1,4 ]diazepine-1-carboxylate (165f)

Methyl iodide (213 mg, 1.5 mmol) was added to a mixture of compound **165e** (640 mg, 1.4 mmol) and cesium carbonate (593 mg, 1.8 mmol) in DMF (10 mL) at room temperature under nitrogen protection, and the reaction mixture was stirred at room temperature for 6 hours. TLC and LC-MS showed that the reaction was complete. The reaction mixture was poured into ice water (40 mL), and solid precipitated. After stirring for 10 minutes, it was filtered, and the filter cake was collected. Then the filter cake was triturated with petroleum ether (50 mL) containing 15% ethyl acetate to obtain compound **165f** (320 mg, 38% yield) as a white solid.

MS (ES⁺): *m*/*z* 469.9 [M+H]⁺.

### Step 6: Synthesis of (S)-3-amino-8-bromo-1-methyl-1,3,4,5-tetrahydro-2H-benzo[b][1,4]diazepine-2-one hydrochloride (165g)

A solution of hydrogen chloride in 1,4-dioxane (4 N, 5 mL) was added dropwise to a solution of compound **165f** (282 mg, 0.6 mmol) in dichloromethane (5 mL) at room temperature, and the reaction solution was stirred at room temperature for 4 hours. TLC and LC-MS showed that the reaction was complete, and the solvent and other volatiles were removed by concentration under reduced pressure, and then the mixture was triturated with ethyl acetate (30 mL) containing 7% methanol to obtain compound **165g** (152 mg, 74% yield) as a white solid.

MS (ES⁺): *m*/*z* 269.8 [M+H]⁺.

¹H NMR (400 MHz, DMSO-*d*₆): *δ* 9.17 (s, 3H), 7.05 (d, *J* = 8.0 Hz, 1H), 6.76 - 6.72 (m, 2H), 5.09 (br, 2H), 4.59 (s, 1H), 3.84 (t, *J* = 5.6 Hz, 1H), 3.79 - 3.77 (m, 1H), 2.71 (s, 3H).

### Step 7: Synthesis of ethyl (S)-2-((8-bromo-1-methyl-2-oxo-2,3,4,5-tetrahydro-1H-benzo[b][1,4]diazepin-3-yl)amino)-2-oxo acetate (165h)

A solution of monoethyl oxalyl chloride (72 mg, 0.53 mmol) in dichloromethane (1 mL) was added dropwise to a solution of compound **165g** (152 mg, 0.44 mmol) and triethylamine (178 mg, 1.76 mmol) in dichloromethane (8 mL) at 0 °C in an ice bath, and the reaction solution was warmed slowly to room temperature, and stirred for another 3 hours. TLC and LC-MS showed that the reaction was complete, and the reaction mixture was poured into water (10 mL) and extracted with dichloromethane (15 mL × 3). The organic layers were combined, washed with saturated brine (100 mL), dried over anhydrous sodium sulfate and concentrated under reduced pressure, and then purified by silica gel column chromatography (petroleum ether containing 30% ethyl acetate as the eluent) to obtain compound **165h** (117 mg, 72% yield) as a white solid.

MS (ES⁺): *m*/*z* 370.0 [M+H]⁺.

### Step 8: Synthesis of N¹-((S)-8-bromo-1-methyl-2-oxo-2,3,4,5-tetrahydro-1H-benzo[b][1,4]diazepin-3-yl)-N²-((R)-1-p henylethyl)oxalamide (165i)

(*R*)-1-Phenylethylamine **125a** (75 mg, 0.62 mmol) was added to a solution of compound **165h** (100 mg, 0.27 mmol) in ethanol (10 mL), and the reaction solution was heated to 80 °C and stirred for 4 hours. TLC and LC-MS showed that the reaction was complete. The reaction solution was cooled to room temperature and concentrated under reduced pressure, and then triturated with petroleum ether (30 mL) containing 15% ethyl acetate to obtain compound **165i** (96 mg, 80% yield) as a white solid.

MS (ES⁺): *m*/*z* 445.1 [M+H]⁺.

### Step 9: Synthesis of N¹-((S)-1-methyl-8-(3-morpholinoprop-1-yn-1-yl)-2-oxo-2,3,4,5-tetrahydro-1H-benzo[b][1,4]dia zepin-3-yl)-N²-((R)-1-phenylethyl)oxalamide (165)

Compound **165i** (44.5 mg, 0.1 mmol), bis(triphenylphosphine)palladium(II) chloride (7.0 mg, 0.01 mmol), cuprous iodide (1.9 mg, 0.01 mmol), triethylamine (1 mL), DMF (2 mL) and 4-(prop-2-yn-1-yl)morpholine **48a** (25.0 mg, 0.2 mmol) was added in turn to a sealed tube, and the air in the tube was quickly replaced with nitrogen three times. The reaction solution was kept stirring at 80 °C for 15 hours. TLC and LC-MS showed that the reaction was complete. After the reaction solution was cooled to room temperature, it was quenched with water (20 mL) and extracted with ethyl acetate (10 mL × 3). The combined organic layers were washed with saturated brine (10 mL), dried over anhydrous sodium sulfate, and concentrated under reduced pressure to obtain a crude product, which was then purified by silica gel column chromatography (dichloromethane containing 0.5% methanol as the eluent) to obtain the title compound 165 (30.3 mg, 62% yield) as a white solid.

MS (ES⁺): *m*/*z* 490.2 [M+H]⁺.

¹H NMR (400 MHz, CDCl₃): *δ* 8.09 (d, *J* = 8.0 Hz, 1H), 7.60 (d, *J* = 8.4 Hz, 1H), 7.37 - 7.29 (m, 5H), 6.75 - 6.73 (m, 3H), 5.74 (br, 1H), 5.06 (qn, *J* = 7.2 Hz, 1H), 4.99 - 4.92 (m, 1H), 3.95 - 3.91 (m, 1H), 3.87 - 3.83 (m, 1H), 3.78 (t, *J* = 4.4 Hz, 4H), 3.50 (s, 2H), 2.85 (s, 3H), 2.65 (t, *J* = 4.4 Hz, 4H), 1.55 (d, *J* = 6.9 Hz, 3H).

### Example 166: Synthesis of N¹-((S)-1,5-dimethyl-8-(3-morpholinoprop-1-yn-1-yl)-2-oxo-2,3,4,5-tetrahydro-1H-benzo[b][1,4] diazepin-3-yl)-N²-((R)-1-phenylethyl)oxalamide (166)

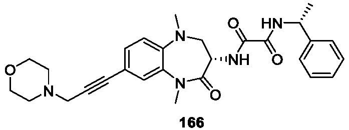

### Synthesis route:

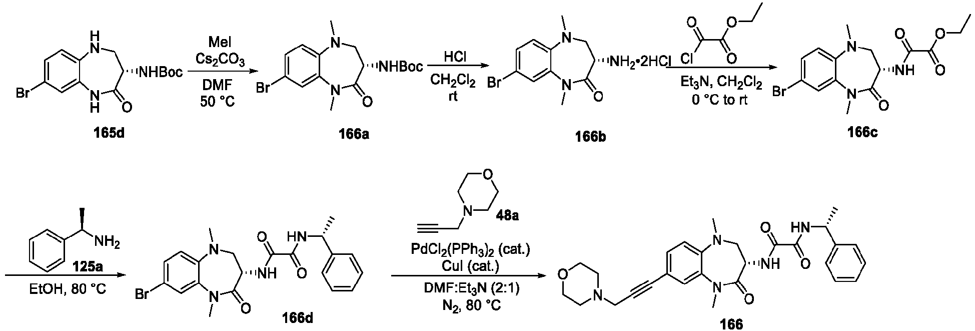

The synthesis route and method of steps 1-5 were the same as steps 5-9 of Example 165, except that the reaction temperature of step 5 of Example 165 was changed from room temperature to 50 °C and compound **165e** was replaced with an equivalent amount of compound **165d** to obtain the title compound **166.**

MS (ES⁺): *m*/*z* 504.3 [M+H]⁺.

¹H NMR (400 MHz, CDCl₃): *δ* 8.30 (d, *J* = 7.6 Hz, 1H), 7.50 (d, *J* = 8.4 Hz, 1H), 7.36 - 7.27 (m, 6H), 7.23 (d, *J =* 1.8 Hz, 1H), 6.99 (d, *J =* 8.4 Hz, 1H), 5.07 (qn, *J* = 7.3 Hz, 1H), 4.54 (dt, *J =* 11.5, 7.2 Hz, 1H), 3.78 (t, *J =* 4.6 Hz, 4H), 3.50 (s, 2H), 3.44 (dd, *J* = 9.4, 6.8 Hz, 1H), 3.37 (s, 3H), 3.32 (dd, *J =* 11.4, 9.6 Hz, 1H), 2.75 (s, 3H), 2.64 (t, *J =* 4.4 Hz, 4H), 1.53 (d, *J =* 6.9 Hz, 3H).

### Example 167: Synthesis of N¹-(3-fluorophenethyl)-N²-(8-(4-hydroxybut-1-yn-1-yl)-1-methyl-2-oxo-2,3,4,5-tetrahydro-1H-b enzo[b]azepin-3-yl)oxalamide (167)

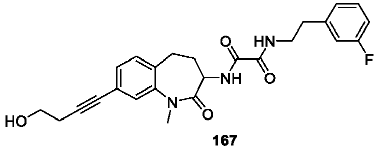

### Synthesis route:

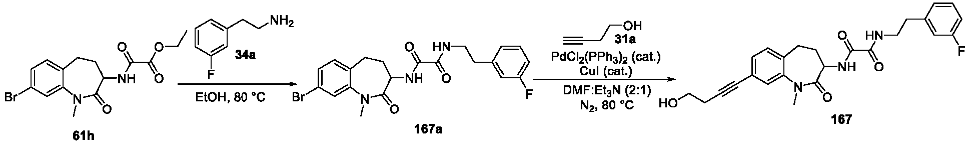

The synthesis route and method were the same as Example 34, except that compound **1h** in step 1 was replaced with an equivalent amount of compound **61h** to obtain the title compound **167.**

MS (ES⁺): *m*/*z* 452.2 [M+H]⁺.

¹H NMR (400 MHz, CDCl₃): *δ* 8.25 (d, *J* = 7.5 Hz, 1H), 7.33 (t, *J* = 6.0 Hz, 1H), 7.28 - 7.23 (m, 3H), 7.16 (d, *J =* 7.7 Hz, 1H), 7.00 - 6.87 (m, 3H), 4.38 (dt, *J =* 11.0, 7.6 Hz, 1H), 3.83 (q, *J =* 6.3 Hz, 2H), 3.58 - 3.52 (m, 2H), 3.40 (s, 3H), 2.89 - 2.80 (m, 3H), 2.70 (t, *J* = 6.3 Hz, 2H), 2.65 - 2.53 (m, 2H), 2.05 - 1.96 (m, 1H), 1.78 (t, *J* = 6.3 Hz, 1H).

### Example 168: Synthesis of N¹-(8-(4-hydroxybutyl)-1-methyl-2-oxo-2,3,4,5-tetrahydro-1H-benzo[b]azepin-3-yl)-N²-phenethy loxalamide (168)

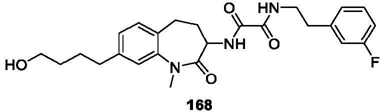

### Synthesis route:

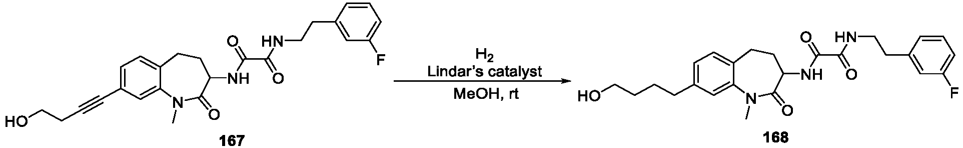

Compound **167** (13 mg, 0.03 mmol), Lindlar catalyst (3 mg) and methanol (1 mL) were added in turn to a sealed tube at room temperature. The air in the tube was quickly replaced with hydrogen three times, and a hydrogen balloon was added. Under the protection of hydrogen atmosphere, the reaction solution was stirred at room temperature for 15 hours. The reaction solution was quenched with water (5 mL) and extracted with dichloromethane (5 mL × 3). The combined organic layers were concentrated under reduced pressure to obtain a crude product, which was purified by Prep-HPLC (water containing 30 - 80% acetonitrile as the mobile phase) to obtain compound **168** (3 mg, 22% yield) as a white solid.

MS (ES⁺): *m*/*z* 456.2 [M+H]⁺.

¹H NMR (400 MHz, CDCl₃): *δ* 8.27 (d, *J =* 6.3 Hz, 1H), 7.34 - 7.27 (m, 3H), 7.23 - 7.17 (m, 3H), 7.12 (d, *J =* 7.6 Hz, 1H), 7.02 - 6.98 (m, 2H), 4.41 (dt, *J =* 11.1, 7.6 Hz, 1H), 3.68 (q, *J* = 5.4 Hz, 2H), 3.62 - 3.49 (m, 2H), 3.40 (s, 3H), 2.86 - 2.78 (m, 3H), 2.66 - 2.53 (m, 4H), 2.05 - 1.94 (m, 1H), 1.75 - 1.60 (m, 4H).

### In vitro kinase activity assay

RIPK1 kinase activity was assayed using the ADP-Glo assay and RIPK1 kinase reaction system (Promega, catalog number: VA7593). The RIPK1 kinase activity assay was performed according to the Promega kit mannual.

### Inhibition of RIPK1 kinase by compounds (1 µM)

The experimental method was briefly described as below. The concentration of RIPK1 kinase in the RIPK1 kinase reaction system was determined by kinase titration and S/B10. Compounds were diluted with 1× kinase buffer D (1% DMSO), and 1 µL of 100 µM compound DMSO stock solution was added to 19 µL 1× kinase buffer D. RIPK1 kinase was diluted with 1× kinase buffer D. 2.5× ATP/substrate was diluted with 1× kinase buffer D. The final concentration of ATP in the kinase reaction system was 10 µM, and the final concentration of MBP substrate was 100 µg/mL. 1 µL of diluted compound and 2 µL of diluted RIPK1 kinase were mixed, incubated at room temperature for 15 minutes, 2 µL of 2.5× ATP/substrate was added, mixed, and incubated at room temperature for 1 hour. 5 µL of ADP-Glo reagent was added to each well and incubated at room temperature for 1 hour. 10 µL of ADP detection reagent was added to each well and incubated at room temperature for another 0.5-1 hour. The chemiluminescent signal was read by a microplate reader and the inhibition rate of kinase activity was calculated.

The inhibition rate range of key compounds on RIPK1 kinase activity at 1 µM was showed in Table 1: A: ≥75%; B: 50 - 74%; C: 25 - 49%.

**Table 1 Inhibition rate range of compounds (1 µM) on RIPK1 kinase activity**

| **Compound No.** | **RIPK1 kinase activity inhibition rate range** |
|---|---|
| 1 | B |
| 2 | A |
| 3 | A |
| 5 | A |
| 6 | B |
| 8 | A |
| 9 | A |
| 10 | A |
| 11 | A |
| 12 | A |
| 13 | A |
| 14 | A |
| 15 | A |
| 16 | B |
| 17 | A |
| 18 | A |
| 19 | A |
| 20 | B |
| 21 | A |
| 22 | A |
| 23 | B |
| 24 | B |
| 25 | A |
| 26 | A |
| 27 | B |
| 28 | A |
| 29 | A |
| 30 | B |
| 31 | A |
| 32 | A |
| 33 | A |
| 34 | B |
| 35 | B |
| 36 | A |
| 37 | B |
| 38 | B |
| 39 | A |
| 40 | A |
| 41 | A |
| 42 | A |
| 43 | A |
| 44 | B |
| 45 | A |
| 46 | A |
| 47 | A |
| 48 | B |
| 49 | B |
| 50 | A |
| 51 | C |
| 52 | A |
| 53 | C |
| 54 | B |
| 55 | B |
| 56 | C |
| 57 | A |
| 58 | C |
| 61 | A |
| 62 | A |
| 63 | C |
| 65 | A |
| 66 | B |
| 68 | A |
| 69 | A |
| 71 | B |
| 72 | A |
| 73 | A |
| 74 | B |
| 75 | C |
| 76 | B |
| 77 | B |
| 78 | B |
| 79 | B |
| 80 | A |
| 81 | A |
| 82 | A |
| 83 | A |
| 84 | A |
| 85 | A |
| 86 | B |
| 87 | C |
| 88 | A |
| 89 | B |
| 91 | A |
| 92 | B |
| 93 | A |
| 94 | A |
| 95 | A |
| 96 | A |
| 97 | A |
| 98 | A |
| 99 | B |
| 100 | A |
| 101 | C |
| 102 | C |
| 103 | C |
| 104 | A |
| 105 | A |
| 106 | A |
| 107 | B |
| 108 | A |
| 109 | A |
| 110 | A |
| 113 | A |
| 114 | A |
| 115 | B |
| 116 | B |
| 117 | B |
| 118 | A |
| 119 | C |
| 120 | A |
| 121 | A |
| 122 | A |
| 123 | B |
| 124 | B |
| 126 | A |
| 128 | A |
| 130 | A |
| 131 | A |
| 132 | B |
| 134 | B |
| 135 | C |
| 136 | C |
| 137 | A |
| 138 | A |
| 139 | A |
| 140 | A |
| 142 | A |
| 143 | A |
| 144 | A |
| 145 | A |
| 146 | B |
| 147 | A |
| 148 | A |
| 149 | A |
| 151 | C |
| 152 | B |
| 153 | B |
| 154 | A |
| 155 | A |
| 156 | B |
| 158 | A |
| 159 | A |
| 160 | A |
| 161 | B |
| 162 | A |
| 163 | A |
| 164 | A |
| 166 | C |
| 167 | A |
| 168 | A |

### In vitro kinase activity IC50 assay

The experimental method was briefly described as below. The concentration of RIPK1 kinase in the RIPK1 kinase reaction system was determined by kinase titration and S/B10. The compound was diluted with 1× kinase buffer D (1% DMSO), 1 µL of 1 mM compound DMSO stock solution was added to 19 µL 1× kinase buffer D, and the compound was serially diluted with 1× kinase buffer D at a ratio of 1:5, for a total of 8 concentration points, and a control with the same DMSO volume was set. RIPK1 kinase was diluted with 1× kinase buffer D. 2.5× ATP/substrate was diluted with 1× kinase buffer D. The final concentration of ATP in the kinase reaction system was 10 µM. The final concentration of MBP substrate was 100 µg/mL. 1 µL of diluted compound and 2 µL of diluted RIPK1 kinase were mixed, incubated at room temperature for 15 minutes, 2 µL of 2.5× ATP/substrate was added, mixed, and incubated at room temperature for 1 hour. 5 µL ADP-Glo reagent was added to each well and incubated at room temperature for 1 hour. 10 µL ADP detection reagent was added to each well and incubated at room temperature for another 0.5-1 hour. The chemiluminescent signal was read with a microplate reader and the kinase activity inhibition rate was calculated. IC50 was obtained by fitting the nonlinear regression curve log [inhibitor] vs. response - variable slope (four parameters) in GraphPad Prism.

Table 2 below provides the IC50 range of some compounds on RIPK1 kinase activity: A<100 nM.

**Table 2 IC50 range of compounds for RIPK1 kinase activity**

| **Compound No.** | **RIPK1 kinase activity inhibition rate range** |
|---|---|
| 13 | A |
| 17 | A |
| 18 | A |
| 19 | A |
| 21 | A |
| 22 | A |
| 24 | A |
| 25 | A |
| 29 | A |
| 31 | A |
| 32 | A |
| 33 | A |
| 34 | A |
| 35 | A |
| 36 | A |
| 37 | A |
| 39 | A |
| 40 | A |
| 41 | A |
| 42 | A |
| 43 | A |
| 44 | A |
| 45 | A |
| 46 | A |
| 47 | A |
| 48 | A |
| 50 | A |
| 51 | A |
| 52 | A |
| 61 | A |
| 62 | A |
| 65 | A |
| 66 | A |
| 74 | A |
| 76 | A |
| 77 | A |
| 78 | A |
| 79 | A |
| 80 | A |
| 81 | A |
| 82 | A |
| 84 | A |
| 85 | A |
| 86 | A |
| 88 | A |
| 89 | A |
| 91 | A |
| 92 | A |
| 94 | A |
| 96 | A |
| 97 | A |
| 98 | A |
| 104 | A |
| 106 | A |
| 107 | A |
| 109 | A |
| 110 | A |
| 113 | A |
| 114 | A |
| 115 | A |
| 117 | A |
| 118 | A |
| 120 | A |
| 122 | A |
| 123 | A |
| 125 | A |
| 126 | A |
| 128 | A |
| 130 | A |
| 132 | A |
| 137 | A |
| 138 | A |
| 139 | A |
| 140 | A |
| 144 | A |
| 145 | A |
| 146 | A |
| 148 | A |
| 154 | A |
| 155 | A |
| 156 | A |
| 158 | A |
| 159 | A |
| 167 | A |
| 168 | A |

### Inhibitory activity of necroptosis in U937 cells

The biological activity of RIPK1 inhibitors was further evaluated in the inhibition of necroptosis in U937 cells. U937 cell line was purchased from Huatuo Biotechnology and stored in liquid nitrogen. U937 cells were revived before the experiment and cultured in RPMI-1640 medium (supplemented with 10% fetal bovine serum, 100 U/mL penicillin and 100 µg/mL streptomycin) at 37 °C, 5% CO₂. The activity was further evaluated by the cellular ATP level detected by the Promega CellTiter-Glo 2.0 Assay cell viability assay kit (Catalog No.: G9242).

### Inhibition rate of compound (1 µM) on necroptosis of U937 cells

The experimental method was briefly described as below. U937 cells in the logarithmic growth phase were collected by centrifugation. Cells were diluted at the density of 5×10⁵ cells/mL with RPMI 1640 medium (supplemented with 10% fetal bovine serum, 100 U/mL penicillin and 100 µg/mL streptomycin). The cell suspension was added to a white sterile 384-well cell culture plate at 15 µL/well. The test compound was dissolved in DMSO to prepare a 10 mM stock solution and diluted to 10 µM with culture medium. At the same time, a control with the same DMSO volume was set up. Two replicates were added for each concentration. The diluted compound was added to the 384-well cell culture plate containing cells at 2.5 µL/well and incubated at 37 °C, 5% CO₂ for 15 minutes. Z-VAD-FMK (Biyuntian, catalog number: C1202-5 mg) was dissolved in DMSO to prepare a 20 mM stock solution. SM-164 (Biyuntian, catalog number: SC0114-10 mM) was dissolved in DMSO to prepare a 10 mM stock solution and diluted to 0.1 mM. hTNF-α (Sino Biological Inc., catalog number: 10602-HNAE) was dissolved in sterile water to 10 µg/mL. RPMI 1640 medium (supplemented with 10% fetal bovine serum, 100 U/mL penicillin and 100 µg/mL streptomycin) was used to prepare the TSZ mixture, containing 133.3 µM Z-VAD-FMK, 33.3 ng/mL hTNF-α and 0.3 µM SM-164. After 15 minutes' coculture of compounds and cells, 7.5 µL of TSZ mixture was added to each well, mixed evenly, and cultured at 37 °C, 5% CO₂ for 20 hours. After 20 hours, the culture plate was removed and placed at room temperature for 30 minutes. 10 µL of CellTiter-Glo reagent pre-equilibrated at room temperature was added to each well and incubated for 10 minutes from light. The chemiluminescence was read by a microplate reader. The inhibition rate was calculated as Inh% = (1-(no TSZ-TSZ & compound)/(no TSZ-TSZ))×100.

Table 3 below provides the inhibition rate range of some compounds at 1 µM on U937 cell necroptosis: A: ≥90%; B: 50 - 89%.

**Table 3 Inhibition rate range of compounds (1 µM) on U937 cell necroptosis**

| **Compound No.** | **RIPK1 kinase activity inhibition rate range** |
|---|---|
| 1 | A |
| 2 | A |
| 3 | A |
| 4 | B |
| 5 | A |
| 6 | A |
| 8 | A |
| 9 | A |
| 10 | A |
| 11 | A |
| 12 | A |
| 13 | A |
| 14 | A |
| 15 | A |
| 16 | A |
| 17 | A |
| 18 | A |
| 19 | A |
| 20 | A |
| 21 | A |
| 22 | B |
| 23 | A |
| 24 | A |
| 25 | A |
| 26 | A |
| 27 | A |
| 28 | A |
| 29 | A |
| 30 | A |
| 31 | A |
| 32 | A |
| 33 | A |
| 34 | A |
| 35 | A |
| 36 | A |
| 37 | A |
| 38 | A |
| 39 | A |
| 40 | A |
| 41 | A |
| 42 | A |
| 43 | A |
| 44 | A |
| 45 | A |
| 46 | A |
| 47 | A |
| 48 | A |
| 49 | A |
| 50 | A |
| 51 | A |
| 52 | A |
| 53 | A |
| 54 | B |
| 55 | A |
| 56 | A |
| 57 | A |
| 58 | B |
| 61 | A |
| 62 | A |
| 63 | A |
| 64 | B |
| 65 | A |
| 66 | A |
| 67 | A |
| 68 | B |
| 69 | B |
| 71 | B |
| 72 | A |
| 73 | A |
| 74 | A |
| 75 | A |
| 76 | A |
| 77 | A |
| 78 | A |
| 79 | A |
| 80 | A |
| 81 | A |
| 82 | A |
| 83 | A |
| 84 | A |
| 85 | B |
| 86 | A |
| 87 | B |
| 88 | A |
| 89 | A |
| 90 | B |
| 91 | A |
| 92 | A |
| 93 | A |
| 94 | B |
| 95 | B |
| 96 | B |
| 97 | A |
| 98 | A |
| 99 | B |
| 100 | B |
| 102 | B |
| 104 | A |
| 105 | A |
| 106 | B |
| 107 | A |
| 108 | A |
| 109 | A |
| 110 | A |
| 112 | A |
| 113 | A |
| 114 | A |
| 115 | A |
| 116 | A |
| 117 | A |
| 118 | A |
| 119 | A |
| 120 | A |
| 121 | B |
| 122 | A |
| 123 | B |
| 124 | A |
| 125 | A |
| 126 | A |
| 127 | B |
| 128 | B |
| 129 | B |
| 130 | A |
| 131 | A |
| 132 | A |
| 133 | B |
| 134 | A |
| 135 | A |
| 136 | A |
| 137 | A |
| 138 | A |
| 139 | B |
| 140 | A |
| 141 | A |
| 142 | A |
| 143 | A |
| 144 | A |
| 145 | A |
| 146 | A |
| 147 | A |
| 148 | A |
| 149 | A |
| 150 | A |
| 151 | A |
| 152 | A |
| 153 | A |
| 154 | A |
| 155 | A |
| 156 | A |
| 158 | A |
| 159 | A |
| 160 | A |
| 161 | A |
| 162 | A |
| 163 | A |
| 164 | A |
| 165 | A |
| 166 | A |
| 167 | A |
| 168 | A |

### EC50 of necroptosis inhibition in U937 cells

The experimental method was briefly described as below. U937 cells in the logarithmic growth phase were collected by centrifugation. Cells were diluted to the cell density of 5×10⁵ cells/mL with RPMI 1640 medium (supplemented with 10% fetal bovine serum, 100 U/mL penicillin and 100 µg/mL streptomycin). Cell suspension was added to a white sterile 384-well cell culture plate at 15 µL/well. The test compound was dissolved in DMSO to prepare a 10 mM stock solution. The compound was serially diluted with culture medium at a ratio of 1:3, for a total of 10 concentration points. A control with the same DMSO volume was set, with two replicates for each concentration. The diluted compound was added to the 384-well cell culture plate containing cells at 2.5 µL/well and incubated at 37 °C, 5% CO₂ for 15 minutes. Z-VAD-FMK (Biyuntian, catalog number: C1202-5 mg) was dissolved in DMSO to prepare a 20 mM stock solution. SM-164 (Biyuntian, catalog number: SC0114-10 mM) was dissolved in DMSO to prepare a 10 mM stock solution and diluted to 0.1 mM. hTNF-α (Sino Biological Inc., catalog number: 10602-HNAE) was dissolved in sterile water to 10 µg/mL. RPMI 1640 medium (supplemented with 10% fetal bovine serum, 100 U/mL penicillin and 100 µg/mL streptomycin) was used to prepare the TSZ mixture, containing 133.3 µM Z-VAD-FMK, 33.3 ng/mL hTNF-α and 0.3 µM SM-164. After 15 minutes' coculture of compounds and cells, 7.5 µL of TSZ mixture was added to each well, mixed evenly, and cultured at 37 °C, 5% CO₂ for 20 hours. After 20 hours, the culture plate was removed and placed at room temperature for 30 minutes. Then, 10 µL of CellTiter-Glo reagent pre-equilibrated at room temperature was added to each well and incubated. After 10 minutes' incubation at room temperature in the dark, the chemiluminescence were measured using a microplate reader. The inhibition rate was calculated as Inh% = (1-(no TSZ-TSZ & compound)/(no TSZ-TSZ))×100. The EC50 was obtained by fitting the nonlinear regression curve log [inhibitor] vs. response - variable slope (four parameters) in GraphPad Prism.

Table 4 below provides the EC50 intervals of necroptosis inhibition in U937 cells for some compounds: A<100 nM; B: 100 - 500 nM; C: 500 - 2000 nM.

**Table 4 EC50 intervals of necroptosis inhibition in U937 cells**

| **Compound No.** | **RIPK1 kinase activity inhibition rate range** |
|---|---|
| 1 | B |
| 2 | A |
| 3 | C |
| 4 | C |
| 5 | B |
| 6 | B |
| 8 | B |
| 9 | B |
| 10 | B |
| 11 | A |
| 12 | A |
| 13 | A |
| 14 | A |
| 15 | A |
| 16 | A |
| 17 | A |
| 18 | A |
| 19 | A |
| 20 | A |
| 21 | A |
| 22 | A |
| 23 | A |
| 24 | A |
| 25 | A |
| 26 | A |
| 27 | A |
| 28 | A |
| 29 | A |
| 30 | B |
| 31 | A |
| 32 | A |
| 33 | A |
| 34 | A |
| 35 | B |
| 36 | A |
| 37 | B |
| 38 | B |
| 39 | B |
| 40 | A |
| 41 | A |
| 42 | A |
| 43 | A |
| 44 | A |
| 45 | A |
| 46 | A |
| 47 | A |
| 48 | A |
| 49 | B |
| 50 | A |
| 51 | A |
| 52 | A |
| 53 | A |
| 54 | C |
| 55 | A |
| 56 | A |
| 57 | B |
| 58 | C |
| 61 | A |
| 62 | A |
| 63 | B |
| 64 | C |
| 65 | A |
| 66 | A |
| 67 | B |
| 68 | C |
| 69 | C |
| 71 | C |
| 72 | A |
| 73 | A |
| 74 | A |
| 75 | A |
| 76 | A |
| 77 | A |
| 78 | A |
| 79 | A |
| 80 | A |
| 81 | A |
| 82 | A |
| 83 | A |
| 84 | A |
| 85 | A |
| 86 | A |
| 87 | C |
| 88 | A |
| 89 | A |
| 90 | B |
| 91 | A |
| 92 | A |
| 93 | B |
| 94 | B |
| 95 | C |
| 96 | A |
| 97 | B |
| 98 | B |
| 100 | B |
| 102 | C |
| 103 | C |
| 104 | A |
| 105 | A |
| 106 | A |
| 107 | A |
| 108 | A |
| 109 | A |
| 110 | A |
| 112 | A |
| 113 | A |
| 114 | A |
| 115 | A |
| 116 | B |
| 117 | A |
| 118 | A |
| 119 | A |
| 120 | A |
| 121 | A |
| 122 | A |
| 123 | A |
| 124 | B |
| 125 | A |
| 126 | A |
| 127 | B |
| 128 | A |
| 129 | B |
| 130 | A |
| 131 | A |
| 132 | A |
| 133 | B |
| 134 | A |
| 135 | A |
| 136 | A |
| 137 | A |
| 138 | A |
| 139 | A |
| 140 | A |
| 141 | A |
| 142 | A |
| 143 | A |
| 144 | A |
| 145 | A |
| 146 | A |
| 147 | A |
| 148 | A |
| 149 | A |
| 150 | A |
| 151 | A |
| 152 | B |
| 153 | B |
| 154 | A |
| 155 | A |
| 156 | A |
| 158 | A |
| 159 | A |
| 160 | A |
| 161 | A |
| 162 | B |
| 163 | A |
| 164 | B |
| 165 | A |
| 166 | A |
| 167 | A |
| 168 | A |

## Claims

1. A compound of formula (I): 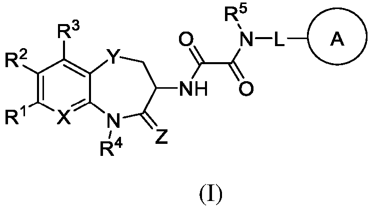
or a mesomer, a racemate, an enantiomer, a diastereoisomer or a mixture thereof, or a pharmaceutically acceptable salt thereof,
wherein,
X is selected from the group consisting of CH and N;
Y is selected from the group consisting of -CR^{a}R^{b}-, -O-, -NR^{a}- and -S(O)ₘ-;
Z is selected from the group consisting of -O and -S;
L is selected from the group consisting of a single bond, -(CR^{a}R^{b})n-, -(CR^{a}Rb)ₙO-, -(CR^{a}R^{b})ₙS-, -(CR^{a}Rb)ₙNR^{a}-, -O-, -NR^{a}- and -S(O)ₘ-;
ring A is selected from the group consisting of C₅-C₁₀ aryl, heteroaryl, cycloalkyl and heterocyclyl, wherein the aryl, heteroaryl, cycloalkyl and heterocyclyl are optionally further substituted by one or more R⁶;
R¹ is independently selected from the group consisting of hydrogen, halogen, amino, nitro, cyano, oxo, hydroxyl, sulfydryl, alkyl, alkoxy, alkenyl, alkynyl, aryl, heteroaryl, cycloalkyl, heterocyclyl, -NR^{a}R^{b}, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, wherein the alkyl, alkoxy, alkenyl, alkynyl, aryl, heteroaryl, cycloalkyl or heterocyclyl is optionally further substituted by one or more groups selected from deuterium atom, halogen, amino, nitro, cyano, oxo, hydroxyl, sulfhydryl, carboxyl, ester group, alkyl, alkoxy, alkenyl, alkynyl, aryl, heteroaryl, cycloalkyl, heterocyclyl and R⁷; preferably, R¹ is selected from the group consisting of the following alkynyl, alkenyl and allenyl group: 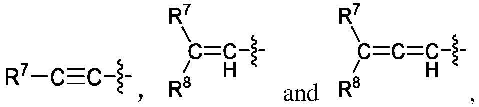
R² and R³ are each independently selected from the group consisting of hydrogen, halogen, amino, nitro, cyano, oxo, hydroxyl, sulfydryl, alkyl, alkoxy, alkenyl, alkynyl, aryl, heteroaryl, cycloalkyl and heterocyclyl; or
R¹ and R² together with the atoms to which they are attached form a C₅-C₁₀ aryl, heteroaryl, heterocyclyl or cycloalkyl, or R² and R³ together with the atoms to which they are attached form a C₅-C₁₀ aryl, heteroaryl, heterocyclyl or cycloalkyl, wherein the C₅-C₁₀ aryl, heteroaryl, heterocyclyl or cycloalkyl is optionally further substituted by one or more R⁹;
R⁴ and R⁵ are each independently selected from the group consisting of hydrogen, alkyl, haloalkyl, cycloalkyl and halocycloalkyl;
R⁶, R⁷, R⁸ and R⁹ are each independently selected from the group consisting of hydrogen, halogen, amino, nitro, cyano, oxo, hydroxyl, sulfydryl, alkyl, alkoxy, alkenyl, alkynyl, aryl, heteroaryl, cycloalkyl, heterocyclyl, -NR^{a}R^{b}, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, wherein the alkyl, alkoxy, alkenyl, alkynyl, aryl, heteroaryl, cycloalkyl, heterocyclyl is optionally further substituted by one or more groups selected from deuterium atom, halogen, amino, nitro, cyano, oxo, hydroxyl, sulfydryl, carboxyl, ester group, alkyl, alkoxy, hydroxyalkyl, alkenyl, alkynyl, aryl, aryloxy, heteroaryl, cycloalkyl and heterocyclyl;
R^{a} and R^{b} are each independently selected from the group consisting of hydrogen, deuterium atom, halogen, amino, cyano, oxo, hydroxyl, sulfydryl, alkyl, alkoxy, aryl, heteroaryl, cycloalkyl and heterocyclyl; or
R^{a} and R^{b} together with the atoms to which they are attached form a cycloalkyl or heterocyclyl, wherein the cycloalkyl or heterocyclyl is optionally further substituted by one or more groups selected from the group consisting of deuterium atom, halogen, amino, nitro, cyano, oxo, hydroxyl, sulfydryl, carboxyl, ester group, alkyl, alkoxy, alkenyl, alkynyl, aryl, heteroaryl, cycloalkyl and heterocyclyl;
m is 0, 1 or 2; and
n is 0, 1, 2 or 3.

2. The compound of formula I or a mesomer, a racemate, an enantiomer, a diastereoisomer or a mixture thereof, or a pharmaceutically acceptable salt thereof according to claim 1, wherein the compound of formula I has the following structure of formula (II): 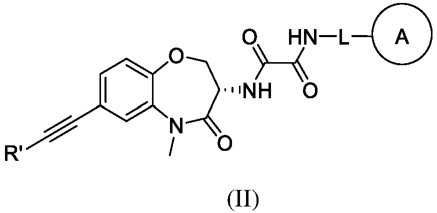 wherein,
R' is selected from the group consisting of hydrogen, halogen, amino, nitro, cyano, oxo, hydroxyl, sulfydryl, alkyl, alkoxy, alkenyl, alkynyl, aryl, heteroaryl, cycloalkyl, heterocyclyl, -NR^{a}R^{b}, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, wherein the alkyl, alkoxy, alkenyl, alkynyl, aryl, heteroaryl, cycloalkyl, heterocyclyl is optionally further substituted by one or more groups selected from the group consisting of deuterium atom, halogen, amino, nitro, cyano, oxo, hydroxyl, sulfydryl, carboxyl, ester group, alkyl, alkoxy, hydroxyalkyl, alkenyl, alkynyl, aryl, aryloxy, heteroaryl, cycloalkyl and heterocyclyl;
m is 0, 1 or 2; and
L and ring A are as defined in claim 1.

3. The compound of formula I or a mesomer, a racemate, an enantiomer, a diastereomer or a mixture thereof, or a pharmaceutically acceptable salt thereof according to claim 1, wherein the compound of formula I has the following structure of formula (III): 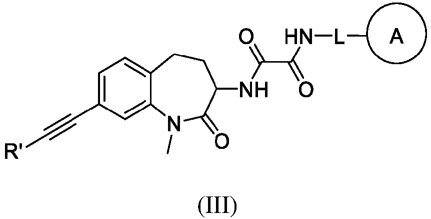 wherein,
L and ring A are as defined in claim 1; and
R' is as defined in claim 2.

4. The compound or a mesomer, a racemate, an enantiomer, a diastereomer or a mixture thereof, or a pharmaceutically acceptable salt thereof according to any of claims 1 to 3, wherein the compound is selected from the group consisting of the following compounds:
| Compound No. | Structure | Name |
|---|---|---|
| 1 | 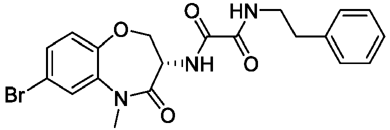 | (*S*)-*N*¹-(7-bromo-5-methyl-4-o xo-2,3,4,5-tetrahydrobenzo[*b*][ 1,4]oxazepin-3-yl)-*N*²-pheneth yloxalamide |
| 2 | 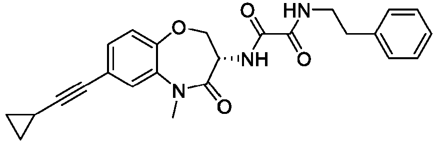 | (*S*)-*N*¹-(7-(cyclopropylethynyl )-5-methyl-4-oxo-2,3,4,5-tetra hydrobenzo[*b*][1,4]oxazepin-3 -yl)-*N*²-phenethyloxalamide |
| 3 | 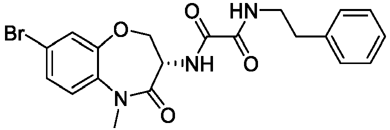 | (*S*)-*N*¹-(8-bromo-5-methyl-4-o xo-2,3,4,5-tetrahydrobenzo[*b*][ 1,4]oxazepin-3-yl)-*N*²-pheneth yloxalamide |
| 4 | 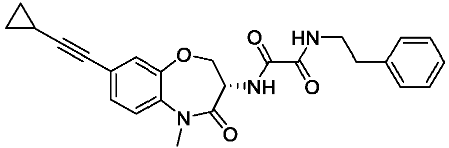 | (*S*)-*N*¹-(8-(cyclopropylethynyl )-5-methyl-4-oxo-2,3,4,5-tetra hydrobenzo[*b*][1,4]oxazepin-3 -yl)-*N*²-phenethyloxalamide |
| 5 | 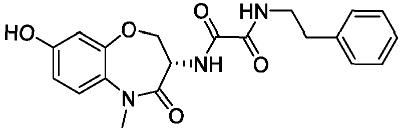 | (*S*)-*N*¹-(8-hydroxy-5-methyl-4 -oxo-2,3,4,5-tetrahydrobenzo [ *b*][1,4]oxazepin-3-yl)-*N*²-phen ethyloxalamide |
| 6 | 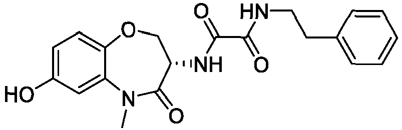 | (*S*)-*N*¹-(7-hydroxy-5-methyl-4 -oxo-2,3,4,5-tetrahydrobenzo[ *b*][1,4]oxazepin-3-yl)-*N*²-phen ethyloxalamide |
| 7 | 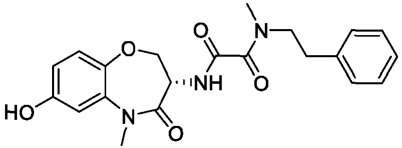 | (*S*)-*N*¹-(7-hydroxy-5-methyl-4 -oxo-2,3,4,5-tetrahydrobenzo[ *b*][1,4]oxazepin-3-yl)-*N*²-meth yl-*N*²-phenethyloxalamide |
| 8 | 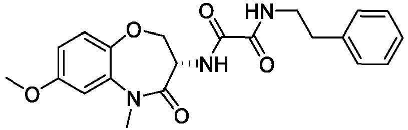 | (*S*)-*N*¹-(7-methoxy-5-methyl-4 -oxo-2,3,4,5-tetrahydrobenzo[ *b*][1,4]oxazepin-3-yl)-*N*²-phen ethyloxalamide |
| 9 | 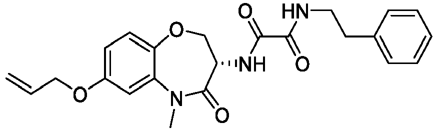 | (*S*)-*N*¹-(7-(allyloxy)-5-methyl-4-oxo-2,3,4,5-tetrahydrobenzo [*b*][1,4]oxazepin-3-yl)-*N*²-phe nethyloxalamide |
| 10 | 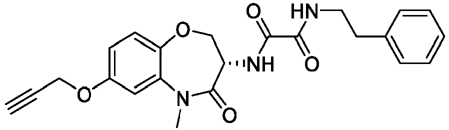 | (*S*)-*N*¹-(5-methyl-4-oxo-7-(pro p-2-yn-1-yloxy)-2,3,4,5-tetrah ydrobenzo[*b*][1,4]oxazepin-3-yl)-*N*²-phenethyloxalamide |
| 11 | 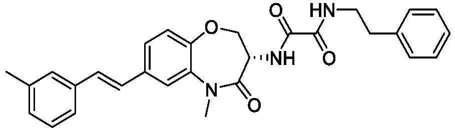 | (*S*,*E*)-*N*¹-(5-methyl-7-(3-meth ylstyryl)-4-oxo-2,3,4,5-tetrahy drobenzo[*b*][1,4]oxazepin-3-yl )-*N*²-phenethyloxalamide |
| 12 | 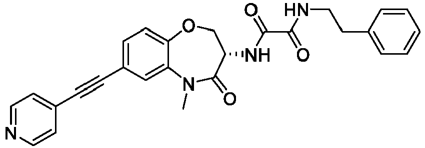 | (*S*)-*N*¹-(5-methyl-4-oxo-7-(pyr idin-4-ylethynyl)-2,3,4,5-tetra hydrobenzo[*b*][1,4]oxazepin-3 -yl)-*N*²-phenethyloxalamide |
| 13 | 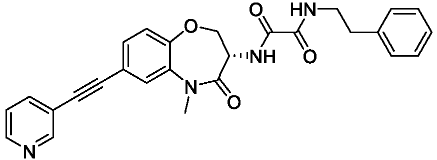 | (*S*)-*N*¹-(5-methyl-4-oxo-7-(pri din-3-ylethynyl)-2,3,4,5-tetrah ydrobenzo[*b*][1,4]oxazepin-3-yl)-*N*²-phenethyloxalamide |
| 14 | 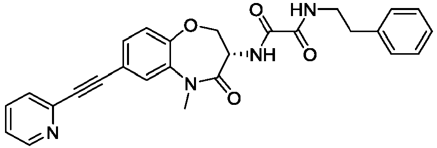 | (*S*)-*N*¹-(5-methyl-4-oxo-7-(pyr idin-2-ylethynyl)-2,3,4,5-tetra hydrobenzo[*b*][1,4]oxazepin-3 -yl)-*N*²-phenethyloxalamide |
| 15 | 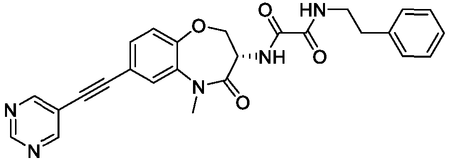 | (*S*)-*N*¹-(5-methyl-4-oxo-7-(pyr imidin-5-ylethynyl)-2,3,4,5-tet rahydrobenzo[*b*][1,4]oxazepin -3-yl)-*N*²-phenethyloxalamide |
| 16 | 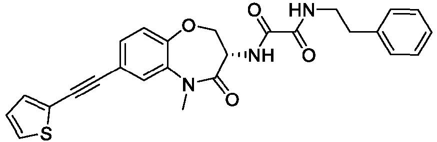 | (*S*)-*N*¹-(5-methyl-4-oxo-7-(thi ophen-2-ylethynyl)-2,3,4,5-tet rahydrobenzo[*b*][1,4]oxazepin -3-yl)-*N*²-phenethyloxalamide |
| 17 | 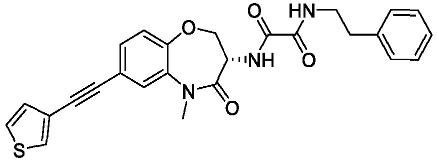 | (*S*)-*N*¹-(5-methyl-4-oxo-7-(thi ophen-3-ylethynyl)-2,3,4,5-tet rahydrobenzo[*b*][1,4]oxazepin -3-yl)-*N*²-phenethyloxalamide |
| 18 | 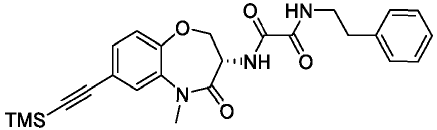 | (*S*)-*N*¹-(5-methyl-4-oxo-7-((tri methylsilyl)ethynyl)-2,3,4,5-te trahydrobenzo[*b*][1,4]oxazepi n-3-yl)-*N*²-phenethyloxalamid e |
| 19 | 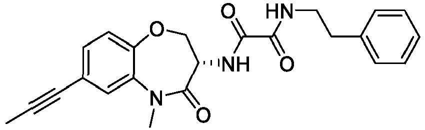 | (*S*)-*N*¹-(5-methyl-4-oxo-7-(pro p-1-yn-1-yl)-2,3,4,5-tetrahydr obenzo[*b*][1,4]oxazepin-3-yl)-*N*²-phenethyloxalamide |
| 20 | 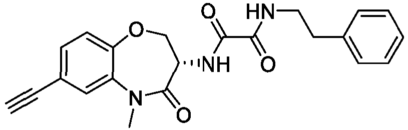 | (*S*)-*N*¹-(7-ethynyl-5-methyl-4-oxo-2,3,4,5-tetrahydrobenzo[*b* ][1,4]oxazepin-3-yl)-*N*²-phene thyloxalamide |
| 21 | 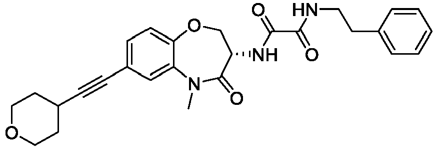 | (*S*)-*N*¹-(5-methyl-4-oxo-7-((tet rahydro-2*H*-pyran-4-yl)ethyny l)-2,3,4,5-tetrahydrobenzo[*b*][ 1,4]oxazepin-3-yl)-*N*²-pheneth yloxalamide |
| 22 | 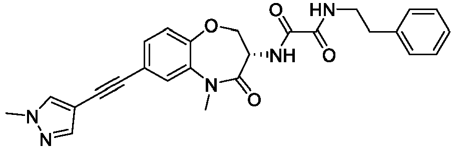 | (*S*)-*N*¹-(5-methyl-7-((1-methyl -1H-pyrazol-4-yl)ethynyl)-4-o xo-2,3,4,5-tetrahydrobenzo[*b*][ 1,4]oxazepin-3-yl)-*N*²-pheneth yloxalamide |
| 23 | 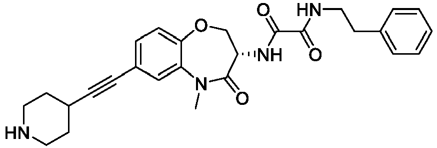 | (*S*)-*N*¹-(5-methyl-4-oxo-7-(pip eridin-4-ylethynyl)-2,3,4,5-tetr ahydrobenzo[*b*][1,4]oxazepin-3-yl)-*N*²-phenethyloxalamide |
| 24 | 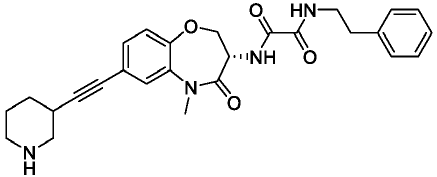 | *N*¹-((3*S*)-5-methyl-4-oxo-7-(pi peridin-3-ylethynyl)-2,3,4,5-te trahydrobenzo[*b*][1,4]oxazepi n-3-yl)-*N*²-phenethyloxalamid e |
| 25 | 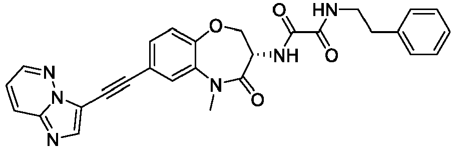 | (*S*)-*N*¹-(7-(imidazo[1,2-*b*]pyrid azin-3-ylethynyl)-5-methyl-4-oxo-2,3,4,5-tetrahydrobenzo[*b* ][1,4]oxazepin-3-yl)-*N*²-phene thyloxalamide |
| 26 | 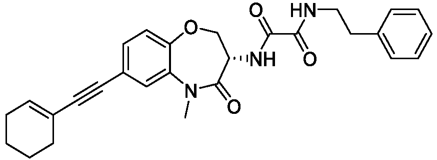 | (*S*)-*N*¹-(7-(cyclohex-1-en-1-yl ethynyl)-5-methyl-4-oxo-2,3,4 ,5-tetrahydrobenzo[*b*][1,4]oxa zepin-3-yl)-*N*²-phenethyloxalamide |
| 27 | 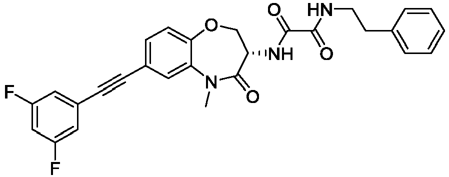 | (*S*)-*N*¹-(7-((3,5-difluorophenyl )ethynyl)-5-methyl-4-oxo-2,3, 4,5-tetrahydrobenzo[*b*][1,4]ox azepin-3-yl)-*N*²-phenethyloxal amide |
| 28 | 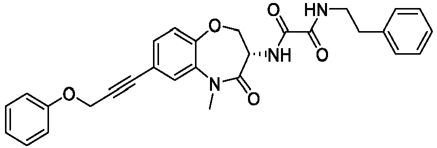 | (*S*)-*N*¹-(5-methyl-4-oxo-7-(3-p henyloxyprop-1-yn-1-yl)-2,3,4 ,5-tetrahydrobenzo[*b*][1,4]oxa zepin-3-yl)-*N*²-phenethyloxala mide |
| 29 | 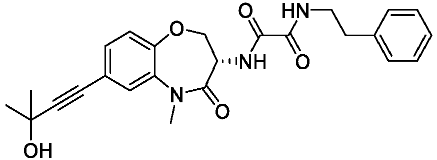 | (*S*)-*N*¹-(7-(3-hydroxy-3-methy lbut-1-yn-1-yl)-5-methyl-4-ox o-2,3,4,5-tetrahydrobenzo[*b*][1 ,4]oxazepin-3-yl)-*N*²-phenethy loxalamide |
| 30 | 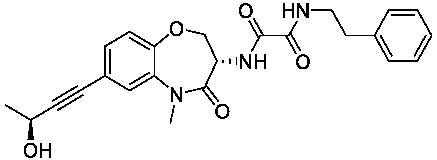 | *N*¹-((*S*)-7-((*S*)-3-hydroxybut-1 -yn-1-yl)-5-methyl-4-oxo-2,3, 4,5-tetrahydrobenzo[*b*][1,4]ox azepin-3-yl)-*N*²-phenethyloxal amide |
| 31 | 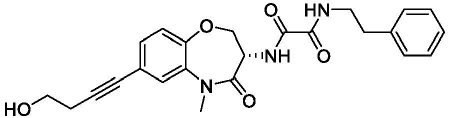 | (*S*)-*N*¹-(7-(4-hydroxybut-1-yn-1-yl)-5-methyl-4-oxo-2,3,4,5-t etrahydrobenzo[*b*][1,4]oxazepi n-3-yl)-*N*²-phenethyloxalamid e |
| 32 | 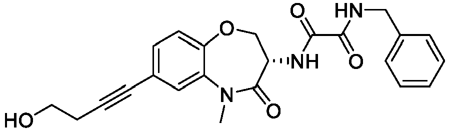 | (*S*)-*N*¹-benzyl-*N*²-(7-(4-hydrox ybut-1-yn-1-yl)-5-methyl-4-ox o-2,3,4,5-tetrahydrobenzo[*b*][1 ,4]oxazepin-3-yl)oxalamide |
| 33 | 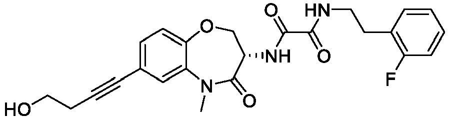 | (*S*)-*N*¹-(2-fluorophenethyl)-*N*²-(7-(4-hydroxybut-1-yn-1-yl)-5 -methyl-4-oxo-2,3,4,5-tetrahy drobenzo[*b*][1,4]oxazepin-3-yl )oxalamide |
| 34 | 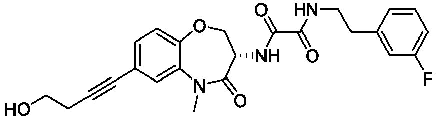 | (*S*)-*N*¹-(3-fluorophenethyl)-*N*²-(7-(4-hydroxybut-1-yn-1-yl)-5 -methyl-4-oxo-2,3,4,5-tetrahy drobenzo[*b*][1,4]oxazepin-3-yl )oxalamide |
| 35 | 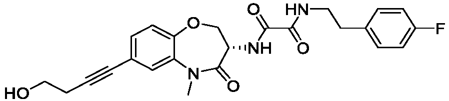 | (*S*)-*N*¹-(4-fluorophenethyl)-*N*²-(7-(4-hydroxybut-1-yn-1-yl)-5 -methyl-4-oxo-2,3,4,5-tetrahy drobenzo[*b*][1,4]oxazepin-3-yl )oxalamide |
| 36 | 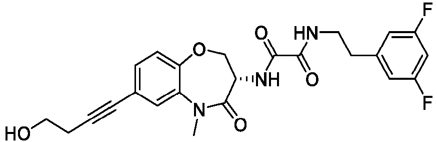 | (*S*)-*N*¹-(3,5-difluorophenethyl) -*N*²-(7-(4-hydroxybut-1-yn-1-yl)-5-methyl-4-oxo-2,3,4,5-tet rahydrobenzo[b] [1,4]oxazepin -3-yl)oxalamide |
| 37 | 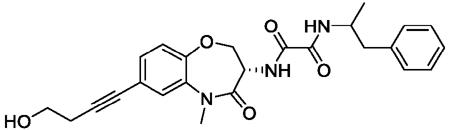 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-(1-phenylpropan-2-yl)oxalamide |
| 38 | 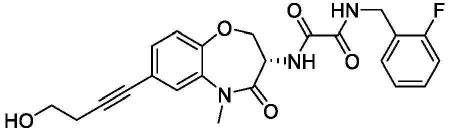 | (*S*)-*N*¹-(2-fluorobenzyl)-*N*²-(7-(4-hydroxybut-1-yn-1-yl)-5-m ethyl-4-oxo-2,3,4,5-tetrahydro benzo[*b*][1,4]oxazepin-3-yl)ox alamide |
| 39 | 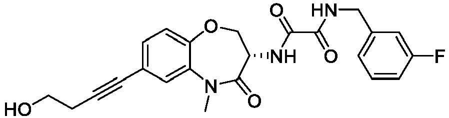 | (*S*)-*N*¹-(3-fluorobenzyl)-*N*²-(7-(4-hydroxybut-1-yn-1-yl)-5-m ethyl-4-oxo-2,3,4,5-tetrahydro benzo[*b*][1,4]oxazepin-3-yl)ox alamide |
| 40 | 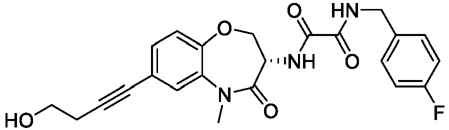 | (*S*)-*N*¹-(4-fluorobenzyl)-*N*²-(7-(4-hydroxybut-1-yn-1-yl)-5-m ethyl-4-oxo-2,3,4,5-tetrahydro benzo[*b*][1,4]oxazepin-3-yl)ox alamide |
| 41 | 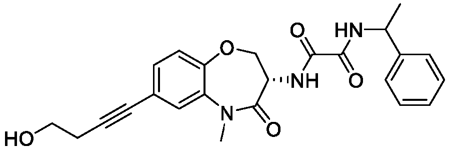 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-(1-phenylethyl)oxal amide |
| 42 | 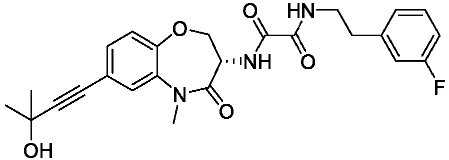 | (*S*)-*N*¹-(3-fluorophenethyl)-*N*²-(7-(3-hydroxy-3-methylbut-1-yn-1-yl)-5-methyl-4-oxo-2,3,4 ,5-tetrahydrobenzo[*b*][1,4]oxa zepin-3-yl)oxalamide |
| 43 | 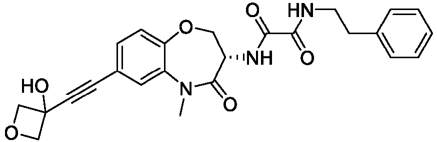 | (*S*)-*N*¹-(7-((3-hydroxyoxetan-3 -yl)ethynyl)-5-methyl-4-oxo-2 ,3,4,5-tetrahydrobenzo[*b*][1,4] oxazepin-3-yl)-*N*²-phenethylo xalamide |
| 44 | 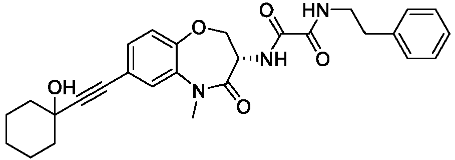 | (*S*)-*N*¹-(7-((1-hydroxycyclohe xyl)ethynyl)-5-methyl-4-oxo-2 ,3,4,5-tetrahydrobenzo[*b*][1,4] oxazepin-3-yl)-*N*²-phenethylo xalamide |
| 45 | 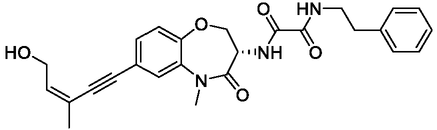 | (*S*,*Z*)-*N*¹-(7-(5-hydroxy-3-met hylpent-3-en-1-yn-1-yl)-5-met hyl-4-oxo-2,3,4,5-tetrahydrobe nzo[*b*][1,4]oxazepin-3-yl)-*N*²-phenethyloxalamide |
| 46 | 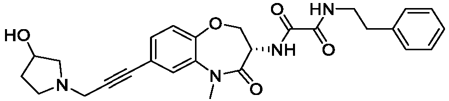 | *N*¹-((3*S*)-7-(3-(3-hydroxypyrro lidin-1-yl)prop-1-yn-1-yl)-5-m ethyl-4-oxo-2,3,4,5-tetrahydro benzo[b] [1,4]oxazepin-3-yl)-*N* ²-phenethyloxalamide |
| 47 | 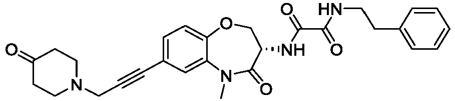 | (*S*)-*N*¹-(5-methyl-4-oxo-7-(3-( 4-oxopiperidin-1-yl)prop-1-yn -1-yl)-2,3,4,5-tetrahydrobenzo [*b*][1,4]oxazepin-3-yl)-*N*²-phe nethyloxalamide |
| 48 | 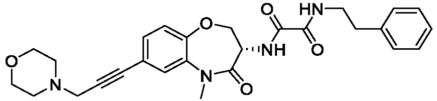 | (*S*)-*N*¹-(5-methyl-7-(3-morpho linoprop-1-yn-1-yl)-4-oxo-2,3, 4,5-tetrahydrobenzo[*b*][1,4]oxazepin-3-yl)-*N*²-phenethyloxal amide |
| 49 | 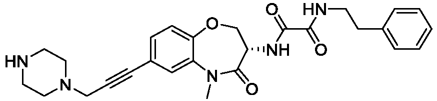 | (*S*)-*N*¹-(5-methyl-4-oxo-7-(3-( piperazin-1-yl)prop-1-yn-1-yl) -2,3,4,5-tetrahydrobenzo[*b*][1, 4]oxazepin-3-yl)-*N*²-phenethyl oxalamide |
| 50 | 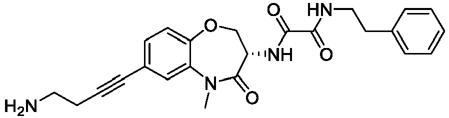 | (*S*)-*N*¹-(7-(4-aminobut-1-yn-1-yl)-5-methyl-4-oxo-2,3,4,5-tet rahydrobenzo[*b*][1,4]oxazepin -3-yl)-*N*²-phenethyloxalamide |
| 51 | 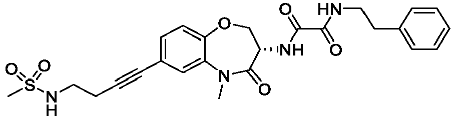 | (*S*)-*N*¹-(5-methyl-7-(4-(methyl sulfonamido)but-1-yn-1-yl)-4-oxo-2,3,4,5-tetrahydrobenzo[*b* ][1,4]oxazepin-3-yl)-*N*²-phene thyloxalamide |
| 52 | 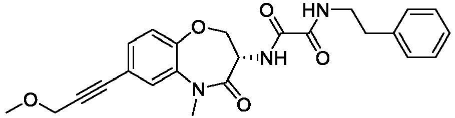 | (*S*)-*N*¹-(7-(3-methoxyprop-1-y n-1-yl)-5-methyl-4-oxo-2,3,4, 5-tetrahydrobenzo[*b*][1,4]oxaz epin-3-yl)-*N*²-phenethyloxala mide |
| 53 | 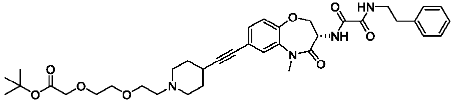 | *tert*-butyl (*S*)-2-(2-(2-(4-((5-methyl-4-ox o-3-(2-oxo-2-(phenethylamino )acetamido)-2,3,4,5-tetrahydro benzo[*b*][1,4]oxazepin-7-yl)et hynyl)piperidin-1-yl)ethoxy)et hoxy)acetate |
| 54 | 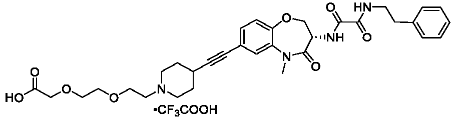 | (*S*)-1-(2-(2-(carboxymethoxy) ethoxy)ethyl)-4-((5-methyl-4-oxo-3-(2-oxo-2-(phenethylami no)acetamido)-2,3,4,5-tetrahy drobenzo[*b*][1,4]oxazepin-7-yl )ethynyl)piperidin-1-ium 2,2,2-trifluoroacetate |
| 55 | 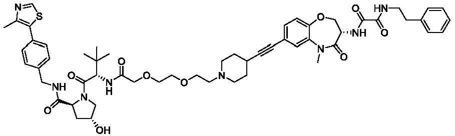 | *N*¹-((*S*)-7-((1-(2-(2-(2-(((*S*)-1-( (2*S*,4*R*)-4-hydroxy-2-((4-(4-m ethylthiazol-5-yl)benzyl)carba moyl)pyrrolidin-1-yl)-3,3-dimethyl-1-oxobutan-2-yl) amino)-2-oxoethoxy)ethoxy)ethyl)pip eridin-4-yl)ethynyl)-5-methyl-4-oxo-2,3,4,5-tetrahydrobenzo [*b*][1,4]oxazepin-3-yl)-*N*²-phe nethyloxalamide |
| 56 | 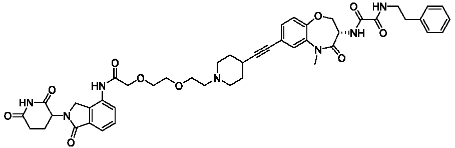 | *N*¹-((3*S*)-7-((1-(2-(2-(2-((2-(2, 6-dioxopiperidin-3-yl)-1-oxois oindolin-4-yl)amino)-2-oxoeth oxy)ethoxy)ethyl)piperidin-4-yl)ethynyl)-5-methyl-4-oxo-2, 3,4,5-tetrahydrobenzo[*b*][1,4]o xazepin-3-yl)-*N*²-phenethylox alamide |
| 57 | 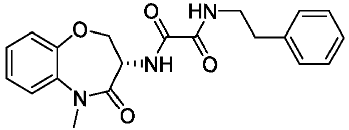 | (*S*)-*N*¹-(5-methyl-4-oxo-2,3,4, 5-tetrahydrobenzo[*b*][1,4]oxaz epin-3-yl)-*N*²-phenethyloxala mide |
| 58 | 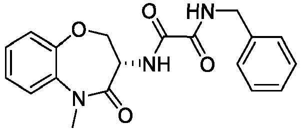 | (*S*)-*N*¹-benzyl-*N*²-(5-methyl-4-oxo-2,3,4,5-tetrahydrobenzo[*b* ][1,4]oxazepin-3-yl)oxalamide |
| 59 | 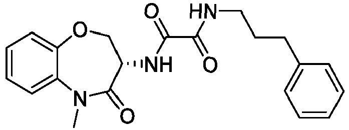 | (*S*)-*N*¹-(5-methyl-4-oxo-2,3,4, 5-tetrahydrobenzo[*b*][1,4]oxaz epin-3-yl)-*N*²-(3-phenylpropyl)oxalamide |
| 60 | 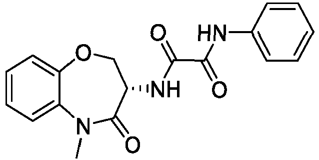 | (*S*)-*N*¹-(5-methyl-4-oxo-2,3,4, 5-tetrahydrobenzo[*b*][1,4]oxaz epin-3-yl)-*N*²-phenyloxalamid e |
| 61 | 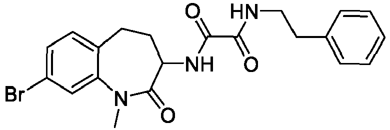 | *N*¹-(8-bromo-1-methyl-2-oxo-2,3,4,5-tetrahydro-1*H*-benzo[*b* ]azepin-3-yl)-*N*²-phenethyloxa lamide |
| 62 | 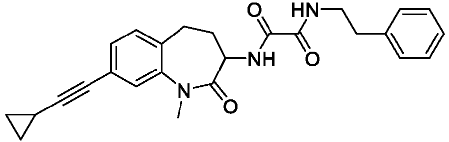 | *N*¹-(8-(cyclopropylethynyl)-1-methyl-2-oxo-2,3,4,5-tetrahyd ro-1*H*-benzo[*b*]azepin-3-yl)-*N* ²-phenethyloxalamide |
| 63 | 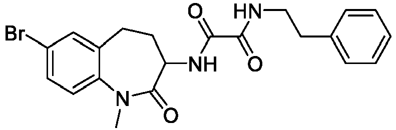 | *N*¹-(7-bromo-1-methyl-2-oxo-2,3,4,5-tetrahydro-1*H*-benzo [*b*]azepin-3-yl)-*N*²-phenethylo xalamide |
| 64 | 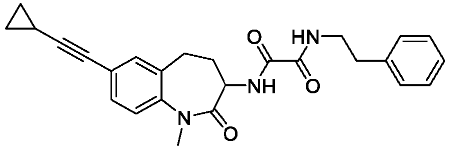 | *N*¹-(7-(cyclopropylethynyl)-1-methyl-2-oxo-2,3,4,5-tetrahyd ro-1*H*-benzo[*b*]azepin-3-yl)-*N* ²-phenethyloxalamide |
| 65 | 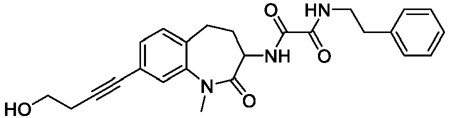 | *N*¹-(8-(4-hydroxybut-1-yn-1-yl )-1-methyl-2-oxo-2,3,4,5-tetra hydro-1*H*-benzo[*b*]azepin-3-yl )-*N*²-phenethyloxalamide |
| 66 | 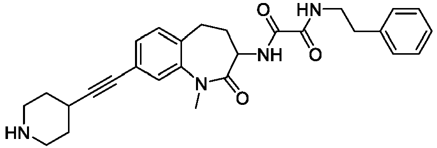 | *N*¹-(1-methyl-2-oxo-8-(piperid in-4-ylethynyl)-2,3,4,5-tetrahy dro-1*H*-benzo[*b*]azepin-3-yl)-*N*²-phenethyloxalamide |
| 67 | 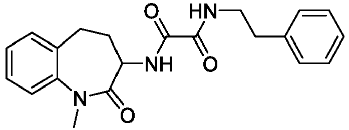 | *N*¹-(1-methyl-2-oxo-2,3,4,5-tet rahydro-1*H*-benzo[b]azepin-3-yl)-*N*²-phenethyloxalamide |
| 68 | 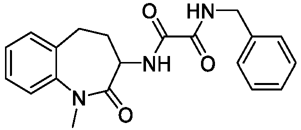 | *N*¹-benzyl-*N*²-(1-methyl-2-oxo -2,3,4,5-tetrahydro-1*H*-benzo[ *b*]azepin-3-yl)oxalamide |
| 69 | 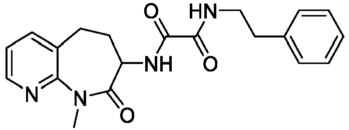 | *N*¹-(9-methyl-8-oxo-6,7,8,9-tet rahydro-5*H*-pyrido[2,3-*b*]azep in-7-yl)-*N*²-phenethyloxalamid e |
| 70 | 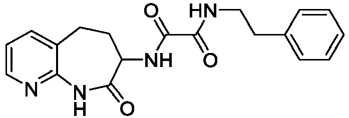 | *N*¹-(8-oxo-6,7,8,9-tetrahydro-5 *H*-pyrido[2,3-*b*]azepin-7-yl)-*N* ²-phenethyloxalamide |
| 71 | 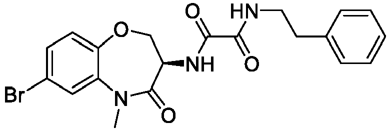 | (*R*)-*N*¹-(7-bromo-5-methyl-4-o xo-2,3,4,5-tetrahydrobenzo[*b*][ 1,4]oxazepin-3-yl)-*N*²-pheneth yloxalamide |
| 72 | 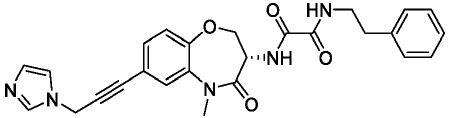 | (*S*)-*N*¹-(7-(3-(1*H*-imidazol-1-y 1)prop-1-yn-1-yl)-5-methyl-4-oxo-2,3,4,5-tetrahydrobenzo[*b* ][1,4]oxazepin-3-yl)-*N*²-phene thyloxalamide |
| 73 | 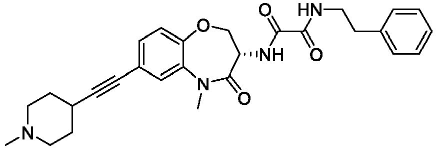 | (*S*)-*N*¹-(5-methyl-7-((1-methyl piperidin-4-yl)ethynyl)-4-oxo-2,3,4,5-tetrahydrobenzo[*b*][1,4 ]oxazepin-3-yl)-*N*²-phenethylo xalamide |
| 74 | 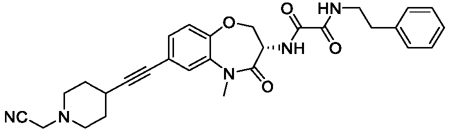 | (*S*)-*N*¹-(7-((1-(cyanomethyl)pi peridin-4-yl)ethynyl)-5-methyl -4-oxo-2,3,4,5-tetrahydrobenz *o*[*b*][1,4]oxazepin-3-yl)-*N*²-ph enethyloxalamide |
| 75 | 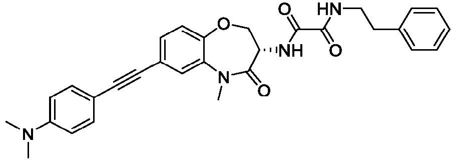 | (*S*)-*N*¹-(7-((4-(dimethylamino) phenyl)ethynyl)-5-methyl-4-o xo-2,3,4,5-tetrahydrobenzo[*b*][ 1,4]oxazepin-3-yl)-*N*²-pheneth yloxalamide |
| 76 | 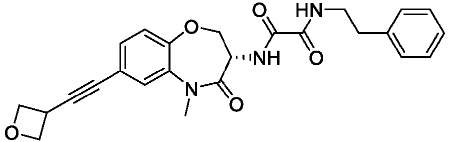 | (*S*)-*N*¹-(5-methyl-7-(oxetan-3-ylethynyl)-4-oxo-2,3,4,5-tetra hydrobenzo[*b*][1,4]oxazepin-3 -yl)-*N*²-phenethyloxalamide |
| 77 | 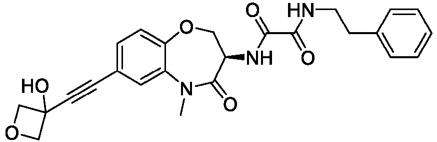 | (*R*)-*N*¹-(7-((3-hydroxyoxetan-3-yl)ethynyl)-5-methyl-4-oxo-2,3,4,5-tetrahydrobenzo[*b*][1,4 ]oxazepin-3-yl)-*N*²-phenethylo xalamide |
| 78 | 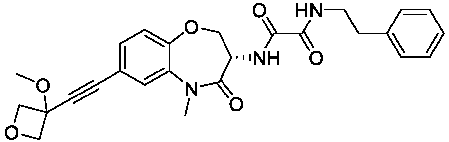 | (*S*)-*N*¹-(7-((3-methoxyoxetan-3-yl)ethynyl)-5-methyl-4-oxo-*2,3,4,5-tetrahydrobenzo[b*][1,4 ]oxazepin-3-yl)-*N*²-phenethylo xalamide |
| 79 | 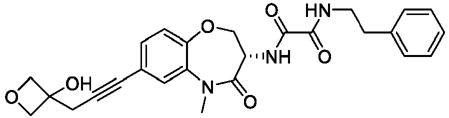 | (*S*)-*N*¹-(7-(3-(3-hydroxyoxetan -3-yl)prop-1-yn-1-yl)-5-methy 1-4-oxo-2,3,4,5-tetrahydrobenz o[*b*][1,4]oxazepin-3-yl)-*N*²-ph enethyloxalamide |
| 80 | 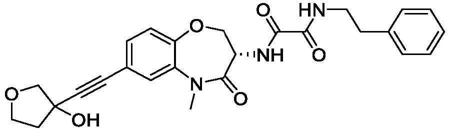 | *N*¹-((3*S*)-7-((3-hydroxytetrahy drofuran-3-yl)ethynyl)-5-meth yl-4-oxo-2,3,4,5-tetrahydroben zo[*b*][1,4]oxazepin-3-yl)-*N*²-phenethyloxalamide |
| 81 | 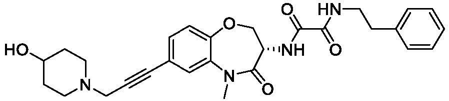 | (*S*)-*N*¹-(7-(3-(4-hydroxypiperi din-1-yl)prop-1-yn-1-yl)-5-me thyl-4-oxo-2,3,4,5-tetrahydrob enzo[*b*][1,4]oxazepin-3-yl)-*N*² -phenethyloxalamide |
| 82 | 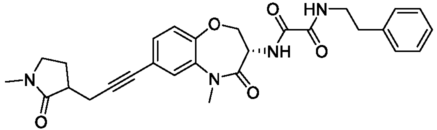 | *N*¹-((3*S*)-5-methyl-7-(3-(1-met hyl-2-oxopyrrolidin-3-yl)prop-1-yn-1-yl)-4-oxo-2,3,4,5-tetra hydrobenzo[*b*][1,4]oxazepin-3 -yl)-*N*²-phenethyloxalamide |
| 83 | 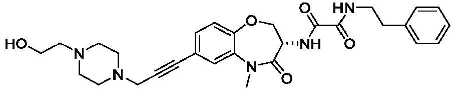 | (*S*)-*N*¹-(7-(3-(4-(2-hydroxyeth yl)piperazin-1-yl)prop-1-yn-1-yl)-5-methyl-4-oxo-2,3,4,5-tet rahydrobenzo[*b*][1,4]oxazepin -3-yl)-*N*²-phenethyloxalamide |
| 84 | 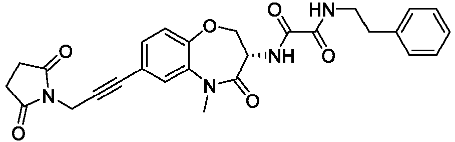 | (*S*)-*N*¹-(7-(3-(2,5-dioxopyrroli din-1-yl)prop-1-yn-1-yl)-5-me thyl-4-oxo-2,3,4,5-tetrahydrob enzo[*b*][1,4]oxazepin-3-yl)-*N*² -phenethyloxalamide |
| 85 | 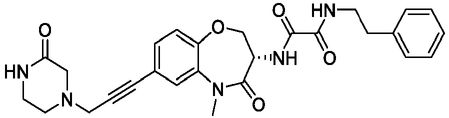 | (*S*)-*N*¹-(5-methyl-4-oxo-7-(3-( 3-oxopiperazin-1-yl)prop-1-yn -1-yl)-2,3,4,5-tetrahydrobenzo [*b*][1,4]oxazepin-3-yl)-*N*²-phe nethyloxalamide |
| 86 | 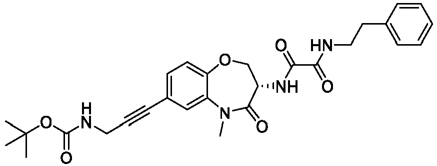 | *tert*-butyl (*S*)-(3-(5-methyl-4-oxo-3-(2-o xo-2-(phenethylamino)acetami do)-2,3,4,5-tetrahydrobenzo[*b*] [1,4]oxazepin-7-yl)prop-2-yn-1-yl)carbamate |
| 87 | 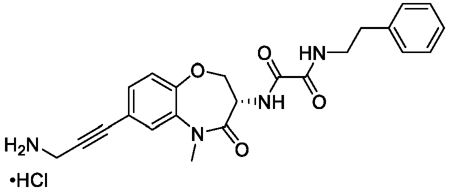 | (*S*)-*N*¹-(7-(3-aminoprop-1-yn-1-yl)-5-methyl-4-oxo-2,3,4,5-t etrahydrobenzo[*b*][1,4]oxazepi n-3-yl)-*N*²-phenethyloxalamid e hydrochloride |
| 88 | 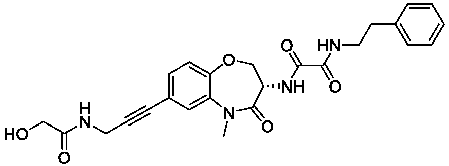 | (*S*)-*N*¹-(7-(3-(2-hydroxyaceta mido)prop-1-yn-1-yl)-5-methy 1-4-oxo-2,3,4,5-tetrahydrobenz o[*b*][1,4]oxazepin-3-yl)-*N*²-ph enethyloxalamide |
| 89 | 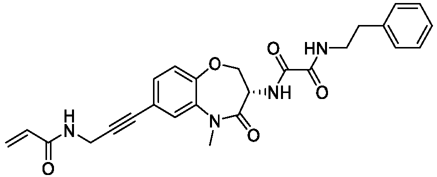 | (*S*)-*N*¹-(7-(3-acrylamidoprop-1 -yn-1-yl)-5-methyl-4-oxo-2,3, 4,5-tetrahydrobenzo[*b*][1,4]ox azepin-3-yl)-*N*²-phenethyloxal amide |
| 90 | 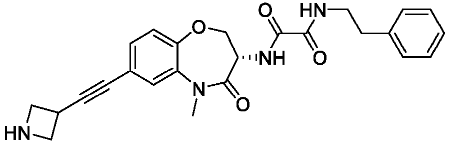 | (*S*)-*N*¹-(7-(azetidin-3-ylethyny l)-5-methyl-4-oxo-2,3,4,5-tetra hydrobenzo[*b*][1,4]oxazepin-3 -yl)-*N*²-phenethyloxalamide |
| 91 | 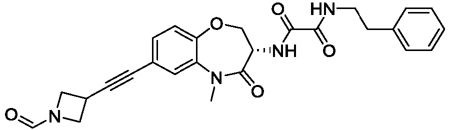 | (*S*)-*N¹*-(7-((1-formylazetidin-3 -yl)ethynyl)-5-methyl-4-oxo-2 ,3,4,5-tetrahydrobenzo[*b*][1,4]oxazepin-3-yl)-*N*²-phenethylo xalamide |
| 92 | 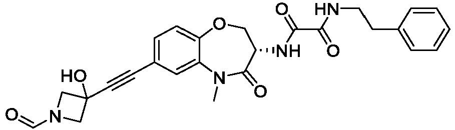 | (*S*)-*N*¹-(7-((1-formyl-3-hydrox yazetidin-3-yl)ethynyl)-5-met hyl-4-oxo-2,3,4,5-tetrahydrobe nzo[*b*][1,4]oxazepin-3-yl)-*N*²-phenethyloxalamide |
| 93 | 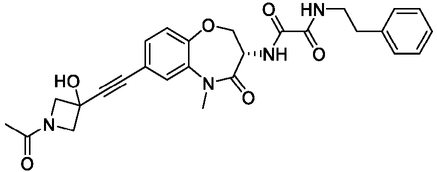 | (*S*)-*N*¹-(7-((1-acetyl-3-hydroxy azetidin-3-yl)ethynyl)-5-meth yl-4-oxo-2,3,4,5-tetrahydroben zo[*b*][1,4]oxazepin-3-yl)-*N*²-p henethyloxalamide |
| 94 | 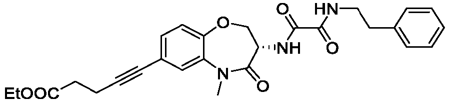 | ethyl (*S*)-5-(5-methyl-4-oxo-3-(2-ox o-2-(phenethylamino)acetamid o)-2,3,4,5-tetrahydrobenzo[*b*][ 1,4]oxazepin-7-yl)pent-4-ynoa te |
| 95 | 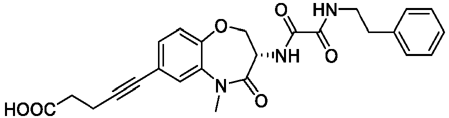 | (*S*)-5-(5-methyl-4-oxo-3-(2-ox o-2-(phenethylamino)acetamid o)-2,3,4,5-tetrahydrobenzo[*b*][ 1,4]oxazepin-7-yl)pent-4-ynoi c acid |
| 96 | 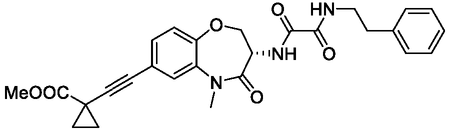 | methyl (*S*)-1-((5-methyl-4-oxo-3-(2-o xo-2-(phenethylamino)acetami do)-2,3,4,5-tetrahydrobenzo[*b*] [1,4]oxazepin-7-yl)ethynyl)cyclopropane-1-carboxylate |
| 97 | 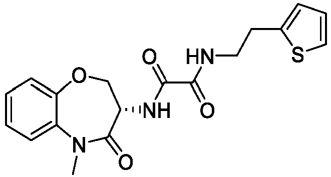 | (*S*)-*N*¹-(5-methyl-4-oxo-2,3,4, 5-tetrahydrobenzo[*b*][1,4]oxaz epin-3-yl)-*N*²-(2-(thiophen-2-y l)ethyl)oxalamide |
| 98 | 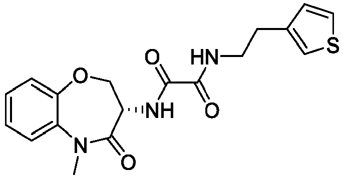 | (*S*)-*N*¹-(5-methyl-4-oxo-2,3,4, 5-tetrahydrobenzo[*b*][1,4]oxaz epin-3-yl)-*N*²-(2-(thiophen-3-y l)ethyl)oxalamide |
| 99 | 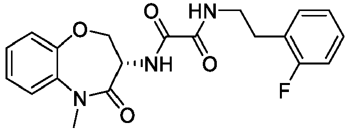 | (*S*)-*N*¹-(2-fluorophenethyl)-*N*²-(5-methyl-4-oxo-2,3,4,5-tetrah ydrobenzo[*b*][1,4]oxazepin-3-yl)oxalamide |
| 100 | 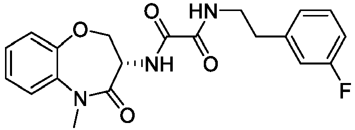 | (*S*)-*N*¹-(3-fluorophenethyl)-*N*²-(5-methyl-4-oxo-2,3,4,5-tetrah ydrobenzo[*b*][1,4]oxazepin-3-yl)oxalamide |
| 101 | 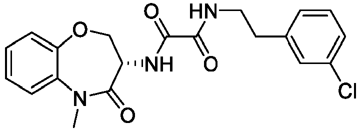 | (*S*)-*N*¹-(3-chlorophenethyl)-*N*² -(5-methyl-4-oxo-2,3,4,5-tetra hydrobenzo[*b*][1,4]oxazepin-3 -yl)oxalamide |
| 102 | 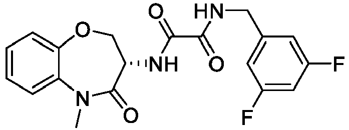 | (*S*)-*N*¹-(3,5-difluorobenzyl)-*N*² -(5-methyl-4-oxo-2,3,4,5-tetra hydrobenzo[*b*][1,4]oxazepin-3 -yl)oxalamide |
| 103 | 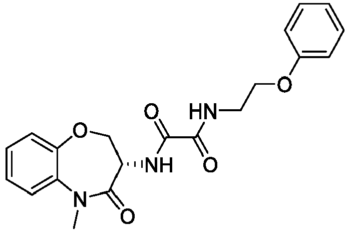 | (*S*)-*N*¹-(5-methyl-4-oxo-2,3,4, 5-tetrahydrobenzo[*b*][1,4]oxaz epin-3-yl)-*N*²-(2-phenoxyethyl )oxalamide |
| 104 | 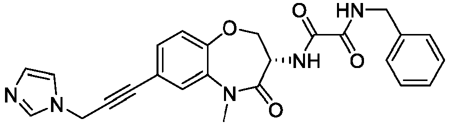 | (*S*)-*N*¹-(7-(3-(1*H*-imidazol-1-y l)prop-1-yn-1-yl)-5-methyl-4-oxo-2,3,4,5-tetrahydrobenzo[*b* ][1,4]oxazepin-3-yl)-*N*²-benzy loxalamide |
| 105 | 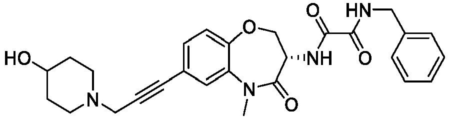 | (*S*)-*N*¹-benzyl-*N*²-(7-(3-(4-hyd roxypiperidin-1-yl)prop-1-yn-1-yl)-5-methyl-4-oxo-2,3,4,5-t etrahydrobenzo[*b*][1,4]oxazepi n-3-yl)oxalamide |
| 106 | 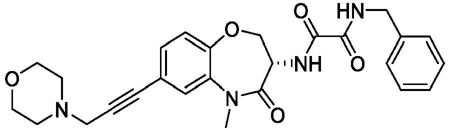 | (*S*)-*N*¹-benzyl-*N*²-(5-methyl-7-(3-morpholinoprop-1-yn-1-yl) -4-oxo-2,3,4,5-tetrahydrobenz o[*b*][1,4]oxazepin-3-yl)oxalam ide |
| 107 | 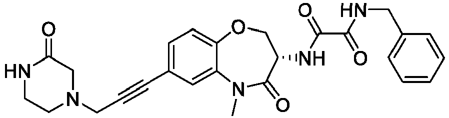 | (*S*)-*N*¹-benzyl-*N*²-(5-methyl-4-oxo-7-(3-(3-oxopiperazin-1-yl )prop-1-yn-1-yl)-2,3,4,5-tetrah ydrobenzo[*b*][1,4]oxazepin-3-yl)oxalamide |
| 108 | 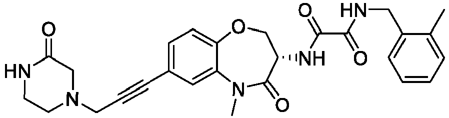 | (*S*)-*N*¹-(5-methyl-4-oxo-7-(3-( 3-oxopiperazin-1-yl)prop-1-yn -1-yl)-2,3,4,5-tetrahydrobenzo [*b*][1,4]oxazepin-3-yl)-*N*²-(2-methylbenzyl)oxalamide |
| 109 | 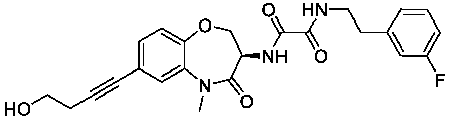 | (*R*)-*N*¹-(3-fluorophenethyl)-*N*² -(7-(4-hydroxybut-1-yn-1-yl)-5-methyl-4-oxo-2,3,4,5-tetrah ydrobenzo[*b*][1,4]oxazepin-3-yl)oxalamide |
| 110 | 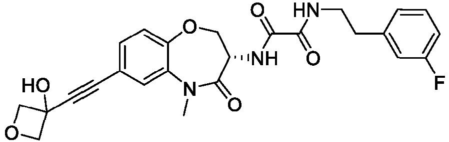 | (*S*)-*N*¹-(3-fluorophenethyl)-*N*²-(7-((3-hydroxyoxetan-3-yl)eth ynyl)-5-methyl-4-oxo-2,3,4,5-t etrahydrobenzo[*b*][1,4]oxazepi n-3-yl)oxalamide |
| 111 | 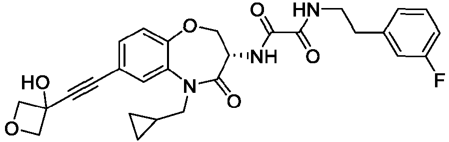 | (*S*)-*N*¹-(5-(cyclopropylmethyl) -7-((3-hydroxyoxetan-3-yl)eth ynyl)-4-oxo-2,3,4,5-tetrahydro benzo[*b*][1,4]oxazepin-3-yl)-*N* ²-(3-fluorophenethyl)oxalamid e |
| 112 | 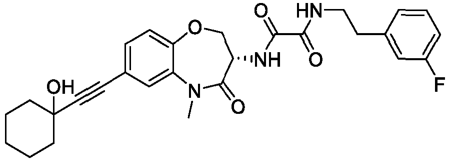 | (*S*)-*N*¹-(3-fluorophenethyl)-*N*²-(7-((1-hydroxycyclohexyl)eth ynyl)-5-methyl-4-oxo-2,3,4,5-t etrahydrobenzo[*b*][1,4]oxazepi n-3-yl)oxalamide |
| 113 | 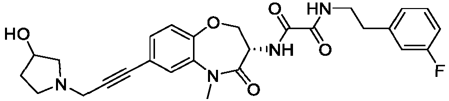 | *N*¹-(3-fluorophenethyl)-*N*²-((3 *S*)-7-(3-(3-hydroxypyrrolidin-1-yl)prop-1-yn-1-yl)-5-methyl -4-oxo-2,3,4,5-tetrahydrobenz o[*b*][1,4]oxazepin-3-yl)oxalam ide |
| 114 | 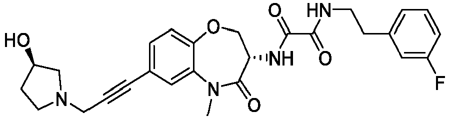 | *N*¹-(3-fluorophenethyl)-*N*²-((*S*) -7-(3-((*R*)-3-hydroxypyrrolidi n-1-yl)prop-1-yn-1-yl)-5-meth yl-4-oxo-2,3,4,5-tetrahydroben zo[*b*][1,4]oxazepin-3-yl)oxala mide |
| 115 | 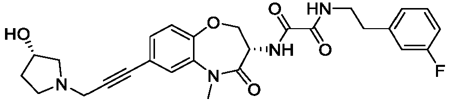 | *N*¹-(3-fluorophenethyl)-*N*²-((*S*) -7-(3-((*S*)-3-hydroxypyrrolidin -1-yl)prop-1-yn-1-yl)-5-methy 1-4-oxo-2,3,4,5-tetrahydrobenz o[*b*][1,4]oxazepin-3-yl)oxalam ide |
| 116 | 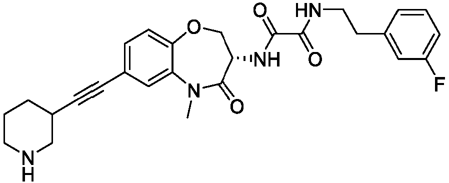 | *N*¹(3-fluorophenethyl)-*N*²-((3 *S*)-5-methyl-4-oxo-7-(piperidi n-3-ylethynyl)-2,3,4,5-tetrahy drobenzo[*b*][1,4]oxazepin-3-yl )oxalamide |
| 117 | 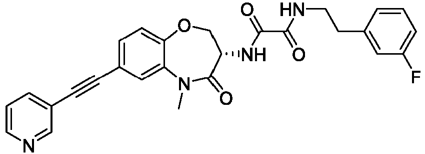 | (*S*)-*N*¹-(3-fluorophenethyl)-*N*²-(5-methyl-4-oxo-7-(pyridin-3-ylethynyl)-2,3,4,5-tetrahydrob enzo[*b*][1,4]oxazepin-3-yl)oxa lamide |
| 118 | 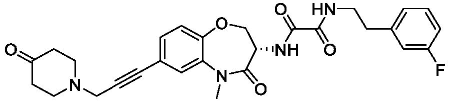 | (*S*)-*N*¹-(3-fluorophenethyl)-*N*²-(5-methyl-4-oxo-7-(3-(4-oxopi peridin-1-yl)prop-1-yn-1-yl)-2 ,3,4,5-tetrahydrobenzo[*b*][1,4] oxazepin-3-yl)oxalamide |
| 119 | 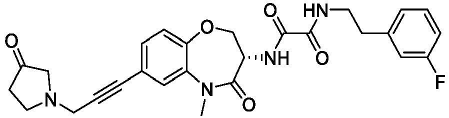 | (*S*)-*N*¹-(3-fluorophenethyl)-*N*²-(5-methyl-4-oxo-7-(3-(3-oxop yrrolidin-1-yl)prop-1-yn-1-yl)-2,3,4,5-tetrahydrobenzo[*b*][1,4 ]oxazepin-3-yl)oxalamide |
| 120 | 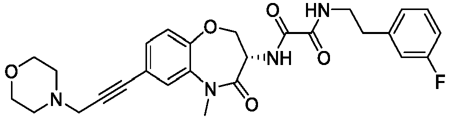 | (*S*)-*N*¹-(3-fluorophenethyl)-*N*²-(5-methyl-7-(3-morpholinopro p-1-yn-1-yl)-4-oxo-2,3,4,5-tetr ahydrobenzo[*b*][1,4]oxazepin-3-yl)oxalamide |
| 121 | 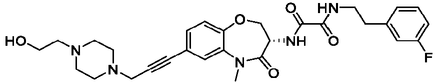 | (*S*)-*N*¹-(3-fluorophenethyl)-*N*²-(7-(3-(4-(2-hydroxyethyl)piper azin-1-yl)prop-1-yn-1-yl)-5-m ethyl-4-oxo-2,3,4,5-tetrahydro benzo[*b*][1,4]oxazepin-3-yl)ox alamide |
| 122 | 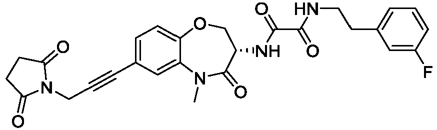 | (*S*)-*N*¹-(7-(3-(2,5-dioxopyrroli din-1-yl)prop-1-yn-1-yl)-5-me thyl-4-oxo-2,3,4,5-tetrahydrob enzo[*b*][1,4]oxazepin-3-yl)-*N*² -(3-fluorophenethyl)oxalamide |
| 123 | 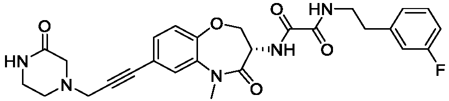 | (*S*)-*N*¹-(3-fluorophenethyl)-*N*²-(5-methyl-4-oxo-7-(3-(3-oxopi perazin-1-yl)prop-1-yn-1-yl)-2 ,3,4,5-tetrahydrobenzo[*b*][1,4] oxazepin-3-yl)oxalamide |
| 124 | 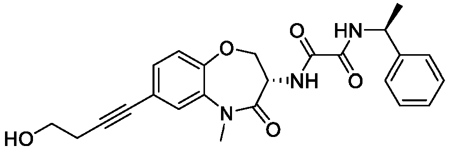 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*S*)-1-phenylethyl) oxalamide |
| 125 | 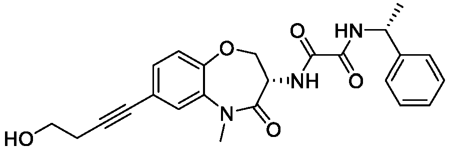 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*R*)-1-phenylethyl) oxalamide |
| 126 | 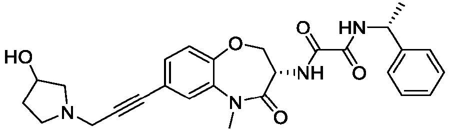 | *N*¹-((3*S*)-7-(3-(3-hydroxypyrro lidin-1-yl)prop-1-yn-1-yl)-5-m ethyl-4-oxo-2,3,4,5-tetrahydro benzo[b] [1,4]oxazepin-3-yl)-*N* ²-((*R*)-1-phenylethyl)oxalamid e |
| 127 | 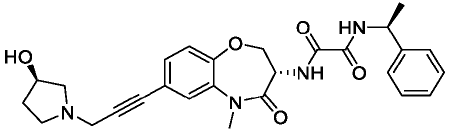 | *N*¹-((*S*)-7-(3-((*R*)-3-hydroxypy rrolidin-1-yl)prop-1-yn-1-yl)-5 -methyl-4-oxo-2,3,4,5-tetrahy drobenzo[*b*][1,4]oxazepin-3-yl )-*N*²-((*S*)-1-phenylethyl)oxala mide |
| 128 | 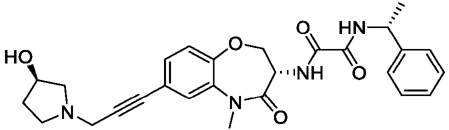 | *N*¹-((*S*)-7-(3-((*R*)-3-hydroxypy rrolidin-1-yl)prop-1-yn-1-yl)-5 -methyl-4-oxo-2,3,4,5-tetrahy drobenzo[*b*][1,4]oxazepin-3-yl )-*N*²-((*R*)-1-phenylethyl)oxala mide |
| 129 | 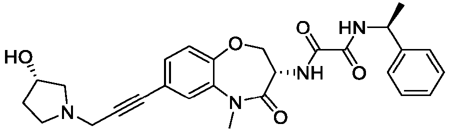 | *N*¹-((*S*)-7-(3-((*S*)-3-hydroxypy rrolidin-1-yl)prop-1-yn-1-yl)-5 -methyl-4-oxo-2,3,4,5-tetrahy drobenzo[*b*][1,4]oxazepin-3-yl )-*N*²-((*S*)-1-phenylethyl)oxala mide |
| 130 | 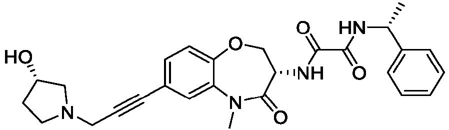 | *N*¹-((*S*)-7-(3-((*S*)-3-hydroxypy rrolidin-1-yl)prop-1-yn-1-yl)-5 -methyl-4-oxo-2,3,4,5-tetrahy drobenzo[*b*][1,4]oxazepin-3-yl )-*N*²-((*R*)-1-phenylethyl)oxala mide |
| 131 | 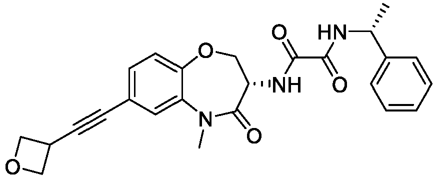 | (*S*)-*N*¹-(5-methyl-7-(oxetan-3-ylethynyl)-4-oxo-2,3,4,5-tetra hydrobenzo[*b*][1,4]oxazepin-3 -yl)-*N*²-((*R*)-1-phenylethyl)oxalamide |
| 132 | 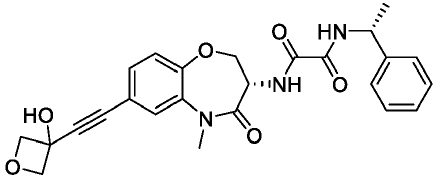 | *N*¹-((*S*)-7-((3-hydroxyoxetan-3 -yl)ethynyl)-5-methyl-4-oxo-2 ,3,4,5-tetrahydrobenzo[*b*][1,4] oxazepin-3-yl)-*N*²-((*R*)-1-phen ylethyl)oxalamide |
| 133 | 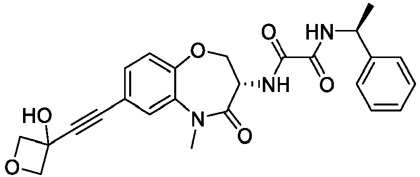 | *N*¹-((*S*)-7-((3-hydroxyoxetan-3 -yl)ethynyl)-5-methyl-4-oxo-2 ,3,4,5-tetrahydrobenzo[*b*][1,4] oxazepin-3-yl)-*N*²-((*S*)-1-phen ylethyl)oxalamide |
| 134 | 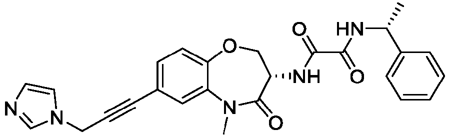 | *N*¹-((*S*)-7-(3-(1*H*-imidazol-1-y l)prop-1-yn-1-yl)-5-methyl-4-oxo-2,3,4,5-tetrahydrobenzo[*b* ][1,4]oxazepin-3-yl)-*N*²-((*R*)-1 -phenylethyl)oxalamide |
| 135 | 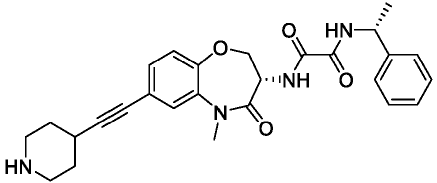 | *N*¹-((*S*)-5-methyl-4-oxo-7-(pip eridin-4-ylethynyl)-2,3,4,5-tetr ahydrobenzo[*b*][1,4]oxazepin-3-yl)-*N*²-((*R*)-1-phenylethyl)o xalamide |
| 136 | 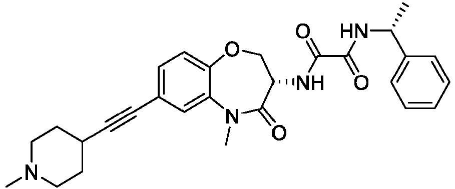 | *N*¹-((*S*)-5-methyl-7-((1-methyl piperidin-4-yl)ethynyl)-4-oxo-2,3,4,5-tetrahydrobenzo[*b*][1,4 ]oxazepin-3-yl)-*N*²-((*R*)-1-phe nylethyl)oxalamide |
| 137 | 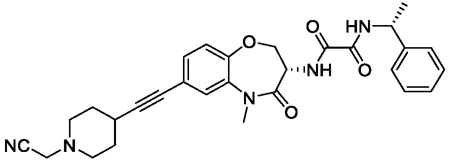 | *N*¹-((*S*)-7-((1-(cyanomethyl)pi peridin-4-yl)ethynyl)-5-methyl -4-oxo-2,3,4,5-tetrahydrobenz o[*b*][1,4]oxazepin-3-yl)-*N*²-((*R* )-1-phenylethyl)oxalamide |
| 138 | 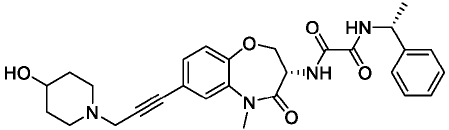 | *N*¹-((*S*)-7-(3-(4-hydroxypiperi din-1-yl)prop-1-yn-1-yl)-5-me thyl-4-oxo-2,3,4,5-tetrahydrob enzo[*b*][1,4]oxazepin-3-yl)-*N*² -((*R*)-1-phenylethyl)oxalamide |
| 139 | 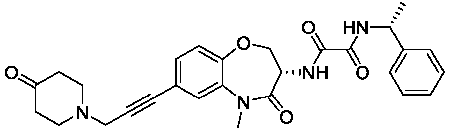 | *N*¹-((*S*)-5-methyl-4-oxo-7-(3-( 4-oxopiperidin-1-yl)prop-1-yn -1-yl)-2,3,4,5-tetrahydrobenzo [*b*][1,4]oxazepin-3-yl)-*N*²-((*R*) -1-phenylethyl)oxalamide |
| 140 | 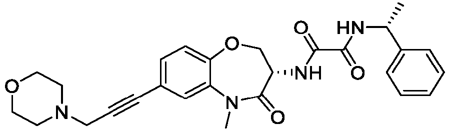 | *N*¹-((*S*)-5-methyl-7-(3-morpho linoprop-1-yn-1-yl)-4-oxo-2,3, 4,5-tetrahydrobenzo[*b*][1,4]ox azepin-3-yl)-*N*²-((*R*)-1-phenyl ethyl)oxalamide |
| 141 | 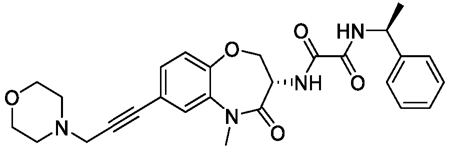 | *N*¹-((*S*)-5-methyl-7-(3-morpho linoprop-1-yn-1-yl)-4-oxo-2,3, 4,5-tetrahydrobenzo[*b*][1,4]ox azepin-3-yl)-*N*²-((*S*)-1-phenyle thyl)oxalamide |
| 142 | 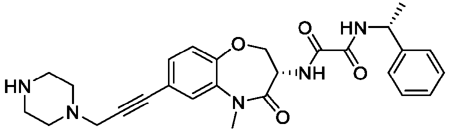 | *N*¹-((*S*)-5-methyl-4-oxo-7-(3-( piperazin-1-yl)prop-1-yn-1-yl) -2,3,4,5-tetrahydrobenzo[*b*][1,4]oxazepin-3-yl)-*N*²-((*R*)-1-ph enylethyl)oxalamide |
| 143 | 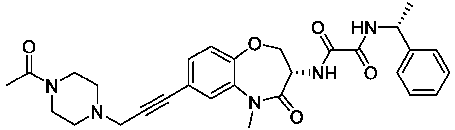 | *N*¹-((*S*)-7-(3-(4-acetylpiperazi n-1-yl)prop-1-yn-1-yl)-5-meth yl-4-oxo-2,3,4,5-tetrahydroben zo[*b*][1,4]oxazepin-3-yl)-*N*²-(( *R*)-1-phenylethyl)oxalamide |
| 144 | 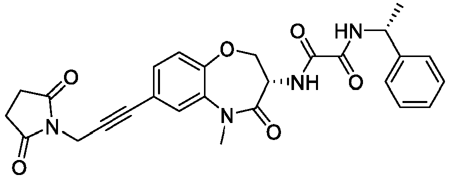 | *N*¹-((*S*)-7-(3-(2,5-dioxopyrroli din-1-yl)prop-1-yn-1-yl)-5-me thyl-4-oxo-2,3,4,5-tetrahydrob enzo[*b*][1,4]oxazepin-3-yl)-*N*² -((*R*)-1-phenylethyl)oxalamide |
| 145 | 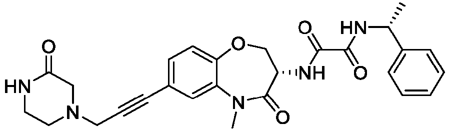 | *N*¹-((*S*)-5-methyl-4-oxo-7-(3-( 3-oxopiperazin-1-yl)prop-1-yn -1-yl)-2,3,4,5-tetrahydrobenzo [*b*][1,4]oxazepin-3-yl)-*N*²-((*R*) -1-phenylethyl)oxalamide |
| 146 | 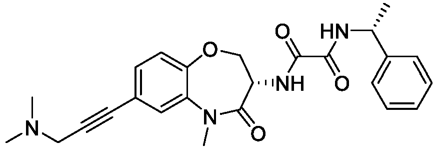 | *N*¹-((*S*)-7-(3-(dimethylamino)p rop-1-yn-1-yl)-5-methyl-4-oxo -2,3,4,5-tetrahydrobenzo[*b*][1, 4]oxazepin-3-yl)-*N*²-((*R*)-1-ph enylethyl)oxalamide |
| 147 | 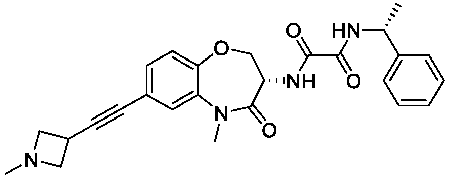 | *N*¹-((*S*)-5-methyl-7-((1-methyl azetidin-3-yl)ethynyl)-4-oxo-2 ,3,4,5-tetrahydrobenzo[*b*][1,4] oxazepin-3-yl)-*N*²-((*R*)-1-phen ylethyl)oxalamide |
| 148 | 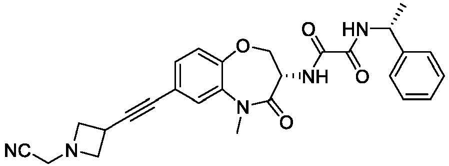 | *N*¹-((*S*)-7-((1-(cyanomethyl)az etidin-3-yl)ethynyl)-5-methyl-4-oxo-2,3,4,5-tetrahydrobenzo [*b*][1,4]oxazepin-3-yl)-*N*²-((*R*) -1-phenylethyl)oxalamide |
| 149 | 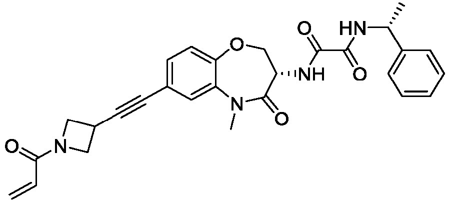 | *N*¹-((*S*)-7-((1-acryloylazetidin-3-yl)ethynyl)-5-methyl-4-oxo-2,3,4,5-tetrahydrobenzo[*b*][1,4 ]oxazepin-3-yl)-*N*²-((*R*)-1-phe nylethyl)oxalamide |
| 150 | 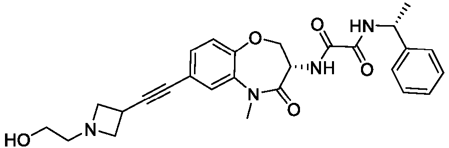 | *N*¹-((*S*)-7-((1-(2-hydroxyethyl) azetidin-3-yl)ethynyl)-5-meth yl-4-oxo-2,3,4,5-tetrahydroben zo[*b*][1,4]oxazepin-3-yl)-*N*²-(( R)-1-phenylethyl)oxalamide |
| 151 | 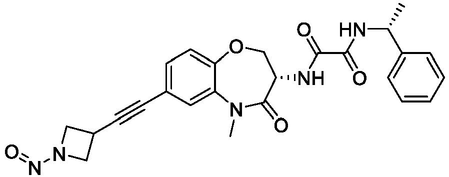 | *N*¹-((*S*)-5-methyl-7-((1-nitroso azetidin-3-yl)ethynyl)-4-oxo-2 ,3,4,5-tetrahydrobenzo[*b*][1,4] oxazepin-3-yl)-*N*²-((*R*)-1-phen ylethyl)oxalamide |
| 152 | 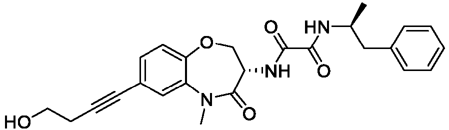 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*S*)-1-phenylpropa n-2-yl)oxalamide |
| 153 | 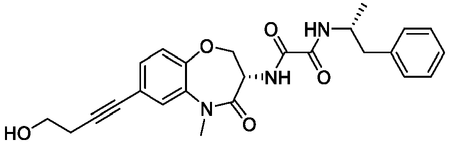 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*R*)-1-phenylpropa n-2-yl)oxalamide |
| 154 | 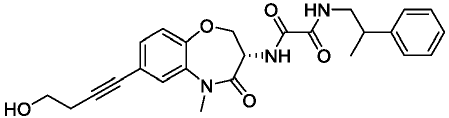 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-(2-phenylpropyl)ox alamide |
| 155 | 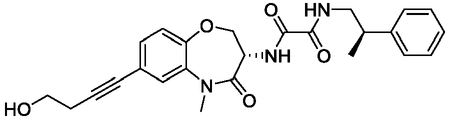 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*R*)-2-phenylpropyl)oxalamide |
| 156 | 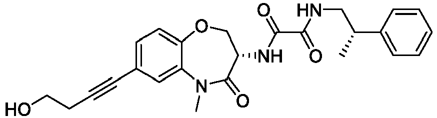 | *N*¹-((*S*)-7-(4-hydroxybut-1-yn-1-yl)-5-methyl-4-oxo-2,3,4,5-t etrahydrobenzo[*b*][1,4]oxazepi n-3-yl)-*N*²-((*S*)-2-phenylpropyl)oxalamide |
| 157 | 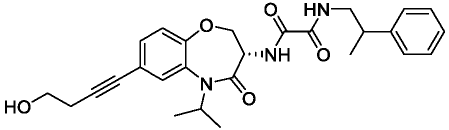 | *N*¹-((*S*)-7-(4-hydroxybut-1-yn-1-yl)-5-isopropyl-4-oxo-2,3,4, 5-tetrahydrobenzo[*b*][1,4]oxaz epin-3-yl)-*N*²-(2-phenylpropyl )oxalamide |
| 158 | 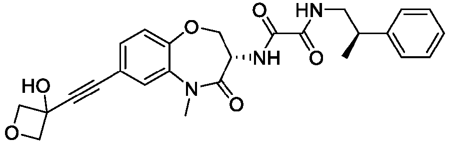 | *N*¹-((*S*)-7-((3-hydroxyoxetan-3 -yl)ethynyl)-5-methyl-4-oxo-2 ,3,4,5-tetrahydrobenzo[*b*][1,4] oxazepin-3-yl)-*N*²-((*R*)-2-phen ylpropyl)oxalamide |
| 159 | 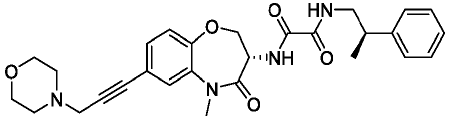 | *N*¹-((*S*)-5-methyl-7-(3-morpho linoprop-1-yn-1-yl)-4-oxo-2,3, 4,5-tetrahydrobenzo[*b*][1,4]ox azepin-3-yl)-*N*²-((*R*)-2-phenyl propyl)oxalamide |
| 160 | 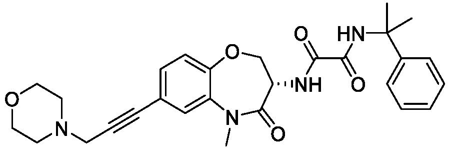 | (*S*)-*N*¹-(5-methyl-7-(3-morpho linoprop-1-yn-1-yl)-4-oxo-2,3, 4,5-tetrahydrobenzo[*b*][1,4]ox azepin-3-yl)-*N*²-(2-phenylpropan-2-yl)oxalamide |
| 161 | 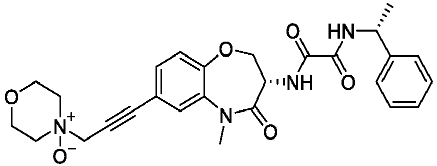 | 4-(3-((*S*)-5-methyl-4-oxo-3-(2 -oxo-2-(((*R*)-1-phenylethyl)am ino)acetamido)-2,3,4,5-tetrahy drobenzo[*b*][1,4]oxazepin-7-yl )prop-2-yn-1-yl)morpholine 4-oxide |
| 162 | 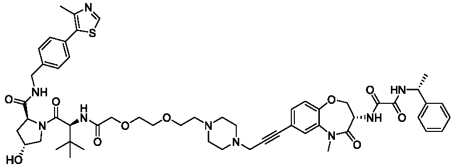 | *N*¹-((*S*)-7-(3-(4-(2-(2-(2-(((*S*)-1-((2*S*,4*R*)-4-hydroxy-2-((4-(4 -methylthiazol-5-yl)benzyl)car bamoyl)pyrrolidin-1-yl)-3,3-di methyl-1-oxobutan-2-yl)amin o)-2-oxoethoxy)ethoxy)ethyl) piperazin-1-yl)prop-1-yn-1-yl) -5-methyl-4-oxo-2,3,4,5-tetrahydrobenzo[*b*][1,4]oxazepin-3-yl)-*N*²-((*R*)-1-phenylethyl)oxal amide |
| 163 | 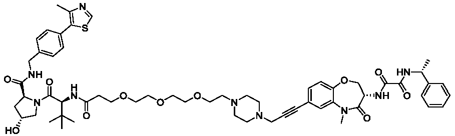 | *N*¹-((*S*)-7-(3-(4-((*S*)-14-((2*S*,4 *R*)-4-hydroxy-2-((4-(4-methylt hiazol-5-yl)benzyl)carbamoyl) pyrrolidine-1-carbonyl)-15,15-dimethyl-12-oxo-3,6,9-trioxa-13-azahexadecyl)piperazin-1-yl)prop-1-yn-1-yl)-5-methyl-4 -oxo-2,3,4,5-tetrahydrobenzo[ *b*][1,4]oxazepin-3-yl)-*N*²-((*R*)-1-phenylethyl)oxalamide |
| 164 | 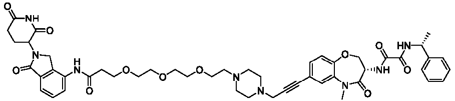 | *N*¹-((3*S*)-7-(3-(4-(2-(2-(2-(3-(( 2-(2,6-dioxopiperidin-3-yl)-1-oxoisoindolin-4-yl)amino)-3-o xopropoxy)ethoxy)ethoxy)eth yl)piperazin-1-yl)prop-1-yn-1-yl)-5-methyl-4-oxo-2,3,4,5-tet rahydrobenzo[*b*][1,4]oxazepin -3-yl)-*N*²-((*R*)-1-phenylethyl)o xalamide |
| 165 | 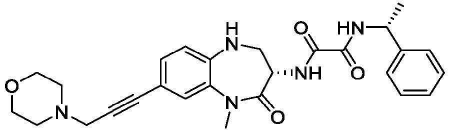 | *N*¹-((*S*)-1-methyl-8-(3-morpho linoprop-1-yn-1-yl)-2-oxo-2,3, 4,5-tetrahydro-1*H*-benzo[*b*][1, 4]diazepin-3-yl)-*N*²-((*R*)-1-phe nylethyl)oxalamide |
| 166 | 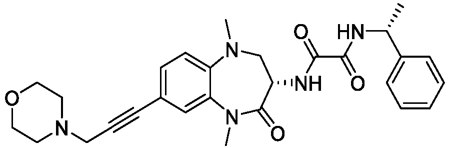 | *N*¹-((*S*)-1,5-dimethyl-8-(3-mor pholinoprop-1-yn-1-yl)-2-oxo-2,3,4,5-tetrahydro-1*H*-benzo[*b* ][1,4]diazepin-3-yl)-*N*²-((*R*)-1-phenylethyl)oxalamide |
| 167 | 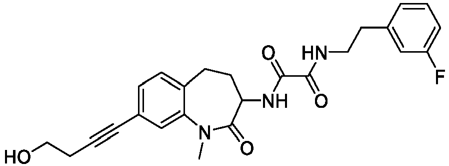 | *N*¹-(3-fluorophenethyl)-*N*²-(8-( 4-hydroxybut-1-yn-1-yl)-1-me thyl-2-oxo-2,3,4,5-tetrahydro-1*H*-benzo[*b*]azepin-3-yl)oxala mide |
| 168 | 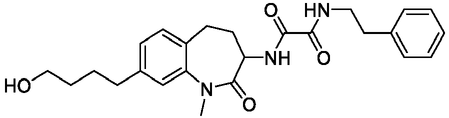 | *N*¹-(8-(4-hydroxybutyl)-1-met hyl-2-oxo-2,3,4,5-tetrahydro-1 *H*-benzo[*b*]azepin-3-yl)-*N*²-ph enethyloxalamide . |

5. A pharmaceutical composition, comprising the compound or a mesomer, a racemate, an enantiomer, a diastereomer or a mixture thereof, or a pharmaceutically acceptable salt thereof according to any one of claims 1 to 4, and an optionally pharmaceutically acceptable carrier.

6. Use of the compound or a mesomer, a racemate, an enantiomer, a diastereomer or a mixture thereof, or a pharmaceutically acceptable salt thereof according to any one of claims 1 to 4 in the manufacture of a medicament for treating or preventing a disease or condition associated with **RIPK1** kinase activity.

7. The use according to claim 6, wherein the disease or condition associated with **RIPK1** kinase activity is selected from the group consisting of neurodegenerative diseases and inflammatory diseases, stroke, coronary heart disease and myocardial infarction, retinal degenerative disease, inflammatory bowel disease, kidney disease, liver disease, and a lesion caused by COVID-19.
